# EUROPEAN PATENT APPLICATION

(11) **EP 4 700 021 A1**
(43) Date of publication of application: **25.02.2026**
(21) Application number: 24904413.2
(22) Date of filing: 12.12.2024
(51) Int. Cl.: C07D 403/12, A61K 31/498, A61P 43/00, A61P 1/16, A61P 13/12, C07D 401/12, C07D 405/12, C07D 401/14, C07D 405/14, C07D 401/04

(54) **NOVEL COMPOUND AND PHARMACEUTICAL COMPOSITION COMPRISING SAME AS ACTIVE INGREDIENT**

(30) Priority: 12.12.2023 KR 20230180038
(71) Applicant: Sapiensbio Inc., Seongnam-si, Gyeonggi-do 13207 (KR)
(72) Inventor: YOON, Dong-Oh, Seongnam-si, Gyeonggi-do 13207 (KR); RYU, Incheol, Seongnam-si, Gyeonggi-do 13207 (KR); RYOO, Kanghyun, Seongnam-si, Gyeonggi-do 13207 (KR); LEE, Myongwoo, Seongnam-si, Gyeonggi-do 13207 (KR); CHO, Hyewon, Seongnam-si, Gyeonggi-do 13207 (KR); DU, Eunjo, Seongnam-si, Gyeonggi-do 13207 (KR); KIM, Daebong, Seongnam-si, Gyeonggi-do 13207 (KR)
(74) Representative: Dehns
(86) International application number: PCT/KR2024/096870
(87) International publication number: WO 2025/127848

(57) **Abstract**

The present invention relates to a novel compound having a fibrosis inhibition effect. The compound or a pharmaceutically acceptable salt thereof, according to the present invention, has been confirmed to effectively inhibit the expression (formation) of α-SMA, COL1A1, and F-actin, which are major causative factors of fibrosis. Thus, the novel compound, according to the present invention, is expected to be usefully employed for the prevention and treatment of fibrosis-related diseases as a therapeutic agent having the effect of reducing or inhibiting fibrosis.

## Description

### [Technical Field]

The present invention relates to a novel compound and a pharmaceutical composition comprising the same as an active ingredient.

This application claims priority to and the benefit of Korean Patent Application No. 10-2023-0180038, filed on December 12, 2023, the disclosure of which is incorporated herein by reference in its entirety.

### [Background Art]

Fibrosis affects virtually every tissue in the body, is the final stage of many chronic inflammatory diseases, and occurs by a variety of mediators and signaling mechanisms.

When cells are affected and damaged by external stimuli, pro-inflammatory proteins such as TGF-β and TNF-α are produced in the damaged tissue and secreted extracellularly. These proteins activate functional or structural changes in the cytoskeleton by various signaling pathways, thereby initiating the healing process. Here, under normal conditions, following the healing process, the pro-inflammatory proteins and the accumulated extracellular matrix (ECM) are reduced, thereby initiating the recovery process. However, under pathological conditions, such a normal recovery process does not proceed, leading to the accumulation of the secreted ECM. This is one of the key mechanisms that causes fibrosis. In response to external physical stimuli, cells undergo changes in intracellular signal transmission (e.g., mechanotransduction) and changes in cell-cell interaction, resulting in changes in gene expression, cell morphology, and the cytoskeleton. These cellular changes become a critical driving force for cell movement or the remodeling of a cell surface. When damage caused by various external factors is not restored to its original state through healing and recovery, the wound progresses into a process that causes fibrosis.

Epithelial-mesenchymal transition (EMT) and fibroblast-myofibroblast transition (FMT) are the key mechanisms leading to fibrosis. In damaged tissue such as lungs, liver, or kidneys, inflammatory cytokines, such as TGF-β, are secreted, and an activated epithelial layer undergoes EMT due to the disruption of cell connective tissue caused by inflammatory signals, and epithelial cells are converted into fibroblasts. In addition, when fibroblasts present in a subepithelial layer are mechanically and physically damaged, they are effectively activated by cytokines that cause fibrosis while stress fibers are expressed through a mechanotransduction pathway, and converted into myofibroblasts through FMT.

The regulation of cytoskeleton remodeling is particularly important in the fibrotic process, and an increase in intracellular F-actin, which is important in the cellular cytoskeleton, activates the transcription cofactor, myocardin-related transcription factor (MRTF), and through the MRTF-serum response factor (SRF) signaling process, the expression of fibrosis-related factors increases. Accordingly, an abnormal fibrosis mechanism can be improved by regulating actin polymerization. Cells regulate cell motility or form stress fibers through actin polymerization, resulting in cytoskeleton remodeling, and a target protein involved in this remodeling is an actin-binding protein. The Arp2/3 complex, one of the target proteins, acts in the nucleation stage among several stages in regulating cytoskeletal motility to induce the formation of filament branches, and consists of 7 subunits (Arp2, Arp3, ARPC1, ARPC2, ARPC3, ARPC4, and ARPC5). Here, the Arp2/3 complex affects the amplification of actin microfibers through the formation of actin branches, and affects the rate of actin network formation in fibroblasts. Therefore, it is possible to regulate cellular deformation by suppressing the actin network and cytoskeleton remodeling, caused by actin polymerization, by regulating the function of the Arp2/3 complex.

Cytoskeleton remodeling is involved in myofibroblast activation (FMT) and EMT, and the key biomarker for the activity of myofibroblasts is known to be α-smooth muscle actin (α-SMA). In the process of fibrosis of lungs, liver and kidneys, fibroblast transition and myofibroblast activation have been reported as key factors of fibrosis, so the extent of α-SMA, a key biomarker, can be used to evaluate myofibroblast activity.

Therefore, the present inventors aimed to develop, as a substance for regulating the function of the Arp2/3 complex involved in actin polymerization that regulates cytoskeletal remodeling, a substance that regulates the expression (formation) of fibrosis-related factors, such as α-SMA and F-actin, and a drug for treating a fibrosis-related disease.

### [Disclosure]

### [Technical Problem]

The present invention was devised to solve the above-mentioned problems, and the inventors of the present invention synthesized a novel compound and confirmed that the compound is capable of treating lung, liver, and kidney fibrosis-related diseases by regulating the expression (formation) of fibrosis-related factors such as α-SMA, F-actin, and COL1A1, thereby completing the present invention.

Therefore, a main object of the present invention is to provide a compound represented by the following Chemical Formula 1, a stereoisomer thereof, or a pharmaceutically acceptable salt thereof:

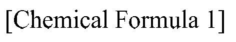

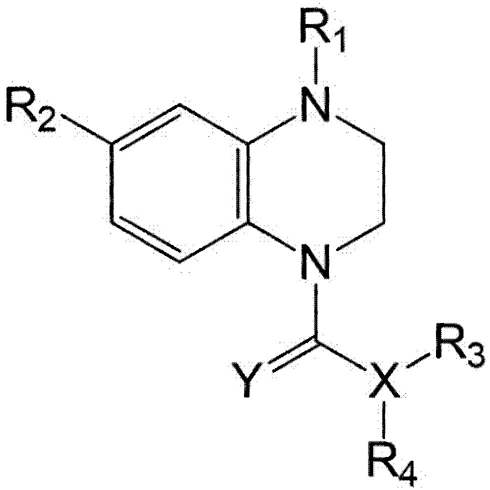

Another object of the present invention is to provide a pharmaceutical composition for preventing or treating fibrosis-related diseases, comprising the compound, a stereoisomer thereof, or a pharmaceutically acceptable salt thereof as an active ingredient.

Yet another object of the present invention is to provide a kit for preventing or treating fibrosis-related diseases, comprising the composition.

However, technical problems to be solved in the present invention are not limited to the above-described problems, and other problems which are not described herein will be fully understood by those of ordinary skill in the art from the following descriptions.

### [Technical Solution]

The present invention provides a compound represented by the following Chemical Formula 1, a stereoisomer thereof, or a pharmaceutically acceptable salt thereof: 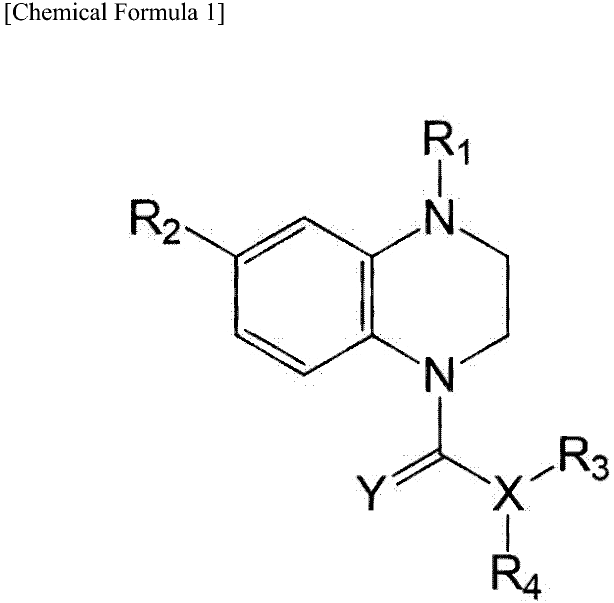 (In Chemical Formula 1,
X is N, S, or O;
Y is O or S;
R₁ is a C₁-C₁₀ alkyl, a C₃-C₂₀ cycloalkyl, a C₂-C₂₀ heterocycloalkyl, a 3- to 10-membered aromatic cyclic group, a 3- to 10-membered aromatic heterocyclic group, - CO-(C₁-C₆ alkyl), or -CO-(halogen-substituted or unsubstituted C₆-C₁₂ aryl),
one or more H atoms of R₁ are substituted with a halogen, a halogen-substituted or unsubstituted C₆-C₁₂ aryl, or a halogen-substituted or unsubstituted C₅-C₁₂ heteroaryl;
R₂ is hydrogen or a halogen;
R₃ and R₄ are linked to each other to form a 5- or 6-membered ring, where the ring has one or more H atoms that are substituted with -NH₂, dimethylamine, -NH-(C₁-C₅ alkyl), or a C₁-C₅ alkyl, and
when R₃ and R₄ do not form a ring, R₄ is absent or hydrogen, R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ are substituted with -NH-(C₁-C₅ alkyl), or a C₁-C₅ alkyl; and
the heterocycloalkyl, a heterocyclic group, and heteroaryl each independently comprise one or more heteroatoms selected from the group consisting of N, S, and O.)

In one embodiment of the present invention, R₃ and R₄ are linked to each other to form a 5- or 6-membered heterocycloalkyl, or 5- or 6-membered aromatic heterocyclic, where the 5- or 6-membered heterocycloalkyl or 5- or 6-membered aromatic heterocyclic comprises one or two N or O atoms, and X is N, and in the 5- or 6-membered heterocycloalkyl or 5- or 6-membered aromatic heterocyclic formed by R₃ and R₄, one or more H atoms may be substituted with -NH₂, dimethylamine, -NH-(C₁-C₅ alkyl), or a C₁-C₅ alkyl, but is not limited thereto.

In another embodiment of the present invention, R₁ is a 6-membered aromatic cyclic group, a 6-membered aromatic heterocyclic group, -CH₂-aryl, -CH₂-heteroaryl, a 6-membered cycloalkyl, a 6-membered heterocycloalkyl, -CO-aryl, or -CO-(C₄-C₅ alkyl), where the 6-membered aromatic heterocyclic group and the heteroaryl comprise one or more N atoms, and the 6-membered heterocycloalkyl comprises one or more O atoms, and one or more H atoms of R₁ may be substituted with a halogen, but is not limited thereto.

In yet another embodiment of the present invention, R₁ is a 6-membered aromatic cyclic group, a 6-membered aromatic heterocyclic group, -CH₂-aryl, -CH₂-heteroaryl, or -CO-(C₅ alkyl), where the 6-membered aromatic heterocyclic group and the heteroaryl comprise one or more N atoms,
one or more H atoms of R₁ may be substituted with a halogen;
R₂ is hydrogen;
R₃ and R₄ are linked to each other to form a 5- or 6-membered heterocycloalkyl, or 5- or 6-membered aromatic heterocyclic, where the 5- or 6-membered heterocycloalkyl or 5- or 6-membered aromatic heterocyclic comprises one or more N or O atoms, and X is N, and
in the 5- or 6-membered heterocycloalkyl or 5- or 6-membered aromatic heterocyclic formed by R₃ and R₄, one or more H atoms may be substituted with -NH₂, or -NH-(C₁-C₅ alkyl),
when R₃ and R₄ do not form a ring, R₄ is absent or hydrogen, R₃ is a C₄-C₆ heterocycloalkyl, -CH₂-(C₃ cycloalkyl), or -CH₂-(C₄-C₆ heterocycloalkyl), where one or more H atoms of R₃ may be substituted with a C₁-C₄ alkyl, but is not limited thereto.

In yet another embodiment of the present invention, the compound represented by Chemical Formula 1 may be any one selected from the group consisting of the following compounds, but is not limited thereto:
(1) (*R*)-6-fluoro-4-(4-fluorophenyl)-*N*-(pyrrolidin-2-ylmethyl)-3,4-dihydroquinoxaline-1(2*H*)-carboxamide;
(2) 6-fluoro-4-(4-fluorophenyl)-*N*-((isopropylpiperidin-4-yl)methyl)-3,4-dihydroquinoxaline-1(2*H*)-carboxamide;
(3) *N*-(cyclopropylmethyl)-6-fluoro-4-(4-fluorophenyl)-3,4-dihydroquinoxaline-1(2*H*)-carboxamide;
(4) (*R*)-4-(4-fluorophenyl)-*N*-(pyrrolidin-2-ylmethyl)-3,4-dihydroquinoxaline-1(2*H*)-carboxamide;
(5) 4-(4-fluorophenyl)-*N*-((isopropylpiperidin-4-yl)methyl)-3,4-dihydroquinoxaline-1(2*H*)-carboxamide;
(6) *N*-(cyclopropylmethyl)-4-(4-fluorophenyl)-3,4-dihydroquinoxaline-1(2*H*)-carboxamide;
(7) (*S*)-4-(4-fluorophenyl)-*N*-(tetrahydrofuran-3-yl)-3,4-dihydroquinoxaline-1(2*H*)-carboxamide;
(8) (4-(2-chlorophenyl)-3,4-dihydroquinoxaline-1(2*H*)-yl)(pyrrolidin-1-yl)methanone;
(9) (4-(2-chlorophenyl)-3,4-dihydroquinoxaline-1(2*H*)-yl)(morpholino)methanone;
(10) (4-(2-chlorophenyl)-3,4-dihydroquinoxaline-1(2*H*)-yl)(piperazin-1-yl)methanone;
(11) (*S*)-*N*-(1-isopropylpyrrolidin-3-yl)-4-(pyridin-2-yl)-3,4-dihydroquinoxaline-1(2*H*)-carboxamide;
(12) (*S*)-(3-aminopyrrolidin-1-yl)(4-(pyridin-2-yl)-3,4-dihydroquinoxaline-1(*2H*)-yl)methanone;
(13) *N*-(piperidin-4-yl)-4-(pyridin-2-yl)-3,4-dihydroquinoxaline-1(2*H*)-carboxamide;
(14) (*R*)-4-(pyridin-2-yl)-*N*-(pyrrolidin-3-ylmethyl)-3,4-dihydroquinoxaline-1(2*H*)-carboxamide;
(15) (*R*)-4-(pyridin-2-yl)-*N*-(pyrrolidin-2-ylmethyl)-3,4-dihydroquinoxaline-1(2*H*)-carboxamide;
(16) (*R*)-4-(pyridin-3-yl)-*N*-(tetrahydrofuran-3-yl)-3,4-dihydroquinoxaline-1(2*H*)-carboxamide;
(17) *N*-(cyclopropylmethyl)-4-(pyridin-4-yl)-3,4-dihydroquinoxaline-1(2*H*)-carboxamide;
(18) 4-(5-fluoropyridin-2-yl)-*N*-(1-methylpiperidin-4-yl)-3,4-dihydroquinoxaline-1(2*H*)-carboxamide;
(19) (*S*)-4-(pyrazin-2-yl)-N-(pyrrolidin-3-yl)-3,4-dihydroquinoxaline-1(*2H*)-carboxamide;
(20) (*S*)-*N*-(1-methylpyrrolidin-3-yl)-4-(pyrazin-2-yl)-3,4-dihydroquinoxaline-1(2*H*)-carboxamide;
(21) (*S*)-*N*-(1-isopropylpyrrolidin-3-yl)-4-(pyrazin-2-yl)-3,4-dihydroquinoxaline-1(2*H*)-carboxamide;
(22) (*S*)-*N*-(1-isobutylpyrrolidin-3-yl)-4-(pyrazin-2-yl)-3,4-dihydroquinoxaline-1(*2H*)-carboxamide;
(23) *N*-(piperidin-4-yl)-4-(pyrazin-2-yl)-3,4-dihydroquinoxaline-1(2*H*)-carboxamide;
(24) *N*-(1-methylpiperidin-4-yl)-4-(pyrazin-2-yl)-3,4-dihydroquinoxaline-1(2*H*)-carboxamide oxalic acid salt;
(25) *N*-(1-methylpiperidin-4-yl)-4-(pyrazin-2-yl)-3,4-dihydroquinoxaline-1(2*H*)-carboxamide fumaric acid salt;
(26) *N*-(1-isopropylpiperidin-4-yl)-4-(pyrazin-2-yl)-3,4-dihydroquinoxaline-1(2*H*)-carboxamide;
(27) *N*-(1-isobutylpiperidin-4-yl)-4-(pyrazin-2-yl)-3,4-dihydroquinoxaline-1(2*H*)-carboxamide;
(28) imidazole-1-yl-(4-pyrazin-2-yl-2,3-dihydroquinoxaline-1-yl)methanethione;
(29) *N*-(1-methylpiperidin-4-yl)-4-(pyrazin-2-yl)-3,4-dihydroquinoxaline-1(*2H*)-carbothioamide;
(30) 1-methylpiperidin-4-yl-4-(pyrazin-2-yl)-3,4-dihydroquinoxaline-1(*2H*)-carboxylate;
(31) (*R*)-(1-methylpyrrolidin-3-yl)methyl-4-(pyrazin-2-yl)-3,4-dihydroquinoxaline-1(2*H*)-carboxylate;
(32) (*S*)-(1-methylpiperidin-4-yl)-4-(pyrazin-2-yl)-3,4-dihydroquinoxaline-1(2*H*)-carbothioate;
(33) (*R*)-4-benzyl-*N*-(pyrrolidin-3-yl)-3,4-dihydroquinoxaline-1(2*H*)-carboxamide;
(34) (*R*)-4-benzyl-N-(1-methylpyrrolidin-3-yl)-3,4-dihydroquinoxaline-1(2*H*)-carboxamide;
(35) (*S*)-(3-aminopyrrolidin-1-yl)(4-benzyl-3,4-dihydroquinoxaline-1(*2H*)-yl)methanone;
(36) (*S*)-(4-benzyl-3,4-dihydroquinoxaline-1(2*H*)-yl)(3-(methylamino)pyrrolidin-1-yl)methanone;
(37) (*S*)-(3-aminopyrrolidin-1-yl)(4-(2-fluorobenzyl)-3,4-dihydroquinoxaline-1(*2H*)-yl)methanone;
(38) (*S*)-(4-(2-fluorobenzyl)-3,4-dihydroquinoxaline-1(2*H*)-yl)(3-(methylamino)pyrrolidin-1-yl)methanone;
(39) (*S*)-(3-aminopyrrolidin-1-yl)(4-(3-fluorobenzyl)-3,4-dihydroquinoxaline-1(*2H*)-yl)methanone;
(40) (*S*)-(4-(3-fluorobenzyl)-3,4-dihydroquinoxaline-1(*2H*)-yl)(3-(methylamino)pyrrolidin-1-yl)methanone;
(41) (*S*)-(3-aminopyrrolidin-1-yl)(4-(4-fluorobenzyl)-3,4-dihydroquinoxaline-1(2*H*)-yl)methanone fumaric acid salt;
(42) (*S*)-(4-(4-fluorobenzyl)-3,4-dihydroquinoxaline-1(2*H*)-yl)(3-(isopropylamino)pyrrolidin-1-yl)methanone;
(43) (*S*)-4-(pyridin-2-ylmethyl)-*N*-(pyrrolidin-3-yl)-3,4-dihydroquinoxaline-1(2*H*)-carboxamide;
(44) (*S*)-*N*-(1-isobutylpyrrolidin-3-yl)-4-(pyrazin-2-ylmethyl)-3,4-dihydroquinoxaline-1(2*H*)-carboxamide;
(45) (*S*)-(3-aminopyrrolidin-1-yl)(4-(pyrazin-2-ylmethyl)-3,4-dihydroquinoxaline-1(2*H*)-yl)methanone;
(46) (*S*)-(3-(dimethylamino)pyrrolidin-1-yl)(4-(pyridin-2-ylmethyl)-3,4-dihydroquinoxaline-1(*2H*)-yl)methanone;
(47) (*S*)-(3-(isopropylamino)pyrrolidin-1-yl)(4-(pyridin-2-ylmethyl)-3,4-dihydroquinoxaline-1(2*H*)-yl)methanone;
(48) (*S*)-(3-(isobutylamino)pyrrolidin-1-yl)(4-(pyridin-2-ylmethyl)-3,4-dihydroquinoxaline-1(*2H*)-yl)methanone oxalic acid salt;
(49) (*S*)-(3-(isobutylamino)pyrrolidin-1-yl)(4-(pyridin-2-ylmethyl)-3,4-dihydroquinoxaline-1(*2H*)-yl)methanone succinic acid salt;
(50) (*S*)-(3-(isobutylamino)pyrrolidin-1-yl)(4-(pyridin-2-ylmethyl)-3,4-dihydroquinoxaline-1(*2H*)-yl)methanone maleic acid salt;
(51) (*S*)-(3-(isobutylamino)pyrrolidin-1-yl)(4-(pyridin-2-ylmethyl)-3,4-dihydroquinoxaline-1(2*H*)-yl)methanone fumaric acid salt;
(52) (*R*)-4-(pyridin-2-ylmethyl)-N-(pyrrolidin-2-ylmethyl)-3,4-dihydroquinoxaline-1(2*H*)-carboxamide;
(53) *N*-(piperidin-4-yl)-4-(pyridin-2-ylmethyl)-3,4-dihydroquinoxaline-1(2*H*)-carboxamide;
(54) *N*-(oxetan-3-yl)-4-(pyridin-2-ylmethyl)-3,4-dihydroquinoxaline-1(*2H*)-carboxamide;
(55) *N*-(3-methyloxetan-3-yl)-4-(pyridin-2-ylmethyl)-3,4-dihydroquinoxaline-1(2*H*)-carboxamide;
(56) *N*-(oxetan-3-ylmethyl)-4-(pyridin-2-ylmethyl)-3,4-dihydroquinoxaline-1(2*H*)-carboxamide;
(57) *N*-((3-methyloxetan-3-yl)methyl)-4-(pyridin-2-ylmethyl)-3,4-dihydroquinoxaline-1(2*H*)-carboxamide;
(58) 4-(pyridin-2-ylmethyl)-*N*-((tetrahydrofuran-3-yl)methyl)-3,4-dihydroquinoxaline-1(2*H*)-carboxamide;
(59) (*S*)-4-(pyridin-2-ylmethyl)-*N*-((tetrahydrofuran-3-yl)methyl)-3,4-dihydroquinoxaline-1(2*H*)-carboxamide;
(60) (*R*)-4-(pyridin-2-ylmethyl)-N-((tetrahydrofuran-3-yl)methyl)-3,4-dihydroquinoxaline-1(2*H*)-carboxamide;
(61) *N*-(cyclopropylmethyl)-4-(pyridin-2-ylmethyl)-3,4-dihydroquinoxaline-1(*2H*)-carboxamide;
(62) 1-methylpiperidin-4-yl-4-(pyridin-2-ylmethyl)-3,4-dihydroquinoxaline-1(*2H*)-carboxylate;
(63) (*S*)-(1-methylpiperidin-4-yl)-4-(pyridin-2-ylmethyl)-3,4-dihydroquinoxaline-1(2*H*)-carbothioate;
(64) (*R*)-(1-methylpyrrolidin-3-yl)methyl-4-(pyridin-2-ylmethyl)-3,4-dihydroquinoxaline-1(2*H*)-carboxylate;
(65) (1-methylpiperidin-4-yl)methyl-4-(pyridin-2-ylmethyl)-3,4-dihydroquinoxaline-1(2*H*)-carboxylate;
(66) 4-((5-fluoropyridine-2-yl)methyl)-*N*-(piperidin-4-ylmethyl)-3,4-dihydroquinoxaline-1(*2H*)-carboxamide oxalic acid salt;
(67) 4-((5-fluoropyridin-2-yl)methyl)-*N*-((1-methylpiperidin-4-yl)methyl)-3,4-dihydroquinoxaline-1(2*H*)-carboxamide;
(68) 4-((3-fluoropyridin-2-yl)methyl)-*N*-(piperidin-4-ylmethyl)-3,4-dihydroquinoxaline-1(2*H*)-carboxamide;
(69) 4-((3-fluoropyridin-2-yl)methyl)-*N*-((1-methylpiperidin-4-yl)methyl)-3,4-dihydroquinoxaline-1(2*H*)-carboxamide;
(70) (4-cyclohexyl-3,4-dihydroquinoxaline-1(2*H*)-yl)(4-methylpiperazin-1-yl)methanone;
(71) 4-cyclohexyl-3,4-dihydroquinoxaline-1(*2H*)-yl)(piperidin-1-yl)methanone;
(72) (*R*)-*N*-(1-methylpyrrolidin-3-yl)-4-(tetrahydro-2H-pyran-4-yl)-3,4-dihydroquinoxaline-1(2*H*)-carboxamide;
(73) (*S*)-(3-aminopyrrolidin-1-yl)(4-(tetrahydro-2H-pyran-4-yl)-3,4-dihydroquinoxaline-1(2*H*)-yl)methanone;
(74) (*S*)-4-benzoyl-*N*-(pyrrolidin-3-ylmethyl)-3,4-dihydroquinoxaline-1(*2H*)-carboxamide;
(75) (*R*)-4-benzoyl-*N*-((1-methylpirrolidin-3-yl)methyl)-3,4-dihydroquinoxaline-1(2*H*)-carboxamide;
(76) 4-(4-fluorobenzoyl-*N*-(piperidin-4-yl)-3,4-dihydroquinoxaline-1(*2H*)-carboxamide;
(77) 4-(3-methylbutanoyl)-*N*-(piperidin-4-ylmethyl)-3,4-dihydroquinoxaline-1(2*H*)-carboxamide;
(78) 3,3-dimethyl-1-(4-(piperazine-1-carbonyl)-3,4-dihydroquinoxaline-1(2*H*)-yl)butan-1-one; and
(79) 4-(3,3-dimethylbutanoyl)-*N*-(piperidin-4-ylmethyl)-3,4-dihydroquinoxaline-1(2*H*)-carboxamide.

Additionally, the present invention provides a pharmaceutical composition for preventing or treating a fibrosis-related disease, comprising the compound represented by Chemical Formula 1, a stereoisomer thereof, or a pharmaceutically acceptable salt thereof as an active ingredient.

Additionally, the present invention provides a method of preventing or treating a fibrosis-related disease, comprising administering the compound represented by Chemical Formula 1, a stereoisomer thereof, or a pharmaceutically acceptable salt thereof to a subject in need thereof at a pharmaceutically effective amount.

Additionally, the present invention provides a use of the compound represented by Chemical Formula 1, a stereoisomer thereof, or a pharmaceutically acceptable salt thereof for the prevention or treatment of a fibrosis-related disease.

Additionally, the present invention provides a use of the compound represented by Chemical Formula 1, a stereoisomer thereof, or a pharmaceutically acceptable salt thereof for the preparation of a drug for preventing or treating a fibrosis-related disease.

In one embodiment of the present invention, the composition may inhibit F-actin formation, but is not limited thereto.

In another embodiment of the present invention, the composition may decrease the level or activity of α-SMA protein, but is not limited thereto.

In yet another embodiment of the present invention, the composition may reduce the expression or activity of one or more mRNAs or proteins selected from the group consisting of α-SMA, COL1A1, and F-actin, but is not limited thereto.

In yet another embodiment of the present invention, the fibrosis-related disease may be a fibrosis-related disease induced by an inflammatory cytokine, but is not limited thereto.

In yet another embodiment of the present invention, the fibrosis-related disease may be a disease associated with liver, renal, or lung fibrosis, but is not limited thereto.

In yet another embodiment of the present invention, the liver fibrosis-related disease may be one or more selected from the group consisting of liver fibrosis, cirrhosis, biliary cirrhosis, alcoholic or non-alcoholic steatohepatitis, viral hepatitis (types A, B, C, D, E, or G), autoimmune hepatitis, and sclerosing cholangitis, but is not limited thereto.

In yet another embodiment of the present invention, the renal fibrosis-related disease may be one or more selected from the group consisting of renal fibrosis, end-stage kidney disease (ESKD), diabetic nephropathy (DN), IgA nephropathy (IgAN), HIV-related nephropathy, non-diabetic chronic kidney disease, focal segmental glomerulosclerosis (FSGS), minimal change disease (MCD), and xanthine oxidase deficiency, but is not limited thereto.

In yet another embodiment of the present invention, the lung fibrosis-related disease may be one or more selected from the group consisting of lung fibrosis, idiopathic pulmonary fibrosis, desquamative interstitial pneumonia, idiopathic interstitial pneumonia, non-specific interstitial pneumonia, cryptogenic organizing pneumonia, interstitial lung disease associated with respiratory bronchiolitis, acute interstitial pneumonia, lymphocytic interstitial pneumonia, idiopathic parenchymal elastosis, interstitial lung disease, and chronic obstructive pulmonary disease (COPD), but is not limited thereto.

Additionally, the present invention provides a kit for preventing or treating a fibrosis-related disease, the kit comprising the compound represented by Chemical Formula 1, a stereoisomer thereof, or a pharmaceutically acceptable salt thereof; or the composition.

Additionally, the present invention provides a pharmaceutical composition for inhibiting or improving a fibrosis-related disease, comprising the compound represented by Chemical Formula 1, a stereoisomer thereof, or a pharmaceutically acceptable salt thereof as an active ingredient.

Additionally, the present invention provides a method of inhibiting or improving a fibrosis-related disease, comprising administering the compound represented by Chemical Formula 1, a stereoisomer thereof, or a pharmaceutically acceptable salt thereof to a subject in need thereof at a pharmaceutically effective amount.

Additionally, the present invention provides a use of the compound represented by Chemical Formula 1, a stereoisomer thereof, or a pharmaceutically acceptable salt thereof for inhibiting or improving a fibrosis-related disease.

Additionally, the present invention provides a use of the compound represented by Chemical Formula 1, a stereoisomer thereof, or a pharmaceutically acceptable salt thereof for the preparation of a drug for inhibiting or improving a fibrosis-related disease.

### [Advantageous Effects]

The present invention relates to a novel compound having an anti-fibrotic effect, and the compound according to the present invention or a pharmaceutically acceptable salt thereof has been confirmed to effectively inhibit the expression (formation) of α-SMA, F-actin, and COL1A1, which are major causative factors of fibrosis. Therefore, the novel compound according to the present invention is expected to be usefully employed as a therapeutic agent having an effect of improving or inhibiting fibrosis, and for the prevention and treatment of fibrosis-related diseases.

### [Description of Drawings]

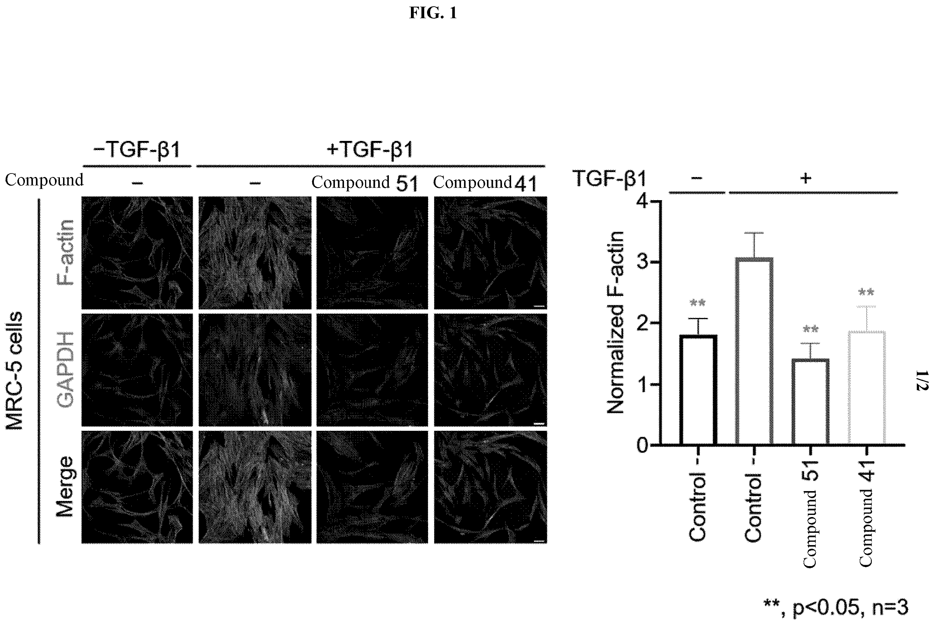

FIG. 1 shows the results of comparing the degree of F-actin formation after treating human-derived pulmonary fibroblasts (MRC-5 cells) with an inflammatory cytokine (TGF-β1) in order to measure the inhibitory activity of the compound according to the present invention on F-actin formation.

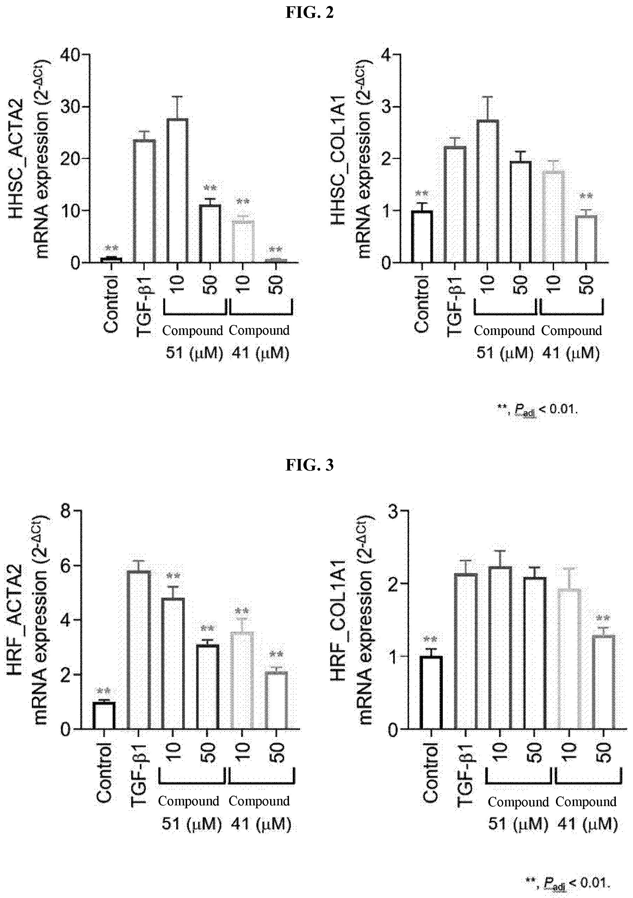

FIG. 2 shows the results of evaluating the therapeutic efficacy of the compound according to the present invention for liver fibrosis by treating human hepatic stellate cells (HHSC) with the compound according to the present invention together with an inflammatory cytokine (TGF-β1), followed by determining the mRNA expression levels of α-SMA *(ACTA2)* and *COL1A1* using qRT-PCR.
FIG. 3 shows the results of evaluating the therapeutic efficacy of the compound according to the present invention for renal fibrosis by treating human renal fibroblasts (HRF) with the compound according to the present invention together with an inflammatory cytokine (TGF-β1), followed by determining the mRNA expression levels of α-SMA *(ACTA2)* and *COLIA1* using qRT-PCR.

### [Best Mode]

The present invention relates to a novel compound, and the present invention was completed by confirming that the compound not only regulates abnormal cytoskeleton formation by inhibiting the formation of F-actin, but also has the effect of inhibiting the expression of fibrosis-related factors α-SMA and *COL1A1.*

Specifically, in one example of the present invention, a novel compound according to the present invention was synthesized (Experimental Example 1).

In another example of the present invention, the inhibitory effect of the compound of the present invention on the fibrosis-related factor α-SMA was verified, and it was confirmed that the compound of the present invention has an excellent inhibitory effect on α-SMA expression in MRC-5 cells, thereby confirming its efficacy against fibrosis (Experimental Example 2).

In yet another example of the present invention, when the compound according to the present invention was administered to MRC-5 cells and the change in the level of the fibrosis-related factor F-actin was measured, it was confirmed that the formation of F-actin was significantly reduced by the treatment with the compound of the present invention (Experimental Example 3).

In yet another example of the present invention, when the compound of the present invention was administered to human hepatic stellate cells together with an inflammatory cytokine, it was confirmed that the increase in mRNA expression of α-SMA (*ACTA2*) and *COLIA1* induced by the inflammatory cytokine was significantly inhibited, thereby confirming that the compound of the present invention can effectively inhibit liver fibrosis (Experimental Example 4).

In yet another example of the present invention, when the compound according to the present invention was administered to human renal fibroblasts together with an inflammatory cytokine, it was confirmed that the increase in mRNA expression of α-SMA (*ACTA2*) and *COLIA1* induced by the inflammatory cytokine was significantly inhibited, thereby confirming that the compound of the present invention can effectively inhibit renal fibrosis (Experimental Example 5).

These results demonstrate that the novel compound according to the present invention can effectively inhibit fibrosis-related factors that induce fibrosis, and the compound of the present invention is expected to be variously utilized in the therapeutic field of various diseases associated with fibrosis.

Hereinafter, the present invention will be described in detail.

The present invention provides a compound represented by the following Chemical Formula 1, a stereoisomer thereof, or a pharmaceutically acceptable salt thereof: 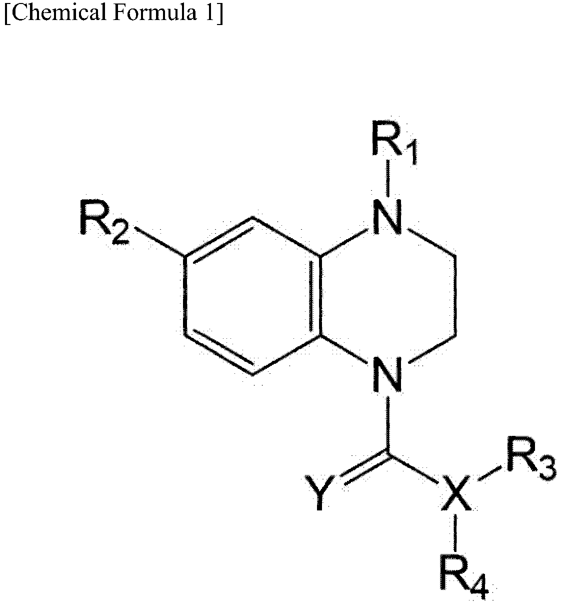 (In Chemical Formula 1,
X is N, S, or O;
Y is O or S;
R₁ is a C₁-C₁₀ alkyl, a C₃-C₂₀ cycloalkyl, a C₂-C₂₀ heterocycloalkyl, a 3- to 10-membered aromatic cyclic group, a 3- to 10-membered aromatic heterocyclic group, - CO-(C₁-C₆ alkyl), or -CO-(halogen-substituted or unsubstituted C₆-C₁₂ aryl),
one or more H atoms of R₁ are substituted with a halogen, a halogen-substituted or unsubstituted C₆-C₁₂ aryl, or a halogen-substituted or unsubstituted C₅-C₁₂ heteroaryl;
R₂ is hydrogen or a halogen;
R₃ and R₄ are linked to each other to form a 5- or 6-membered ring, where the ring has one or more H atoms that are substituted with -NH₂, dimethylamine, -NH-(C₁-C₅ alkyl), or a C₁-C₅ alkyl, and
when R₃ and R₄ do not form a ring, R₄ is absent (when X is O or S) or hydrogen, R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ are substituted with -NH-(C₁-C₅ alkyl), or a C₁-C₅ alkyl; and
the heterocycloalkyl, a heterocyclic group, and heteroaryl each independently comprise one or more heteroatoms selected from the group consisting of N, S, and O.)

Here, the -CO-(C₁-C₆ alkyl) means a C₁-C₆ alkyl connected to CO. For example, when R₁ is -CO-(C₁-C₆ alkyl), it means a C₁-C₆ alkyl connected to a nitrogen atom (N) through CO.

In the present invention, specifically, when X is O or S, R₄ is absent, and R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ may be substituted with -NH-(C₁-C₅ alkyl) or a C₁-C₅ alkyl,
when X is N, R₃ and R₄ may be linked to each other to form a 5- or 6-membered ring, where one or more H atoms of the ring may be substituted with -NH₂, dimethylamine, -NH-(C₁-C₅ alkyl), or a C₁-C₅ alkyl,
and when R₃ and R₄ do not form a ring, R₄ is hydrogen, and R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ may be substituted with -NH-(C₁-C₅ alkyl) or a C₁-C₅ alkyl, but is not limited thereto.

In the present invention, the statement that R₃ and R₄ are linked to each other to form a 5- or 6-membered ring means that R₃ and R₄ are linked to each other through another atom to form a ring structure. The ring may be composed of a single bond, multiple bonds (double bond, triple bond, etc.), or a combination thereof (i.e., including both cycloalkyl and aromatic rings), and may be a ring consisting entirely of carbon atoms or a heterocyclic group including one or more heteroatoms. Preferably, when R₃ and R₄ are linked to each other to form a 5- or 6-membered ring, both R₃ and R₄ are carbon atoms.

In one embodiment of the present invention, R₃ and R₄ are linked to each other to form a 5- or 6-membered heterocycloalkyl, or a 5- or 6-membered aromatic heterocyclic, and the 5- or 6-membered heterocycloalkyl or the 5- or 6-membered aromatic heterocyclic includes 1 to 2 N or O atoms in the ring, wherein X may be N, but is not limited thereto. In addition, the 5- or 6-membered heterocycloalkyl or the 5- or 6-membered aromatic heterocyclic formed by R₃ and R₄ may have one or more H atoms substituted with -NH₂, dimethylamine, -NH-(C₁-C₅ alkyl), or C₁-C₅ alkyl, but is not limited thereto.

In another embodiment of the present invention, R₁ is a 6-membered aromatic cyclic group (e.g., aryl), a 6-membered aromatic heterocyclic group (e.g., heteroaryl), - CH₂-aryl, -CH₂-heteroaryl, a 6-membered cycloalkyl, a 6-membered heterocycloalkyl, - CO-aryl, or -CO-(C₄-C₅ alkyl), where the 6-membered aromatic heterocyclic group and the heteroaryl may include one or more N atoms, and the 6-membered heterocycloalkyl may include one or more O atoms, but is not limited thereto. In addition, one or more H atoms of R₁ may be substituted with a halogen, but is not limited thereto.

In yet another embodiment of the present invention, when R₃ and R₄ do not form a ring, when X is O or S, R₄ is absent, and R₃ is a C₂-C₁₀ heterocycloalkyl or -CH₂-(C₂-C₆ heterocycloalkyl), where one or more H atoms of R₃ may be substituted with a C₁-C₅ alkyl,
when X is N, R₄ is hydrogen, and R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ may be substituted with -NH-(C₁-C₅ alkyl) or a C₁-C₅ alkyl. Here, the heterocycloalkyl may be a 4- to 6-membered heterocycloalkyl including 1 to 2 N or O atoms, but is not limited thereto.

In yet another embodiment of the present invention, R₁ is a 6-membered aromatic cyclic group, a 6-membered aromatic heterocyclic group, -CH₂-aryl, -CH₂-heteroaryl, or -CO-(C₅ alkyl), where the 6-membered aromatic heterocyclic group and the heteroaryl include one or more N atoms,
one or more H atoms of R₁ may be substituted with a halogen;
R₂ is hydrogen;
R₃ and R₄ are linked to each other to form a 5- or 6-membered heterocycloalkyl, or 5- or 6-membered aromatic heterocyclic, where the 5- or 6-membered heterocycloalkyl or 5- or 6-membered aromatic heterocyclic includes one or more N or O atoms, and X is N, and
in the 5- or 6-membered heterocycloalkyl or 5- or 6-membered aromatic heterocyclic formed by R₃ and R₄, one or more H atoms may be substituted with -NH₂, or -NH-(C₁-C₅ alkyl),
when R₃ and R₄ do not form a ring, R₄ is absent (when X is O or S) or hydrogen, R₃ is a C₄-C₆ heterocycloalkyl, -CH₂-(C₃ cycloalkyl), or -CH₂-(C₄-C₆ heterocycloalkyl), where one or more H atoms of R₃ may be substituted with a C₁-C₄ alkyl, but is not limited thereto.

In another embodiment of the present invention, the compound may be represented by the following Chemical Formula 1-1, but is not limited thereto: 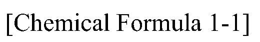 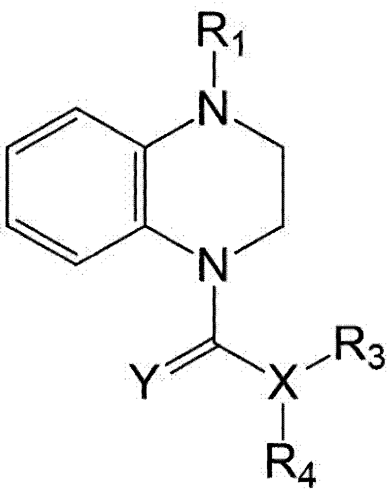 (In Chemical Formula 1-1,
R₁ is a 6-membered aromatic cyclic group, a 6-membered aromatic heterocyclic group, -CH₂-aryl, -CH₂-heteroaryl, or -CO-(C₅ alkyl), where the 6-membered aromatic heterocyclic group and the heteroaryl include one or more N atoms,
one or more H atoms of R₁ may be substituted with a halogen;
R₃ and R₄ are linked to each other to form a 5- or 6-membered heterocycloalkyl or a 5- or 6-membered aromatic heterocyclic, where the 5- or 6-membered heterocycloalkyl or the 5- or 6-membered aromatic heterocyclic includes one or more N or O atoms in the ring, wherein X is N,
the 5- or 6-membered heterocycloalkyl or the 5- or 6-membered aromatic heterocyclic formed by R₃ and R₄ may have one or more H atoms substituted with -NH₂ or -NH-(C₁-C₅ alkyl),
and when R₃ and R₄ do not form a ring, R₄ is absent or hydrogen, and R₃ is a C₄-C₆ heterocycloalkyl, -CH₂-(C₃ cycloalkyl), or -CH₂-(C₄-C₆ heterocycloalkyl), where one or more H atoms of R₃ may be substituted with a C₁-C₄ alkyl;
the heterocycloalkyl, heterocyclic group, and heteroaryl each independently include one or more heteroatoms selected from the group consisting of N, S, and O.)

Preferably, the compound may be any one of the compounds shown in Table 1 below, but is not limited thereto.

Throughout this specification, substituents may be denoted with omission of the term "-group."

In the present invention, the term "halogen" includes F, Cl, Br, or I.

In the present invention, the term "alkyl" means a fully saturated branched or unbranched (i.e., straight-chain or linear) hydrocarbon. In addition, the alkyl according to the present invention also includes a straight-chain or branched hydrocarbon containing a hydrocarbon cyclic group at the terminal or in the middle. In the present invention, the alkyl may be a C₁-C₂₀, C₁-C₁₅, C₁-C₁₂, C₁-C₁₀, C₁-C₈, C₁-C₆, C₁-C₅, C₁-C₄, or C₁-C₃ alkyl, but is not limited thereto. The alkyl is, for example, methyl, ethyl, n-propyl, isopropyl, n-butyl, isobutyl, sec-butyl, n-pentyl, isopentyl, neopentyl, iso-amyl, n-hexyl, 3-methylhexyl, 2,2-dimethylpentyl, 2,3-dimethylpentyl, or n-heptyl. In one embodiment of the present invention, the alkyl may be an aminoalkyl or a haloalkyl. The term "aminoalkyl" may mean an alkyl group in which one or more H atoms are substituted with a nitrogen atom (e.g., -NH-(C₁-C₅ alkyl)), and the term "haloalkyl" may mean an alkyl group in which one or more H atoms are substituted with a halogen atom.

In the present invention, the term "amino" refers to a functional group (-NH₂) in which a hydrogen atom is bound to a nitrogen atom.

The compound according to the present invention may include a cyclic substituent (e.g., a cycloalkyl group, an aryl group, etc.) comprising a single bond, multiple bonds (such as a double bond or a triple bond), or a combination thereof. That is, the compound according to the present invention may include a (hetero)cyclic group having a single bond and/or multiple bonds.

In the present invention, the term "cycloalkyl" means a saturated or partially unsaturated non-aromatic cyclic hydrocarbon group. In the present invention, the cycloalkyl may be a C₃-C₂₀, C₃-C₁₅, C₃-C₁₂, C₃-C₁₀, C₃-C₈, C₃-C₆, or C₃-C₅ cycloalkyl, but is not limited thereto. Specific examples of the cycloalkyl include cyclopropyl, cyclobutyl, cyclopentyl, cyclohexyl, cyclohexenyl, cycloheptyl, cycloheptenyl, cyclooctyl, and cyclooctenyl. In the present invention, the cycloalkyl includes "bicycloalkyl" or "tricycloalkyl." The terms "bicycloalkyl" or "tricycloalkyl," unless otherwise stated, mean a structure composed of two or more cycloalkyl portions sharing two or more atoms in common. In addition, the cycloalkyl group may be a polycyclic ring.

In the present invention, the term "heterocycloalkyl" means a cyclic hydrocarbon group including one or more heteroatoms in addition to carbon atoms in the ring. The heteroatoms may be one or more selected from the group consisting of N, O, P, and S. In one embodiment of the present invention, the heterocycloalkyl may be a cyclic amine or a heterocyclic amine containing an N atom, and may be preferably a 3- to 6-membered cyclic amine. For example, the heterocycloalkyl may be selected from the group consisting of piperidine, pyrrolidine, pyridine, morpholine, piperazine, pyrazine, pyrrolopyrazine, and azetidine. Alternatively, the heterocycloalkyl may be a 4- to 6-membered ring containing one or more O atoms. In the present invention, the heterocycloalkyl includes a "biheterocycloalkyl." The biheterocycloalkyl means a structure composed of two or more rings sharing two or more atoms in common, wherein at least one of the two or more rings is a heterocycloalkyl.

In the present invention, the term "aryl" means an aromatic system containing one or more rings used alone or in combination, and also includes a group in which the aromatic ring is fused with one or more carbon rings. In the present invention, the aryl may be a C₃-C₂₀, C₃-C₁₅, C₃-C₁₂, C₃-C₁₀, C₃-C₈, or C₃-C₆ aryl, but is not limited thereto. The aryl may mean, for example, phenyl, benzyl, naphthyl, or tetrahydronaphthyl, but is not limited thereto. The aryl includes heteroaryl.

In the present invention, the term "heteroaryl" means a monocyclic or bicyclic organic compound containing one or more heteroatoms in addition to carbon atoms in the ring. The heteroatoms may be one or more selected from the group consisting of N, O, P, and S. In one embodiment of the present invention, the heteroaryl may include one or more N atoms (i.e., one or more C atoms constituting the ring are substituted with N).

In addition, in the present invention, the heteroaryl may be an aromatic amine. In another embodiment of the present invention, the heteroaryl may include 1 to 3, 1 to 2, or 1 N atom.

The aryl and heteroaryl (aromatic rings) according to the present invention may include, as non-limiting examples, phenyl, biphenyl, benzyl, benzoyl, benzidine, toluyl, thienyl, furyl, naphthyl, pyrimidinyl, imidazolyl (imidazole), pyrrolyl, thiazolyl, oxazolyl, isoxazolyl, pyranyl, pyrazinyl, pyrrolinyl, pyridinyl (pyridyl), piperazinyl, pyrimidinyl, pyrazinyl, pyridazinyl, pyrazolyl, thiadiazolyl, triazolyl, indolyl, azaindolyl, indazolyl, azaindazolyl, benzimidazolyl, benzofuranyl, benzothienyl, benzisoxazolyl, benzoxazolyl, benzopyrazolyl, benzothiazolyl, benzothiadiazolyl, benzotriazolyl, adenylyl, quinolinyl, isoquinolinyl, naphthalenyl, tetrahydronaphthyl, and isomers thereof.

In addition, the compound according to the present invention may include a polycyclic ring in which a plurality of rings are fused. Specifically, the polycyclic ring means a substituent composed of two or more rings arranged linearly at an angle or in a condensed structure. For example, the compound may include a polycyclic ring in which 2 to 5 rings are fused (i.e., a bicyclic to pentacyclic ring), a polycyclic ring in which 2 to 4 rings are fused, or a polycyclic ring in which 2 to 3 rings are fused. Each of the rings constituting the polycyclic ring may include not only the rings described in the present specification but also any rings known in the art. For example, the ring may be a 3-membered ring, a 4-membered ring, a 5-membered ring, a 6-membered ring, a 10-membered ring, or a combination thereof, and is preferably a 5-membered ring and/or a 6-membered ring. In addition, each of the rings constituting the polycyclic ring may be formed only of single bonds, or may include multiple bonds (such as double bonds or triple bonds). Each of the rings constituting the polycyclic ring may be of the same or different types. For example, each of the rings may independently be a cycloalkyl, a heterocycloalkyl, a 3- to 10-membered aromatic cyclic group, or a 3- to 10-membered aromatic heterocyclic group.

In addition, the compound according to the present invention may include a heterocyclic group. The ring may include a single bond or multiple bonds (such as a double bond or a triple bond). In one embodiment of the present invention, the heterocyclic group may include one or more heteroatoms selected from the group consisting of N, O, and S.

In the present invention, the term "aromatic cyclic group" means a substituent in which a carbon compound is cyclically bonded through unsaturated bonds (e.g., a double bond), lone pairs, empty orbitals, and the like. In addition, the term "aromatic heterocyclic group" means a heterocyclic group in which the types of elements constituting the aromatic ring are two or more. In the present invention, the aromatic (hetero)cyclic group may be a 3- to 10-membered, 3- to 8-membered, 3- to 6-membered, 3- to 5-membered, 4- to 6-membered, 4- to 5-membered, or 5- to 6-membered ring, but is not limited thereto.

In the present invention, a substituent containing a heteroatom (such as a heterocyclic group, a heterocycloalkyl group, a heteroaryl group, and various other heterocyclic groups) may include one or more heteroatoms in its backbone structure. For example, a substituent containing a heteroatom may include 1 to 5, 1 to 4, 1 to 3, 1 to 2, or 1 heteroatom(s), but is not limited thereto.

In the present invention, the term "substituted" in "substituted or unsubstituted" means that one or more hydrogen atoms in an organic compound are replaced by other atomic groups to form a derivative, and the substituent refers to the introduced atomic group. That is, in the present invention, when any functional group is substituted, it means that one or more hydrogen atoms of the functional group are replaced with other atomic groups. In the present invention, each functional group (such as alkyl, haloalkyl, cycloalkyl, heterocycloalkyl, aryl, and heteroaryl) may independently have one or more hydrogen atoms substituted with other atomic groups. In the present invention, the term "substituted" includes monosubstitution, disubstitution, trisubstitution, and tetrasubstitution.

In the present invention, unless otherwise specified, the expression "substituted or unsubstituted" may mean that one or more H (hydrogen atoms) are substituted or unsubstituted with other functional groups. For example, the expression "C₃-C₁₀ aryl group substituted or unsubstituted with halogen" may mean that the C₃-C₁₀ aryl group is unsubstituted, or that one or more H atoms of the aryl group are substituted with halogen.

In the present invention, the term "isomer" refers to a compound having the same molecular formula but a different connectivity or spatial arrangement of constituent atoms within the molecule. Isomers include, for example, structural isomers and stereoisomers. The stereoisomer may be a diastereomer or an enantiomer. An enantiomer refers to an isomer that is not superimposable on its mirror image, like the relationship between the left and right hands, and is also called an optical isomer. Enantiomers are classified as R (Rectus: clockwise) and S (Sinister: counterclockwise) when four or more different substituents are attached to a chiral center carbon. Diastereomers refer to stereoisomers that are not in a mirror-image relationship and may be divided into cis-trans isomers arising from differences in the spatial arrangement of atoms.

In the present invention, the term "pharmaceutically acceptable salt" includes a salt derived from a pharmaceutically acceptable inorganic acid, organic acid, or base.

As used herein, the term "pharmaceutically acceptable" means a compound or composition which is suitable for use in contact with the tissues of a subject (for example, a human) without undue toxicity, irritation, allergic response, or other problems or complications, commensurate with a reasonable benefit/risk ratio, and within the scope of sound medical judgment.

Examples of suitable acids include hydrochloric acid, hydrobromic acid, sulfuric acid, nitric acid, perchloric acid, hydroiodic acid, fumaric acid, maleic acid, phosphoric acid, glycolic acid, lactic acid, salicylic acid, succinic acid, p-toluenesulfonic acid, tartaric acid, (+)-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetaminobenzoic acid, (+)-camphoric acid, camphorsulfonic acid, (+)-(1S)-camphorsulfonic acid, capric acid, caproic acid, caprylic acid, cinnamic acid, cyclamic acid, dodecylsulfuric acid, ethanedisulfonic acid, ethanesulfonic acid, 2-hydroxyethanesulfonic acid, galactaric acid, gentisic acid, glucoheptonic acid, D-gluconic acid, D-glucuronic acid, L-glutamic acid, α-ketoglutaric acid, hippuric acid, (+)-L-lactic acid, (±)-DL-lactic acid, lactobionic acid, (-)-L-malic acid, (±)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, palmitic acid, pamoic acid, L-pyroglutamic acid, salicylic acid, 4-aminosalicylic acid, sebacic acid, stearic acid, tannic acid, thiocyanic acid, camsylic acid, and undecylic acid. An acid addition salt may be prepared by conventional methods, for example, by dissolving the compound in an excess of an aqueous solution of acid and precipitating the salt using a water-miscible organic solvent such as methanol, ethanol, acetone, or acetonitrile. In addition, the acid or alcohol in water may be heated with an equimolar amount of the compound, followed by evaporating the mixture to dryness, or the precipitated salt may be obtained by suction filtration.

A salt derived from a suitable base may include alkali metals such as sodium and potassium, alkaline earth metals such as magnesium, and ammonium, but is not limited thereto. The alkali metal or alkaline earth metal salt may be obtained, for example, by dissolving the compound in an excess of an alkali metal hydroxide or alkaline earth metal hydroxide solution, filtering the insoluble compound salt, and evaporating and drying the filtrate. In this case, it is pharmaceutically suitable to prepare particularly sodium, potassium, or calcium salts as the metal salt, and the corresponding silver salt may be obtained by reacting the alkali metal or alkaline earth metal salt with an appropriate silver salt (e.g., silver nitrate).

The scope of the compounds of the present invention includes not only pharmaceutically acceptable salts but also all isomers, hydrates, and solvates that may be prepared by conventional methods.

In addition, the present invention provides a pharmaceutical composition for preventing or treating a fibrosis-related disease, comprising as an active ingredient the compound represented by Chemical Formula 1, a stereoisomer thereof, or a pharmaceutically acceptable salt thereof.

In the present invention, the term "fibrosis-related disease" includes all diseases caused directly or indirectly by fibrosis, and may also include diseases accompanied by fibrosis. Fibrosis refers to a phenomenon in which connective tissue is formed in a tissue by secretion of extracellular matrix, including collagen, by activated fibroblasts. More specifically, cellular changes such as epithelial-mesenchymal transition (EMT), activation of fibroblasts, infiltration of monocytes/macrophages, and cellular apoptosis, as well as activation of signaling molecules such as TGF-β (transforming growth factor beta) and angiotensin II, may cause fibrosis. Fibrosis may contribute to the healing process by forming scar tissue and the like, but when connective tissue accumulates excessively, it may impair the normal structure and function of the organ.

According to one example of the present invention, the fibrosis-related disease may be a fibrosis-related disease induced by an inflammatory cytokine (TGF-β1), occurring in the liver, kidney, or lung, that is, a liver, kidney, or lung fibrosis-related disease, and according to one example of the present invention, the fibrosis-related disease may be liver fibrosis, kidney fibrosis, or pulmonary fibrosis induced by TGF-β1, but is not limited thereto.

In the present invention, the liver fibrosis-related disease may be one or more selected from the group consisting of liver fibrosis, cirrhosis, hepatic cirrhosis, biliary cirrhosis, alcoholic or non-alcoholic steatohepatitis, viral hepatitis (including hepatitis A, B, C, D, E, and G), autoimmune hepatitis, and sclerosing cholangitis, but is not limited thereto, and any disease in which liver fibrosis appears as a symptom or is directly or indirectly caused by liver fibrosis is included without limitation. Preferably, in the present invention, the liver fibrosis-related disease may be accompanied by overexpression of α-SMA, COL1A1, and/or F-actin.

In the present invention, the term "hepatic fibrosis" refers to a condition in which an abnormally large amount of scar tissue is formed in the liver due to various causes, and specifically means a state in which excessive accumulation of fibrous connective tissue in the liver destroys the normal liver structure and results in hardening. Causes of hepatic fibrosis include drugs, alcohol abuse, viral hepatitis, non-alcoholic fatty liver, obesity, diabetes, hereditary metabolic disorders, autoimmune diseases, heart failure, portal vein thrombosis, and blood flow obstruction. When hepatic fibrosis progresses, cirrhosis and hepatic cirrhosis may occur, and complications resulting therefrom (e.g., portal hypertension) may also be induced.

In the present invention, the kidney fibrosis-related disease may be one or more selected from the group consisting of renal fibrosis, end-stage kidney disease (ESKD), diabetic nephropathy (DN), IgA nephropathy (IgAN), HIV-associated nephropathy, nondiabetic chronic kidney disease, focal segmental glomerulosclerosis (FSGS), minimal change disease (MCD), and xanthine oxidase deficiency, but is not limited thereto, and any disease in which renal fibrosis appears as a symptom or is directly or indirectly caused by renal fibrosis is included without limitation. Preferably, in the present invention, the kidney fibrosis-related disease may be accompanied by overexpression of α-SMA, COL1A1, and/or F-actin.

In the present invention, the term "renal fibrosis" refers to a state in which excessive deposition of fibrous connective tissue occurs in the kidney, thereby destroying the normal renal structure and resulting in hardening. In particular, renal fibrosis is characterized by tubulointerstitial fibrosis and glomerulosclerosis. Renal fibrosis is a pathological feature commonly observed when various chronic kidney diseases progress to end-stage renal disease and is also regarded as a final manifestation of chronic kidney disease. Renal fibrosis is known to be induced not only by various inflammatory factors but also by activation of tubular epithelial cells or fibroblasts.

In the present invention, the pulmonary fibrosis-related disease may be one or more selected from the group consisting of pulmonary fibrosis, idiopathic pulmonary fibrosis, idiopathic interstitial pneumonia, desquamative interstitial pneumonia, nonspecific interstitial pneumonia, cryptogenic organizing pneumonia, respiratory bronchiolitis-associated interstitial lung disease, acute interstitial pneumonia, lymphocytic interstitial pneumonia, idiopathic pulmonary elastic fibrosis, interstitial lung disease, and chronic obstructive pulmonary disease (COPD), but is not limited thereto, and any disease in which pulmonary fibrosis is a symptom or is directly or indirectly caused by pulmonary fibrosis is included without limitation. In the present invention, the pulmonary fibrosis-related disease may be preferably accompanied by overexpression of α-SMA, COL1A1, and/or F-actin.

In the present invention, the term "pulmonary fibrosis" refers to a respiratory disease in which lung tissue becomes hardened and causes respiratory disorders, and specifically means a state in which excessive accumulation of fibrous connective tissue in the lung destroys the normal lung structure and results in hardening. Such excessive formation of fibrous connective tissue in an organ is referred to as fibrosis, and when fibrosis progresses in the lung, the lung wall thickens, thereby reducing the amount of oxygen supplied to the blood and causing shortness of breath and the like. The most common type of pulmonary fibrosis is idiopathic pulmonary fibrosis of unknown cause, the main symptom of which is dyspnea during exercise. For the treatment of idiopathic pulmonary fibrosis, the antifibrotic agent nintedanib is used, but the therapeutic effect is known to be only about 10%.

In the present invention, the term "interstitial lung disease" refers to diseases characterized by abnormal collagen deposition accompanied by proliferation of the interstitial compartment of the lung, infiltration of various inflammatory cells, and sometimes fibrosis, and includes idiopathic interstitial pneumonia, idiopathic pulmonary fibrosis, nonspecific interstitial pneumonia, respiratory bronchiolitis-associated interstitial lung disease, desquamative interstitial pneumonia, and acute interstitial pneumonia.

In the present invention, the composition may reduce the expression or activity of one or more mRNA or proteins selected from the group consisting of α-SMA, fibronectin, collagen type 1 (COL1A1), collagen type 4 (COL4A1), F-actin, and phosphorylated-SMAD2 (p-SMAD2). The genes or proteins are fibrosis-related factors that may induce fibrosis, and in particular, α-SMA is a biomarker of activated myofibroblasts, which are major effector cells of fibrosis. Myofibroblasts with increased expression of α-SMA are known to produce fibrotic proteins such as collagen, thereby causing fibrosis. The inventors confirmed through specific examples that the compound according to the present invention inhibited the increase in expression (formation) of α-SMA and F-actin in pulmonary fibroblasts (MRC-5 cells), and confirmed that it significantly inhibited the increase in mRNA expression of α-SMA (ACTA2) and COL1A1 induced by the inflammatory cytokine (TGF-β1) in hepatic stellate cells and renal fibroblasts, which demonstrates that the compound according to the present invention has preventive or therapeutic activity against pulmonary, hepatic, and renal fibrosis.

According to one example of the present invention, the compound of the present invention may inhibit F-actin expression (formation), and the inhibition of F-actin expression (formation) may be achieved through inhibition of the function of the actin-binding protein Arp2/3 complex. Actin filaments are assembled from actin monomers through actin polymerization reactions that hydrolyze ATP to ADP. Actin polymerization is necessary for the regulation of cell motility, but excessive actin polymerization promotes the expression of α-SMA and induces the activation of myofibroblasts, a heterogeneous cell population composed of fibrosis-promoting cells, thereby causing tissue fibrosis. The inventors confirmed through specific experiments that the compound according to the present invention can effectively inhibit F-actin and α-SMA formation (expression) caused by actin polymerization, and therefore, the compound fundamentally treats and improves fibrosis symptoms.

The content of the compound of the present invention in the composition of the present invention may be appropriately adjusted depending on the symptoms of the disease, the degree of progression of the symptoms, the condition of the patient, and the like, and may be, for example, 0.0001 to 99.9% by weight, or 0.001 to 50% by weight, based on the total weight of the composition, but is not limited thereto. The content ratio is based on the dried weight after removal of the solvent.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be formulated and used, by conventional methods, in the form of tablets, sustained-release tablets, enteric-coated tablets, sublingual tablets, troches, pills, capsules, hard capsules, soft capsules, sustained-release capsules, enteric-coated capsules, granules, sustained-release granules, enteric-coated granules, powders, dry syrups, solutions, suspensions, soft extracts, fluid extracts, lemonades, aromatic waters, emulsions, tinctures, inhalants, elixirs, injections, perfusion solutions, sterile injectable solutions, eye drops, gargles, lotions, pastes, sprays, patches, or external preparations such as aerosols, and the external preparations may be in the form of creams, gels, patches, sprays, ointments, gargles, lotions, liniments, pastes, or cataplasms.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

In addition, the present invention provides a kit for preventing or treating a fibrosis-related disease, comprising the composition according to the present invention. In the present invention, the term "kit" means a combination of materials or devices for preventing or treating a fibrosis-related disease using the compound according to the present invention, and is not limited in its specific form. The kit according to the present invention may include not only the compound of the present invention for preventing and/or treating a fibrosis-related disease, but also one or more other component compositions, solutions, or devices suitable for prevention, amelioration, or treatment of the disease. In addition, the kit may further include an instruction manual or directions containing information related to the compound of the present invention.

Throughout the specification of the present invention, when a part is described as "comprising" a component, this means that unless otherwise specified, it does not exclude other components but may further include other components. In the entire specification of the present invention, terms of degree such as "about" and "substantially" are used to indicate values within or close to the stated numerical ranges in view of manufacturing and material tolerances inherent in the stated meaning, and are used to prevent unscrupulous infringers from unfairly taking advantage of disclosures of exact or absolute values for the purpose of understanding the present invention.

Throughout the specification of the present invention, the term "combinations thereof" included in Markush-type expressions means a mixture or combination of one or more selected from the group consisting of the components described in the Markush-type expression, and indicates that one or more selected from the group consisting of the components are included.

### [Modes of the Invention]

Hereinafter, preferred examples will be presented to allow the present invention to be better understood. However, the following examples are merely provided in order for the present invention to be more easily understood, and the content of the present invention is not limited by the following examples.

### [Experimental Examples]

### Experimental Example 1. Preparation of compounds

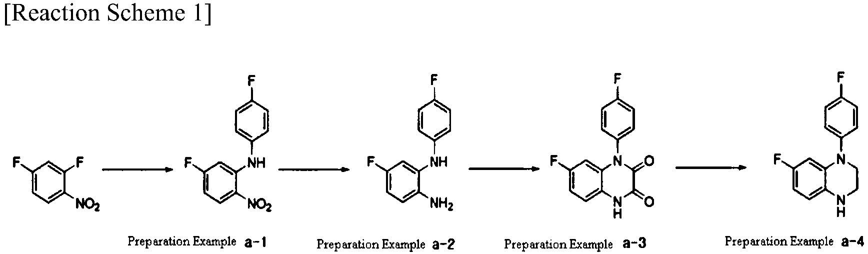

### <Preparation Method a> Preparation of 7-fluoro-1-(4-fluorophenyl)-1,2,3,4-tetrahydroquinoxaline

The 7-fluoro-1-(4-fluorophenyl)-1,2,3,4-tetrahydroquinoxaline according to the present invention was prepared through the procedures described in Preparation Examples a-1 to a-4 below.

### <Preparation Example a-1> Preparation of 5-fluoro-N-(4-fluorophenyl)-2-nitroaniline

A solution in which 2,4-difluoro-1-nitrobenzene (4.0 g, 25.14 mmol) and 4-fluoroaniline (2.8 g, 25.14 mmol) were mixed was stirred for 24 hours at 130 °C and then cooled to room temperature. After stopping the reaction by adding water to the reaction solution, extraction was performed using dichloromethane. An organic layer was washed with brine, dried over MgSO₄, filtered and then concentrated under reduced pressure. The residue obtained thereby was purified by silica column chromatography using a mixture of n-hexane and ethyl acetate to prepare 5-fluoro-N-(4-fluorophenyl)-2-nitroaniline.

Yellow solid (yield: 63%); ¹H NMR (400 MHz, CDCl₃) δ 9.54 (s, 1H), 8.26 (dd, J = 9.5, 6.0 Hz, 1H), 7.27-7.23 (m, 2H), 7.18-7.13 (m, 2H), 6.63 (dd, J = 11.3, 2.7 Hz, 1H). 6.48 (ddd, J = 9.5, 7.1, 2.7 Hz, 1H).

### <Preparation Example a-2> Preparation of 5-fluoro-N¹-(4-fluorophenyl)benzene-1,2-diamine

A mixture in which the 5-fluoro-N-(4-fluorophenyl)-2-nitroaniline (4.0 g, 15.98 mmol) obtained in Preparation Example a-1 and tin(II) chloride dihydrate (10.8 g, 47.96 mmol) were dissolved in ethyl acetate (0.5 M) was stirred for 5 hours at 90 °C. After cooling the reaction solution to room temperature, it was added to water, neutralized with a 10M NaOH aqueous solution, and extracted with ethyl acetate. An organic layer was washed with brine, dried over MgSO₄, filtered and then concentrated under reduced pressure. The residue obtained thereby was purified by silica column chromatography using a mixture of n-hexane and ethyl acetate to prepare 5-fluoro-N1-(4-fluorophenyl)benzene-1,2-diamine.

Orange solid (yield: 56%); ¹H NMR (400 MHz, CDCl₃) δ 6.99-6.94 (m, 2H), 6.86-6.82 (m, 2H), 6.79 (dd, J = 10.0, 2.8 Hz, 1H), 6.73 (dd, J = 8.6, 5.5 Hz, 1H), 6.62 (ddd, J = 8.6, 8.0, 2.8 Hz, 1H), 527 (brs, NH), 3.49 (brs, NH2); LC/MS ESI (+): 220.6 (M+1).

### <Preparation Example a-3> Preparation of 7-fluoro-1-(4-fluorophenyl)quinoxaline-2,3(1H,4H)-dione

The 5-fluoro-N¹-(4-fluorophenyl)benzene-1,2-diamine (3.0 g, 14.83 mmol) obtained in Preparation Example a-2 was dissolved in diethyl oxalate (13.0 g, 89.00 mmol) and then stirred for 24 hours at 160 °C. The reaction solution was cooled to room temperature, filtered and dried, thereby preparing 7-fluoro-1-(4-fluorophenyl)quinoxaline-2,3(1H,4H)-dione.

Ivory solid (yield: 68%); ¹H NMR (400 MHz, DMSO-d₆) δ 11.98 (brs, NH), 7.32 (d, J = 6.8 Hz, 4H), 7.08 (dd, J = 8.8, 5.5 Hz, 1H), 6.87 (ddd, J = 8.8, 8.0, 2.7 Hz, 1H), 5.93 (dd, J = 10.3, 2.7 Hz, 1H); LC/MS ESI (+): 274.9 (M+1).

### <Preparation Example a-4> Preparation of 7-fluoro-1-(4-fluorophenyl)-1,2,3,4-tetrahydroquinoxaline

A borane-THF complex (1 M) (22 mL, 21.88 mmol) was slowly added at room temperature to the 7-fluoro-1-(4-fluorophenyl)quinoxaline-2,3(1H,4H)-dione (2.0 g, 7.29 mmol) obtained in Preparation Example a-3 dissolved in THF (0.5 M), and stirred for 16 hours at 65 °C. After cooling the reaction solution to room temperature and adding water to terminate the reaction, the resulting solution was neutralized with a saturated NaHCO₃ aqueous solution and extracted with ethyl acetate. An organic layer was washed with brine, dried over MgSO₄, filtered and then concentrated under reduced pressure. The residue obtained thereby was purified by silica column chromatography using a mixture of n-hexane and ethyl acetate to prepare 7-fluoro-1-(4-fluorophenyl)-1,2,3,4-tetrahydroquinoxaline.

Light purple solid (yield: 72%); ¹H NMR (400 MHz, CDCl₃) δ 7.21-7.16 (m, 2H), 7.09-7.04 (m, 2H), 6.49 (dd, J = 8.5, 5.6 Hz, 1H), 6.36-6.27 (m, 2H), 3.64 (dd, J = 5.8, 4.5 Hz, 2H), 3.45 (dd, J = 5.8, 4.5 Hz, 2H); LC/MS ESI (+): 246.8 (M+1).

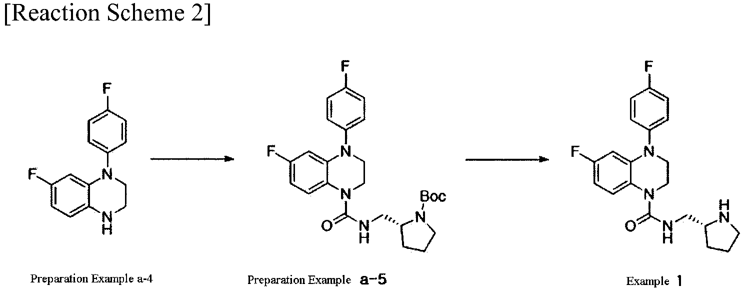

### <Preparation Example a-5> Preparation of tert-butyl (R)-2-((6-fluoro-4-(4-fluorophenyl)-1,2,3,4-tetrahydroquinoxaline-1-carboxamido)methyl)pyrrolidin-1-carboxylate

The 7-fluoro-1-(4-fluorophenyl)-1,2,3,4-tetrahydroquinoxaline (1.0 equiv.) obtained in Preparation Example a-4 was dissolved in dichloromethane (0.3 M), TEA (4.0 equiv.) and triphosgene (0.6 equiv.) were slowly added at 0 °C and then stirred for 1 hour at 0 °C. TEA (4.0 equiv.) and (R)-1-Boc-2-(aminomethyl)pyrrolidine (1.5 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (R)-2-((6-fluoro-4-(4-fluorophenyl)-1,2,3,4-tetrahydroquinoxaline-1-carboxamido)methyl)pyrrolidin-1-carboxylate.

White solid (yield: 90%); ¹H NMR (400 MHz, CDCl₃) δ 7.23-7.18 (m, 3H), 7.12-7.08 (m, 2H), 6.42 (ddd, J = 8.3, 8.0 2.8 Hz, 1H), 6.23 (dd, J = 11.6, 2.8 Hz, 1H), 3.97-3.92 (m, 2H), 3.87-3.82 (m, 1H), 3.62-3.59 (m, 3H), 3.49-3.45 (m, 1H), 3.35-3.26 (m, 2H), 2.01-1.96 (m, 1H),1.86-1.78 (m, 2H), 1.75-1.68 (m, 1H), 1.39 (s, 9H).

### <Example 1> Synthesis of (R)-6-fluoro-4-(4-fluorophenyl)-N-(pyrrolidin-2-ylmethyl)-3,4-dihydroquinoxaline-1(2H)-carboxamide

(R)-6-fluoro-4-(4-fluorophenyl)-N-(pyrrolidin-2-ylmethyl)-3,4-dihydroquinoxaline-1(2H)-carboxamide was synthesized by deprotecting the Boc group of the tert-butyl (R)-2-((6-fluoro-4-(4-fluorophenyl)-1,2,3,4-tetrahydroquinoxaline-1-carboxamido)methyl)pyrrolidin-1-carboxylate obtained in Preparation Example a-5 with TFA.

Light yellow solid (yield: 53%); ¹H NMR (400 MHz, CD₃OD) δ 7.33-7.30 (m, 2H), 7.24-7.18 (m, 3H), 6.45 (ddd, J = 8.8, 8.0, 2.8 Hz, 1H), 6.19 (dd, J = 11.4, 2.8 Hz, 1H), 3.88 (dd, J = 5.8, 4.5 Hz, 2H), 3.65 (dd, J = 5.8, 4.5 Hz, 2H), 3.39-3.33 (m, 2H), 3.27-3.23 (m, 1H), 3.03-2.90 (m, 2H), 1.99-1.91 (m, 1H), 1.88-1.76 (m, 2H), 1.56-1.48 (m, 1H); LC/MS ESI (+): 373.3 (M+1).

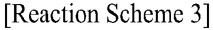

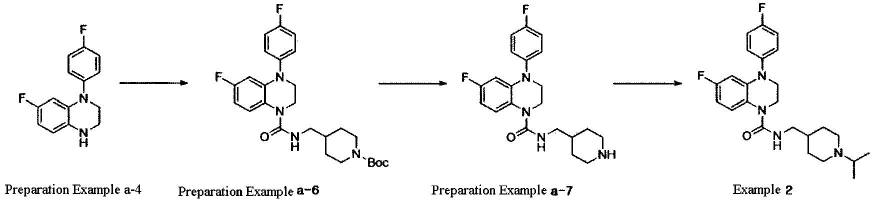

### <Preparation Example a-6> Preparation of tert-butyl 4-((6-fluoro-4-(4-fluorophenyl)-1,2,3,4-tetrahydroquinoxaline-1-carboxamido)methyl)piperidin-1-carboxylate

Tert-butyl 4-((6-fluoro-4-(4-fluorophenyl)-1,2,3,4-tetrahydroquinoxaline-1-carboxamido)methyl)piperidin-1-carboxylate was prepared in the same manner as described in Preparation Example a-5 using 1-Boc-4-(aminomethyl)piperidine instead of (R)-1-Boc-2-(aminomethyl)pyrrolidine.

White solid (yield: 95%); ¹H NMR (400 MHz, CDCl₃) δ 7.24-7.19 (m, 2H), 7.15-7.08 (m, 3H), 6.41 (ddd, J = 8.7, 7.8, 2.8 Hz, 1H), 6.25 (dd, J = 11.4, 2.8 Hz, 1H), 5.34-5.31 (m, 1H), 3.91 (t, J = 5.1 Hz, 2H), 3.60 (t, J = 5.1 Hz, 2H), 3.18-3.15 (m, 2H), 2.72-2.66 (m, 2H), 1.73-1.64 (m, 4H), 1.45 (s, 9H), 1.17-1.07 (m, 2H).

### <Preparation Example a-7> Preparation of 6-fluoro-4-(4-fluorophenyl)-N-(piperidin-4-ylmethyl)-3,4-dihydroquinoxaline-1(2H)-carboxamide

6-fluoro-4-(4-fluorophenyl)-N-(piperidin-4-ylmethyl)-3,4-dihydroquinoxaline-1(2H)-carboxamide was prepared by deprotecting the Boc group of the tert-butyl 4-((6-fluoro-4-(4-fluorophenyl)-1,2,3,4-tetrahydroquinoxaline-1-carboxamido)methyl)piperidin-1-carboxylate obtained in Preparation Example a-6 with TFA.

White solid (yield: 80%); ¹H NMR (400 MHz, CD₃OD) δ 7.37-7.27 (m, 2H), 7.25-7.15 (m, 3H), 6.45 (ddd, J = 8.8, 8.0, 2.8 Hz, 1H), 6.20 (dd, J = 11.4, 2.8 Hz, 1H), 3.86 (dd, J = 5.8, 4.5 Hz, 2H), 3.64 (dd, J = 5.8, 4.5 Hz, 2H), 3.13-3.05 (m, 4H), 2.63-2.57 (m, 2H), 1.74-1.66 (m, 3H), 1.23-1.16 (m, 2H); LC/MS ESI (+): 387.1 (M+1).

### <Example 2> Synthesis of 6-fluoro-4-(4-fluorophenyl)-N-((1-isopropylpiperidin-4-yl)methyl)-3,4-dihydroquinoxaline-1(2H)-carboxamide

The 6-fluoro-4-(4-fluorophenyl)-N-(piperidin-4-ylmethyl)-3,4-dihydroquinoxaline-1(2H)-carboxamide (1.0 equiv.) obtained in Preparation Example a-7 was dissolved in dichloromethane (0.3 M), sodium triacetoxyborohydride (1.5 equiv.) and acetone (2.0 equiv.) were added, and then the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize 6-fluoro-4-(4-fluorophenyl)-N-((1-isopropylpiperidin-4-yl)methyl)-3,4-dihydroquinoxaline-1(2H)-carboxamide.

White solid (yield: 67%); ¹H NMR (400 MHz, CD₃OD) δ 7.33-7.29 (m, 2H), 7.23-7.18 (m, 3H), 6.45 (ddd, J = 8.8, 8.0, 2.8 Hz, 1H), 6.20 (dd, J = 11.4, 2.8 Hz, 1H), 3.86 (dd, J = 5.8, 4.5 Hz, 2H), 3.64 (dd, J = 5.8, 4.5 Hz, 2H), 3.23-3.20 (m, 2H), 3.15 (d, J = 6.7 Hz, 2H), 2.68-2.62 (m, 2H), 1.90-1.87 (m, 2H), 1.75-1.70 (m, 1H), 1.46-1.36 (m, 3H), 1.23 (d, J = 6.6 Hz, 6H); LC/MS ESI (+): 429.3 (M+1).

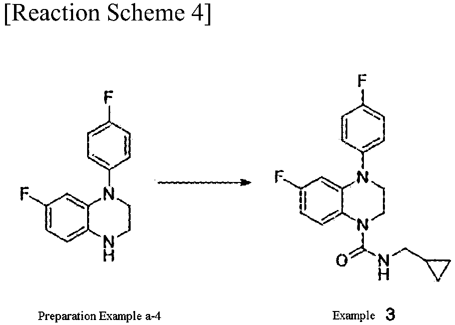

### <Example 3> Synthesis of N-(cyclopropylmethyl)-6-fluoro-4-(4-fluorophenyl)-3,4-dihydroquinoxaline-1(2H)-carboxamide

N-(cyclopropylmethyl)-6-fluoro-4-(4-fluorophenyl)-3,4-dihydroquinoxaline-1(2H)-carboxamide was synthesized in the same manner as described in Preparation Example a-5 using (cyclopropyl)methylamine instead of (R)-1-Boc-2-(aminomethyl)pyrrolidine.

White solid (yield: 57%); ¹H NMR (400 MHz, CDCl₃) δ 7.24-7.20 (m, 2H), 7.16-7.10 (m, 3H), 6.42 (ddd, J = 8.8, 7.8, 2.8 Hz, 1H), 6.26 (dd, J = 11.4, 2.8 Hz, 1H), 5.33 (brs, NH), 3.92 (dd, J = 5.7, 4.6 Hz, 2H), 3.61 (dd, J = 5.7, 4.6 Hz, 2H), 3.16 (dd, J = 7.0, 5.4 Hz, 2H), 1.03-0.93 (m, 1H), 0.52-0.48 (m, 2H), 0.21-0.18 (m, 2H); LC/MS ESI (+): 344.3 (M+1).

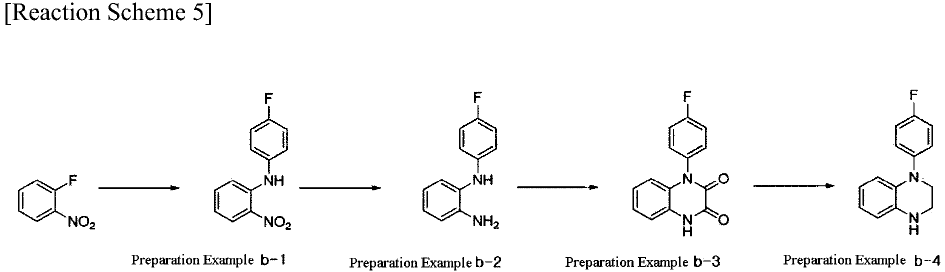

### <Preparation Method b> Preparation of 1-(4-fluorophenyl)-1,2,3,4-tetrahydroquinoxaline

1-(4-fluorophenyl)-1,2,3,4-tetrahydroquinoxaline according to the present invention was prepared through the procedures described in Preparation Examples b-1 to b-4 below.

### <Preparation Example b-1> Preparation of N-(4-fluorophenyl)-2-nitroaniline

N-(4-fluorophenyl)-2-nitroaniline was prepared in the same manner as described in Preparation Example a-1 using 1-fluoro-2-nitrobenzene instead of 2,4-difluoro-1-nitrobenzene.
yellow solid (yield: 30%); ¹H NMR (400 MHz, CDCl₃) δ 9.39 (brs, NH), 8.21 (dd, J = 8.6, 1.6 Hz, 1H), 7.36 (ddd, J = 8.7, 6.8, 1.6 Hz, 1H), 7.27-7.23 (m, 2H), 7.14-7.10 (m, 2H), 7.05 (dd, J = 8.7, 1.3 Hz, 1H), 6.77 (ddd, J = 8.6, 6.8, 1.3 Hz, 1H).

### <Preparation Example b-2> Preparation of N¹-(4-fluorophenyl)benzene-1,2-diamine

N¹-(4-fluorophenyl)benzene-1,2-diamine was prepared in the same manner as described in Preparation Example a-2 using the N-(4-fluorophenyl)-2-nitroaniline obtained in Preparation Example b-1 instead of 5-fluoro-N-(4-fluorophenyl)-2-nitroaniline.

Orange solid (yield: 61%); ¹H NMR (400 MHz, CDCl₃) δ 7.06 (dd, J = 7.8, 1.5 Hz, 1H), 7.01 (ddd, J = 7.9, 7.6, 1.5 Hz, 1H), 6.95-6.89 (m, 2H), 6.81 (dd, J = 7.9, 1.5 Hz, 1H), 6.76 (ddd, J = 7.8, 7.6, 1.5 Hz, 1H), 6.73-6.68 (m, 2H), 5.09 (brs, NH), 3.74 (brs, NH2); LC/MS ESI (+): 202.6 (M+1).

### <Preparation Example b-3> Preparation of 1-(4-fluorophenyl)quinoxaline-2,3(1H,4H)-dione

1-(4-fluorophenyl)quinoxaline-2,3(1H,4H)-dione was prepared in the same manner as described in Preparation Example a-3 using the N¹-(4-fluorophenyl)benzene-1,2-diamine obtained in Preparation Example b-2 instead of 5-fluoro-N¹-phenylbenzene-1,2-diamine.

White solid (yield: 81%); ¹H NMR (400 MHz, DMSO-d₆) δ 12.12 (brs, NH), 7.46 (d, J = 6.7 Hz, 4H), 7.23 (dd, J = 8.0, 1.5 Hz, 1H), 7.16 (ddd, J = 8.0, 7.6, 1.2 Hz, 1H), 7.01 (ddd, J = 8.6, 7.6, 1.5 Hz, 1H), 6.34 (dd, J = 8.6, 1.2 Hz, 1H); LC/MS ESI (+): 256.6 (M+1).

### <Preparation Example b-4> Preparation of 1-(4-fluorophenyl)-1,2,3,4-tetrahydroquinoxaline

1-(4-fluorophenyl)-1,2,3,4-tetrahydroquinoxaline was prepared in the same manner as described in Preparation Example a-4 using the 1-(4-fluorophenyl)quinoxaline-2,3(1H,4H)-dione obtained in Preparation Example b-3 instead of 7-fluoro-1-phenylquinoxaline-2,3(1H,4H)-dione.

Brown solid (yield: 38%); ¹H NMR (400 MHz, CDCl₃) δ 7.18-7.13 (m, 2H), 7.05-6.99 (m, 2H), 6.71-6.64 (m, 2H), 6.60-6.53 (m, 2H), 3.86 (brs, NH), 3.66 (dd, J = 6.4, 4.8 Hz, 2H), 3.47 (dd, J = 6.4, 4.8 Hz, 2H); LC/MS ESI (+): 228.8 (M+1).

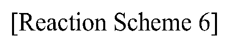

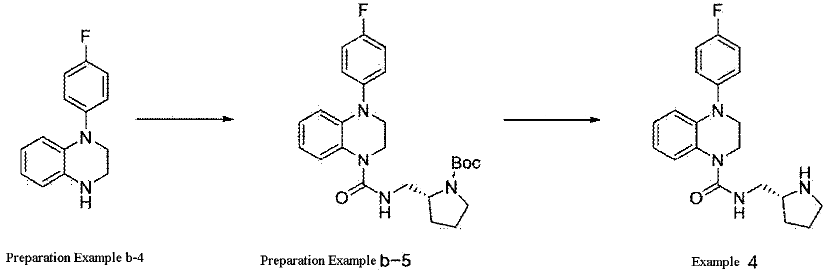

### <Preparation Example b-5> Preparation of tert-butyl (R)-2-((4-(4-fluorophenyl)-1,2,3,4-tetrahydroquinoxaline-1-carboxamido)methyl)pyrrolidine-1-carboxylate

The 1-(4-fluorophenyl)-1,2,3,4-tetrahydroquinoxaline (1.0 equiv.) obtained in Preparation Example b-4 was dissolved in dichloromethane (0.3 M), TEA (4.0 equiv.) and triphosgene (0.6 equiv.) were slowly added at 0 °C, and the resulting mixture was stirred for 1 hour at 0 °C. TEA (4.0 equiv.) and (R)-1-Boc-2-(aminomethyl)pyrrolidine (1.5 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (R)-2-((4-(4-fluorophenyl)-1,2,3,4-tetrahydroquinoxaline-1-carboxamido)methyl)pyrrolidine-1-carboxylate.

Light yellow solid (yield: 93%); ¹H NMR (400 MHz, CDCl₃) δ 7.31-7.28 (m, 1H), 7.23-7.18 (m, 2H), 7.09-7.05 (m, 2H), 6.89-6.86 (m, 1H), 6.74 (ddd, J = 8.0, 7.6, 1.5 Hz, 1H), 6.61 (d, J = 8.2 Hz, 1H), 6.40 (brs, 1H), 3.96-3.90 (m, 3H), 3.64-3.62 (m, 2H), 3.50-3.47 (m, 1H), 3.35-3.24 (m, 3H), 1.99-1.91 (m, 2H) 1.84-1.77 (m, 2H), 1.40 (s, 9H).

### <Example 4> Synthesis of (R)-4-(4-fluorophenyl)-N-(pyrrolidin-2-ylmethyl)-3,4-dihydroquinoxaline-1(2H)-carboxamide

(R)-4-(4-fluorophenyl)-N-(pyrrolidin-2-ylmethyl)-3,4-dihydroquinoxaline-1(2H)-carboxamide was synthesized by deprotecting the Boc group of the tert-butyl (R)-2-((4-(4-fluorophenyl)-1,2,3,4-tetrahydroquinoxaline-1-carboxamido)methyl)pyrrolidine-1-carboxylate obtained in Preparation Example b-5 with TFA.

Yellow oil (yield: 65%); ¹H NMR (400 MHz, CDCl₃) δ 7.23-7.20 (m, 3H), 7.11-7.07 (m, 2H), 6.89 (ddd, J = 8.3, 7.3, 1.6 Hz, 1H), 6.72 (ddd, J = 8.0, 7.3, 1.4 Hz, 1H), 6.62 (dd, J = 8.3, 1.4 Hz, 1H), 5.82 (t, J = 6.9 Hz, 1H), 3.94 (dd, J = 5.2, 4.6 Hz, 2H), 3.63 (dd, J = 5.2, 4.6 Hz, 2H), 3.42-3.34 (m, 2H), 3.18-3.13 (m, 1H), 2.97-2.86 (m, 2H), 1.90-1.85 (m, 1H), 1.80-1.70 (m, 2H), 1.47-1.40 (m, 1H); LC/MS ESI (+): 355.3 (M+1).

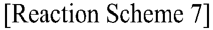

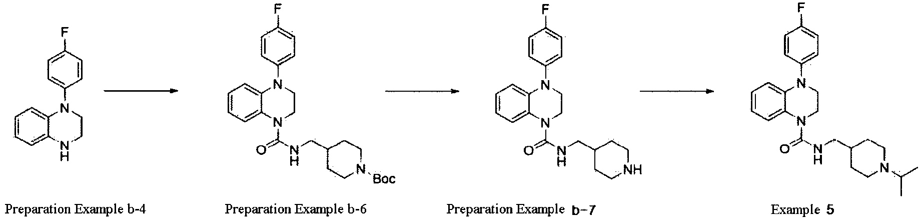

### <Preparation Example b-6> Preparation of tert-butyl 4-((4-(4-fluorophenyl)-1,2,3,4-tetrahydroquinoxaline-1-carboxamido)methyl)piperidin-1-carboxylate

Tert-butyl 4-((4-(4-fluorophenyl)-1,2,3,4-tetrahydroquinoxaline-1-carboxamido)methyl)piperidin-1-carboxylate was prepared in the same manner as described in Preparation Example b-5 using 1-Boc-4-(aminomethyl)piperidine instead of (R)-1-Boc-2-(aminomethyl)pyrrolidine.

White solid (yield: 67%); ¹H NMR (400 MHz, CDCl₃) δ 7.22-7.17 (m, 3H), 7.12-7.07 (m, 2H), 6.95-6.88 (m, 1H), 6.75-6.71 (m, 1H), 6.63 (ddd, J = 8.3, 7.3, 1.4 Hz, 1H), 5.45 (t, J = 6.0 Hz, 1H), 4.15-4.09 (m, 2H), 3.94 (dd, J = 6.2, 4.8 Hz, 2H), 3.63 (dd, J = 6.2, 4.8 Hz, 2H), 3.18-3.15 (m, 2H), 2.72-2.65 (m, 2H), 1.72-1.65 (m, 2H), 1.45 (s, 9H), 1.29-1.24 (m, 1H), 1.18-1.08 (m, 2H).

### <Preparation Example b-7> Preparation of 4-(4-fluorophenyl)-N-(piperidin-4-ylmethyl)-3,4-dihydroquinoxaline-1(2H)-carboxamide

4-(4-fluorophenyl)-N-(piperidin-4-ylmethyl)-3,4-dihydroquinoxaline-1(2H)-carboxamide was prepared by deprotecting the Boc group of the tert-butyl 4-((4-(4-fluorophenyl)-1,2,3,4-tetrahydroquinoxaline-1-carboxamido)methyl)piperidin-1-carboxylate obtained in Preparation Example b-6 with TFA.

White solid (yield: 96%); ¹H NMR (400 MHz, CDCl₃) δ 7.23-7.17 (m, 3H), 7.12-7.07 (m, 2H), 6.90 (ddd, J = 8.3, 7.3, 1.6 Hz, 1H), 6.73 (ddd, J = 8.3, 7.3, 1.4 Hz, 1H), 6.63 (dd, J = 8.3, 1.4 Hz, 1H), 5.44 (t, J = 5.8 Hz, 1H), 3.94 (dd, J = 5.7, 4.6 Hz, 2H), 3.63 (dd, J = 5.7, 4.6 Hz, 2H), 3.18-3.10 (m, 3H), 2.90-2.86 (m, 2H), 2.65-2.58 (m, 2H), 1.72-1.68 (m, 2H), 1.22-1.16 (m, 2H); LC/MS ESI (+): 369.2 (M+1).

### <Example 5> Synthesis of 4-(4-fluorophenyl)-N-((1-isopropylpiperidin-4-yl)methyl)-3,4-dihydroquinoxaline-1(2H))-carboxamide

The 4-(4-fluorophenyl)-N-(piperidin-4-ylmethyl)-3,4-dihydroquinoxaline-1(2H)-carboxamide (1.0 equiv.) obtained in Preparation Example b-7 was dissolved in dichloromethane (0.3 M), sodium triacetoxyborohydride (1.5 equiv.) and acetone (2.0 equiv.) were added and then stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize 4-(4-fluorophenyl)-N-((1-isopropylpiperidin-4-yl)methyl)-3,4-dihydroquinoxaline-1(2H))-carboxamide.

Light yellow solid (yield: 81%); ¹H NMR (400 MHz, CDCl₃) δ 7.23-7.17 (m, 3H), 7.12-7.07 (m, 2H), 6.90 (ddd, J = 8.3, 7.3, 1.6 Hz, 1H), 6.73 (ddd, J = 8.3, 7.3, 1.4 Hz, 1H), 6.63 (dd, J = 8.3, 1.4 Hz, 1H), 5.44 (t, J = 5.8 Hz, 1H), 3.94 (dd, J = 5.8, 4.5 Hz, 2H), 3.63 (dd, J = 5.8, 4.5 Hz, 2H), 3.17 (dd, J = 6.7, 5.8 Hz, 2H), 2.90-2.86 (m, 2H), 2.73-2.65 (m, 1H), 2.14-2.08 (m, 2H), 1.72-1.68 (m, 2H), 1.55-1.49 (m, 1H), 1.30-1.20 (m, 2H), 1.03 (d, J = 6.6 Hz, 6H); LC/MS ESI (+): 411.3 (M+1).

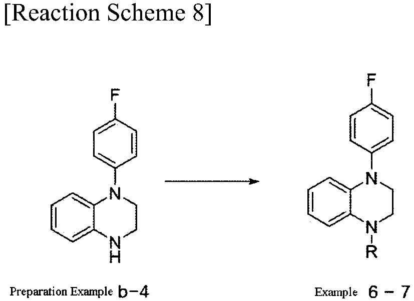

### <Example 6> Synthesis of N-(cyclopropylmethyl)-4-(4-fluorophenyl)-3,4-dihydroquinoxaline-1(2H)-carboxamide

N-(cyclopropylmethyl)-4-(4-fluorophenyl)-3,4-dihydroquinoxaline-1(2H)-carboxamide was synthesized in the same manner as described in Preparation Example b-5 using cyclopropylmethylamine instead of (R)-1-Boc-2-(aminomethyl)pyrrolidine.

White solid (yield: 77%); ¹H NMR (400 MHz, CDCl₃) δ 7.24-7.19 (m, 3H), 7.12-7.07 (m, 2H), 6.90 (ddd, J = 8.5, 7.3, 1.6 Hz, 1H), 6.74 (ddd, J = 8.0, 7.3, 1.4 Hz, 1H), 6.63 (dd, J = 8.5, 1.4 Hz, 1H), 5.46 (t, J = 5.3 Hz, 1H), 3.95 (dd, J = 5.8, 4.5 Hz, 2H), 3.64 (dd, J = 5.8, 4.5 Hz, 2H), 3.16 (dd, J = 5.3, 4.6 Hz, 2H), 1.04-0.94 (m, 1H), 0.52-0.47 (m, 2H), 0.22-0.18 (m, 2H); LC/MS ESI (+): 326.3 (M+1).

### <Example 7> Synthesis of ((S)-4-(4-fluorophenyl)-N-(tetrahydrofuran-3-yl)-3,4-dihydroquinoxaline-1(2H)-carboxamide

((S)-4-(4-fluorophenyl)-N-(tetrahydrofuran-3-yl)-3,4-dihydroquinoxaline-1(2H)-carboxamide was synthesized in the same manner as described in Preparation Example b-5 using (S)-3-aminotetrahydrofuran instead of (R)-1-Boc-2-(aminomethyl)pyrrolidine.

Brown solid (yield: 67%); ¹H NMR (400 MHz, CDCl₃) δ 7.24-7.19 (m, 2H), 7.16 (dd, J = 8.0, 1.6 Hz, 1H), 7.13-7.07 (m, 2H), 6.90 (ddd, J = 8.3, 7.3, 1.6 Hz, 1H), 6.72 (ddd, J = 8.0, 7.3, 1.4 Hz, 1H), 6.62 (dd, J = 8.3, 1.4 Hz, 1H), 5.47 (d, J = 7.0 Hz, 1H), 4.52-4.46 (m, 1H), 4.01-3.95 (m, 1H), 3.93-3.84 (m, 3H), 3.81-3.76 (m, 1H), 3.70-3.66 (m, 1H), 3.63 (t, J = 5.2 Hz, 2H), 2.32-2.24 (m, 1H), 1.83-1.75 (m, 1H); LC/MS ESI (+): 342.3 (M+1).

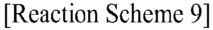

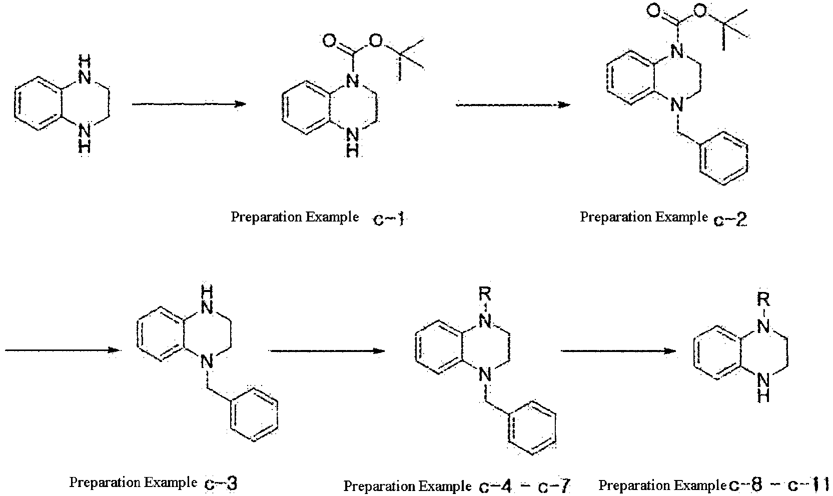

### <Preparation Method c> Preparation of chlorine-substituted phenyl or pyridin-1,2,3,4-tetrahydroquinoxaline

Chlorine-substituted phenyl or pyridin-1,2,3,4-tetrahydroquinoxaline according to the present invention was prepared through the procedures described in Preparation Examples c-1 to c-11.

### <Preparation Example c-1> Preparation of tert-butyl 3,4-dihydroquinoxaline-1(2H)-carboxylate

A 0.02 M NaOH aqueous solution (50 mL) was added to a mixed solution in which 1,2,3,4-tetrahydroquinoxaline (10.8 g, 80.48 mmol) and di-tert-butyl-dicarbonate (17.6 g, 80.48 mmol) were dissolved in THF (200 mL) and stirred for 24 hours at room temperature. The reaction solution was concentrated under reduced pressure, and extracted with dichloromethane. An organic layer was dried over MgSO₄, filtered, and concentrated under reduced pressure. The residue obtained thereby was purified by silica column chromatography using a mixture of n-hexane and ethyl acetate to prepare tert-butyl 3,4-dihydroquinoxaline-1(2H)-carboxylate.

Yellow solid (yield: 94%); ¹H NMR (400 MHz, CDCl₃) δ 7.49 (d, J = 8.5 Hz, 1H), 6.89 (ddd, J = 8.0, 7.2, 1.4 Hz, 1H), 6.65 (ddd, J = 8.5, 7.2, 1.5 Hz, 1H), 6.56 (dd, J = 8.0, 1.5 Hz, 1H), 3.79-3.75 (m, 2H), 3.42-3.39 (m, 2H), 1.52 (s, 9H).

### <Preparation Example c-2> Preparation of tert-butyl 4-benzyl-3,4-dihydroquinoxaline-1(2H)-carboxylate

Benzyl bromide (1.7 g, 10.24 mmol) was added to a mixed solution in which the tert-butyl 3,4-dihydroquinoxaline-1(2H)-carboxylate (2.0 g, 8.54 mmol) obtained in Preparation Example c-1 and DIPEA (4.4 g, 34.15 mmol) were dissolved in DMF (15.0 mL), and stirred for 2 hours at 130 °C. The reaction solution was cooled to room temperature, water was poured, and then the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl 4-benzyl-3,4-dihydroquinoxaline-1(2H)-carboxylate.

Brown oil (yield: 99%); ¹H NMR (400 MHz, CD₃CN) δ 7.40-7.29 (m, 3H), 7.28-7.19 (m, 3H), 6.84 (ddd, J = 8.6, 7.3, 1.6 Hz, 1H), 6.62-6.51 (m, 2H), 4.53 (s, 2H), 3.80-3.73 (m, 2H), 3.48-3.41 (m, 2H), 1.47 (s, 9H).

### <Preparation Example c-3> Preparation of 1-benzyl-1,2,3,4-tetrahydroquinoxaline

A mixed solution in which the tert-butyl 4-benzyl-3,4-dihydroquinoxaline-1(2H)-carboxylate (2.7 g, 8.40 mmol) obtained in Preparation Example c-2 and TFA (9.6 g, 84.00 mmol) were dissolved in dichloromethane (15.0 mL) was stirred for 12 hours at room temperature. After the termination of the reaction, toluene was added to the reaction solution, concentrated under reduced pressure, water was added, and then extracted with dichloromethane. An organic layer was washed with brine, dried over MgSO₄, filtered and then concentrated under reduced pressure. The residue obtained thereby was purified by silica column chromatography using an n-hexane/ethylacetate mixture to prepare 1-benzyl-1,2,3,4-tetrahydroquinoxaline.

Brown solid (yield: 80%); ¹H NMR (400 MHz, CD₃CN) δ 7.37-7.19 (m, 5H), 6.47-6.39 (m, 4H), 4.41 (s, 2H), 4.29 (brs, NH), 3.37 (s, 4H); LC/MS ESI (+): 225.2 (M+1).

### <Preparation Example c-4> Preparation of 1-benzyl-4-(2-chlorophenyl)-1,2,3,4-tetrahydroquinoxaline

The 1-benzyl-1,2,3,4-tetrahydroquinoxaline (1.0 equiv.) obtained in Preparation Example c-3 and sodium tert-butoxide (2.0 equiv.), 1-chloro-2-iodobenzene (3.0 equiv.) were sufficiently dissolved in toluene (0.3 M). A mixed solution in which Pd₂dba3 (0.05 equiv.) and XPhos (0.15 equiv.) were dissolved in toluene (0.6 M), which was heated for 5 minutes at 110 °C was slowly added to the reaction solution, and stirred for 15 hours at 110 °C. After cooling to room temperature, the reaction solution was filtered through a Celite pad, water was added, and the resulting solution was extracted with dichloromethane. An organic layer was dried over MgSO₄, filtered, and concentrated under reduced pressure. The residue obtained thereby was purified by silica column chromatography using a mixture of n-hexane and ethyl acetate to prepare 1-benzyl-4-(2-chlorophenyl)-1,2,3,4-tetrahydroquinoxaline.

Colorless oil (yield: 99%); ¹H NMR (400 MHz, DMSO-d₆) δ 7.57 (dd, J = 8.0, 1.4 Hz, 1H), 7.47-7.31 (m, 6H), 7.30-7.21 (m, 2H), 6.61-6.49 (m, 2H), 6.37 (dd, J = 7.4, 1.9 Hz, 1H), 5.99 (dd, J = 8.0, 0.9 Hz, 1H), 3.72-3.59 (m, 2H), 3.57-3.42 (m, 2H); LC/MS ESI (+): 335.1 (M+1).

### <Preparation Example c-5> Preparation of 1-benzyl-4-(pyridin-2-yl)-1,2,3,4-tetrahydroquinoxaline

1-benzyl-4-(pyridin-2-yl)-1,2,3,4-tetrahydroquinoxaline was prepared in the same manner as described in Preparation Example c-4 using 2-bromopyridine instead of 1-chloro-2-iodobenzene.

Yellow solid (yield: 73%); ¹H NMR (400 MHz, DMSO-d₆) δ 8.22 (dd, J = 5.1, 1.2 Hz, 1H), 7.53 (ddd, J = 8.7, 6.9, 1.9 Hz, 1H), 7.35-7.19 (m, 5H), 7.15 (dd, J = 7.8, 1.6 Hz, 1H), 7.03 (d, J = 8.6 Hz, 1H), 6.86 (ddd, J = 8.6, 7.3, 1.5 Hz, 1H), 6.77 (dd, J = 7.0, 5.5 Hz, 1H), 6.70 (dd, J = 8.2, 1.2 Hz, 1H), 6.60-6.53 (m, 1H), 4.50 (s, 2H), 4.10-4.02 (m, 2H), 3.40 (t, J = 5.1 Hz, 2H); LC/MS ESI (+): 302.2 (M+1).

### <Preparation Example c-6> Preparation of 1-benzyl-4-(pyridin-3-yl)-1,2,3,4-tetrahydroquinoxaline

1-benzyl-4-(pyridin-3-yl)-1,2,3,4-tetrahydroquinoxaline was prepared in the same manner as described in Preparation Example c-4 using 3-iodopyridine instead of 1-chloro-2-iodobenzene.

Yellow oil (yield: 100%); ¹H NMR (400 MHz, CD₃CN) δ 8.46 (d, J = 2.7 Hz, 1H), 8.19 (dd, J = 4.7, 1.2 Hz, 1H), 7.50 (ddd, J = 8.4, 2.9, 1.6 Hz, 1H), 7.40-7.31 (m, 4H), 7.30-7.24 (m, 2H), 6.82 (dd, J = 8.2, 1.6 Hz, 1H), 6.78-6.72 (m, 1H), 6.70-6.65 (m, 1H), 6.55-6.49 (m, 1H), 4.57 (s, 2H), 3.80-3.74 (m, 2H), 3.52-3.46 (m, 2H); LC/MS ESI (+): 302.2 (M+1).

### <Preparation Example c-7> Preparation of 1-benzyl-4-(pyridin-4-yl)-1,2,3,4-tetrahydroquinoxaline

1-benzyl-4-(pyridin-4-yl)-1,2,3,4-tetrahydroquinoxaline was prepared in the same manner as described in Preparation Example c-4 using 4-iodopyridine instead of 1-chloro-2-iodobenzene.

Red oil (yield: 100%); ¹H NMR (400 MHz, DMSO-d₆) δ 8.26-8.19 (m, 2H), 7.31 (dd, J = 8.1, 6.8 Hz, 2H), 7.26-7.18 (m, 3H), 7.09 (dd, J = 7.9, 1.5 Hz, 1H), 7.05-6.99 (m, 2H), 6.89 (ddd, J = 8.6, 7.2, 1.5 Hz, 1H), 6.73 (dd, J = 8.3, 1.3 Hz, 1H), 6.62-6.53 (m, 1H), 4.56 (s, 2H), 3.84 (t, J = 5.1 Hz, 2H), 3.40 (t, J = 5.1 Hz, 2H); LC/MS ESI (+): 302.2 (M+1).

### <Preparation Example c-8> Preparation of 1-(2-chlorophenyl)-1,2,3,4-tetrahydroquinoxaline

A mixed solution in which 1-benzyl-4-(2-chlorophenyl)-1,2,3,4-tetrahydroquinoxaline (1.0 equiv.) obtained in Preparation Example c-4 and Pd(OH)₂ (0.6 equiv.) were dissolved in MeOH/THF (v/v = 2: 1, 0.3M) was stirred for 3 hours at room temperature under a hydrogen gas condition. The reaction solution was filtered through a Celite pad, water was added, and the resulting solution was extracted with dichloromethane. An organic layer was dried over MgSO₄, filtered, and concentrated under reduced pressure. The residue obtained thereby was purified by silica column chromatography using a mixture of n-hexane and ethyl acetate to prepare 1-(2-chlorophenyl)-1,2,3,4-tetrahydroquinoxaline.

Gray solid (yield: 62%); ¹H NMR (400 MHz, DMSO-d₆) δ 7.55 (dd, J = 8.0, 1.4 Hz, 1H), 7.43-7.35 (m, 1H), 7.34-7.30 (m, 1H), 7.28-7.21 (m, 1H), 6.54-6.49 (m, 2H), 6.31 (ddd, J = 8.2, 5.1, 3.5 Hz, 1H), 5.96 (d, J = 7.8 Hz, 1H), 3.54 (dd, J = 4.9, 2.2 Hz, 2H), 3.35 (dd, J = 4.9, 2.2 Hz, 2H); LC/MS ESI (+): 245.1 (M+1).

### <Preparation Example c-9> Preparation of 1-(pyridin-2-yl)-1,2,3,4-tetrahydroquinoxaline

1-(pyridin-2-yl)-1,2,3,4-tetrahydroquinoxaline was prepared in the same manner as described in Preparation Example c-8 using the 1-benzyl-4-(pyridin-2-yl)-1,2,3,4-tetrahydroquinoxaline obtained in Preparation Example c-5 instead of 1-benzyl-4-(2-chlorophenyl)-1,2,3,4-tetrahydroquinoxaline.

Yellow solid (yield: 78%); ¹H NMR (400 MHz, CDCl₃) δ 8.26 (d, J = 4.1 Hz, 1H), 7.42 (ddd, J = 8.5, 7.1, 2.0 Hz, 1H), 7.22 (dd, J = 8.4, 1.5 Hz, 1H), 7.16 (d, J = 8.5 Hz, 1H), 6.93-6.83 (m, 1H), 6.70 (ddd, J = 7.1, 4.9, 1.0 Hz, 1H), 6.67-6.55 (m, 2H), 4.08 (t, J = 4.8 Hz, 2H), 3.99 (brs, NH), 3.41 (t, J = 4.8 Hz, 2H); LC/MS ESI (+): 212.2 (M+1).

### <Preparation Example c-10> Preparation of 1-(pyridin-3-yl)-1,2,3,4-tetrahydroquinoxaline

1-(pyridin-3-yl)-1,2,3,4-tetrahydroquinoxaline was prepared in the same manner as described in Preparation Example c-8 using the 1-benzyl-4-(pyridin-3-yl)-1,2,3,4-tetrahydroquinoxaline obtained in Preparation Example c-6 instead of 1-benzyl-4-(2-chlorophenyl)-1,2,3,4-tetrahydroquinoxaline.

Brown oil (yield: 83%); ¹H NMR (400 MHz, CD₃CN) δ 8.45 (d, J = 2.7 Hz, 1H), 8.17 (dd, J = 4.7, 1.6 Hz, 1H), 7.50 (ddd, J = 8.3, 2.8, 1.8 Hz, 1H), 7.26 (dd, J = 8.2, 4.7 Hz, 1H), 6.81-6.71 (m, 2H), 6.66-6.61 (m, 1H), 6.54-6.47 (m, 1H), 4.63 (brs, NH), 3.68-3.63 (m, 2H), 3.39-3.34 (m, 2H); LC/MS ESI (+): 212.2 (M+1).

### <Preparation Example c-11> Preparation of 1-(pyridin-4-yl)-1,2,3,4-tetrahydroquinoxaline

1-(pyridin-4-yl)-1,2,3,4-tetrahydroquinoxaline was prepared in the same manner as described in Preparation Example c-8 using the 1-benzyl-4-(pyridin-4-yl)-1,2,3,4-tetrahydroquinoxaline obtained in Preparation Example c-7 instead of 1-benzyl-4-(2-chlorophenyl)-1,2,3,4-tetrahydroquinoxaline.

Yellow solid (yield: 77%); ¹H NMR (400 MHz, DMSO-d₆) δ 8.25-8.18 (m, 2H), 7.06-7.00 (m, 3H), 6.85 (ddd, J = 8.4, 7.2, 1.4 Hz, 1H), 6.65 (dd, J = 8.1, 1.5 Hz, 1H), 6.50 (ddd, J = 8.0, 7.2, 1.5 Hz, 1H), 6.09 (brs, NH), 3.70-3.63 (m, 2H), 3.29-3.21 (m, 2H); LC/MS ESI (+): 212.1 (M+1).

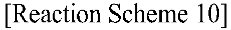

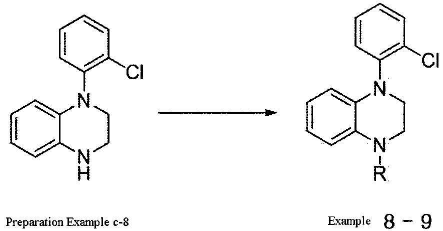

### <Example 8> Synthesis of (4-(2-chlorophenyl)-3,4-dihydroquinoxaline-1(2H)-yl)(pyrrolidin-1-yl)methanone

The 1-(2-chlorophenyl)-1,2,3,4-tetrahydroquinoxaline (1.0 equiv.) obtained in Preparation Example c-8 was dissolved in dichloromethane (0.3 M), TEA (3.0 equiv.) and triphosgene (0.6 equiv.) were slowly added at 0 °C, and then the resulting mixture was stirred for 1 hour at 0 °C. TEA (2.0 equiv.) and pyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (4-(2-chlorophenyl)-3,4-dihydroquinoxaline-1(2H)-yl)(pyrrolidin-1-yl)methanone.

Light yellow solid (yield: 55%); ¹H NMR (400 MHz, CDCl₃) δ 7.56 (ddd, J = 8.0, 7.6, 1.1 Hz, 1H), 7.35-7.32 (m, 2H), 7.29-7.24 (m, 1H), 6.95 (dd, J = 8.0, 1.6 Hz, 1H), 6.73 (ddd, J = 8.2, 7.3, 1.6 Hz, 1H), 6.66 (ddd, J = 8.0, 7.3, 1.5 Hz, 1H), 6.19 (dd, J = 8.2, 1.5 Hz, 1H), 3.90 (t, J = 5.1 Hz, 2H), 3.72 (t, J = 5.1 Hz, 2H), 3.38-3.34 (m, 4H), 1.86-1.80 (m, 4H); LC/MS ESI (+): 342.3 (M+1).

### <Example 9> Synthesis of (4-(2-chlorophenyl)-3,4-dihydroquinoxaline-1(2H)-yl)(morpholino)methanone

(4-(2-chlorophenyl)-3,4-dihydroquinoxaline-1(2H)-yl)(morpholino)methanone was synthesized in the same manner as described in Example 8 using morpholine instead of pyrrolidine.

White solid (yield: 46%); ¹H NMR (400 MHz, C_{D3}OD) δ 7.56 (ddd, J = 8.0, 7.6, 1.1 Hz, 1H), 7.43-7.40 (m, 2H), 7.37-7.32 (m, 1H), 7.03 (dd, J = 8.0, 1.5 Hz, 1H), 6.75 (ddd, J = 8.2, 7.3, 1.5 Hz, 1H), 6.67 (ddd, J = 8.0, 7.3, 1.4 Hz, 1H), 6.13 (dd, J = 8.2, 1.4 Hz, 1H), 3.81 (dd, J = 5.8, 4.3 Hz, 2H), 3.69 (dd, J = 5.8, 4.3 Hz, 2H), 3.66-3.64 (m, 4H), 3.41-3.39 (m, 4H); LC/MS ESI (+): 358.2 (M+1).

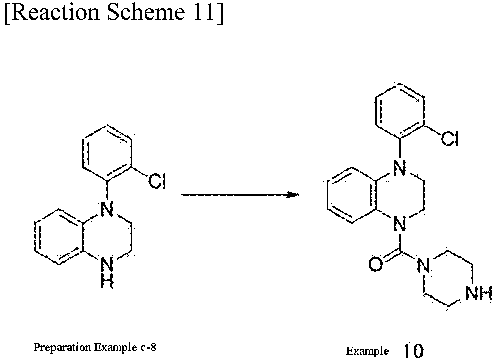

### <Example 10> Synthesis of (4-(2-chlorophenyl)-3,4-dihydroquinoxaline-1(2H)-yl)(piperazin-1-yl)methanone

The 1-(2-chlorophenyl)-1,2,3,4-tetrahydroquinoxaline (1.0 equiv.) obtained in Preparation Example c-8 was dissolved in dichloromethane (0.4 M), TEA (3.0 equiv.) and triphosgene (0.6 equiv.) were slowly added at 0 °C, and the resulting mixture was stirred for 1 hour at 0 °C. TEA (2.0 equiv.) and 1-Boc-piperzine (1.2 equiv.) were added to the reaction solution, and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The obtained concentrate was dissolved in dichloromethane (0.5 M), TFA (10.0 equiv.) was added, and the resulting mixture was stirred for 24 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (4-(2-chlorophenyl)-3,4-dihydroquinoxaline-1(2H)-yl)(piperazin-1-yl)methanone.

White solid (yield: 74%); ¹H NMR (400 MHz, CD₃OD) δ 7.57 (ddd, J = 8.0, 7.6, 1.1 Hz, 1H), 7.43-7.40 (m, 2H), 7.37-7.31 (m, 1H), 7.00 (dd, J = 8.0, 1.5 Hz, 1H), 6.75 (ddd, J = 8.2, 7.3, 1.5 Hz, 1H), 6.66 (ddd, J = 8.0, 7.3, 1.4 Hz, 1H), 6.13 (dd, J = 8.2, 1.4 Hz, 1H), 3.81 (dd, J = 5.8, 4.3 Hz, 2H), 3.69 (dd, J = 5.8, 4.3 Hz, 2H), 3.41-3.38 (m, 4H), 2.82-2.80 (m, 4H); LC/MS ESI (+): 357.2 (M+1).

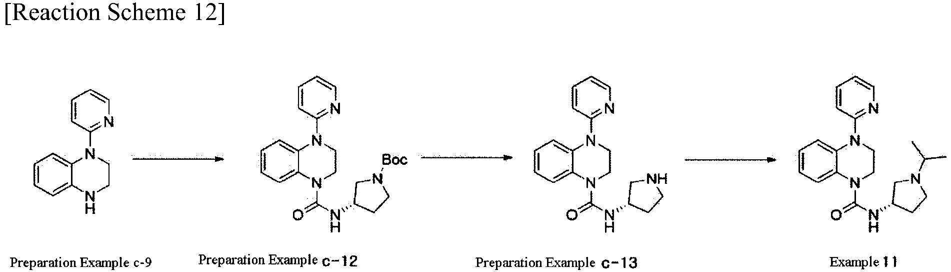

### <Preparation Example c-12> Preparation of tert-butyl (S)-3-(4-(pyridin-2-yl)-1,2,3,4-tetrahydroquinoxaline-1-carboxamido)pyrrolidin-1-carboxylate

The 1-(pyridin-2-yl)-1,2,3,4-tetrahydroquinoxaline (1.0 equiv.) obtained in Preparation Example c-9 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-1-Boc-3-aminopyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (S)-3-(4-(pyridin-2-yl)-1,2,3,4-tetrahydroquinoxaline-1-carboxamido)pyrrolidin-1-carboxylate.

White solid (yield: 97%); ¹H NMR (400 MHz, CD₃OD) δ 8.24 (ddd, J = 5.0, 2.0, 0.9 Hz, 1H), 7.62 (ddd, J = 8.5, 7.2, 1.8 Hz, 1H), 7.43 (dd, J = 7.8, 1.8 Hz, 1H), 7.31 (dd, J = 8.1, 1.8 Hz, 1H), 7.19 (ddd, J = 8.5, 1.8, 0.9 Hz, 1H), 7.08-7.00 (m, 2H), 6.90 (ddd, J = 7.3, 5.0, 0.9 Hz, 1H), 4.31-4.28 (m, 1H), 4.02-4.00 (m, 2H), 3.99-3.96 (m, 3H), 3.89-3.81 (m, 2H), 3.62-3.58 (m, 1H), 3.46-3.40 (m, 1H), 3.36-3.34 (m, 1H), 3.22-3.18 (m, 1H), 2.15-2.11 (m, 1H), 1.93-1.87 (m, 1H), 1.45 (s, 9H).

### <Preparation Example c-13> Preparation of (S)-4-(pyridin-2-yl)-N-(pyrrolidin-3-yl)-3,4-dihydroquinoxaline-1(2H)-carboxamide

(S)-4-(pyridin-2-yl)-N-(pyrrolidin-3-yl)-3,4-dihydroquinoxaline-1(2H)-carboxamide was prepared by deprotecting the Boc group of the tert-butyl (S)-3-(4-(pyridin-2-yl)-1,2,3,4-tetrahydroquinoxaline-1-carboxamido)pyrrolidin-1-carboxylate obtained in Preparation Example c-12 with TFA.

White solid (yield: 48%); ¹H NMR (400 MHz, CDCl₃) δ 8.29 (ddd, J = 5.0, 2.0, 0.9 Hz, 1H), 7.52 (ddd, J = 8.5, 7.2, 1.0 Hz, 1H), 7.44 (dd, J = 8.2, 1.5 Hz, 1H), 7.34 (dd, J = 7.9, 1.6 Hz, 1H), 7.15 (dd, J = 8.5, 0.9 Hz, 1H), 7.04 (ddd, J = 8.2, 7.3, 1.6 Hz, 1H), 6.9-6.94 (m, 1H), 6.82 (ddd, J = 7.2, 4.9, 0.9 Hz, 1H), 5.41 (d, J = 6.9 Hz, 1H), 4.36-4.28 (m, 1H), 4.00 (ddd, J = 6.9, 5.4, 1.3 Hz, 2H), 3.94 (ddd, J = 6.9, 5.4, 1.3 Hz, 2H), 3.31 (brs, NH), 3.16 (dd, J = 11.4, 6.5 Hz, 1H), 3.05-3.02 (m, 1H), 2.96-2.89 (m, 1H), 2.84-2.80 (m, 1H), 2.19-2.10 (m, 1H), 1.66-1.56 (m, 1H); LC/MS ESI (+): 324.2 (M+1).

### <Example 11> Synthesis of (S)-N-(1-isopropylpyrrolidin-3-yl)-4-(pyridin-2-yl)-3,4-dihydroquinoxaline-1(2H)-carboxamide

The (S)-4-(pyridin-2-yl)-N-(pyrrolidin-3-yl)-3,4-dihydroquinoxaline-1(2H)-carboxamide (1.0 equiv.) obtained in Preparation Example c-13 was dissolved in dichloromethane (0.3 M), sodium triacetoxyborohydride (1.5 equiv.) and acetone (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (S)-N-(1-isopropylpyrrolidin-3-yl)-4-(pyridin-2-yl)-3,4-dihydroquinoxaline-1(2H)-carboxamide.

Light yellow oil (yield: 48%); ¹H NMR (400 MHz, CD₃OD) δ 8.24 (ddd, J = 5.0, 2.0, 0.9 Hz, 1H), 7.62 (ddd, J = 8.5, 7.2, 2.0 Hz, 1H), 7.49-7.46 (m, 1H), 7.36-7.32 (m, 1H), 7.21 (ddd, J = 8.5, 7.6, 1.0 Hz, 1H), 7.10-7.02 (m, 2H), 6.90 (ddd, J = 7.2, 5.0, 0.9 Hz, 1H), 4.37-4.31 (m, 1H), 3.99-3.97 (m, 2H), 3.93-3.81 (m, 2H), 2.90-2.82 (m, 2H), 2.63-2.59 (m, 1H), 2.52-2.50 (m, 1H), 2.46-2.40 (m, 1H), 2.32-2.23 (m, 1H), 1.09 (d, J = 7.4 Hz, 6H); LC/MS ESI (+): 366.3 (M+1).

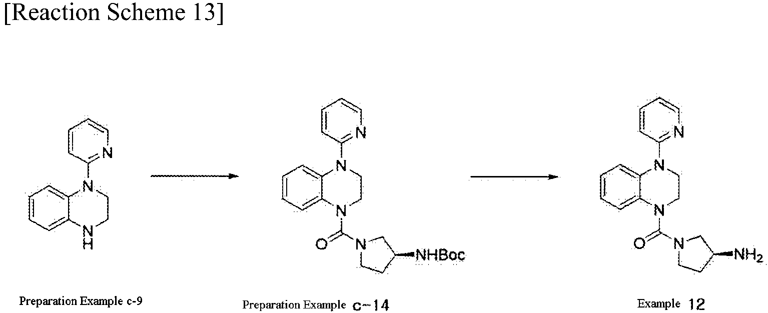

### <Preparation Example c-14> Preparation of tert-butyl (S)-1-(4-(pyridin-2-yl)-1,2,3,4-tetrahydroquinoxaline-1-carboxamido)pyrrolidin-1-carboxylate

Tert-butyl (S)-1-(4-(pyridin-2-yl)-1,2,3,4-tetrahydroquinoxaline-1-carboxamido)pyrrolidin-1-carboxylate was prepared in the same manner as described in Preparation Example c-12 using (S)-3-(Boc-amino)pyrrolidine instead of (S)-(-)-1-Boc-3-aminopyrrolidine.

White solid (yield: 89%); ¹H NMR (400 MHz, CD₃OD) δ 8.22 (ddd, J = 5.0, 2.0, 0.9 Hz, 1H), 7.60 (ddd, J = 8.5, 7.2, 2.0 Hz, 1H), 7.34-7.32 (m, 1H), 7.20-7.17 (m, 1H), 7.09-7.06 (m, 1H), 7.00-6.96 (m, 2H), 6.88 (ddd, J = 7.2, 5.0, 0.9 Hz, 1H), 4.02-3.99 (m, 2H), 3.84-3.78 (m, 1H), 3.76-3.70 (m, 1H), 3.51-3.44 (m, 2H), 3.41-3.35 (m, 2H), 3.17-3.14 (m, 1H), 2.06-2.01 (m, 1H), 1.85-1.77 (m, 1H), 1.39 (s, 9H).

### <Example 12> Synthesis of (S)-(3-aminopyrrolidin-1-yl)(4-(pyridin-2-yl)-3,4-dihydroquinoxaline-1(2H)-yl)methanone

(S)-(3-aminopyrrolidin-1-yl)(4-(pyridin-2-yl)-3,4-dihydroquinoxaline-1(2H)-yl)methanone was synthesized by deprotecting the Boc group of the tert-butyl (S)-1-(4-(pyridin-2-yl)-1,2,3,4-tetrahydroquinoxaline-1-carboxamido)pyrrolidin-1-carboxylate obtained in Preparation Example c-14 with TFA.

White solid (yield: 78%); ¹H NMR (400 MHz, CD₃OD) δ 8.23 (ddd, J = 5.0, 2.0, 0.9 Hz, 1H), 7.61 (ddd, J = 8.5, 7.2, 2.0 Hz, 1H), 7.36-7.32 (m, 1H), 7.21-7.17 (m, 1H), 7.09-7.06 (m, 1H), 7.02-6.96 (m, 2H), 6.89 (ddd, J = 7.2, 5.0, 0.9 Hz, 1H), 4.01 (t, J = 6.3 Hz, 2H), 3.84-3.72 (m, 2H), 3.55-3.49 (m, 2H), 3.44-3.38 (m, 1H), 3.14-3.08 (m, 1H), 2.13-2.04 (m, 1H), 1.78-1.69 (m, 1H); LC/MS ESI (+): 324.2 (M+1).

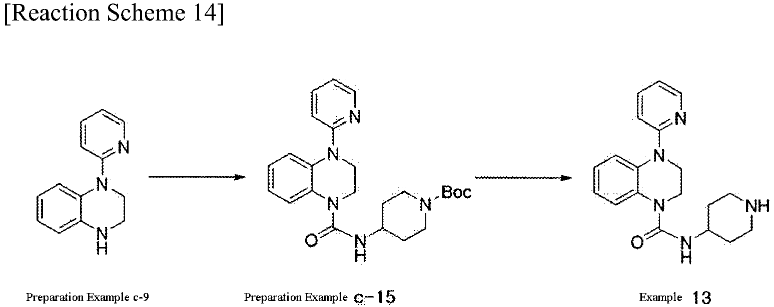

### <Preparation Example c-15> Preparation of tert-butyl 4-(4-(pyridin-2-yl)-1,2,3,4-tetrahydroquinoxaline-1-carboxamido)piperidin-1-carboxylate

Tert-butyl 4-(4-(pyridin-2-yl)-1,2,3,4-tetrahydroquinoxaline-1-carboxamido)piperidin-1-carboxylate was prepared in the same manner as described in Preparation Example c-12 using 4-amino-1-Boc-piperidine instead of (S)-(-)-1-Boc-3-aminopyrrolidine.

White solid (yield: 95%); ¹H NMR (400 MHz, CD₃OD) δ 8.23 (ddd, J = 5.0, 2.0, 0.9 Hz, 1H), 7.62 (ddd, J = 8.5, 7.2, 2.0 Hz, 1H), 7.47-7.45 (m, 1H), 7.33-7.30 (m, 1H), 7.20-7.18 (m, 1H), 7.08-7.00 (m, 2H), 6.90 (ddd, J = 7.2, 5.0, 0.9 Hz, 1H), 4.04-4.00 (m, 2H), 3.99-3.96 (m, 2H), 3.87-3.84 (m, 2H), 3.82-3.74 (m, 1H), 2.91-2.89 (m, 2H), 1.89-1.81 (m, 2H), 1.45 (s, 9H), 1.41-1.37 (m, 2H).

### <Example 13> Synthesis of N-(piperidin-4-yl)-4-(pyridin-2-yl)-3,4-dihydroquinoxaline-1(2H)-carboxamide

N-(piperidin-4-yl)-4-(pyridin-2-yl)-3,4-dihydroquinoxaline-1(2H)-carboxamide was synthesized by deprotecting the Boc group of the tert-butyl 4-(4-(pyridin-2-yl)-1,2,3,4-tetrahydroquinoxaline-1-carboxamido)piperidin-1-carboxylate obtained in Preparation Example c-15 with TFA.

White solid (yield: 80%); ¹H NMR (400 MHz CDCl₃) δ 8.31 (ddd, J = 5.0, 2.0, 0.9 Hz, 1H), 7.53 (ddd, J = 8.4, 7.2, 2.0 Hz, 1H), 7.47 (dd, J = 8.2, 1.4 Hz, 1H), 7.32 (dd, J = 8.0, 1.6 Hz, 1H), 7.15 (ddd, J = 8.4, 2.0, 0.9 Hz, 1H), 7.06 (ddd, J = 8.2, 7.3, 1.6 Hz, 1H), 6.96 (ddd, J = 8.0, 7.3, 1.4 Hz, 1H), 6.83 (ddd, J = 7.2, 5.0, 2.0 Hz, 1H), 5.08 (d, J = 7.6 Hz, 1H), 4.02-3.98 (m, 2H), 3.96-3.93 (m, 2H), 3.84-3.75 (m, 1H), 3.07-3.02 (m, 2H), 2.73-2.66 (m, 2H), 1.99-1.96 (m, 2H), 1.38-1.25 (m, 2H); LC/MS ESI (+): 338.2 (M+1).

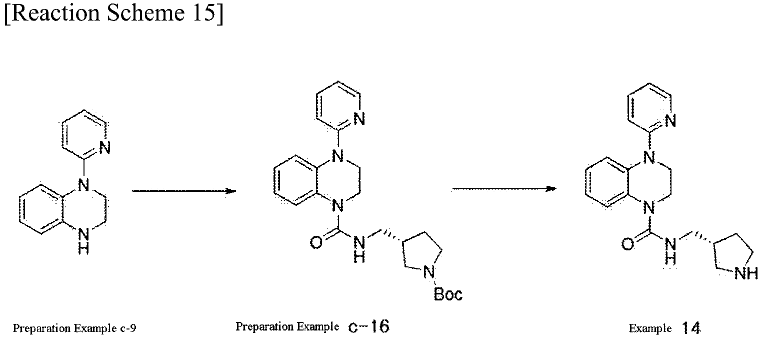

### <Preparation Example c-16> Preparation of tert-butyl (S)-3-((4-pyridin-2-yl)-1,2,3,4-tetrahydroquinoxaline-1-carboxamido)methyl)pyrrolidin-1-carboxylate

Tert-butyl (S)-3-((4-pyridin-2-yl)-1,2,3,4-tetrahydroquinoxaline-1-carboxamido)methyl)pyrrolidin-1-carboxylate was prepared in the same manner as described in Preparation Example c-12 using (S)-1-Boc-3-(aminomethyl)pyrrolidine instead of (S)-(-)-1-Boc-3-aminopyrrolidine.

Yellow oil (yield: 100%); ¹H NMR (400 MHz, CDCl₃) δ 8.31 (ddd, J = 4.9, 2.0, 0.9 Hz, 1H), 7.55-7.52 (m, 1H), 7.49-7.45 (m, 1H), 7.30 (dd, J = 7.9, 1.6 Hz, 1H), 7.16-7.14 (m, 1H), 7.10-7.06 (m, 1H), 6.98 (ddd, J = 8.0, 7.3, 1.4 Hz, 1H), 6.86-6.83 (m, 1H), 5.29 (d, J = 5.8 Hz, 1H), 4.02-3.94 (m, 4H), 3.53-3.43 (m, 2H), 3.39-3.37 (m, 1H), 3.30-3.22 (m, 2H), 3.16-3.12 (m, 1H), 3.05-2.94 (m, 1H), 2.42-2.39 (m, 1H), 1.99-1.93 (m, 1H), 1.45 (s, 9H).

### <Example 14> Synthesis of (R)-4-(pyridin-2-yl)-N-(pyrrolidin-3-ylmethyl)-3,4-dihydroquinoxaline-1(2H)-carboxamide

(R)-4-(pyridin-2-yl)-N-(pyrrolidin-3-ylmethyl)-3,4-dihydroquinoxaline-1(2H)-carboxamide was synthesized by deprotecting the Boc group of the tert-butyl (S)-3-((4-pyridin-2-yl)-1,2,3,4-tetrahydroquinoxaline-1-carboxamido)methyl)pyrrolidin-1-carboxylate obtained in Preparation Example c-16 with TFA.

White solid (yield: 53%); ¹H NMR (400 MHz CD₃OD) δ 8.24 (ddd, J = 5.0, 2.0, 0.9 Hz, 1H), 7.64 (ddd, J = 8.5, 7.2, 2.0 Hz, 1H), 7.45 (dd, J = 7.8, 1.6 Hz, 1H), 7.35 (dd, J = 8.2, 1.6 Hz, 1H), 7.20 (ddd, J = 8.4, 2.0, 0.9 Hz, 1H), 7.11 (ddd, J = 8.2, 7.3, 1.6 Hz, 1H), 7.05 (ddd, J = 7.8, 7.3, 1.6 Hz, 1H), 6.92 (ddd, J = 7.2, 5.0, 2.0 Hz, 1H), 4.00-3.97 (m, 2H), 3.89-3.87 (m, 2H), 3.36-3.34 (m, 3H), 3.29-3.25 (m, 2H), 3.00-2.95 (m, 1H), 2.62-2.53 (m, 1H), 2.14-2.04 (m, 1H), 1.79-1.69 (m, 1H); LC/MS ESI (+): 338.2 (M+1).

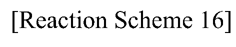

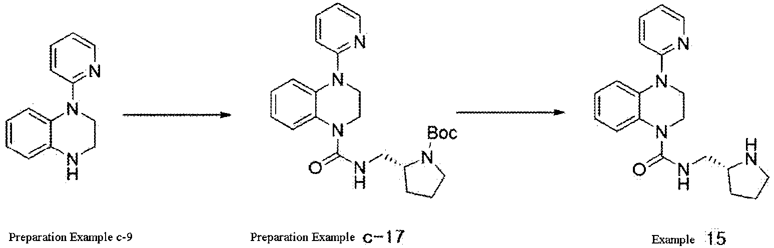

### <Preparation Example c-17> Preparation of tert-butyl (R)-2-((4-pyridin-2-yl)-1,2,3,4-tetrahydroquinoxaline-1-carboxamido)methyl)pyrrolidin-1-carboxylate

Tert-butyl (S)-3-((4-pyridin-2-yl)-1,2,3,4-tetrahydroquinoxaline-1-carboxamido)methyl)pyrrolidin-1-carboxylate was prepared in the same manner as described in Preparation Example c-12 using (R)-1-Boc-2-(aminomethyl)pyrrolidine instead of (S)-(-)-1-Boc-3-aminopyrrolidine.

Yellow oil (yield: 100%); ¹H NMR (400 MHz, CDCl₃) δ 8.29 (d, J = 2.9 Hz, 1H), 7.60-7.55 (m, 1H), 7.48-7.44 (m, 1H), 7.38-7.36 (m, 1H), 7.14 (ddd, J = 8.5, 7.3, 1.0 Hz, 1H), 7.03-7.01 (m, 1H), 6.79-6.77 (m, 1H), 6.58-6.53 (m, 1H), 4.06-4.05 (m, 2H), 3.97-3.91 (m, 2H), 3.89-3.84 (m, 1H), 3.41-3.39 (m, 1H), 3.33-3.23 (m, 3H), 1.98-1.86 (m, 2H), 1.82-1.78 (m, 1H), 1.74-1.68 (m, 1H), 1.40 (s, 9H).

### <Example 15> Synthesis of (R)-4-(pyridin-2-yl)-N-(pyrrolidin-3-ylmethyl)-3,4-dihydroquinoxaline-1(2H)-carboxamide

(R)-4-(pyridin-2-yl)-N-(pyrrolidin-3-ylmethyl)-3,4-dihydroquinoxaline-1(2H)-carboxamide was synthesized by deprotecting the Boc group of the tert-butyl (S)-3-((4-pyridin-2-yl)-1,2,3,4-tetrahydroquinoxaline-1-carboxamido)methyl)pyrrolidin-1-carboxylate obtained in Preparation Example c-17 with TFA.

White solid (yield: 100%); ¹H NMR (400 MHz, CD₃OD) δ 8.24 (ddd, J = 5.0, 2.0, 0.9 Hz, 1H), 7.64 (ddd, J = 8.5, 7.2, 2.0 Hz, 1H), 7.50 (dd, J = 8.0, 1.6 Hz, 1H), 7.36 (dd, J = 8.0, 1.5 Hz, 1H), 7.20 (ddd, J = 8.5, 2.0, 0.9 Hz, 1H), 7.13 (ddd, J = 8.0, 7.3, 1.6 Hz, 1H), 7.07 (ddd, J = 8.0, 7.3, 1.5 Hz, 1H), 6.92 (ddd, J = 7.2, 5.0, 2.0 Hz, 1H), 4.03-3.99 (m, 2H), 3.94-3.90 (m, 2H), 3.70-3.64 (m, 1H), 3.52-3.42 (m, 2H), 3.29-3.19 (m, 2H), 3.15-2.06 (m, 1H), 2.03-1.92 (m, 2H), 1.79-1.70 (m, 1H); LC/MS ESI (+): 338.3 (M+1).

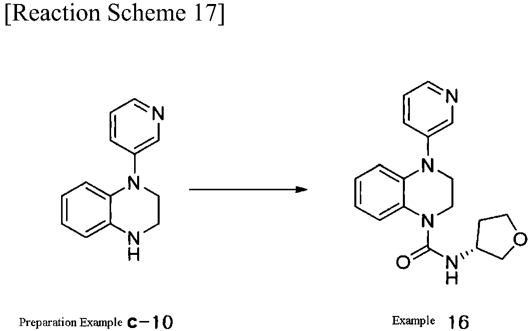

### <Example 16> Synthesis of (R)-4-(pyridin-3-yl)-N-(tetrahydrofuran-3-yl)-3,4-dihydroquinoxaline-1(2H)-carboxamide

The 1-(pyridin-3-yl)-1,2,3,4-tetrahydroquinoxaline (1.0 equiv.) obtained in Preparation Example c-10 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (R)-3-aminotetrahydrofuran (1.2 equiv.) were added to the reaction solution, and the resulting solution was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (R)-4-(pyridin-3-yl)-N-(tetrahydrofuran-3-yl)-3,4-dihydroquinoxaline-1(2H)-carboxamide.

Yellow solid (yield: 97%); ¹H NMR (400 MHz, CDCl₃) δ 8.58 (dd, J = 2.7, 0.7 Hz, 1H), 8.41 (dd, J = 4.7, 1.5 Hz, 1H), 7.59 (ddd, J = 8.2, 2.7, 1.5 Hz, 1H), 7.32 (ddd, J = 8.2, 4.7, 0.7 Hz, 1H), 7.23-7.20 (m, 1H), 6.97-6.93 (m, 1H), 6.84-6.80 (m, 2H), 5.45 (d, J = 6.9 Hz, 1H), 4.53-4.46 (m, 1H), 4.03-3.95 (m, 2H), 3.93-3.85 (m, 2H), 3.82-3.78 (m, 1H), 3.74-3.67 (m, 3H), 2.33-2.24 (m, 1H), 1.84-1.76 (m, 1H); LC/MS ESI (+): 325.0 (M+1).

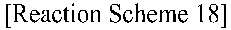

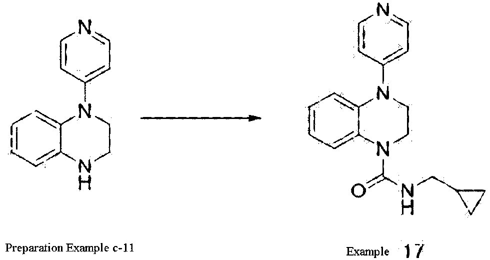

### <Example 17> Synthesis of N-(cyclopropylmethyl)-4-(pyridin-4-yl)-3,4-dihydroquinoxaline-1(2H)-carboxamide

The 1-(pyridin-3-yl)-1,2,3,4-tetrahydroquinoxaline (1.0 equiv.) obtained in Preparation Example c-11 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and cyclopropylmethylamine (1.2 equiv.) were added to the reaction solution, and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize N-(cyclopropylmethyl)-4-(pyridin-4-yl)-3,4-dihydroquinoxaline-1(2H)-carboxamide.

Light yellow solid (yield: 37%); ¹H NMR (400 MHz, CDCl₃) δ 8.39-8.37 (m, 2H), 7.41 (dd, J = 8.1, 1.5 Hz, 1H), 7.37 (dd, J = 7.9, 1.6 Hz, 1H), 7.13-7.10 (m, 1H), 7.07-7.03 (m, 3H), 5.26 (t, J = 5.2 Hz, 1H), 3.98 (t, J = 6.1 Hz, 2H), 3.82 (t, J = 6.1 Hz, 2H), 3.13 (dd, J = 7.0, 5.2 Hz, 2H), 0.99-0.93 (m, 1H), 0.51-0.46 (m, 2H), 0.20-0.16 (m, 2H); LC/MS ESI (+): 309.1 (M+1).

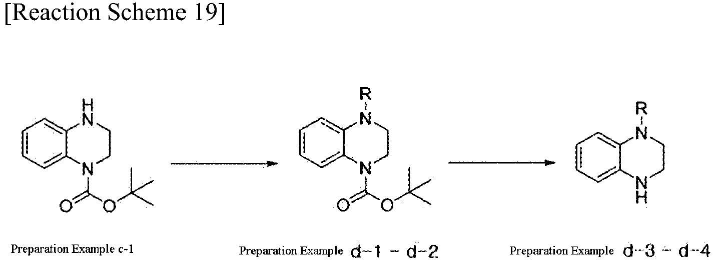

### <Preparation Method d> Preparation of halogen-substituted pyridine or pyrazin-1,2,3,4-tetrahydroquinoxaline

A halogen-substituted pyridine or pyrazin-1,2,3,4-tetrahydroquinoxaline according to the present invention was prepared through the procedures described in Preparation Examples d-1 to d-4 below.

### <Preparation Example d-1> Preparation of tert-butyl 4-(5-fluoropyridin-2-yl)-3,4-dihydroquinoxaline-1(2H)-carboxylate

The tert-butyl 3,4-dihydroquinoxaline-1(2H)-carboxylate (1.0 equiv.) obtained in Preparation Example c-1, sodium butoxide (2.0 equiv.), and 2-bromo-5-fluoropyridine (3.0 equiv.) were sufficiently dissolved in toluene (0.3 M). A mixed solution in which Pd₂dba (0.05 equiv.) and XPhos (0.15 equiv.) were dissolved in toluene (0.6 M), which was heated for 5 minutes at 110 °C was slowly added to the reaction solution, and stirred for 15 hours at 110 °C. After cooling to room temperature, the resulting solution was filtered through a Celite pad and extracted with dichloromethane. An organic layer was dried over MgSO₄, filtered, and concentrated under reduced pressure. The residue obtained thereby was purified by silica column chromatography using a mixture of n-hexane and ethyl acetate to prepare tert-butyl 4-(5-fluoropyridin-2-yl)-3,4-dihydroquinoxaline-1(2H)-carboxylate.

Red oil (yield: 93%); ¹H NMR (400 MHz, CDCl₃) δ 8.14 (d, J = 3.0 Hz, 1H), 7.81-7.77 (m, 1H), 7.27-7.21 (m, 2H), 7.13 (ddd, J = 9.2, 3.0, 0.7 Hz, 1H), 7.00-6.95 (m, 2H), 4.02 (dd, J = 5.8, 4.6 Hz, 2H), 3.87 (dd, J = 5.8, 4.6 Hz, 2H), 1.53 (s, 9H).

### <Preparation Example d-2> Preparation of tert-butyl 4-(pyrazin-2-yl)-3,4-dihydroquinoxaline-1(2H)-carboxylate

Tert-butyl 4-(pyrazin-2-yl)-3,4-dihydroquinoxaline-1(2H)-carboxylate was prepared in the same manner as described in Preparation Example d-1 using 2-chloropyrazine instead of 2-bromo-5-fluoropyridine.

Orange solid (yield: 85%); ¹H NMR (400 MHz, CDCl₃) δ 8.59 (d, J = 1.6 Hz, 1H), 8.16 (dd, J = 2.7, 1.5 Hz, 1H), 7.98 (d, J = 2.7 Hz, 1H), 7.84 (d, J = 8.0 Hz, 1H), 7.40-7.34 (m, 1H), 7.12-7.00 (m, 2H), 4.12-4.04 (m, 2H), 3.93-3.86 (m, 2H), 1.53 (s, 9H).

### <Preparation Example d-3> Preparation of 1-(5-fluoropyridin-2-yl)-1,2,3,4-tetrahydroquinoxaline

1-(5-fluoropyridin-2-yl)-1,2,3,4-tetrahydroquinoxaline was prepared by deprotecting the Boc group of the tert-butyl 4-(5-fluoropyridin-2-yl)-3,4-dihydroquinoxaline-1(2H)-carboxylate obtained in Preparation Example d-1 with TFA.

Red oil (yield: 91%); ¹H NMR (400 MHz, CDCl₃) δ 8.12 (d, J = 3.0 Hz, 1H), 7.22-7.20 (m, 1H), 7.18-7.11 (m, 2H), 6.89-6.85 (m, 1H), 6.65-6.61 (m, 2H), 4.01 (t, J = 5.0 Hz, 2H), 3.90 (brs, NH), 3.40 (t, J = 5.0 Hz, 2H); LC/MS ESI (+): 230.1 (M+1).

### <Preparation Example d-4> Preparation of 1-(pyrazin-2-yl)-1,2,3,4-tetrahydroquinoxaline

1-(pyrazin-2-yl)-1,2,3,4-tetrahydroquinoxaline was prepared by deprotecting the Boc group of the tert-butyl 4-(pyrazin-2-yl)-3,4-dihydroquinoxaline-1(2H)-carboxylate obtained in Preparation Example d-2 with TFA.

Yellow oil (yield: 88%); ¹H NMR (400 MHz, CDCl₃) δ 8.59 (d, J = 1.6 Hz, 1H), 8.13 (dd, J = 2.7, 1.6 Hz, 1H), 7.91 (d, J = 2.7 Hz, 1H), 7.26-7.21 (m, 1H), 6.99-6.89 (m, 1H), 6.72-6.63 (m, 2H), 4.11-4.01 (m, 3H), 3.48-3.40 (m, 2H); LC/MS ESI (+): 213.2 (M+1).

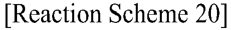

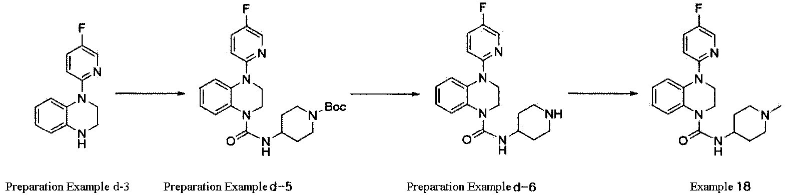

### <Preparation Example d-5> Preparation of tert-butyl 4-((4-(5-fluoropyridin-2-yl)-1,2,3,4-tetrahydroquinoxalin-1-carboxamido)piperidin-1-carboxylate

The 1-(5-fluoropyridin-2-yl)-1,2,3,4-tetrahydroquinoxaline (1.0 equiv.) obtained in Preparation Example d-3 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and 4-amino-1-Boc-piperidine (1.2 equiv.) were added to the reaction solution, and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl 4-((4-(5-fluoropyridin-2-yl)-1,2,3,4-tetrahydroquinoxalin-1-carboxamido)piperidin-1-carboxylate.

Light yellow oil (yield: 88%); ¹H NMR (400 MHz, CDCl₃) δ 8.18 (d, J = 3.0 Hz, 1H), 7.36 (dd, J = 8.2, 1.4 Hz, 1H), 7.33-7.27 (m, 2H), 7.15 (dd, J = 9.1, 3.0 Hz, 1H), 7.07-7.03 (m, 1H), 6.95 (ddd, J = 8.0, 7.3, 1.4 Hz, 1H), 5.07 (d, J = 7.6 Hz, 1H), 3.99-3.96 (m, 2H), 3.95 (s, 4H), 3.89-3.80 (m, 1H), 2.90-2.84 (m, 2H), 1.96-1.90 (m, 2H), 1.44 (s, 9H), 1.33-1.23 (m, 2H).

### <Preparation Example d-6> Preparation of 4-(5-Fluoropyridin-2-yl)-N-(piperidin-4-yl)-3,4-dihydroquinoxaline-1 (2H)-carboxamide

4-(5-Fluoropyridin-2-yl)-N-(piperidin-4-yl)-3,4-dihydroquinoxaline-1(2H)-carboxamide was prepared by deprotecting the Boc group of the tert-butyl 4-((4-(5-fluoropyridin-2-yl)-1,2,3,4-tetrahydroquinoxalin-1-carboxamido)piperidin-1-carboxylate obtained in Preparation Example d-5 with TFA.

White solid (yield: 82%); ¹H NMR (400 MHz, CDCl₃) δ 8.18 (d, J = 3.0 Hz, 1H), 7.36 (dd, J = 8.2, 1.4 Hz, 1H), 7.33-7.28 (m, 2H), 7.16 (ddd, J = 9.1, 3.0, 0.6 Hz, 1H), 7.07-7.02 (m, 1H), 6.95 (ddd, J = 8.0, 7.3, 1.4 Hz, 1H), 5.09 (d, J = 7.7 Hz, 1H), 3.95 (s, 4H), 3.84-3.75 (m, 1H), 3.06-3.00 (m, 2H), 2.72-2.65 (m, 2H), 2.00-1.93 (m, 2H), 1.32-1.23 (m, 2H); LC/MS ESI (+): 356.3 (M+1).

<Example 18> Synthesis of 4-(5-Fluoropyridin-2-yl)-N-(1-methylpiperidin-4-yl)-3,4-dihydroquinoxaline-1(2H)-carboxamide

The 4-(5-Fluoropyridin-2-yl)-N-(piperidin-4-yl)-3,4-dihydroquinoxaline-1(2H)-carboxamide (1.0 equiv.) obtained in Preparation Example d-6 was dissolved in dichloromethane (0.3 M), sodium triacetoxyborohydride (1.3 equiv.) and formaldehyde (1.1 equiv.) were added, and the resulting mixture was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize 4-(5-Fluoropyridin-2-yl)-N-(1-methylpiperidin-4-yl)-3,4-dihydroquinoxaline-1(2H)-carboxamide.

White solid (yield: 95%); ¹H NMR (400 MHz, CDCl₃) δ 8.18 (dd, J = 3.0, 0.6 Hz, 1H), 7.36 (dd, J = 8.2, 1.4 Hz, 1H), 7.31-7.28 (m, 2H), 7.16 (ddd, J = 9.1, 3.0, 0.6 Hz, 1H), 7.05 (ddd, J = 8.2, 7.4, 1.6 Hz, 1H), 6.94 (ddd, J = 7.9, 7.4, 1.4 Hz, 1H), 5.06 (d, J = 7.6 Hz, 1H), 3.95 (s, 4H), 3.76-3.66 (m, 1H), 2.74-2.72 (m, 2H), 2.26 (s, 3H), 2.12-2.07 (m, 2H), 1.98-1.93 (m, 2H), 1.48-1.42 (m, 2H); LC/MS ESI (+): 370.3 (M+1).

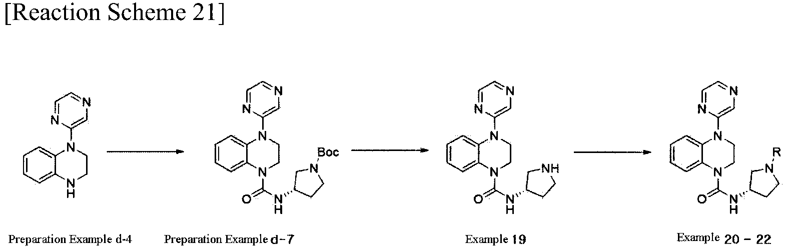

### <Preparation Example d-7> Preparation of tert-butyl (S)-3-(4-(pyrazin-2-yl)-1,2,3,4-tetrahydroquinoxaline-1-carboxamido)pyrrolidin-1-carboxylate

The 1-(pyrazin-2-yl)-1,2,3,4-tetrahydroquinoxaline (1.0 equiv.) obtained in Preparation Example d-4 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-1-Boc-3-aminopyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (S)-3-(4-(pyrazin-2-yl)-1,2,3,4-tetrahydroquinoxaline-1-carboxamido)pyrrolidin-1-carboxylate.

Light yellow solid (yield: 88%); ¹H NMR (400 MHz, CDCl₃) δ 8.61 (d, J = 1.5 Hz, 1H), 8.21 (dd, J = 2.6, 1.5 Hz, 1H), 8.05 (d, J = 2.6 Hz, 1H), 7.54 (dd, J = 8.1, 1.5 Hz, 1H), 7.32 (dd, J = 7.9, 1.6 Hz, 1H), 7.16-7.12 (m, 1H), 7.09-7.04 (m, 1H), 5.18 (d, J = 6.7 Hz, 1H), 4.42-4.37 (m, 1H), 4.05-3.91 (m, 4H), 3.67-3.62 (m, 1H), 3.42-3.37 (m, 2H), 3.19-3.13 (m, 1H), 2.18-2.12 (m, 1H), 1.82-1.78 (m, 1H), 1.44 (s, 9H).

### <Example 19> Synthesis of (S)-4-(Pyrazin-2-yl)-N-(pyrrolidin-3-yl)-3,4-dihydroquinoxaline-1(2H)-carboxamide

(S)-4-(Pyrazin-2-yl)-N-(pyrrolidin-3-yl)-3,4-dihydroquinoxaline-1(2H)-carboxamide was synthesized by deprotecting the Boc group of the tert-butyl (S)-3-(4-(pyrazin-2-yl)-1,2,3,4-tetrahydroquinoxaline-1-carboxamido)pyrrolidin-1-carboxylate obtained in Preparation Example d-7 with TFA.

Light yellow solid (yield: 77%); ¹H NMR (400 MHz, CDCl₃) δ 8.60 (d, J = 1.5 Hz, 1H), 8.19 (dd, J = 2.7, 1.5 Hz, 1H), 8.02 (d, J = 2.7 Hz, 1H), 7.50 (dd, J = 8.1, 1.5 Hz, 1H), 7.42 (dd, J = 7.8, 1.7 Hz, 1H), 7.14-7.04 (m, 2H), 5.47 (d, J = 7.4 Hz, 1H), 4.40-4.33 (m, 1H), 4.06-4.02 (m, 2H), 3.99-3.95 (m, 2H), 3.22-3.17 (m, 1H), 3.15-3.09 (m, 1H), 3.01-2.95 (m, 1H), 2.93-2.87 (m, 1H), 2.24-2.08 (m, 1H), 2.01-1.98 (m, 1H), 1.65-1.52 (m, 1H); LC/MS ESI (+): 325.2 (M+1).

### <Example 20> Synthesis of (S)-N-(1-methylpyrrolidin-3-yl)-4-(pyrazin-2-yl)-3,4-dihydroquinoxaline-1(2H)-carboxamide

The (S)-4-(Pyrazin-2-yl)-N-(pyrrolidin-3-yl)-3,4-dihydroquinoxaline-1(2H)-carboxamide (1.0 equiv.) obtained in Example 19 was dissolved in dichloromethane (0.3 M), sodium triacetoxyborohydride (1.5 equiv.) and formaldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (S)-N-(1-methylpyrrolidin-3-yl)-4-(pyrazin-2-yl)-3,4-dihydroquinoxaline-1(2H)-carboxamide.

Light yellow solid (yield: 82%); ¹H NMR (400 MHz, CDCl₃) δ 8.61 (dd, J = 1.6, 0.4 Hz, 1H), 8.20 (dd, J = 2.7, 1.6 Hz, 1H), 8.03 (dd, J = 2.7, 0.4 Hz, 1H), 7.50 (dd, J = 8.1, 1.5 Hz, 1H), 7.39 (dd, J = 7.8, 1.7 Hz, 1H), 7.13-7.04 (m, 2H), 5.42 (d, J = 7.7 Hz, 1H), 4.43-4.36 (m, 1H), 4.04-4.01 (m, 2H), 3.99-3.89 (m, 2H), 2.82-2.76 (m, 1H), 2.60-2.58 (m, 1H) 2.54-2.50 (m, 1H), 2.36-2.28 (m, 4H), 2.22-2.16 (m, 1H), 1.62-1.54 (m, 1H); LC/MS ESI (+): 339.2 (M+1).

### <Example 21> Synthesis of (S)-N-(1-Isopropylpyrrolidin-3-yl)-4-(pyrazin-2-yl)-3,4-dihydroquinoxaline-1(2H)-carboxamide

(S)-N-(1-isopropylpyrrolidin-3-yl)-4-(pyrazin-2-yl)-3,4-dihydroquinoxaline-1(2H)-carboxamide was synthesized in the same manner as described in Example 20 using acetone instead of formaldehyde.

Light yellow solid (yield: 65%); ¹H NMR (400 MHz, CDCl₃) δ 8.62 (d, J = 1.6 Hz, 1H), 8.20 (dd, J = 2.7, 1.6 Hz, 1H), 8.03 (d, J = 2.7 Hz, 1H), 7.50 (dd, J = 7.9, 1.6 Hz, 1H), 7.39 (dd, J = 7.7, 1.8 Hz, 1H), 7.13-7.04 (m, 2H), 5.43 (d, J = 7.6 Hz, 1H), 4.42-4.35 (m, 1H), 4.05-4.01 (m, 2H), 3.98-3.90 (m, 2H), 2.87-2.81 (m, 1H), 2.71-2.62 (m, 2H), 2.35-2.23 (m, 2H), 1.63-1.53 (m, 1H), 1.06 (d, J = 6.3 Hz, 6H); LC/MS ESI (+): 367.3 (M+1).

### <Example 22> Synthesis of (S)-N-(1-isobutylpyrrolidin-3-yl)-4-(pyrazin-2-yl)-3,4-dihydroquinoxaline-1(2H)-carboxamide

(S)-N-(1-isobutylpyrrolidin-3-yl)-4-(pyrazin-2-yl)-3,4-dihydroquinoxaline-1(2H)-carboxamide was synthesized in the same manner as described in Example 20 using isobutyraldehyde instead of formaldehyde.

Yellow oil (yield: 74%); ¹H NMR (400 MHz, CDCl₃) δ 8.60 (d, J = 1.5 Hz, 1H), 8.20 (dd, J = 2.7, 1.5 Hz, 1H), 8.03 (d, J = 2.7 Hz, 1H), 7.51 (dd, J = 8.0, 1.5 Hz, 1H), 7.38 (dd, J = 7.8, 1.7 Hz, 1H), 7.13-7.03 (m, 2H), 5.54 (d, J = 8.0 Hz, 1H), 4.39-4.32 (m, 1H), 4.04-4.02 (m, 2H), 4.00-3.90 (m, 2H), 2.83-2.78 (m, 1H), 2.59-2.56 (m, 1H), 2.46-2.42 (m, 1H), 2.22-2.14 (m, 3H), 1.72-1.65 (m, 1H), 1.63-1.56 (m, 2H), 0.87 (d, J = 6.3 Hz, 6H); LC/MS ESI (+): 381.3 (M+1).

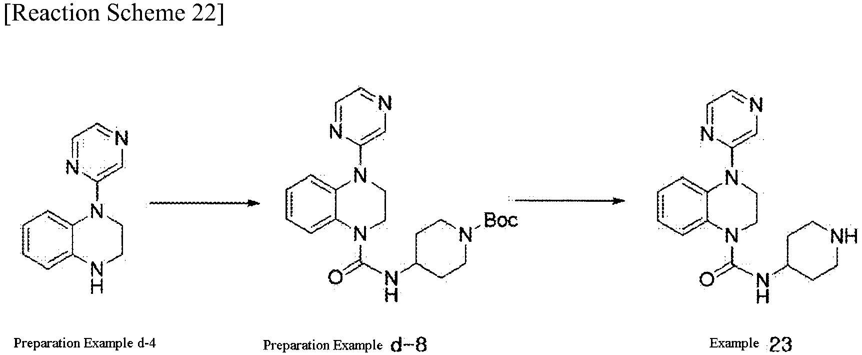

### <Preparation Example d-8> Preparation of tert-butyl (4-(pyrazin-2-yl)-1,2,3,4-tetrahydroquinoxaline-1-carboxamido)piperidin-1-carboxylate

Tert-butyl (4-(pyrazin-2-yl)-1,2,3,4-tetrahydroquinoxaline-1-carboxamido)piperidin-1-carboxylate was prepared in the same manner as described in Preparation Example d-7 using 1-Boc-4-aminopiperidine instead of (S)-(-)-1-Boc-3-aminopyrrolidine.

Yellow solid (yield: 88%); ¹H NMR (400 MHz, CDCl₃) δ 8.59 (d, J = 1.5 Hz, 1H), 8.21 (dd, J = 2.7, 1.5 Hz, 1H), 8.04 (d, J = 2.7 Hz, 1H), 7.53 (dd, J = 8.1, 1.5 Hz, 1H), 7.34 (dd, J = 7.8, 1.6 Hz, 1H), 7.13 (ddd, J = 7.8, 7.4, 1.5 Hz, 1H), 7.07 (ddd, J = 8.1, 7.4, 1.6 Hz, 1H), 5.03 (d, J = 7.6 Hz, 1H), 4.05-4.02 (m, 3H), 3.99-3.97 (m, 3H), 3.89-3.81 (m, 1H), 2.89-2.84 (m, 2H), 1.97-1.91 (m, 2H), 1.44 (s, 9H), 1.34-1.24 (m, 2H).

### <Example 23> Synthesis of N-(1-piperidin-4-yl)-4-(pyrazin-2-yl)-3,4-dihydroquinoxaline-1(2H)-carboxamide

N-(1-piperidin-4-yl)-4-(pyrazin-2-yl)-3,4-dihydroquinoxaline-1(2H)-carboxamide was synthesized by deprotecting the Boc group of the tert-butyl (4-(pyrazin-2-yl)-1,2,3,4-tetrahydroquinoxaline-1-carboxamido)piperidin-1-carboxylate obtained in Preparation Example d-8 with TFA.

Light yellow solid (yield: 85%); ¹H NMR (400 MHz, CDCl₃) δ 8.60 (d, J = 1.5 Hz, 1H), 8.20 (dd, J = 2.7, 1.5 Hz, 1H), 8.04 (d, J = 2.7 Hz, 1H), 7.53 (dd, J = 8.1, 1.5 Hz, 1H), 7.37 (dd, J = 7.8, 1.7 Hz, 1H), 7.37 (ddd, J = 7.8, 7.4, 1.5 Hz, 1H), 7.07 (ddd, J = 8.1, 7.4, 1.7 Hz, 1H), 5.06 (d, J = 7.7 Hz, 1H), 4.05-4.02 (m, 2H), 3.98-3.95 (m, 2H), 3.85-3.75 (m, 1H), 3.07-3.02 (m, 2H), 2.72-2.67 (m, 2H), 1.99-1.94 (m, 2H), 1.34-1.29 (m, 2H); LC/MS ESI (+): 339.2 (M+1).

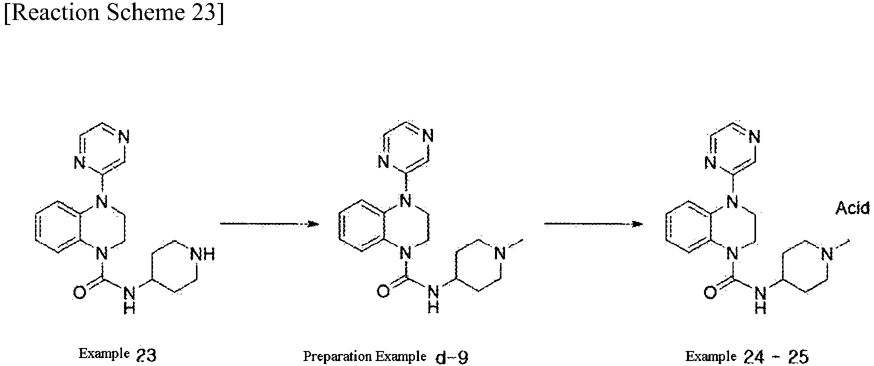

### <Preparation Example d-9> Preparation of N-(1-methylpiperidin-4-yl)-4-(pyrazin-2-yl)-3,4-dihydroquinoxaline-1(2H)-carboxamide

The N-(1-piperidin-4-yl)-4-(pyrazin-2-yl)-3,4-dihydroquinoxaline-1(2H)-carboxamide (1.0 equiv.) obtained in Example 23 was dissolved in dichloromethane (0.3 M), sodium triacetoxyborohydride (1.5 equiv.) and formaldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to prepare N-(1-methylpiperidin-4-yl)-4-(pyrazin-2-yl)-3,4-dihydroquinoxaline-1(2H)-carboxamide.

Light yellow solid (yield: 67%); ¹H NMR (400 MHz, CDCl₃) δ 8.60 (d, J = 1.5 Hz, 1H), 8.20 (dd, J = 2.6, 1.5 Hz, 1H), 8.03 (d, J = 2.6 Hz, 1H), 7.53 (dd, J = 8.0, 1.5 Hz, 1H), 7.36 (dd, J = 7.9, 1.6 Hz, 1H),7.14-7.04 (m, 2H), 5.03 (d, J = 7.6 Hz, 1H), 4.04-4.01 (m, 2H), 3.98-3.95 (m, 2H), 3.75-3.67 (m, 1H), 2.74-2.71 (m, 2H), 2.26 (s, 3H), 2.12-2.06 (m, 2H), 1.98-1.94 (m, 2H), 1.49-1.41 (m, 2H); LC/MS ESI (+): 353.3 (M+1).

### <Example 24> Synthesis of N-(1-methylpiperidin-4-yl)-4-(pyrazin-2-yl)-3,4-dihydroquinoxaline-1(2H)-carboxamide oxalic acid salt

The N-(1-methylpiperidin-4-yl)-4-(pyrazin-2-yl)-3,4-dihydroquinoxaline-1(2H)-carboxamide (1.0 equiv.) obtained in Preparation Example d-9 was dissolved in ethanol (0.3 M) and stirred for 10 minutes at room temperature. Oxalic acid (1.0 equiv.) was added to the reaction solution and stirred for 10 minutes at 70 °C until it dissolved to form a clear solution. After cooling the resulting solution again, it was stirred for 1 hour at room temperature. When a solid was formed, ethyl acetate (0.4 M) was added and then the resulting solution was stirred for 2 hours at room temperature. The produced solid was filtered with ethyl acetate, washed and dried to synthesize N-(1-methylpiperidin-4-yl)-4-(pyrazin-2-yl)-3,4-dihydroquinoxaline-1(2H)-carboxamide.

Ivory solid (yield: 77%); ¹H NMR (400 MHz, CD₃OD) δ 8.50 (d, J = 1.5 Hz, 1H), 8.24 (dd, J = 2.7, 1.5 Hz, 1H), 7.98 (d, J = 2.7 Hz, 1H), 7.55-7.53 (m, 1H), 7.51-7.46 (m, 1H), 7.17-7.12 (m, 2H), 4.06 (t, J = 6.7 Hz, 2H), 3.91-3.84 (m, 3H), 3.54-3.50 (m, 2H), 3.11-3.07 (m, 2H), 2.84 (s, 3H), 2.13-2.11 (m, 2H), 1.83-1.79 (m, 2H).

### <Example 25> Synthesis of N-(1-methylpiperidin-4-yl)-4-(pyrazin-2-yl)-3,4-dihydroquinoxaline-1(2H)-carboxamide fumaric acid salt

N-(1-methylpiperidin-4-yl)-4-(pyrazin-2-yl)-3,4-dihydroquinoxaline-1(2H)-carboxamide fumaric acid salt was synthesized in the same manner as described in Example 24 using fumaric acid instead of oxalic acid.

Ivory solid (yield: 72%); ¹H NMR (400 MHz, CD₃OD) δ 8.50 (d, J = 1.5 Hz, 1H), 8.24 (dd, J = 2.7, 1.5 Hz, 1H), 7.98 (d, J = 2.7 Hz, 1H), 7.55-7.53 (m, 1H), 7.50-7.48 (m, 1H), 7.16-7.14 (m, 2H), 6.68 (s, 2H), 4.06 (t, J = 6.2 Hz, 2H), 3.88-3.84 (m, 3H), 3.46-3.43 (m, 2H), 3.09-3.03 (m, 2H), 2.81 (s, 3H), 2.13-2.10 (m, 2H), 1.86-1.77 (m, 2H).

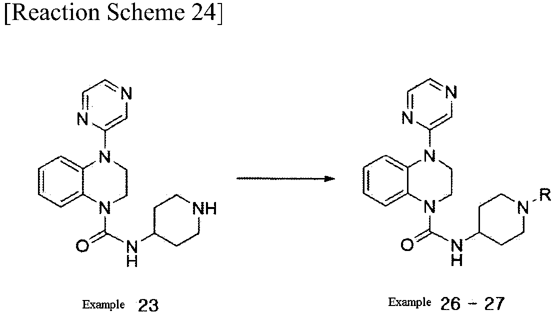

### <Example 26> Synthesis of N-(1-isopropylpiperidin-4-yl)-4-(pyrazin-2-yl)-3,4-dihydroquinoxaline-1 (2H)-carboxamide

The N-(1-piperidin-4-yl)-4-(pyrazin-2-yl)-3,4-dihydroquinoxaline-1(2H)-carboxamide (1.0 equiv.) obtained in Example 23 was dissolved in dichloromethane (0.3 M), sodium triacetoxyborohydride (1.5 equiv.) and acetone (2.0 equiv.) were added, and the resulting mixture was stirred for 1 hour at room temperature. After terminating the reaction by adding water to the reaction solution, the resulting solution as extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize N-(1-isopropylpiperidin-4-yl)-4-(pyrazin-2-yl)-3,4-dihydroquinoxaline-1 (2H)-carboxamide.

Light yellow solid (yield: 82%); ¹H NMR (400 MHz, CDCl₃) δ 8.59 (d, J = 1.6 Hz, 1H), 8.20 (dd, J = 2.6, 1.6 Hz, 1H), 8.03 (d, J = 2.6 Hz, 1H), 7.52 (dd, J = 8.1, 1.4 Hz, 1H), 7.35 (dd, J = 7.9, 1.6 Hz, 1H), 7.11 (ddd, J = 8.1, 7.4, 1.6 Hz, 1H), 7.04 (ddd, J = 7.9, 7.4, 1.4 Hz, 1H), 5.05 (d, J = 7.7 Hz, 1H), 4.04-4.01 (m, 2H), 3.98-3.95 (m, 2H), 3.74-3.66 (m, 1H), 2.79-2.77 (m, 2H), 2.74-2.67 (m, 1H), 2.28-2.22 (m, 2H), 2.01-1.96 (m, 2H), 1.46-1.36 (m, 2H), 1.02 (d, J = 6.6 Hz, 6H); LC/MS ESI (+): 381.3 (M+1).

### <Example 27> Synthesis of N-(1-isobutylpiperidin-4-yl)-4-(pyrazin-2-yl)-3,4-dihydroquinoxaline-1 (2H)-carboxamide

N-(1-isobutylpiperidin-4-yl)-4-(pyrazin-2-yl)-3,4-dihydroquinoxaline-1 (2H)-carboxamide was synthesized in the same manner as described in Example 26 using isobutyraldehyde instead of acetone.

Light yellow solid (yield: 82%) ¹H NMR (400 MHz, CDCl₃) δ 8.60 (d, J = 1.5 Hz, 1H), 8.20 (dd, J = 2.6, 1.5 Hz, 1H), 8.03 (d, J = 2.6 Hz, 1H), 7.52 (dd, J = 8.0, 1.5 Hz, 1H), 7.36 (dd, J = 7.9, 1.6 Hz, 1H), 7.16-7.02 (m, 2H), 5.05 (d, J = 7.7 Hz, 1H), 4.04-4.01 (m, 2H), 3.98-3.95 (m, 2H), 3.75-3.66 (m, 1H), 2.74-2.71 (m, 2H), 2.06-2.00 (m, 4H), 1.96-1.91 (m, 2H), 1.78-1.68 (m, 1H), 1.47-1.38 (m, 2H), 0.86 (d, J = 6.6 Hz, 6H); LC/MS ESI (+): 395.3 (M+1).

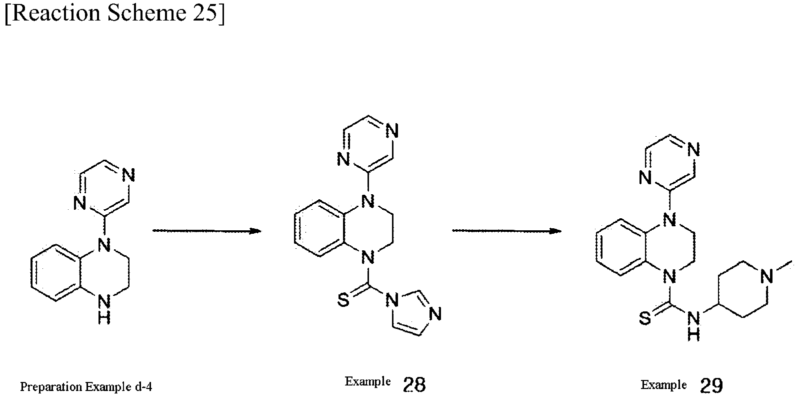

### <Example 28> Synthesis of imidazole-1-yl-(4-pyrazin-2-yl-2,3-dihydroquinoxaline-1-yl)methanethione

1-(pyrazin-2-yl)-1,2,3,4-tetrahydroquinoxaline (1.0 equiv.) obtained in Preparation Example d-4 and TEA (2.0 equiv.) were dissolved in dichloromethane (0.2 M), 1,1'-thiocarbonyldiimidazole (1.5 equiv.) was slowly added at 0 °C, and the resulting mixture was stirred for 6 hours at 0 °C. After terminating the reaction by adding water to the reaction solution, the resulting solution was extracted with dichloromethane. The residue obtained thereby was purified by silica column chromatography using a dichloromethane/methanol mixture to synthesize imidazole-1-yl-(4-pyrazin-2-yl-2,3-dihydroquinoxaline-1-yl)methanethione.

Yellow solid (yield: 85%) ¹H NMR (400 MHz, CDCl₃) δ 8.68 (d, J = 1.5 Hz, 1H), 8.26 (dd, J = 2.6, 1.5 Hz, 1H), 8.13 (d, J = 2.6 Hz, 1H), 7.82 (dd, J = 1.4, 1.1 Hz, 1H), 7.52 (dd, J = 8.2, 1.3 Hz, 1H), 7.18 (ddd, J = 8.5, 7.4, 1.4 Hz, 1H), 7.04 (dd, J = 1.4, 0.2 Hz, 1H), 6.93-6.88 (m, 2H), 6.61 (dd, J = 8.2, 1.4 Hz, 1H), 4.63 (t, J = 6.6 Hz, 2H), 4.23 (t, J = 6.6 Hz, 2H); LC/MS ESI (+): 323.2 (M+1).

### <Example 29> Synthesis of N-(1-methylpiperidin-4-yl)-4-(pyrazin-2-yl)-3,4-dihydroquinoxaline-1 (2H)-carbothioamide

The imidazole-1-yl-(4-pyrazin-2-yl-2,3-dihydroquinoxaline-1-yl)methanethione (1.0 equiv.) obtained in Example 28 and TEA (2.0 equiv.) were dissolved in DMF (0.2 M), 1-methylpiperidine-4-amine (2.0 equiv.) was added, and the resulting mixture was stirred for 24 hours at 70 °C. After terminating the reaction by adding water to the reaction solution, the resulting solution was extracted with dichloromethane. The residue obtained thereby was purified by silica column chromatography using a dichloromethane/methanol mixture to synthesize N-(1-methylpiperidin-4-yl)-4-(pyrazin-2-yl)-3,4-dihydroquinoxaline-1(2H)-carbothioamide.

Ivory solid (yield: 44%) ¹H NMR (400 MHz, CDCl₃) δ 8.61 (d, J = 1.5 Hz, 1H), 8.22 (dd, J = 2.6, 1.5 Hz, 1H), 8.08 (d, J = 2.6 Hz, 1H), 7.65 (dd, J = 8.4, 1.3 Hz, 1H), 7.27-7.21 (m, 2H), 7.08 (ddd, J = 8.4, 7.7, 1.4 Hz, 1H), 6.27 (d, J = 7.8 Hz, 1H), 4.62-4.58 (m, 2H), 4.39-4.32 (m, 1H), 4.07 (t, J = 6.3 Hz, 2H), 2.87-2.84 (m, 2H), 2.33 (s, 3H), 2.25-2.19 (m, 2H), 2.14-2.10 (m, 2H), 1.60-1.57 (m, 2H); LC/MS ESI (+): 369.2 (M+1).

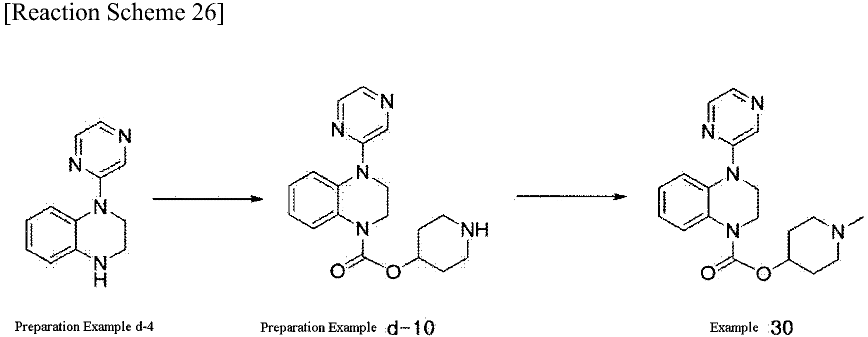

### <Preparation Example d-10> Preparation of piperidine-4-yl 4-(pyrazine-2-yl)-3,4-dihydroquinoxaline-1(2H)-carboxylate

The 1-(pyridine-2-ylmethyl)-1,2,3,4-tetrahydroquinoxyline (1.0 equiv.) obtained in Preparation Example d-4 was dissolved in dichloromethane (0.2 M), TEA (3.0 equiv.) and triphosgene (0.5 equiv.) were slowly added at 0 °C, and the resulting mixture was stirred for 10 minutes at 0 °C. NaH (6.0 equiv.) was dissolved in THF (0.2M) and 1-Boc-4-hydroxypiperidine (3.0 equiv.) was added, and the mixture was stirred for 10 minutes at room temperature and then slowly added to the reaction solution and stirred for 3 hours at room temperature. After terminating the reaction by adding water to the reaction solution, the resulting solution was extracted with dichloromethane. The organic layer obtained thereby was washed with brine, dried over MgSO₄, filtered, and concentrated under reduced pressure, thereby obtaining a concentrate. The concentrate was dissolved in dichloromethane (0.2 M), and a Boc group was deprotected using TFA (10.0 equiv.). After terminating the reaction by adding water to the reaction solution, the resulting solution was extracted with dichloromethane and concentrated under reduced pressure. The residue obtained thereby was purified by silica column chromatography using a dichloromethane/methanol mixture to prepare piperidine-4-yl 4-(pyrazine-2-yl)-3,4-dihydroquinoxaline-1(2H)-carboxylate.

Light yellow solid (yield: 70%); ¹H NMR (400 MHz, CDCl₃) δ 8.58 (d, J = 1.6 Hz, 1H), 8.17 (dd, J = 2.7, 1.6 Hz, 1H), 7.99 (d, J = 2.7 Hz, 1H), 7.89-7.85 (m, 1H), 7.39-7.37 (m, 1H), 7.12-7.04 (m, 2H), 4.95-4.88 (m, 1H), 4.09 (dd, J = 6.4, 5.6 Hz, 2H), 3.96 (dd, J = 6.4, 5.6 Hz, 2H), 3.09-3.03 (m, 2H), 2.78-2.71 (m, 2H), 2.01-1.94 (m, 2H), 1.68-1.60 (m, 2H); LC/MS ESI (+): 340.2 (M+1).

### <Example 30> Synthesis of 1-methylpiperidin-4-yl 4-(pyrazin-2-yl)-3,4-dihydroquinoxaline-1 (2H)-carboxylate

The piperidine-4-yl 4-(pyrazine-2-yl)-3,4-dihydroquinoxaline-1(2H)-carboxylate (1.0 equiv.) obtained in Preparation Example d-10 was dissolved in dichloromethane (0.2 M), sodium triacetoxyborohydride (3.0 equiv.) and formaldehyde (2.0 equiv.) were added, and the resulting mixture was stirred for 2 hours at room temperature. After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize 1-methylpiperidin-4-yl 4-(pyrazin-2-yl)-3,4-dihydroquinoxaline- I (2H)-carboxylate.

Yellow oil (yield: 76%) ¹H NMR (400 MHz, CDCl₃) δ 8.58 (d, J = 1.5 Hz, 1H), 8.17 (dd, J = 2.7, 1.5 Hz, 1H), 7.99 (d, J = 2.7 Hz, 1H), 7.90-7.87 (m, 1H), 7.39-7.37 (m, 1H), 7.12-7.04 (m, 2H), 4.09 (dd, J = 6.8, 5.2 Hz, 2H), 3.95 (dd, J = 6.8, 5.2 Hz, 2H), 2.64-2.58 (m, 2H), 2.36-2.32 (m, 2H), 2.31 (s, 3H), 2.04-1.98 (m, 2H), 1.86-1.78 (m, 2H); LC/MS ESI (+): 354.3 (M+1).

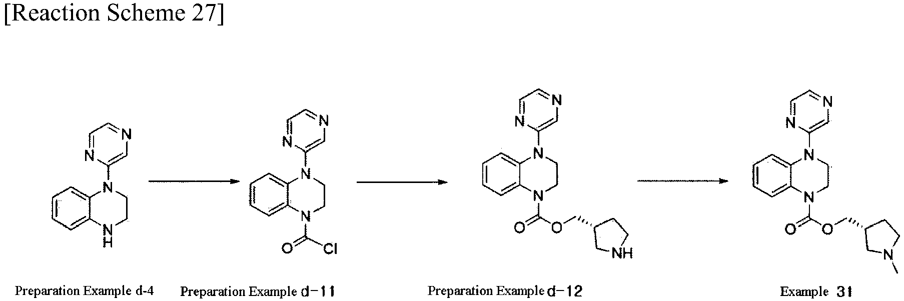

### <Preparation Example d-11> Preparation of 4-(pyrazin-2-yl)-3,4-dihydroquinoxaline-1(2H)-carbonyl chloride

The 1-(pyrazin-2-yl)-1,2,3,4-tetrahydroquinoxaline (1.0 equiv.) obtained in Preparation Example d-4 was dissolved in dichloromethane (0.3 M), TEA (3.0 equiv.) and triphosgene (0.5 equiv.) were slowly added at 0 °C, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare 4-(pyrazin-2-yl)-3,4-dihydroquinoxaline-1(2H)-carbonyl chloride.

Ivory solid (yield: 70%); ¹H NMR (400 MHz, CDCl₃) δ 8.64 (d, J = 1.6 Hz, 1H), 8.22 (dd, J = 2.7, 1.6 Hz, 1H), 8.07 (d, J = 2.7 Hz, 1H), 7.79-7.76 (m, 1H), 7.45 (dd, J = 8.2, 1.5 Hz, 1H), 7.23-7.19 (m, 1H), 7.13 (ddd, J = 8.2, 7.4, 1.6 Hz, 1H), 4.15 (s, 4H); LC/MS ESI (+): 275.1 (M+1).

### <Preparation Example d-12> Preparation of (R)-pyrrolidin-3-ylmethyl 4-(pyrazin-2-yl)-3,4-dihydroquinoxaline-1(2H)-carboxylate

The 4-(pyrazin-2-yl)-3,4-dihydroquinoxaline-1(2H)-carbonyl chloride (1.0 equiv.) obtained in Preparation Example d-11 was dissolved in dichloromethane (0.2 M). NaH (2.0 equiv.) was dissolved in THF (0.2 M) and (R)-1-Boc-(3-hydroxymethyl)pyrrolidine (1.5 equiv.) was added, and the mixture was stirred for 10 minutes at room temperature and then slowly added to the reaction solution and stirred for 3 hours at room temperature. After terminating the reaction by adding water to the reaction solution, the resulting solution was extracted with dichloromethane. The organic layer obtained thereby was washed with brine, dried over MgSO₄, filtered and concentration under reduced pressure, thereby obtaining a concentrate. The concentrate was dissolved in dichloromethane (0.2 M), and a Boc group was deprotected using TFA (10.0 equiv.). After terminating the reaction by adding water to the reaction solution, the resulting solution was extracted with dichloromethane and concentrated under reduced pressure. The residue obtained thereby was purified by silica column chromatography using a dichloromethane/methanol mixture to prepare (R)-pyrrolidin-3-ylmethyl 4-(pyrazin-2-yl)-3,4-dihydroquinoxaline-1 (2H)-carboxylate.

Light yellow oil (yield: 87%); ¹H NMR (400 MHz, CDCl₃) δ 8.58 (d, J = 1.5 Hz, 1H), 8.17 (dd, J = 2.7, 1.5 Hz, 1H), 7.99 (d, J = 2.7 Hz, 1H), 7.84-7.77 (m, 1H), 7.39-7.37 (m, 1H), 7.13-7.05 (m, 2H), 4.21-4.17 (m, 1H), 4.15-4.07 (m, 3H), 3.96-3.92 (m, 2H), 3.13-3.08 (m, 1H), 3.00-2.92 (m, 2H), 2.78-2.71 (m, 1H), 2.55-2.48 (m, 1H), 1.99-1.91 (m, 1H), 1.55-1.46 (m, 1H); LC/MS ESI (+): 340.2 (M+1).

### <Example 31> Synthesis of (R)-(1-methylpyrrolidin-3-yl)methyl 4-(pyrazin-2-yl)-3,4-dihydroquinoxaline-1(2H)-carboxylate

The (R)-pyrrolidin-3-ylmethyl 4-(pyrazin-2-yl)-3,4-dihydroquinoxaline-1(2H)-carboxylate (1.0 equiv.) obtained in Preparation Example d-12 was dissolved in dichloromethane (0.2 M), sodium triacetoxyborohydride (3.0 equiv.) and formaldehyde (2.0 equiv.) were added, and the resulting mixture was stirred for 2 hours at room temperature. After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize (R)-(1-methylpyrrolidin-3-yl)methyl 4-(pyrazin-2-yl)-3,4-dihydroquinoxaline-1(2H)-carboxylate.

Yellow oil (yield: 72%); ¹H NMR (400 MHz, CDCl₃) δ 8.58 (d, J = 1.5 Hz, 1H), 8.17 (dd, J = 2.7, 1.5 Hz, 1H), 7.99 (d, J = 2.7 Hz, 1H), 7.84-7.77 (m, 1H), 7.39-7.36 (m, 1H), 7.12-7.05 (m, 2H), 4.19-4.15 (m, 1H), 4.12-4.08 (m, 3H), 3.96-3.93 (m, 2H), 2.71-2.66 (m, 1H), 2.59-2.45 (m, 3H), 2.34 (s, 3H), 2.32-2.28 (m, 1H), 2.05-1.96 (m, 1H), 1.58-1.49 (m, 1H); LC/MS ESI (+): 354.3 (M+1).

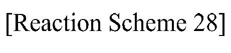

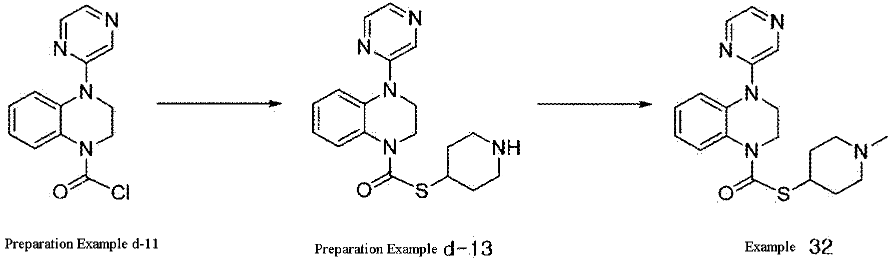

### <Preparation Example d-13> Preparation of (S)-(piperidin-4-yl)4-(pyrazin-2-yl)-3,4-dihydroquinoxaline-1(2H)-carboxylate

The 4-(pyrazin-2-yl)-3,4-dihydroquinoxaline-1(2H)-carbonyl chloride (1.0 equiv.) obtained in Preparation Example d-11 was dissolved in dichloromethane (0.2 M). NaH (2.0 equiv.) was dissolved in THF (0.2 M) and 1-Boc-4-mercapto-piperidine (1.5 equiv.) was added, and the resulting mixture was stirred for 10 minutes at room temperature and then slowly added to the reaction solution and stirred for 3 hours at room temperature. After terminating the reaction by adding water to the reaction solution, the resulting solution was extracted with dichloromethane. The organic layer obtained thereby was washed with brine, dried over MgSO₄, filtered, and concentrated under reduced pressure, thereby obtaining a concentrate. The concentrate was dissolved in dichloromethane (0.2 M), and a Boc group was deprotected using TFA (10.0 equiv.). After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize (S)-(piperidin-4-yl)4-(pyrazin-2-yl)-3,4-dihydroquinoxaline-1(2H)-carboxylate.

White solid (yield: 89%); ¹H NMR (400 MHz, CDCl₃) δ 8.63 (dd, J = 1.5, 0.4 Hz, 1H), 8.19 (dd, J = 2.7, 1.5 Hz, 1H), 8.02 (dd, J = 2.7, 0.4 Hz, 1H), 7.78 (d, J = 8.0 Hz, 1H), 7.44 (dd, J = 8.1, 1.5 Hz, 1H), 7.17 (ddd, J = 8.0, 7.4, 1.6 Hz, 1H), 7.10 (ddd, J = 8.0, 7.4, 1.5 Hz, 1H), 4.09 (ddd, J = 6.4, 5.6, 1.1 Hz, 2H), 4.01 (ddd, J = 6.4, 5.6, 1.1 Hz, 2H), 3.63-3.56 (m, 1H), 3.10-3.05 (m, 2H), 2.79-2.73 (m, 2H), 2.05-2.00 (m, 2H), 1.65-1.55 (m, 2H); LC/MS ESI (+): 356.3 (M+1).

### <Example 32> Synthesis of (S)-(1-methylpiperidin-4-yl)4-(pyrazin-2-yl)-3,4-dihydroquinoxaline-1 (2H)-carbothioate

The (S)-(piperidin-4-yl)4-(pyrazin-2-yl)-3,4-dihydroquinoxaline-1(2H)-carboxylate (1.0 equiv.) obtained in Preparation Example d-13 was dissolved in dichloromethane (0.2 M), sodium triacetoxyborohydride (3.0 equiv.) and formaldehyde (2.0 equiv.) were added, and the resulting mixture was stirred for 2 hours at room temperature. After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize (S)-(1-methylpiperidin-4-yl)4-(pyrazin-2-yl)-3,4-dihydroquinoxaline-1(2H)-carbothioate.

Ivory solid (yield: 48%); ¹H NMR (400 MHz, CDCl₃) δ 8.63 (d, J = 1.5 Hz, 1H), 8.19 (dd, J = 2.7, 1.5 Hz, 1H), 8.02 (d, J = 2.7 Hz, 1H), 7.78 (dd, J = 8.1, 1.6 Hz, 1H), 7.44 (dd, J = 8.1, 1.5 Hz, 1H), 7.16 (ddd, J = 8.1, 7.4, 1.6 Hz, 1H), 7.10 (ddd, J = 8.1, 7.4, 1.5 Hz, 1H), 4.09 (ddd, J = 6.3, 5.5, 1.1 Hz, 2H), 4.01 (ddd, J = 6.3, 5.5, 1.1 Hz, 2H), 3.49-3.44 (m, 1H), 2.78-2.73 (m, 1H), 2.26 (s, 3H), 2.17-2.12 (m, 2H), 2.05-2.01 (m, 2H), 1.76-1.66 (m, 3H); LC/MS ESI (+): 370.3 (M+1).

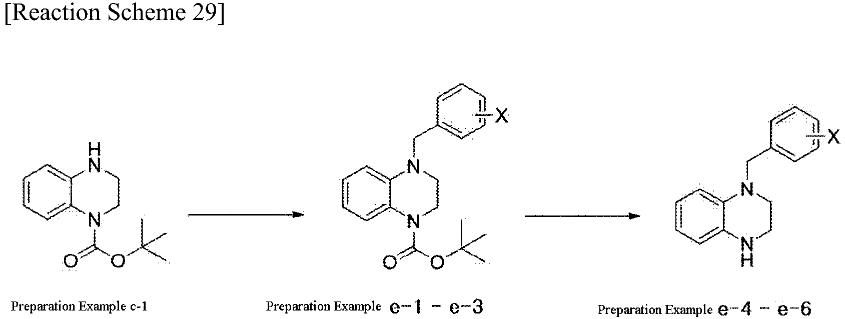

### <Preparation Method e> Preparation of benzyl or halogen-substituted benzyl-1,2,3,4-tetrahydroquinoxaline

Benzyl or halogen-substituted benzyl-1,2,3,4-tetrahydroquinoxaline according to the present invention was prepared through the procedures described in Preparation Examples e-1 to e-6 below.

### <Preparation Example e-1> Preparation of tert-butyl 4-(2-fluorobenzyl)-3,4-dihydroquinoxaline-1(2H)-carboxylate

Tert-butyl 4-(2-fluorobenzyl)-3,4-dihydroquinoxaline-1(2H)-carboxylate was prepared in the same manner as described in Preparation Example c-2 using 2-fluorobenzyl bromide instead of benzyl bromide.

Yellow oil (yield: 90%); ¹H NMR (400 MHz, CDCl₃) δ 7.47 (d, J = 7.9 Hz, 1H), 7.32-7.28 (m, 1H), 7.03 (ddd, J = 7.9, 2.4, 1.1 Hz, 1H), 6.96-6.89 (m, 3H), 6.66 (ddd, J = 8.3, 7.3, 1.4 Hz, 1H), 6.57 (dd, J = 8.3, 1.3 Hz, 1H), 4.51 (s, 2H), 3.85 (dd, J = 5.8, 4.5 Hz, 2H), 3.44 (dd, J = 5.8, 4.4 Hz, 2H), 1.53 (s, 9H).

### <Preparation Example e-2> Preparation of tert-butyl 4-(3-fluorobenzyl)-3,4-dihydroquinoxaline-1(2H)-carboxylate

Tert-butyl 4-(3-fluorobenzyl)-3,4-dihydroquinoxaline-1(2H)-carboxylate was prepared in the same manner as described in Preparation Example c-2 using 3-fluorobenzyl bromide instead of benzyl bromide.

Yellow oil (yield: 96%); ¹H NMR (400 MHz, CDCl₃) δ 7.49 (d, J = 7.9 Hz, 1H), 7.37-7.27 (m, 1H), 7.09-6.89 (m, 4H), 6.73-6.64 (m, 1H), 6.59 (dd, J = 8.3, 1.3 Hz, 1H), 4.53 (s, 2H), 3.87 (dd, J = 5.8, 4.5 Hz, 2H), 3.47 (dd, J = 5.8, 4.4 Hz, 2H), 1.55 (s, 9H).

### <Preparation Example e-3> Preparation of tert-butyl 4-(4-fluorobenzyl)-3,4-dihydroquinoxaline-1(2H)-carboxylate

Tert-butyl 4-(4-fluorobenzyl)-3,4-dihydroquinoxaline- I (2H)-carboxylate was prepared in the same manner as described in Preparation Example c-2 using 4-fluorobenzyl bromide instead of benzyl bromide.

Yellow oil (yield: 95%); ¹H NMR (400 MHz, CDCl₃) δ 7.47 (d, J = 8.1 Hz, 1H), 7.23-7.18 (m, 2H), 7.04-6.98 (m, 2H), 6.92 (ddd, J = 8.3, 7.2, 1.6 Hz, 1H), 6.66 (ddd, J = 8.1, 7.2, 1.4 Hz, 1H), 6.60 (dd, J = 8.3, 1.4 Hz, 1H), 4.48 (s, 2H), 3.84-3.82 (m, 2H), 3.43-3.40 (m, 2H), 1.53 (s, 9H).

### <Preparation Example e-4> Preparation of 1-(2-fluorobenzyl)-1,2,3,4-tetrahydroquinoxaline

1-(2-fluorobenzyl)-1,2,3,4-tetrahydroquinoxaline was prepared by deprotecting the Boc group of the tert-butyl 4-(2-fluorobenzyl)-3,4-dihydroquinoxaline-1(2H)-carboxylate obtained in Preparation Example e-1 with TFA.

Gray solid (yield: 96%); ¹H NMR (400 MHz, CD₃OD) δ 7.33-7.23 (m, 1H), 7.27-7.23 (m, 1H), 7.11-7.06 (m, 1H), 6.57-6.53 (m, 2H), 6.52-6.50 (m, 1H), 6.48-6.45 (m, 2H), 4.48 (s, 2H), 3.43-3.41 (m, 2H), 3.37-3.35 (m, 2H); LC/MS ESI (+): 243.1 (M+1).

### <Preparation Example e-5> Preparation of 1-(3-fluorobenzyl)-1,2,3,4-tetrahydroquinoxaline

1-(3-fluorobenzyl)-1,2,3,4-tetrahydroquinoxaline was prepared by deprotecting the the Boc group of the tert-butyl 4-(3-fluorobenzyl)-3,4-dihydroquinoxaline-1(2H)-carboxylate obtained in Preparation Example e-2 with TFA.

yellow solid (yield: 96%); ¹H NMR (400 MHz, CD₃OD) δ 7.34-7.28 (m, 1H), 7.13-7.10 (m, 1H), 7.03-7.00 (m, 1H), 6.96-6.91 (m, 1H), 6.57-6.52 (m, 2H), 6.51-6.46 (m, 1H), 6.45-6.42 (m, 1H), 4.46 (s, 2H), 3.42-3.35 (m, 4H); LC/MS ESI (+): 243.1 (M+1).

### <Preparation Example e-6> Preparation of 1-(4-fluorobenzyl)-1,2,3,4-tetrahydroquinoxaline

1-(4-fluorobenzyl)-1,2,3,4-tetrahydroquinoxaline was prepared by by deprotecting the Boc group of the tert-Butyl 4-(4-fluorobenzyl)-3,4-dihydroquinoxaline-1(2H)-carboxylate obtained in Preparation Example e-3 with TFA.

Light Brown solid (yield: 93%); ¹H NMR (400 MHz, CD₃OD) δ 7.32-7.28 (m, 2H), 7.05-6.99 (m, 2H), 6.55-6.45 (m, 4H), 4.42 (s, 2H), 3.38-3.34 (m, 4H); LC/MS ESI (+): 243.1 (M+1).

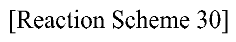

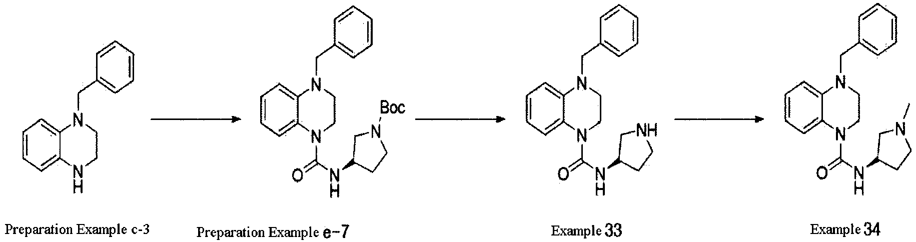

### <Preparation Example e-7> Preparation of tert-butyl (R)-3-(4-benzyl-1,2,3,4-tetrahydroquinoxaline- 1 -carboxamido)pyrrolidin-1-carboxylate

The 1-benzyl-1,2,3,4-tetrahydroquinoxaline (1.0 equiv.) obtained in Preparation Example c-3 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (R)-(+)-1-Boc-3-aminopyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (R)-3-(4-benzyl-1,2,3,4-tetrahydroquinoxaline-1-carboxamido)pyrrolidin-1-carboxylate.

Brown solid (yield: 100%); ¹H NMR (400 MHz, CDCl₃) δ 7.36-7.30 (m, 2H), 7.25-7.19 (m, 3H), 7.08 (d, J = 7.8 Hz, 1H), 6.99-6.96 (m, 1H), 6.69 (d, J = 8.3 Hz, 1H), 6.67-6.62 (m, 1H), 5.34 (d, J = 6.9 Hz, 1H), 5.08-5.03 (m, 1H), 4.56 (s, 2H), 4.43-4.37 (m, 1H), 3.95-3.84 (m, 1H), 3.65-3.63 (m, 1H), 3.46-3.39 (m, 3H), 3.18-3.12, (m, 1H), 3.06-3.01 (m, 1H), 1.94-1.64 (m, 1H), 1.51-1.48 (m, 1H), 1.45 (s, 9H).

### <Example 33> Synthesis of (R)-4-benzyl-N-(pyrrolidin-3-yl)-3,4-dihydroquinoxaline-1 (2H)-carboxamide

(R)-4-benzyl-N-(pyrrolidin-3-yl)-3,4-dihydroquinoxaline-1(2H)-carboxamide was synthesized by deprotecting the Boc group of the tert-butyl (R)-3-(4-benzyl-1,2,3,4-tetrahydroquinoxaline-1-carboxamido)pyrrolidin-1-carboxylate obtained in Preparation Example e-7 with TFA.

Light yellow solid (yield: 49%); ¹H NMR (400 MHz, CDCl₃) δ 7.34-7.28 (m, 2H), 7.27-7.24 (m, 1H), 7.21-7.19 (m, 2H), 7.12 (dd, J = 7.8, 1.6 Hz, 1H), 6.97 (ddd, J = 8.3, 7.3, 1.6 Hz, 1H), 6.69-6.62 (m, 2H), 5.39 (d, J = 6.9 Hz, 1H), 4.55 (s, 2H), 4.43-4.27 (m, 1H), 3.91-3.81 (m, 2H), 3.43 (t, J = 5.2 Hz, 2H), 3.19-3.15 (m, 1H), 3.03-2.97 (m, 1H), 2.93-2.88 (m, 1H), 2.77-2.73 (m, 1H), 2.18-2.10 (m, 1H), 1.59-1.51 (m, 1H); LC/MS ESI (+): 337.1 (M+1).

### <Example 34> Synthesis of (R)-4-Benzyl-N-(1-methylpyrrolidin-3-yl)-3,4-dihydroquinoxaline-1 (2H)-carboxamide

The (R)-4-benzyl-N-(pyrrolidin-3-yl)-3,4-dihydroquinoxaline-1(2H)-carboxamide (1.0 equiv.) obtained in Example 33 was dissolved in dichloromethane (0.2 M), sodium triacetoxyborohydride (3.0 equiv.) and formaldehyde (2.0 equiv.) were added, and the resulting mixture was stirred for 2 hours at room temperature. After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize (R)-4-Benzyl-N-(1-methylpyrrolidin-3-yl)-3,4-dihydroquinoxaline-1(2H)-carboxamide.

Light yellow oil (yield: 27%); ¹H NMR (400 MHz, CD₃OD) 7.32-7.27 (m, 2H), 7.24-7.2 (m, 3H), 7.17 (d, J = 8.0, 1.6 Hz, 1H), 6.95 (ddd, J = 8.3, 7.3, 1.6 Hz, 1H), 6.72 (dd, J = 8.3, 1.3 Hz, 1H), 6.64 (ddd, J = 8.0, 7.3, 1.3 Hz, 1H), 4.57 (s, 2H), 4.41-4.35 (m, 1H), 3.85-3.74 (m, 2H), 3.44 (t, J = 5.2 Hz, 2H), 3.01-2.95 (m, 1H), 2.89-2.85 (m, 1H), 2.77-2.73 (m, 1H), 2.60-2.54 (m, 1H), 2.47 (s, 3H), 2.39-2.31 (m, 1H), 1.79-1.70 (m, 1H); LC/MS ESI (+): 351.0 (M+1).

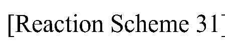

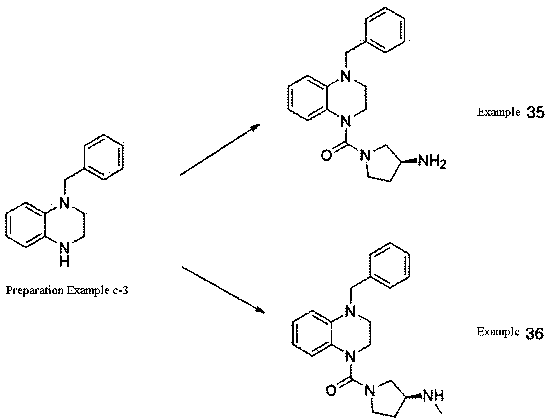

### <Example 35> Synthesis of (S)-(3-aminopyrrolidin-1-yl)(4-benzyl-3,4-dihydroquinoxaline-1 (2H)-yl)methanone

The 1-benzyl-1,2,3,4-tetrahydroquinoxaline (1.0 equiv.) obtained in Preparation Example c-3 was dissolved in dichloromethane (0.4 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-3-(Boc-amino)pyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The obtained concentrate was dissolved in dichloromethane (0.5 M), TFA (10.0 equiv.) was added, and then the resulting solution was stirred for 24 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (S)-(3-aminopyrrolidin-1-yl)(4-benzyl-3,4-dihydroquinoxaline-1 (2H)-yl)methanone.

Yellow oil (yield: 23%); ¹H NMR (400 MHz, CD₃OD) δ 7.32-7.20 (m, 5H), 6.86-6.79 (m, 2H), 6.65 (dd, J = 8.0, 1.3 Hz, 1H), 6.58 (ddd, J = 8.0, 7.3, 1.4 Hz, 1H), 4.56 (s, 2H), 3.77-3.72 (m, 1H), 3.70-3.62 (m, 1H), 3.55-3.49 (m, 5H), 3.41-3.36 (m, 1H), 3.05-3.00 (m, 1H), 2.07-1.95 (m, 1H), 1.71-1.63 (m, 1H); LC/MS ESI (+): 337.1 (M+1).

### <Example 36> Synthesis of (S)-(4-benzyl-3,4-dihydroquinoxaline-1(2H)-yl)(3-(methylamino)pyrrolidin-1-yl)methanone

The 1-benzyl-1,2,3,4-tetrahydroquinoxaline (1.0 equiv.) obtained in Preparation Example c-3 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-3-(Boc-amino)pyrrolidine (1.2 equiv.) were added to the reaction solution, and the resulting solution was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The ivory solid obtained thereby was dissolved in THF (0.3 M), NaH (1.5 equiv.) and iodomethane (1.0 equiv.) were added, and the resulting mixture was stirred for 3 hours at room temperature. After terminating the reaction by adding water to the reaction solution, the resulting solution was extracted with dichloromethane, washed with brine, dried over MgSO₄, filtered, and concentrated under reduced pressure. The light brown oil obtained thereby was dissolved in dichloromethane (0.5 M), TFA (10.0 equiv.) was added, and the resulting mixture was stirred for 3 hours at room temperature, water was added to terminate the reaction, and then the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (S)-(4-benzyl-3,4-dihydroquinoxaline-1(2H)-yl)(3-(methylamino)pyrrolidin-1-yl)methanone.

Yellow oil (yield: 47%); ¹H NMR (400 MHz, CD₃OD) δ 7.32-7.20 (m, 5H), 6.85-6.80 (m, 2H), 6.66 (dd, J = 8.2, 1.3 Hz, 1H), 6.58 (ddd, J = 8.0, 7.3, 1.4 Hz, 1H), 4.57 (s, 2H), 3.79-3.74 (m, 1H), 3.70-3.61 (m, 1H), 3.54-3.49 (m, 5H), 3.40-3.36 (m, 1H), 3.20-3.10 (m, 2H), 2.33 (s, 3H), 2.15-2.03 (m, 1H), 1.76-1.67 (m, 1H); LC/MS ESI (+): 351.3 (M+1).

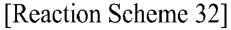

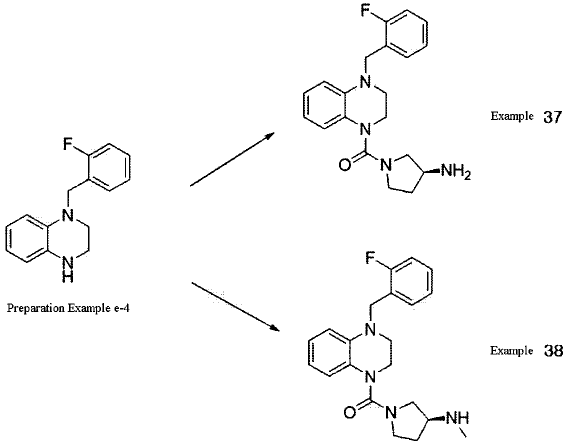

### <Example 37> Synthesis of (S)-(3-aminopyrrolidin-1-yl)(4-(2-fluorobenzyl)-3,4-dihydroquinoxaline-1(2H)-yl)methanone

(*S*)-(3-aminopyrrolidin-1-yl)(4-(2-fluorobenzyl)-3,4-dihydroquinoxaline-1(2H)-yl)methanone was synthesized in the same manner as described in Example 35 using the 1-(2-fluorobenzyl)-1,2,3,4-tetrahydroquinoxaline obtained in Preparation Example e-4 instead of 1-benzyl-1,2,3,4-tetrahydroquinoxaline.

Light yellow oil (yield: 56%); ¹H NMR (400 MHz, CDCl₃) δ 7.25-7.21 (m, 1H), 7.18-7.14 (m, 1H), 7.09-7.04 (m, 2H), 6.90 (dd, J = 7.9, 1.5 Hz, 1H), 6.84 (ddd, J = 8.2, 7.3, 1.5 Hz, 1H), 6.62 (ddd, J = 8.2, 7.3, 1.5 Hz, 1H), 6.58 (dd, J = 8.2, 1.5 Hz, 1H), 4.55 (s, 2H), 3.84-3.74 (m, 2H), 3.54-3.47 (m, 4H), 3.42-3.35 (m, 1H), 3.03-2.98 (m, 1H), 2.07-1.99 (m, 1H), 1.68-1.58 (m, 1H), 0.89-0.78 (m, 1H); LC/MS ESI (+): 355.3 (M+1).

### <Example 38> Synthesis of (S)-(4-(2-fluorobenzyl)-3,4-dihydroquinoxaline-1(2H)-yl)(3-(methylamino)pyrrolidin-1-yl)methanone

(S)-(4-(2-fluorobenzyl)-3,4-dihydroquinoxaline-1(2H)-yl)(3-(methylamino)pyrrolidin-1-yl)methanone was synthesized in the same manner as described in Example 36 using the 1-(2-fluorobenzyl)-1,2,3,4-tetrahydroquinoxaline obtained in Preparation Example e-4 instead of 1-benzyl-1,2,3,4-tetrahydroquinoxaline.

Orange oil (yield: 83%); ¹H NMR (400 MHz, CDCl₃) δ 7.25-7.21 (m, 1H), 7.16 (ddd, J = 8.8, 7.4, 1.7 Hz, 1H), 7.09-7.03 (m, 2H), 6.89 (dd, J = 7.9, 1.5 Hz, 1H), 6.83 (ddd, J = 8.8, 7.4, 1.5 Hz, 1H), 6.63-6.56 (m, 2H), 4.58 (s, 2H), 3.84-3.73 (m, 2H), 3.53 (t, J = 5.5 Hz, 2H), 3.50-3.43 (m, 2H), 3.39-3.34 (m, 1H), 3.22-3.16 (m, 1H), 3.13-3.09 (m, 1H), 2.39 (s, 3H), 2.04-1.97 (m, 1H), 1.72-1.64 (m, 1H); LC/MS ESI (+): 369.3 (M+1).

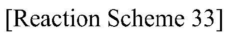

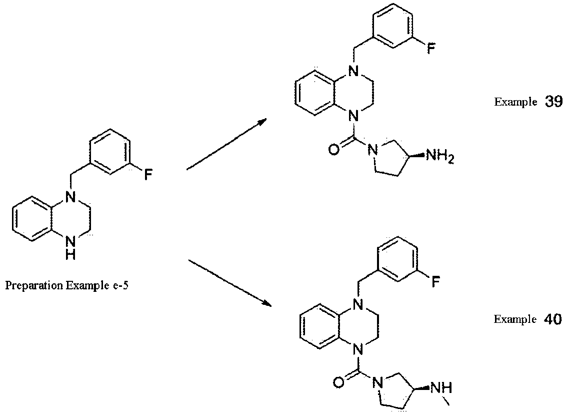

### <Example 39> Synthesis of (S)-(3-aminopyrrolidin-1-yl)(4-(3-fluorobenzyl-3,4-dihydroquinoxaline-1 (2H)-yl)methanone

(S)-(3-Aminopyrrolidin-1-yl)(4-(2-fluorobenzyl)-3,4-dihydroquinoxaline-1(2H)-yl)methanone was synthesized in the same manner as described in Example 35 using the 1-(3-fluorobenzyl)-1,2,3,4-tetrahydroquinoxaline obtained in Preparation Example e-5 instead of 1-benzyl-1,2,3,4-tetrahydroquinoxaline.

Light yellow oil (yield: 56%); ¹H NMR (400 MHz, CDCl₃) δ 7.31-7.27 (m, 2H), 7.03-7.00 (m, 1H), 6.95-6.89 (m, 2H), 6.83 (ddd, J = 8.1, 7.3, 1.5 Hz, 1H), 6.63 (ddd, J = 8.0, 7.3, 1.3 Hz, 1H), 6.56 (dd, J = 8.1, 1.3 Hz, 1H), 4.52 (s, 2H), 3.84-3.74 (m, 2H), 3.55-3.47 (m, 5H), 3.42-3.36 (m, 1H), 3.01-2.98 (m, 1H), 2.07-1.99 (m, 1H), 1.65-1.60 (m, 1H); LC/MS ESI (+): 355.3 (M+1).

### <Example 40> Synthesis of (S)-(4-(3-fluorobenzyl)-3,4-dihydroquinoxaline-1(2H)-yl)(3-(methylamino)pyrrolidin-1-yl)methanone

(S)-(4-(3-fluorobenzyl)-3,4-dihydroquinoxaline-1(2H)-yl)(3-(methylamino)pyrrolidin-1-yl)methanone was synthesized in the same manner as described in Example 36 using the 1-(3-fluorobenzyl)-1,2,3,4-tetrahydroquinoxaline obtained in Preparation Example e-5 instead of 1-benzyl-1,2,3,4-tetrahydroquinoxaline.

Light yellow solid (yield: 78%); ¹H NMR (400 MHz, CDCl₃) δ 7.31-7.27 (m, 1H), 7.03-7.00 (m, 1H), 6.95-6.89 (m, 3H), 6.83 (ddd, J = 8.1, 7.3, 1.5 Hz, 1H), 6.62 (ddd, J = 8.3, 7.3, 1.4 Hz, 1H), 6.56 (dd, J = 8.3, 1.4 Hz, 1H), 4.52 (s, 2H), 3.85-3.73 (m, 2H), 3.52 (t, J = 5.5 Hz, 2H), 3.50-3.43 (m, 2H), 3.38-3.34 (m, 1H), 3.22-3.17 (m, 1H), 3.13-3.09 (m, 1H), 2.40 (s, 3H), 2.06-1.98 (m, 1H), 1.73-1.64 (m, 1H); LC/MS ESI (+): 369.1 (M+1).

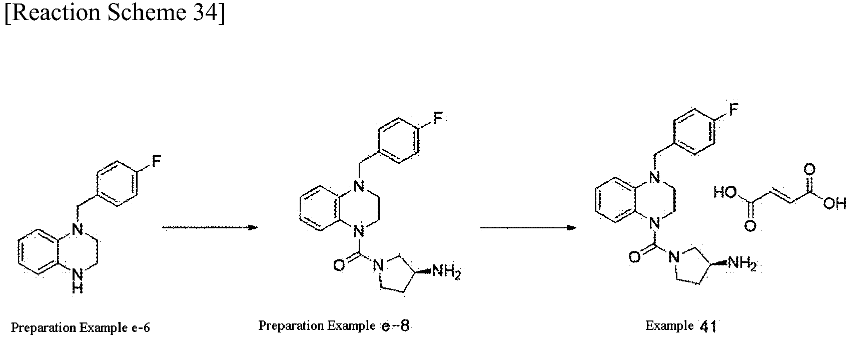

### <Preparation Example e-8> Preparation of (S)-(3-aminopyrrolidin-1-yl)(4-(4-fluorobenzyl)-3,4-dihydroquinoxaline-1(2H)-yl)methanon

(S)-(3-Aminopyrrolidin-1-yl)(4-(4-fluorobenzyl)-3,4-dihydroquinoxaline-1(2H)-yl)methanone was prepared in the same manner as described in Example 35 using the 1-(4-fluorobenzyl)-1,2,3,4-tetrahydroquinoxaline obtained in Preparation Example e-6 instead of 1-benzyl-1,2,3,4-tetrahydroquinoxaline.

White solid (yield: 84%); ¹H NMR (400 MHz, CDCl₃) δ 7.22-7.17 (m, 2H), 7.04-6.97 (m, 2H). 6.90 (dd, J = 7.9, 1.5 Hz, 1H), 6.84 (ddd, J = 8.1, 7.4, 1.5 Hz, 1H), 6.64-6.58 (m, 2H), 4.49 (s, 2H), 3.82-3.72 (m, 2H), 3.55-3.46 (m, 4H), 3.41-3.35 (m, 1H), 3.01-2.97 (m, 1H), 2.06-1.98 (m, 1H), 1.64-1.59 (m, 1H), 0.89-0.79 (m, 1H); LC/MS ESI (+): 355.1 (M+1).

### <Example 41> Synthesis of (S)-(3-aminopyrrolidin-1-yl)(4-(4-fluorobenzyl)-3,4-dihydroquinoxaline-1(2H)-yl)methanone fumaric acid salt

The (S)-(3-aminopyrrolidin-1-yl)(4-(4-fluorobenzyl)-3,4-dihydroquinoxaline-1(2H)-yl)methanone (1.0 equiv.) obtained in Preparation Example e-8 was dissolved in ethanol (0.3 M) and stirred for 10 minutes at room temperature. Fumaric acid (1.0 equiv.) was added to the reaction solution, and stirred for 10 minutes at 70 °C until it dissolved to form a clear solution. After cooling the resulting solution again, it was stirred for 1 hour at room temperature. When a solid was formed, ethyl acetate (0.4 M) was added and then the resulting solution was stirred for 2 hours at room temperature. The produced solid was filtered with ethyl acetate, washed and dried to synthesize (S)-(3-aminopyrrolidin-1-yl)(4-(4-fluorobenzyl)-3,4-dihydroquinoxaline-1(2H)-yl)methanone fumaric acid salt.

White solid (yield: 96%); ¹H NMR (400 MHz, CD₃OD) δ 7.30-7.25 (m, 2H), 7.18-7.02 (m, 2H), 6.90 (dd, J = 8.0, 1.5 Hz, 1H), 6.86 (ddd, J = 8.6, 7.3, 1.5 Hz, 1H), 6.68 (s, 2H), 6.67-6.65 (m, 1H), 6.61 (ddd, J = 8.6, 7.3, 1.3 Hz, 1H), 4.55 (s, 2H), 3.86-3.80 (m, 1H), 3.78-3.74 (m, 1H), 3.71-3.65 (m, 2H), 3.57-3.50 (m, 3H), 3.48-3.42 (m, 1H), 3.40-3.36 (m, 1H), 2.31-2.24 (m, 1H), 2.01-1.93 (m, 1H).

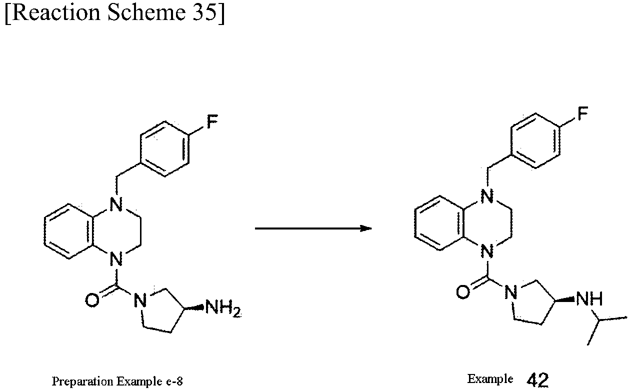

### <Example 42> Synthesis of (S)-(4-(4-fluorobenzyl)-3,4-dihydroquinoxaline-1(2H)-yl)(3-(isopropylamino)pyrrolidin-1-yl)methanone

The (S)-(3-aminopyrrolidin-1-yl)(4-(4-fluorobenzyl)-3,4-dihydroquinoxaline-1(2H)-yl)methanone (1.0 equiv.) obtained in Preparation Example e-8 was dissolved in dichloromethane (0.2 M), sodium triacetoxyborohydride (3.0 equiv.) and acetone (2.0 equiv.) were added, and the resulting mixture was stirred for 2 hours at room temperature. After terminating the reaction by adding water to the reaction solution, the resulting solution was extracted with dichloromethane and concentrated under reduced pressure. The residue obtained thereby was purified by silica column chromatography using a dichloromethane/methanol mixture to synthesize (S)-(4-(4-fluorobenzyl)-3,4-dihydroquinoxaline-1(2H)-yl)(3-(isopropylamino)pyrrolidin-1-yl)methanone.

White solid (yield: 93%); ¹H NMR (400 MHz, CDCl₃) 7.22-7.17 (m, 2H), 7.03-6.97 (m, 2H), 6.88 (dd, J = 7.9, 1.5 Hz, 1H), 6.83 (ddd, J = 8.0, 7.4, 1.5 Hz, 1H), 6.63-6.57 (m, 2H), 4.49 (s, 2H), 3.89-3.84 (m, 1H), 3.68-3.62 (m, 1H), 3.52-3.42 (m, 4H), 3.40-3.33 (m, 1H), 3.01-2.97 (m, 1H), 2.86-2.79 (m, 1H), 2.09-2.02 (m, 1H), 1.65-1.56 (m, 1H), 1.04 (d, J = 6.2 Hz, 3H), 1.02 (d, J = 6.2 Hz, 3H); LC/MS ESI (+): 397.3 (M+1).

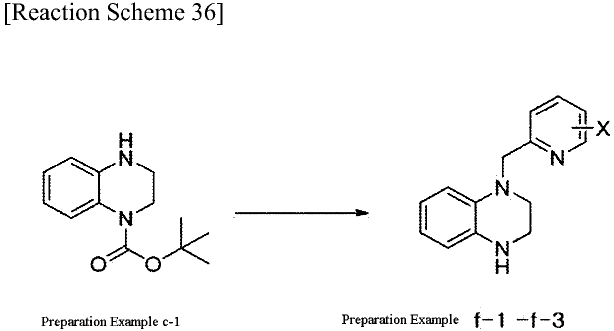

### <Preparation Method f> Preparation of unsubstituted or halogen-substituted 1-(pyridin-2-ylmethyl)-1,2,3,4-tetrahydroquinoxaline

Unsubstituted or halogen-substituted 1-(pyridin-2-ylmethyl)-1,2,3,4-tetrahydroquinoxaline according to the present invention was prepared through the procedures described in Preparation Examples f-1 to f-3 below.

### <Preparation Example f-1> Preparation of 1-(pyridin-2-ylmethyl)-1,2,3,4-tetrahydroquinoxaline

The tert-butyl 3,4-dihydroquinoxaline-1(2H)-carboxylate (1.0 equiv.) obtained in Preparation Example c-1 was dissolved in 1,2-dichloroethane (0.3 M), sodium triacetoxyborohydride (1.5 equiv.) and 2-pyridinecarboxaldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The obtained concentrate was dissolved in dichloromethane (20.0 mL), TFA (10.3 mL, 134.76 mmol) was added, and the resulting mixture was stirred for 24 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare 1-(pyridin-2-ylmethyl)-1,2,3,4-tetrahydroquinoxaline.

Light brown solid (yield: 87%); ¹H NMR (400 MHz, CD₃CN) δ 8.53 (ddd, J = 4.9, 1.9, 1.0 Hz, 1H), 7.66 (ddd, J = 7.9, 7.7, 1.8 Hz, 1H), 7.28 (ddd, J = 8.8, 7.7, 1.0 Hz, 1H), 7.19 (ddd, J = 7.4, 4.9, 1.0 Hz, 1H), 6.46-6.39 (m, 3H), 6.35-6.32 (m, 1H), 4.49 (s, 2H), 4.31 (brs, NH), 3.50-3.48 (m, 2H), 3.40-3.38 (m, 2H); LC/MS ESI (+): 226.1 (M+1).

### <Preparation Example f-2> Preparation of 1-((5-fluoropyridin-2-yl)methyl)-1,2,3,4-tetrahydroquinoxaline

1-((5-fluoropyridin-2-yl)methyl)-1,2,3,4-tetrahydroquinoxaline was prepared in the same manner as described in Preparation Example f-1 using 5-fluoro-2-formylpyridine instead of 2-pyridinecarboxaldehyde.

Yellow oil (yield: 78%); ¹H NMR (400 MHz, CD₃OD) δ 8.42 (ddd, J = 4.7, 3.0, 0.8 Hz, 1H), 7.53 (ddd, J = 9.7, 8.7, 3.0 Hz, 1H), 7.40 (ddd, J = 8.7, 4.7, 0.8 Hz, 1H), 6.57-6.53 (m, 1H), 6.51-6.46 (m, 2H), 6.35-6.31 (m, 1H), 4.50 (s, 2H), 3.49-3.47 (m, 2H), 3.41-3.38 (m, 2H); LC/MS ESI (+): 244.1 (M+1).

### <Preparation Example f-3> Preparation of 1-((3-fluoropyridin-2-yl)methyl)-1,2,3,4-tetrahydroquinoxaline

1-((3-fluoropyridin-2-yl)methyl)-1,2,3,4-tetrahydroquinoxaline was prepared in the same manner as described in Preparation Example f-1 using 3-fluoropyridine-2-carbaldehyde instead of 2-pyridinecarboxaldehyde.

Green oil (yield: 38%); ¹H NMR (400 MHz, CDCl₃) δ 8.38 (ddd, J = 4.7, 3.0, 1.5 Hz, 1H), 7.37 (ddd, J = 9.7, 8.3, 1.4 Hz, 1H), 7.23-7.18 (m, 1H), 6.82 (dd, J = 8.0, 1.4 Hz, 1H), 6.65-6.61 (m, 1H), 6.55 (ddd, J = 7.6, 7.4, 1.4 Hz, 1H), 6.49 (dd, J = 7.6, 1.7 Hz, 1H), 4.59 (d, J = 2.0 Hz, 2H), 3.60 (t, J = 4.7 Hz, 2H), 3.43 (t, J = 4.7 Hz, 2H); LC/MS ESI (+): 244.1 (M+1).

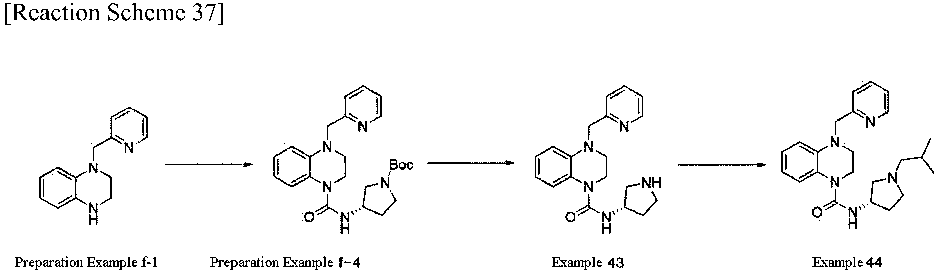

### <Preparation Example f-4> Preparation of tert-butyl (S)-3-(4-(pyridin-2-ylmethyl)-1,2,3,4-tetrahydroquinoxaline-1-carboxamido)pyrrolidin-1-carboxylate

The 1-(pyridin-2-ylmethyl)-1,2,3,4-tetrahydroquinoxaline (1.0 equiv.) obtained in Preparation Example f-1 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-1-Boc-3-aminopyrrolidine (1.2 equiv.) were added to the reaction solution, and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to prepare tert-butyl (S)-3-(4-(pyridin-2-ylmethyl)-1,2,3,4-tetrahydroquinoxaline-1-carboxamido)pyrrolidin-1-carboxylate.

Yellow solid (yield: 90%); ¹H NMR (400 MHz, CD₃OD) δ 8.53-8.51 (m, 1H), 7.76 (ddd, J = 7.9, 7.4, 1.4 Hz, 1H), 7.32-7.28 (m, 1H), 7.15 (dd, J = 7.6, 1.3 Hz, 1H), 6.91 (ddd, J = 8.8, 7.4, 1.8 Hz, 1H), 6.64 (ddd, J = 7.6, 4.9, 1.3 Hz, 1H), 6.60 (dd, J = 8.8, 1.4 Hz, 1H), 4.65 (s, 2H), 4.31-4.28 (m, 1H), 3.86-3.77 (m, 2H), 3.63-3.58 (m, 1H), 3.54 (t, J = 5.5 Hz, 2H), 3.42-3.34 (m, 2H), 3.21-3.18 (m, 1H), 2.18-2.10 (m, 1H), 1.92-1.84 (m, 1H), 1.46 (s, 9H).

### <Example 43> Synthesis of ((S)-4-(pyridin-2ylmethyl)-N-(pyrrolidin-3-yl)-3,4-dihydroquinoxaline-1 (2H)-carboxamide

((S)-4-(pyridin-2ylmethyl)-N-(pyrrolidin-3-yl)-3,4-dihydroquinoxaline-1(2H)-carboxamide was synthesized by deprotecting the Boc group of the tert-butyl (S)-3-(4-(pyridin-2-ylmethyl)-1,2,3,4-tetrahydroquinoxaline-1-carboxamido)pyrrolidin-1-carboxylate obtained in Preparation Example f-4 with TFA.

Yellow oil (yield: 92%); ¹H NMR (400 MHz, CD₃OD) δ 8.52 (ddd, J = 4.5, 1.7, 1.0 Hz, 1H), 7.77 (ddd, J = 7.9, 7.4, 1.8 Hz, 1H), 7.32-7.29 (m, 2H), 7.20 (dd, J = 7.9, 1.6 Hz, 1H), 6.93 (ddd, J = 8.3, 7.4, 1.6 Hz, 1H), 6.66 (ddd, J = 8.8, 7.7, 1.7 Hz, 1H), 6.60 (dd, J = 8.3, 1.8 Hz, 1H), 4.66 (s, 2H), 4.32-4.26 (m, 1H), 3.84 (t, J = 5.8 Hz, 2H), 3.54 (t, J = 5.8 Hz, 2H), 3.19-3.15 (m, 1H), 3.12-3.06 (m, 1H), 2.98-2.92 (m, 1H), 2.87-2.83 (m, 1H), 2.21-2.14 (m, 1H), 1.78-1.71 (m, 1H); LC/MS ESI (+): 338.2 (M+1).

### <Example 44> Synthesis of (S)-N-(1-Isobutylpyrrolidin-3-yl)-4-(pyridin-2-ylmethyl)-3,4-dihydroquinoxaline-1(2H)-carboxamide

The ((S)-4-(pyridin-2ylmethyl)-N-(pyrrolidin-3-yl)-3,4-dihydroquinoxaline-1(2H)-carboxamide (1.0 equiv.) obtained in Example 43 was dissolved in dichloromethane (0.3 M), sodium triacetoxyborohydride (1.5 equiv.) and isobutyraldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (S)-N-(1-Isobutylpyrrolidin-3-yl)-4-(pyridin-2-ylmethyl)-3,4-dihydroquinoxaline-1(2H)-carboxamide.

Light yellow oil (yield: 65%); ¹H NMR (400 MHz, CD₃OD) δ 8.52 (ddd, J = 4.9, 1.8, 0.9 Hz, 1H), 7.76 (ddd, J = 7.9, 7.7, 1.7 Hz, 1H), 7.32-7.27 (m, 2H), 7.17 (dd, J = 7.9, 1.5 Hz, 1H), 6.93 (ddd, J = 8.3, 7.7, 1.5 Hz, 1H), 6.66 (ddd, J = 8.8, 7.6, 1.8 Hz, 1H), 6.60 (dd, J = 8.3, 1.7 Hz, 1H), 4.66 (s, 2H), 4.36-4.30 (m, 1H), 3.91-3.85 (m, 1H), 3.83-3.77 (m, 1H), 3.53 (t, J = 5.2 Hz, 2H), 2.84-2.80 (m, 1H), 2.58-2.54 (m, 2H), 2.29-2.22 (m, 4H), 1.79-1.70 (m, 1H), 1.65-1.58 (m, 1H), 0.91 (d, J = 6.6 Hz, 6H); LC/MS ESI (+): 394.2 (M+1).

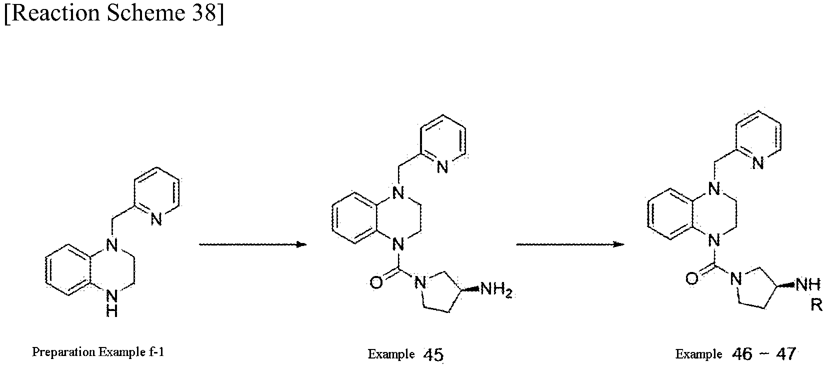

### <Example 45> Synthesis of (S)-(3-aminopyrrolidin-1-yl)(4-(pyridin-2-ylmethyl)-3,4-dihydroquinoxaline-1(2H)-yl)methanone

The 1-(pyridin-2-ylmethyl)-1,2,3,4-tetrahydroquinoxaline (1.0 equiv.) obtained in Preparation Example f-1 was dissolved in dichloromethane (0.4 M), TEA (3.0 equiv.) and triphosgene (0.6 equiv.) were slowly added at 0 °C, and the resulting mixture was stirred for 1 hour at 0 °C. TEA (2.0 equiv.) and (S)-(-)-3-(Boc-amino)pyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The obtained concentrate was dissolved in dichloromethane (0.3 M), TFA (10 equiv.) was added, and the resulting mixture was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a mixture of n-hexane and ethyl acetate to synthesize (S)-(3-aminopyrrolidin-1-yl)(4-(pyridin-2-ylmethyl)-3,4-dihydroquinoxaline-1 (2H)-yl)methanone.

Yellow oil (yield: 70%); ¹H NMR (400 MHz, CD₃OD) δ 8.52 (ddd, J = 4.9, 1.8, 1.0 Hz, 1H), 7.78 (ddd, J = 7.9, 7.3, 1.8 Hz, 1H), 7.33-7.29 (m, 2H), 6.86 (dd, J = 7.9, 1.5 Hz, 1H), 6.79 (ddd, J = 8.2, 7.3, 1.5 Hz, 1H), 6.60 (ddd, J = 7.9, 7.6, 1.3 Hz, 1H), 6.51 (dd, J = 8.2, 1.8 Hz, 1H), 4.64 (s, 2H), 3.83-3.77 (m, 1H), 3.74-3.68 (m, 1H), 3.65-3.62 (m, 2H), 3.55-3.39 (m, 4H), 3.10-3.02 (m, 1H), 2.09-2.02 (m, 1H), 1.73-1.65 (m, 1H); LC/MS ESI (+): 338.2 (M+1).

### <Example 46> Synthesis of (S)-(3-(dimethylamino)pyrrolidin-1-yl)(4-(pyridin-2-ylmethyl)-3,4-dihydroquinoxaline-1(2H)-yl)methanone

The (S)-(3-aminopyrrolidin-1-yl)(4-(pyridin-2-ylmethyl)-3,4-dihydroquinoxaline-1(2H)-yl)methanone (1.0 equiv.) obtained in Example 45 was dissolved in dichloromethane (0.3 M), sodium triacetoxyborohydride (1.5 equiv.) and formaldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (S)-(3-(dimethylamino)pyrrolidin-1-yl)(4-(pyridin-2-ylmethyl)-3,4-dihydroquinoxaline-1 (2H)-yl)methanone.

Light yellow oil (yield: 53%); ¹H NMR (400 MHz, CD₃OD) δ 8.52 (ddd, J = 4.9, 1.8, 1.0 Hz, 1H), 7.76 (ddd, J = 7.9, 7.3, 1.8 Hz, 1H), 7.33-7.28 (m, 2H), 6.85 (dd, J = 7.9, 1.5 Hz, 1H), 6.80 (ddd, J = 8.2, 7.3, 1.5 Hz, 1H), 6.62-6.58 (m, 1H), 6.53 (dd, J = 8.2, 1.8 Hz, 1H), 4.72-4.58 (m, 2H), 4.02-3.93 (m, 1H), 3.73-3.67 (m, 1H), 3.61-3.57 (m, 1H), 3.54-3.49 (m, 3H), 3.44-3.35 (m, 1H), 3.19-3.14 (m, 1H), 2.80-2.72 (m, 1H), 2.23 (s, 6H), 2.14-2.08 (m, 1H), 1.78-1.68 (m, 1H); LC/MS ESI (+): 366.3 (M+1).

### <Example 47> Synthesis of (S)-(3-(isopropylamino)pyrrolidin-1-yl)(4-(pyridin-2-ylmethyl)-3,4-dihydroquinoxaline-1(2H)-yl)methanone

(S)-(3-(isopropylamino)pyrrolidin-1-yl)(4-(pyridin-2-ylmethyl)-3,4-dihydroquinoxaline-1(2H)-yl)methanone was synthesized in the same manner as described in Example 46 using acetone instead of formaldehyde.

Ivory solid (yield: 79%); ¹H NMR (400 MHz, CD₃OD) δ 8.52 (ddd, J = 4.9, 1.8, 1.0 Hz, 1H), 7.77 (ddd, J = 7.9, 7.3, 1.7 Hz, 1H), 7.33-7.29 (m, 2H), 6.86 (dd, J = 7.9, 1.5 Hz, 1H), 6.79 (ddd, J = 8.2, 7.3, 1.5 Hz, 1H), 6.62-6.58 (m, 1H) 6.52 (dd, J = 8.2, 1.7 Hz, 1H), 4.69-4.59 (m, 2H), 3.94-3.88 (m, 1H), 3.70-3.57 (m, 2H), 3.55-3.45 (m, 2H), 3.42-3.36 (m, 3H), 3.08-3.03 (m, 1H), 2.88-2.79 (m, 1H), 2.16-2.09 (m, 1H), 1.72-1.63 (m, 1H), 1.07 (d, J = 6.3 Hz, 6H); LC/MS ESI (+): 380.3 (M+1).

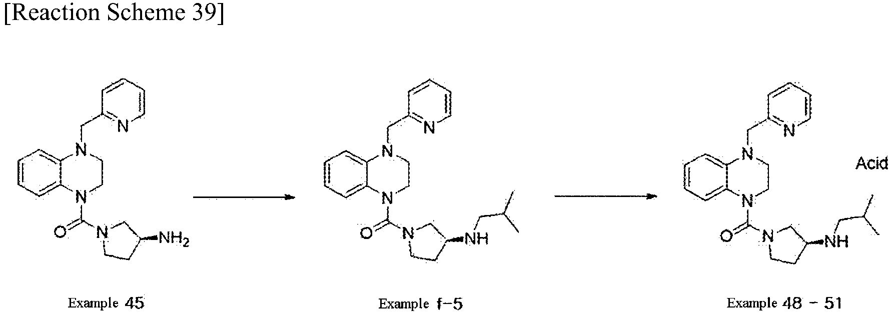

### <Preparation Example f-5> Preparation of (S)-(3-(isobutylamino)pyrrolidin-1-yl)(4-(pyridin-2-ylmethyl)-3,4-dihydroquinoxaline-1(2H)-yl)methanone

(S)-(3-(isobutylamino)pyrrolidin-1-yl)(4-(pyridin-2-ylmethyl)-3,4-dihydroquinoxaline-1(2H)-yl)methanone was prepared in the same manner as described in Example 46 using isobutyraldehyde instead of formaldehyde.

Light yellow oil (yield: 71%); ¹H NMR (400 MHz, CD₃OD) δ 8.52 (ddd, J = 4.9, 1.8, 1.0 Hz, 1H), 7.77 (ddd, J = 7.9, 7.3, 1.8 Hz, 1H), 7.33-7.29 (m, 2H), 6.85 (dd, J = 7.9, 1.5 Hz, 1H), 6.79 (ddd, J = 8.1, 7.3, 1.5 Hz, 1H), 6.66 (ddd, J = 8.8, 7.6, 1.8 Hz, 1H), 6.52 (dd, J = 8.1, 1.8 Hz, 1H), 4.69-4.60 (m, 2H), 3.89-3.83 (m, 1H), 3.68-3.58 (m, 3H), 3.53-3.40 (m, 2H), 3.41-3.34 (m, 1H), 3.27-3.23 (m, 1H), 3.14-3.10 (m, 1H), 2.41 (dd, J = 11.5, 6.9 Hz, 1H), 2.33 (dd, J = 11.5, 6.9 Hz, 1H), 2.13-2.03 (m, 1H), 1.78-1.67 (m, 2H), 0.92 (dd, J = 6.6, 1.0 Hz, 6H); LC/MS ESI (+): 394.3 (M+1).

### <Example 48> Synthesis of (S)-(3-(isobutylamino)pyrrolidin-1-yl)(4-(pyridin-2-ylmethyl)-3,4-dihydroquinoxaline-1(2H)-yl)methanone oxalic acid salt

The (S)-(3-(isobutylamino)pyrrolidin-1-yl)(4-(pyridin-2-ylmethyl)-3,4-dihydroquinoxaline-1(2H)-yl)methanone (1.0 equiv.) obtained in Preparation Example f-5 was dissolved in ethanol (0.3 M), and the resulting mixture was stirred for 10 minutes at room temperature. Oxalic acid (1.0 equiv.) was added to the reaction solution and stirred for 10 minutes at 70 °C until it dissolved to form a clear solution. After cooling the resulting solution again, it was stirred for 1 hour at room temperature. When a solid was formed, ethyl acetate (0.4 M) was added and then the resulting solution was stirred for 2 hours at room temperature. The produced solid was filtered with ethyl acetate, washed and dried to synthesize (S)-(3-(isobutylamino)pyrrolidin-1-yl)(4-(pyridin-2-ylmethyl)-3,4-dihydroquinoxaline-1(2H)-yl)methanone oxalic acid salt.

Yellow solid (yield: 38%); ¹H NMR (400 MHz, CD₃OD) δ 8.53 (ddd, J = 5.0, 1.8, 1.0 Hz, 1H), 7.79 (ddd, J = 7.9, 7.3, 1.8 Hz, 1H), 7.35-7.30 (m, 2H), 6.91 (dd, J = 7.9, 1.5 Hz, 1H), 6.83 (ddd, J = 8.3, 7.3, 1.5 Hz, 1H), 6.62 (ddd, J = 8.8, 7.6, 1.8 Hz, 1H), 6.54 (dd, J = 8.3, 1.8 Hz, 1H), 4.70-4.60 (m, 2H), 3.96-3.91 (m, 1H), 3.87-3.80 (m, 2H), 3.70-3.59 (m, 3H), 3.56-3.42 (m, 2H), 3.44-3.38 (m, 1H), 2.92 (dd, J = 12.4, 7.3 Hz, 1H), 2.84 (dd, J = 12.4, 7.3 Hz, 1H), 2.39-2.32 (m, 1H), 2.08-1.94 (m, 2H), 1.03 (dd, J = 6.6, 0.9 Hz, 6H).

### <Example 49> Synthesis of (S)-(3-(isobutylamino)pyrrolidin-1-yl)(4-(pyridin-2-ylmethyl)-3,4-dihydroquinoxaline-1(2H)-yl)methanone succinic acid salt

(S)-(3-(isobutylamino)pyrrolidin-1-yl)(4-(pyridin-2-ylmethyl)-3,4-dihydroquinoxaline-1(2H)-yl)methanone succinic acid salt was synthesized in the same manner as described in Example 48 using succinic acid instead of oxalic acid.

White solid (yield: 87%); ¹H NMR (400 MHz, CD₃OD) δ 8.53 (ddd, J = 4.9, 1.8, 1.0 Hz, 1H), 7.77 (ddd, J = 7.9, 7.3, 1.8 Hz, 1H), 7.34-7.30 (m, 2H), 6.89 (dd, J = 7.9, 1.5 Hz, 1H), 6.82 (ddd, J = 8.3, 7.3, 1.5 Hz, 1H), 6.61 (ddd, J = 8.8, 7.6, 1.8 Hz, 1H), 6.54 (dd, J = 8.3, 1.8 Hz, 1H), 4.70-4.60 (m, 2H), 3.94-3.89 (m, 1H), 3.70-3.58 (m, 5H), 3.54-3.49 (m, 1H), 3.44-3.38 (m, 2H), 2.73 (dd, J = 12.0, 7.4 Hz, 1H), 2.65 (dd, J = 12.0, 7.4 Hz, 1H), 2.30-2.23 (m, 1H), 2.01 (s, 4H), 1.96-1.86 (m, 2H), 0.99 (dd, J = 6.6, 0.9 Hz, 6H).

### <Example 50> Synthesis of (S)-(3-(isobutylamino)pyrrolidin-1-yl)(4-(pyridin-2-ylmethyl)-3,4-dihydroquinoxaline-1(2H)-yl)methanone maleic acid salt

(S)-(3-(isobutylamino)pyrrolidin-1-yl)(4-(pyridin-2-ylmethyl)-3,4-dihydroquinoxaline-1(2H)-yl)methanone maleic acid salt was synthesized in the same manner as described in Example 48 using maleic acid instead of oxalic acid.

Light yellow solid (yield: 55%); ¹H NMR (400 MHz, CD₃OD) δ 8.54 (ddd, J = 5.0, 1.8, 1.0 Hz, 1H), 7.80 (ddd, J = 7.9, 7.3, 1.8 Hz, 1H), 7.36-7.31 (m, 2H), 6.92 (dd, J = 7.9, 1.5 Hz, 1H), 6.84 (ddd, J = 8.3, 7.3, 1.5 Hz, 1H), 6.62 (ddd, J = 8.8, 7.6, 1.8 Hz, 1H), 6.55 (dd, J = 8.3, 1.8 Hz, 1H), 6.27 (s, 2H), 4.71-4.61 (m, 2H), 3.98-3.93 (m, 1H), 3.87-3.80 (m, 1H), 3.78-3.74 (m, 1H), 3.71-3.60 (m, 2H), 3.54-3.50 (m, 2H), 3.48-3.40 (m, 2H), 2.93 (dd, J = 12.4, 7.3 Hz, 1H), 2.85 (dd, J = 12.4, 7.3 Hz, 1H), 2.41-2.33 (m, 1H), 2.05-1.93 (m, 2H), 1.04 (dd, J = 6.7, 0.8 Hz, 6H).

### <Example 51> Synthesis of (S)-(3-(isobutylamino)pyrrolidin-1-yl)(4-(pyridin-2-ylmethyl)-3,4-dihydroquinoxaline-1(2H)-yl)methanone fumaric acid salt

(S)-(3-(isobutylamino)pyrrolidin-1-yl)(4-(pyridin-2-ylmethyl)-3,4-dihydroquinoxaline-1(2H)-yl)methanone fumaric acid salt was synthesized in the same manner as described in Example 48 using fumaric acid instead of oxalic acid.

Ivory solid (yield: 81%); ¹H NMR (400 MHz, CD₃OD) δ 8.53 (ddd, J = 5.0, 1.8, 1.0 Hz, 1H), 7.78 (ddd, J = 7.9, 7.3, 1.8 Hz, 1H), 7.34-7.30 (m, 2H), 6.91 (dd, J = 7.9, 1.5 Hz, 1H), 6.83 (ddd, J = 8.3, 7.3, 1.5 Hz, 1H), 6.71 (s, 2H), 6.62 (ddd, J = 8.8, 7.6, 1.8 Hz, 1H), 6.55 (dd, J = 8.3, 1.8 Hz, 1H), 4.70-4.60 (m, 2H), 3.97-3.93 (m, 1H), 3.83-3.72 (m, 2H), 3.68-3.60 (m, 2H), 3.54-3.50 (m, 2H), 3.47-3.42 (m, 2H), 2.91 (dd, J = 12.4, 7.3 Hz, 1H), 2.83 (dd, J = 12.4, 7.3 Hz, 1H), 2.40-2.32 (m, 1H), 2.04-1.93 (m, 2H), 1.04 (dd, J = 6.7, 0.8 Hz, 6H).

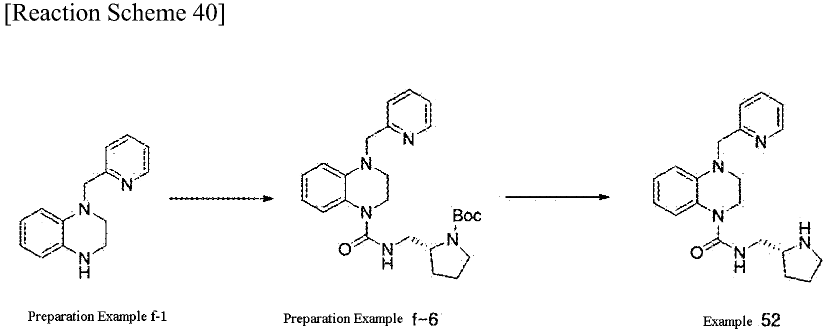

### <Preparation Example f-6> Preparation of tert-butyl (R)-2-((4-(pyridin-2-ylmethyl)-1,2,3,4-tetrahydroquinoxaline-1-carboxamido)methyl)pyrrolidin-1-carboxylate

Tert-butyl (R)-2-((4-(pyridin-2-ylmethyl)-1,2,3,4-tetrahydroquinoxaline-1-carboxamido)methyl)pyrrolidin-1-carboxylate was prepared in the same manner as described in Preparation Example f-4 using (R)-1-Boc-2-(aminomethyl)pyrrolidine instead of (S)-(-)-1-Boc-3-aminopyrrolidine.

Light yellow solid (yield: 90%); ¹H NMR (400 MHz, CD₃OD) δ 8.52 (ddd, J = 4.9, 1.7, 0.9 Hz, 1H), 7.77 (ddd, J = 7.9, 7.3, 1.8 Hz, 1H), 7.31-7.27 (m, 2H), 7.22-7.19 (m, 1H), 6.94 (ddd, J = 8.2, 7.3, 1.5 Hz, 1H), 6.67 (ddd, J = 8.8, 7.6, 1.7 Hz, 1H), 6.61-6.59 (m, 1H), 4.65 (s, 2H), 3.95-3.89 (m, 1H), 3.86-3.81 (m, 2H), 3.53 (t, J = 5.1 Hz, 2H), 3.39-3.25 (m, 4H), 2.03-1.90 (m, 2H), 1.83-1.76 (m, 2H), 1.47 (s, 9H).

### <Example 52> Synthesis of (R)-4-(pyridin-2-ylmethyl)-N-(pyrrolidin-2-ylmethyl)-3,4-dihydroquinoxaline-1(2H)-carboxamide

(R)-4-(pyridin-2-ylmethyl)-N-(pyrrolidin-2-ylmethyl)-3,4-dihydroquinoxaline-1(2H)-carboxamide was synthesized by deprotecting the Boc group of the tert-butyl (R)-2-((4-(pyridin-2-ylmethyl)-1,2,3,4-tetrahydroquinoxaline-1-carboxamido)methyl)pyrrolidin-1-carboxylate obtained in Preparation Example f-6 with TFA.

Yellow oil (yield: 98%); ¹H NMR (400 MHz, CD₃OD) δ 8.52 (ddd, J = 5.0, 1.8, 1.0 Hz, 1H), 7.77 (ddd, J = 7.9, 7.4, 1.8 Hz, 1H), 7.32-7.27 (m, 2H), 7.21 (dd, J = 7.9, 1.6 Hz, 1H), 6.94 (ddd, J = 8.3, 7.4, 1.6 Hz, 1H), 6.69-6.65 (m, 1H), 6.60 (dd, J = 8.3, 1.8 Hz, 1H), 4.66 (s, 2H), 3.89-3.81 (m, 2H), 3.54 (t, J = 5.1 Hz, 2H), 3.36-3.26 (m, 2H), 3.20-3.15 (m, 1H), 2.97-2.83 (m, 2H), 1.93-1.87 (m, 1H), 1.83-1.75 (m, 2H), 1.51-1.42 (m, 1H); LC/MS ESI (+): 352.3 (M+1).

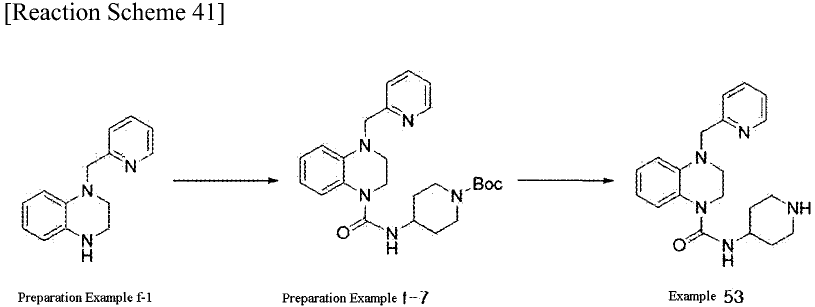

### <Preparation Example f-7> Preparation of tert-butyl 4-(4-(pyridin-2-ylmethyl)-1,2,3,4-tetrahydroquinoxaline-1-carboxamido)piperidin-1-carboxylate

Tert-butyl 4-(4-(pyridin-2-ylmethyl)-1,2,3,4-tetrahydroquinoxaline-1-carboxamido)piperidin-1-carboxylate was prepared in the same manner as described in Preparation Example f-4 using 1-Boc-4-aminopiperidine instead of (S)-(-)-1-Boc-3-aminopyrrolidine.

White solid (yield: 100%); ¹H NMR (400 MHz, CD₃OD) δ 8.51 (ddd, J = 5.0, 1.8, 1.0 Hz, 1H), 7.77 (ddd, J = 7.9, 7.4, 1.8 Hz, 1H), 7.32-7.27 (m, 2H), 7.18 (dd, J = 7.9, 1.6 Hz, 1H), 6.92 (ddd, J = 8.3, 7.4, 1.6 Hz, 1H), 6.68-6.64 (m, 1H), 6.60 (dd, J = 8.3, 1.8 Hz, 1H), 4.66 (s, 2H), 4.03-3.98 (m, 2H), 3.83 (t, J = 5.8 Hz, 2H), 3.80-3.75 (m, 1H), 3.53 (t, J = 5.8 Hz, 2H), 2.93-2.88 (m, 2H), 1.89-1.85 (m, 2H), 1.45 (s, 9H), 1.39-1.32 (m, 2H).

### <Example 53> Synthesis of N-(piperidin-4-yl)-4-(pyridin-2-ylmethyl)-3,4-dihydroquinoxaline-1 (2H)-carboxamide

N-(piperidin-4-yl)-4-(pyridin-2-ylmethyl)-3,4-dihydroquinoxaline-1(2H)-carboxamide was synthesized by deprotecting the Boc group of the tert-butyl 4-(4-(pyridin-2-ylmethyl)-1,2,3,4-tetrahydroquinoxaline-1-carboxamido)piperidin-1-carboxylate obtained in Preparation Example f-7 with TFA.

Ivory solid (yield: 79%); ¹H NMR (400 MHz, CD₃OD) δ 8.52 (ddd, J = 5.0, 1.9, 1.0 Hz, 1H), 7.77 (ddd, J = 7.9, 7.4, 1.8 Hz, 1H), 7.32-7.27 (m, 2H), 7.17 (dd, J = 7.9, 1.6 Hz, 1H), 6.93 (ddd, J = 8.2, 7.4, 1.6 Hz, 1H), 6.66 (ddd, J = 8.8, 7.6, 1.9 Hz, 1H), 6.61 (dd, J = 8.2, 1.8 Hz, 1H), 4.66 (s, 2H), 3.83 (t, J = 5.8 Hz, 2H), 3.77-3.70 (m, 1H), 3.53 (t, J = 5.8 Hz, 2H), 3.06-3.01 (m, 2H), 2.70-2.63 (m, 2H), 1.93-1.88 (m, 2H), 1.46-1.35 (m, 2H); LC/MS ESI (+): 352.3 (M+1).

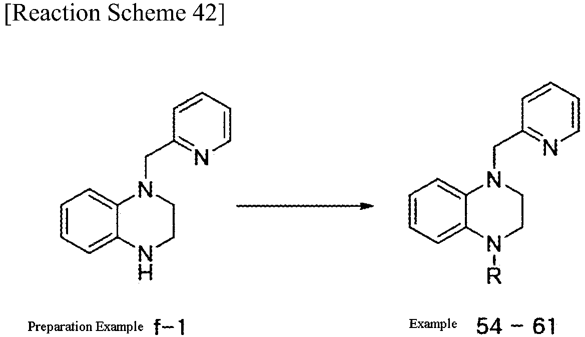

### <Example 54> Synthesis of N-(oxetan-3-yl)-4-(pyridin-2-ylmethyl)-3,4-dihydroquinoxaline-1 (2H)-carboxamide

N-(oxetan-3-yl)-4-(pyridin-2-ylmethyl)-3,4-dihydroquinoxaline-1(2H)-carboxamide was synthesized in the same manner as described in Preparation Example f-4 using 3-oxetanamine instead of (S)-(-)-1-Boc-3-aminopyrrolidine.

White solid (yield: 77%); ¹H NMR (400 MHz, CD₃OD) δ 8.53-8.51 (m, 1H), 7.76 (ddd, J = 7.9, 7.4, 1.8 Hz, 1H), 7.31-7.28 (m, 2H), 7.24 (dd, J = 7.9, 1.6 Hz, 1H), 6.94 (ddd, J = 8.3, 7.4, 1.6 Hz, 1H), 6.69-6.66 (m, 1H), 6.61 (dd, J = 8.3, 1.8 Hz, 1H), 4.92-4.88 (m, 1H), 4.86-4.81 (m, 2H), 4.66 (s, 2H), 4.61-4.58 (m, 2H), 3.84 (t, J = 5.8 Hz, 2H), 3.54 (t, J = 5.8 Hz, 2H); LC/MS ESI (+): 325.2 (M+1).

### <Example 55> Synthesis of N-(3-Methyloxetan-3-yl)-4-(pyridin-2-ylmethyl)-3,4-dihydroquinoxaline-1(2H)-carboxamide

N-(3-Methyloxetan-3-yl)-4-(pyridin-2-ylmethyl)-3,4-dihydroquinoxaline-1(2H)-carboxamide was synthesized in the same manner as described in Preparation Example f-4 using 3-methyl-3-oxetanamine instead of (S)-(-)-1-Boc-3-aminopyrrolidine.

Light yellow oil (yield: 40%); ¹H NMR (400 MHz, CD₃OD) δ 8.52 (ddd, J = 4.9, 1.8, 1.0 Hz, 1H), 7.76 (ddd, J = 7.9, 7.7, 1.8 Hz, 1H), 7.31-7.28 (m, 2H), 7.24 (dd, J = 7.9, 1.6 Hz, 1H), 6.93 (ddd, J = 8.2, 7.7, 1.6 Hz, 1H), 6.67 (ddd, J = 8.8, 7.6, 1.9 Hz, 1H), 6.61 (dd, J = 8.2, 1.8 Hz, 1H), 4.73 (d, J = 6.7 Hz, 2H), 4.66 (s, 2H), 4.39 (d, J = 6.7 Hz, 2H), 3.84 (t, J = 5.8 Hz, 2H), 3.54 (t, J = 5.8 Hz, 2H), 1.63 (s, 3H); LC/MS ESI (+): 339.2 (M+1).

### <Example 56> Synthesis of N-(oxetan-3-ylmethyl)-4-(pyridin-2-ylmethyl)-3,4-dihydroquinoxaline-1 (2H)-carboxamide

N-(oxetan-3-ylmethyl)-4-(pyridin-2-ylmethyl)-3,4-dihydroquinoxaline-1(2H)-carboxamide was synthesized in the same manner as described in Preparation Example f-4 using oxetan-3-ylmethanamine instead of (S)-(-)-1-Boc-3-aminopyrrolidine.

Light yellow solid (yield: 61%); ¹H NMR (400 MHz, CD₃OD) δ 8.52 (ddd, J = 5.0, 1.8, 1.0 Hz, 1H), 7.76 (ddd, J = 7.9, 7.7, 1.8 Hz, 1H), 7.32-7.26 (m, 2H), 7.18 (dd, J = 7.9, 1.6 Hz, 1H), 6.93 (ddd, J = 8.3, 7.7, 1.6 Hz, 1H), 6.66 (ddd, J = 8.8, 7.6, 1.9 Hz, 1H), 6.60 (dd, J = 8.3, 1.8 Hz, 1H), 4.78 (dd, J = 7.9, 6.2 Hz, 2H), 4.66 (s, 2H), 4.47 (dd, J = 6.8, 6.1 Hz, 2H), 3.84 (t, J = 5.8 Hz, 2H), 3.54 (t, J = 5.8 Hz, 2H), 3.48 (d, J = 6.5 Hz, 2H), 3.27-3.17 (m, 1H); LC/MS ESI (+): 339.2 (M+1).

### <Example 57> Synthesis of N-((3-methyloxetan-3-yl)methyl)-4-(pyridin-2-ylmethyl)-3,4-dihydroquinoxaline-1(2H)-carboxamide

N-((3-methyloxetan-3-yl)methyl)-4-(pyridin-2-ylmethyl)-3,4-dihydroquinoxaline-1(2H)-carboxamide was synthesized in the same manner as described in Preparation Example f-4 using (3-methyloxetan-3-yl)methanamine instead of (S)-(-)-1-Boc-3-aminopyrrolidine.

Light yellow solid (yield: 89%); ¹H NMR (400 MHz, CD₃OD) δ 8.52 (ddd, J = 5.0, 1.8, 1.0 Hz, 1H), 7.75 (ddd, J = 7.9, 7.7, 1.8 Hz, 1H), 7.31-7.28 (m, 2H), 7.25 (dd, J = 7.9, 1.6 Hz, 1H), 6.94 (ddd, J = 8.3, 7.7, 1.6 Hz, 1H), 6.67 (ddd, J = 8.8, 7.6, 1.9 Hz, 1H), 6.62 (dd, J = 8.3, 1.8 Hz, 1H), 4.67 (s, 2H), 4.56 (d, J = 6.0 Hz, 2H), 4.34 (d, J = 6.0 Hz, 2H), 3.86 (t, J = 5.8 Hz, 2H), 3.55 (t, J = 5.8 Hz, 2H), 3.34 (s, 2H), 1.30 (s, 3H); LC/MS ESI (+): 353.3 (M+1).

### <Example 58> Synthesis of 4-(pyridin-2-ylmethyl)-N-((tetrahydrofuran-3-yl)methyl)-3,4-dihydroquinoxaline-1(2H)-carboxamide

4-(pyridin-2-ylmethyl)-N-((tetrahydrofuran-3-yl)methyl)-3,4-dihydroquinoxaline-1(2H)-carboxamide was synthesized in the same manner as described in Preparation Example f-4 using (tetrahydrofuran-3-yl) methanamine instead of (S)-(-)-1-Boc-3-aminopyrrolidine.

White solid (yield: 51%); ¹ H NMR (400 MHz, CDCl₃) δ 8.59 (ddd, J = 4.9, 1.8, 0.9 Hz, 1H), 7.61 (ddd, J = 7.9, 7.7, 1.8 Hz, 1H), 7.18 (ddd, J = 7.6, 4.9, 1.1 Hz, 1H), 7.14-7.11 (m, 2H), 6.98 (ddd, J = 8.3, 7.7, 1.6 Hz, 1H), 6.69-6.62 (m, 2H), 5.47 (t, J = 5.8 Hz, 1H), 4.65 (s, 2H), 3.92-3.89 (m, 2H), 3.87-3.84 (m, 1H), 3.84 (dd, J = 6.8, 6.1 Hz, 2H), 3.76-3.71 (m, 1H), 3.54-3.50 (m, 2H), 3.34-3.21 (m, 2H), 2.56-2.45 (m, 1H), 2.07-1.98 (m, 1H), 1.66-1.59 (m, 1H); LC/MS ESI (+): 353.3 (M+1).

### <Example 59> Synthesis of (S)-4-(pyridin-2-ylmethyl)-N-((tetrahydrofuran-3-yl)methyl)-3,4-dihydroquinoxaline-1(2H)-carboxamide

(S)-4-(pyridin-2-ylmethyl)-N-((tetrahydrofuran-3-yl)methyl)-3,4-dihydroquinoxaline-1(2H)-carboxamide was synthesized in the same manner as described in Preparation Example f-4 using (S)-(tetrahydrofuran-3-yl) methanamine instead of (S)-(-)-1-Boc-3-aminopyrrolidine.

Ivory solid (yield: 72%); ¹H NMR (400 MHz, CD₃OD) δ 8.52 (ddd, J = 5.0, 1.8, 0.9 Hz, 1H), 7.75 (ddd, J = 7.9, 7.7, 1.8 Hz, 1H), 7.31-7.26 (m, 2H), 7.16 (dd, J = 7.9, 1.5 Hz, 1H), 6.94 (ddd, J = 8.3, 7.7, 1.6 Hz, 1H), 6.67 (ddd, J = 8.8, 7.6, 1.8 Hz, 1H), 6.61 (dd, J = 8.3, 1.8 Hz, 1H), 4.66 (s, 2H), 3.87-3.83 (m, 3H), 3.80-3.77 (m, 1H), 3.75-3.69 (m, 1H), 3.54-3.51 (m, 3H), 3.26-3.17 (m, 2H), 2.55-2.45 (m, 1H), 2.05-1.98 (m, 1H), 1.69-1.61 (m, 1H); LC/MS ESI (+): 353.3 (M+1).

### <Example 60> Synthesis of (R)-4-(pyridin-2-ylmethyl)-N-((tetrahydrofuran-3-yl)methyl)-3,4-dihydroquinoxaline-1(2H)-carboxamide

(R)-4-(pyridin-2-ylmethyl)-N-((tetrahydrofuran-3-yl)methyl)-3,4-dihydroquinoxaline-1(2H)-carboxamide was synthesized in the same manner as described in Preparation Example f-4 using (R)-(tetrahydrofuran-3-yl) methanamine instead of (S)-(-)-1-Boc-3-aminopyrrolidine.

Ivory solid (yield: 60%); ¹H NMR (400 MHz, CD₃OD) δ 8.52 (ddd, J = 4.9, 1.8, 0.9 Hz, 1H), 7.75 (ddd, J = 7.9, 7.7, 1.8 Hz, 1H), 7.31-7.26 (m, 2H), 7.16 (dd, J = 7.9, 1.6 Hz, 1H), 6.94 (ddd, J = 8.3, 7.7, 1.6 Hz, 1H), 6.67 (ddd, J = 8.8, 7.6, 1.8 Hz, 1H), 6.61 (dd, J = 8.3, 1.8 Hz, 1H), 4.66 (s, 2H), 3.87-3.82 (m, 3H), 3.80-3.77 (m, 1H), 3.75-3.69 (m, 1H), 3.54-3.51 (m, 3H), 3.27-3.17 (m, 2H), 2.56-2.45 (m, 1H), 2.06-1.98 (m, 1H), 1.69-1.60 (m, 1H); LC/MS ESI (+): 353.3 (M+1).

### <Example 61> Synthesis of N-(cyclopropylmethyl)-4-(pyridin-2-ylmethyl)-3,4-dihydroquinoxaline-1 (2H)-carboxamide

N-(cyclopropylmethyl)-4-(pyridin-2-ylmethyl)-3,4-dihydroquinoxaline-1(2H)-carboxamide was synthesized in the same manner as described in Preparation Example f-4 using cyclopropylmethanamine instead of (S)-(-)-1-Boc-3-aminopyrrolidine.

Light yellow solid (yield: 68%); ¹H NMR (400 MHz, CD₃OD) δ 8.52 (ddd, J = 4.9, 1.8, 0.9 Hz, 1H), 7.75 (ddd, J = 7.9, 7.7, 1.8 Hz, 1H), 7.31-7.27 (m, 2H), 7.19 (dd, J = 7.9, 1.6 Hz, 1H), 6.94 (ddd, J = 8.3, 7.7, 1.6 Hz, 1H), 6.67 (ddd, J = 8.8, 7.6, 1.8 Hz, 1H), 6.61 (dd, J = 8.3, 1.8 Hz, 1H), 4.66 (s, 2H), 3.84 (t, J = 5.8 Hz, 2H), 3.54 (t, J = 5.8 Hz, 2H), 3.09 (d, J = 6.9 Hz, 2H), 1.07-0.97 (m, 1H), 0.49-0.41 (m, 2H), 0.25-0.16 (m, 2H); LC/MS ESI (+): 323.3 (M+1).

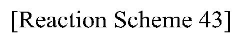

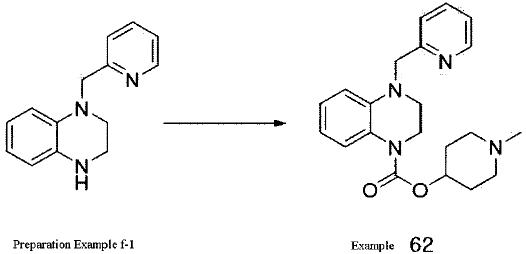

### <Example 62> Synthesis of 1-methylpiperidin-4-yl 4-(pyridin-2-ylmethyl)-3,4-dihydroquinoxaline-1(2H)-carboxylate

The 1-(pyridin-2-ylmethyl)-1,2,3,4-tetrahydroquinoxaline (1.0 equiv.) obtained in Example f-1 was dissolved in dichloromethane (0.2 M), TEA (2.0 equiv.) and triphosgene (0.3 equiv.) were slowly added at 0 °C, and the resulting mixture was stirred for 10 minutes at 0 °C. NaH (3.0 equiv.) was dissolved in THF (0.2M) and 1-Boc-4-hydroxypiperidine (1.5 equiv.) was added, and the resulting mixture was slowly added to the reaction solution and then stirred for 3 hours at room temperature. After terminating the reaction by adding water to the reaction solution, the resulting solution was extracted with dichloromethane. The organic layer obtained thereby was washed with brine, dried over MgSO₄, filtered, and concentrated under reduced pressure, thereby obtaining a concentrate. The concentrate was dissolved in dichloromethane (0.2 M), and a Boc group was deprotected using TFA (10.0 equiv.). The resulting reaction solution was concentrated and dissolved in dichloromethane (0.1 M), and after adding sodium triacetoxyborohydride (2.0 equiv.) and formaldehyde (6.0 equiv.), the resulting solution was stirred for 2 hours at room temperature. Water was added to the reaction solution to terminate the reaction, and then the resulting solution was extracted with dichloromethane and concentrated under reduced pressure. The residue obtained thereby was purified by silica column chromatography using a dichloromethane/methanol mixture to synthesize 1-methylpiperidin-4-yl 4-(pyridin-2-ylmethyl)-3,4-dihydroquinoxaline-1 (2H)-carboxylate.

Light yellow oil (yield: 15%); ¹H NMR (400 MHz, CDCl₃) δ 8.59 (ddd, J = 4.7, 1.8, 1.0 Hz, 1H), 7.61 (ddd, J = 7.9, 7.7, 1.8 Hz, 1H), 7.50-7.46 (m, 1H), 7.20-7.16 (m, 2H), 6.92 (ddd, J = 8.3, 7.7, 1.6 Hz, 1H), 6.66 (ddd, J = 8.4, 7.3, 1.8 Hz, 1H), 6.57 (dd, J = 8.3, 1.8 Hz, 1H), 4.94-4.88 (m, 1H), 4.63 (s, 2H), 3.92 (t, J = 5.8 Hz, 2H), 3.56 (t, J = 5.8 Hz, 2H), 2.67-2.63 (m, 2H), 2.48-2.42 (m, 2H), 2.34 (s, 3H), 2.09-2.03 (m, 2H), 1.90-1.83 (m, 2H); LC/MS ESI (+): 367.3 (M+1).

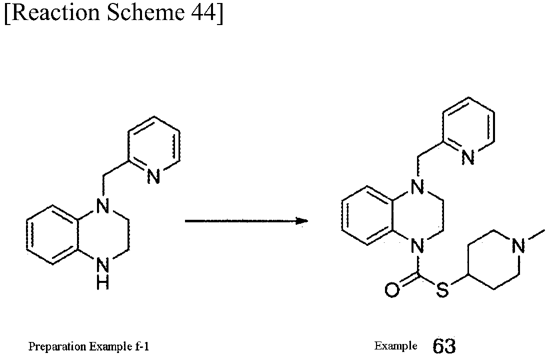

### <Example 63> Synthesis of (S)-(1-Methylpiperidin-4-yl)-4-(pyridin-2-ylmethyl)-3,4-dihydroquinoxaline-1(2H)-carbothioate

(S)-(1-Methylpiperidin-4-yl)-4-(pyridin-2-ylmethyl)-3,4-dihydroquinoxaline-1(2H)-carbothioate was synthesized in the same manner as described in Example 62 using 1-Boc-4-mercapto-piperidine instead of 1-Boc-4-hydroxypiperidine.

Light yellow oil (yield: 33%); ¹H NMR (400 MHz, CDCl₃) δ 8.60 (ddd, J = 4.8, 1.8, 0.9 Hz, 1H), 7.61 (ddd, J = 7.9, 7.6, 1.8 Hz, 1H), 7.50 (d, J = 7.9 Hz, 1H), 7.20-7.14 (m, 2H), 7.02 (ddd, J = 8.3, 7.6, 1.6 Hz, 1H), 6.68 (ddd, J = 8.4, 7.3, 1.8 Hz, 1H), 6.61 (dd, J = 8.3, 1.8 Hz, 1H), 4.65 (s, 2H), 3.96 (dd, J = 5.8, 4.6 Hz, 2H), 3.58 (dd, J = 5.8, 4.6 Hz, 2H), 3.52-3.44 (m, 1H), 2.82-2.78 (m, 2H), 2.29 (s, 3H), 2.21-2.16 (m, 2H), 2.06-2.04 (m, 2H), 1.79-1.69 (m, 2H); LC/MS ESI (+): 383.3 (M+1).

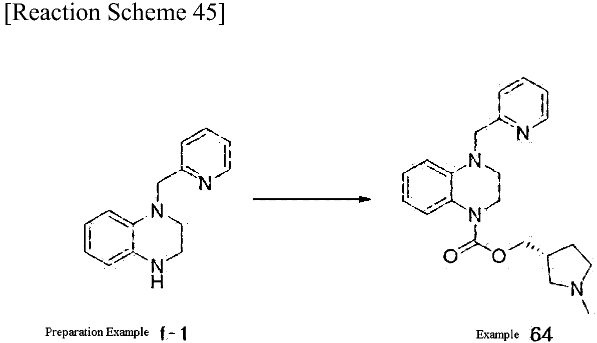

### <Example 64> Synthesis of (R)-(1-methylpyrrolidin-3-yl)methyl 4-(pyridin-2-ylmethyl)-3,4-dihydroquinoxaline-1(2H)-carboxylate

(R)-(1-methylpyrrolidin-3-yl)methyl 4-(pyridin-2-ylmethyl)-3,4-dihydroquinoxaline-1(2H)-carboxylate was synthesized in the same manner as described in Example 62 using (R)-1-Boc-(3-hydroxymethyl)pyrrolidine instead of 1-Boc-4-hydroxypiperidine.

Light yellow solid (yield: 19%); ¹H NMR (400 MHz, CDCl₃) δ 8.59 (ddd, J = 4.7, 1.8, 1.1 Hz, 1H), 7.61 (ddd, J = 7.9, 7.7, 1.8 Hz, 1H), 7.45-7.43 (m, 1H), 7.21-7.16 (m, 2H), 6.92 (ddd, J = 8.2, 7.3, 1.6 Hz, 1H), 6.66 (ddd, J = 8.3, 7.3, 1.3 Hz, 1H), 6.57 (dd, J = 8.2, 1.3 Hz, 1H), 4.63 (s, 2H), 4.19-4.09 (m, 2H), 3.92 (t, J = 5.8 Hz, 2H), 3.57 (t, J = 5.8 Hz, 2H), 2.78-2.74 (m, 1H), 2.63-2.57 (m, 1H), 2.54-2.47 (m, 1H), 2.37 (s, 3H), 2.34-2.32 (m, 1H), 2.04-2.01 (m, 2H), 1.661-1.53 (m, 1H); LC/MS ESI (+): 367.3 (M+1).

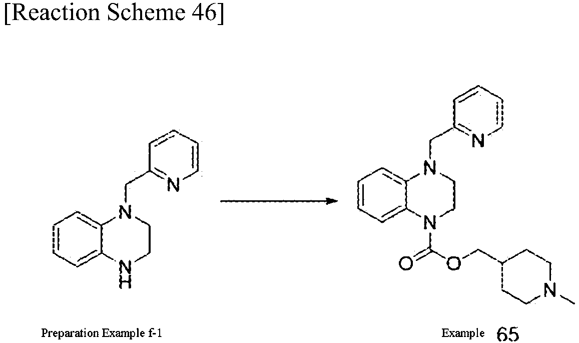

### <Example 65> Synthesis of (1-methylpiperidin-4-yl)methyl-4-(pyridin-2-ylmethyl)-3,4-dihydroquinoxaline-1(2H)-carboxylate

(R)-(1-methylpyrrolidin-3-yl)methyl 4-(pyridin-2-ylmethyl)-3,4-dihydroquinoxaline-1(2H)-carboxylate was synthesized in the same manner as described in Example 62 using (R)-1-Boc-(3-hydroxymethyl)pyrrolidine instead of 1-Boc-4-hydroxypiperidine.

Yellow oil (yield: 38%); ¹H NMR (400 MHz, CDCl₃) δ 8.59 (ddd, J = 4.7, 1.8, 1.0 Hz, 1H), 7.61 (ddd, J = 7.9, 7.7, 1.8 Hz, 1H), 7.50-7.47 (m 1H), 7.19-7.16 (m, 2H), 6.92 (ddd, J = 8.7, 7.3, 1.6 Hz, 1H), 6.66 (ddd, J = 8.4, 7.3, 1.4 Hz, 1H), 6.57 (dd, J = 8.4, 1.6 Hz, 1H), 4.92-4.88 (m, 1H), 4.63 (s, 2H), 3.92 (t, J = 5.8 Hz, 2H), 3.56 (t, J = 5.8 Hz, 2H), 2.69-2.64 (m, 2H), 2.47-2.43 (m, 2H), 2.34 (s, 3H), 2.08-2.02 (m, 2H), 1.90-1.82 (m, 2H); LC/MS ESI (+): 381.3 (M+1).

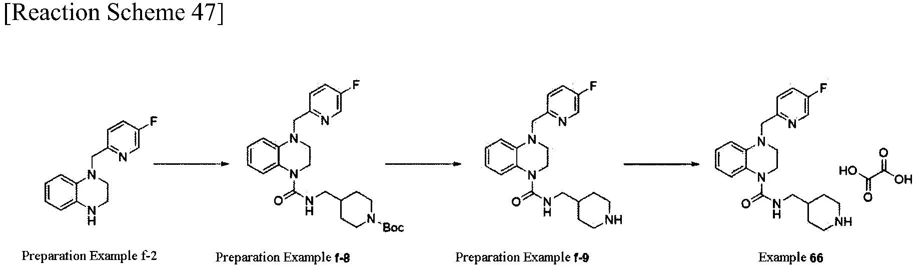

### <Preparation Example f-8> Preparation of tert-butyl 4-((4-((5-fluoropyridin-2-yl)methyl)-1,2,3,4-tetrahydroquinoxaline-1-carboxamido)methyl)piperidin-1-carboxylate

The 1-((5-fluoropyridin-2-yl)methyl)-1,2,3,4-tetrahydroquinoxaline (1.0 equiv.) obtained in Preparation Example f-2 was dissolved in dichloromethane (0.3 M), and TEA (3.0 equiv.) and triphosgene (0.6 equiv.) were slowly added at 0 °C, and the resulting mixture was stirred for 1 hour at 0 °C. TEA (2.0 equiv.) and 1-Boc-4-(aminomethyl)piperidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered, and concentrated under reduced pressure. The residue obtained thereby was purified by silica column chromatography using a hexane/ethyl acetate mixture to prepare tert-butyl 4-((4-((5-fluoropyridin-2-yl)methyl)-1,2,3,4-tetrahydroquinoxaline-1-carboxamido)methyl)piperidin-1-carboxylate.

Light yellow solid (yield: 98%); ¹H NMR (400 MHz, CD₃OD) δ 8.43 (ddd, J = 2.9, 1.5, 0.8 Hz, 1H), 7.54 (ddd, J = 8.6, 4.4, 2.9 Hz, 1H), 7.31 (dd, J = 8.7, 4.4 Hz, 1H), 7.15 (dd, J = 7.9, 1.5 Hz, 1H), 6.94 (ddd, J = 8.3, 7.4, 1.6 Hz, 1H), 6.69-6.62 (m, 2H), 6.46 (t, J = 5.9 Hz, 1H), 4.65 (s, 2H), 4.13-4.06 (m, 3H), 3.82 (t, J = 5.1 Hz, 2H), 3.53 (t, J = 5.1 Hz, 2H), 3.12-3.08 (m, 2H), 2.79-2.72 (m, 2H), 1.75-1.66 (m, 2H), 1.45 (s, 9H), 1.13-1.10 (m, 2H).

### <Preparation Example f-9> Preparation of 4-((5-fluoropyridine-2-yl)methyl)-N-(piperidin-4-ylmethyl)-3,4-dihydroquinoxaline-1(2H)-carboxamide

4-((5-fluoropyridine-2-yl)methyl)-N-(piperidin-4-ylmethyl)-3,4-dihydroquinoxaline-1(2H)-carboxamide was prepared by deprotecting the Boc group of the tert-butyl 4-((4-((5-fluoropyridin-2-yl)methyl)-1,2,3,4-tetrahydroquinoxaline-1-carboxamido)methyl)piperidin-1-carboxylate obtained in Preparation Example f-8 with TFA.

Light yellow solid (yield: 25%); ¹H NMR (400 MHz, CD₃OD) δ 8.43 (ddd, J = 3.0, 1.5, 0.8 Hz, 1H), 7.54 (ddd, J = 8.6, 4.5, 2.9 Hz, 1H), 7.31 (ddd, J = 8.8, 4.5, 0.8 Hz, 1H), 7.17 (dd, J = 7.9, 1.5 Hz, 1H), 6.94 (ddd, J = 8.2, 7.4, 1.6 Hz, 1H), 6.69-6.62 (m, 2H), 4.65 (s, 2H), 3.82 (dd, J = 5.8, 4.5 Hz, 2H), 3.53 (dd, J = 5.8, 4.5 Hz, 2H), 3.13-3.12 (m, 1H), 3.11-3.09 (m, 3H), 2.67-2.60 (m, 2H), 1.75-1.64 (m, 3H), 1.29-1.15 (m, 2H); LC/MS ESI (+): 384.3 (M+1).

### <Example 66> Synthesis of 4-((5-fluoropyridine-2-yl)methyl)-N-(piperidin-4-ylmethyl)-3,4-dihydroquinoxaline-1(2H)-carboxamide oxalic acid salt

The 4-((5-fluoropyridine-2-yl)methyl)-N-(piperidin-4-ylmethyl)-3,4-dihydroquinoxaline-1(2H)-carboxamide (1.0 equiv.) obtained in Preparation Example f-9 was dissolved in DCM/EtOH (v/v = 4: 1, 0.7 M), oxalic acid (1.0 equiv.) was added to the reaction solution and stirred for 5 minutes at 40 °C until it dissolved to form a clear solution. After cooling the resulting solution again, it was stirred for 4.5 hours at room temperature. When a solid was formed, it was filtered with ethyl acetate, washed and dried, thereby synthesizing 4-((5-fluoropyridine-2-yl)methyl)-N-(piperidin-4-ylmethyl)-3,4-dihydroquinoxaline-1(2H)-carboxamide oxalic acid salt.

White solid (yield: 57%); ¹H NMR (400 MHz, CD₃OD) δ 8.43 (d, J = 2.9 Hz, 1H), 7.54 (ddd, J = 8.6, 4.4, 2.9 Hz, 1H), 7.32 (ddd, J = 8.6, 4.4, 0.7 Hz, 1H), 7.18 (dd, J = 7.9, 1.5 Hz, 1H), 6.95 (ddd, J = 8.3, 7.4, 1.5 Hz, 1H), 6.69-6.62 (m, 2H), 4.65 (s, 2H), 3.83 (t, J = 5.5 Hz, 2H), 3.53 (t, J = 5.5 Hz, 2H), 3.43-3.39 (m, 2H), 3.15 (d, J = 6.7 Hz, 2H), 3.00-2.93 (m, 2H), 1.94-1.90 (m, 2H), 1.87-1.82 (m, 1H), 1.47-1.36 (m, 2H).

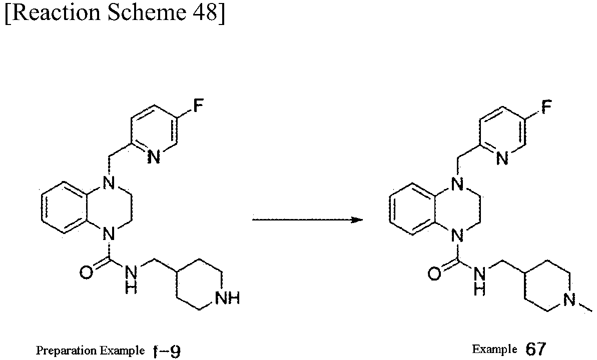

### <Example 67> Synthesis of 4-((5-fluoropyridin-2-yl)methyl)-N-((1-methylpiperidin-4-yl)methyl)-3,4-dihydroquinoxaline-1(2H)-carboxamide

The 4-((5-fluoropyridine-2-yl)methyl)-N-(piperidin-4-ylmethyl)-3,4-dihydroquinoxaline-1(2H)-carboxamide (1.0 equiv.) obtained in Preparation Example f-9 was dissolved in dichloromethane (0.3 M), sodium triacetoxyborohydride (1.3 equiv.) and formaldehyde (1.1 equiv.) were added and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize 4-((5-fluoropyridin-2-yl)methyl)-N-((1-methylpiperidin-4-yl)methyl)-3,4-dihydroquinoxaline-1(2H)-carboxamide.

Yellow solid (yield: 44%); ¹H NMR (400 MHz, CD₃OD) δ 8.43 (ddd, J = 2.9, 1.5, 0.7 Hz, 1H), 7.54 (ddd, J = 8.6, 4.5, 2.9 Hz, 1H), 7.31 (ddd, J = 8.7, 4.5, 0.7 Hz, 1H), 7.17 (dd, J = 7.9, 1.5 Hz, 1H), 6.94 (ddd, J = 8.3, 7.4, 1.6 Hz, 1H), 6.69-6.62 (m, 2H), 4.65 (s, 2H), 3.82 (dd, J = 5.8, 4.5 Hz, 2H), 3.53 (dd, J = 5.8, 4.5 Hz, 2H), 3.11 (d, J = 6.7 Hz, 2H), 2.94-2.91 (m, 2H), 2.30 (s, 3H), 2.09-2.03 (m, 2H), 1.73-1.69 (m, 2H), 1.59-1.52 (m, 1H), 1.34-1.23 (m, 2H); LC/MS ESI (+): 398.3 (M+1).

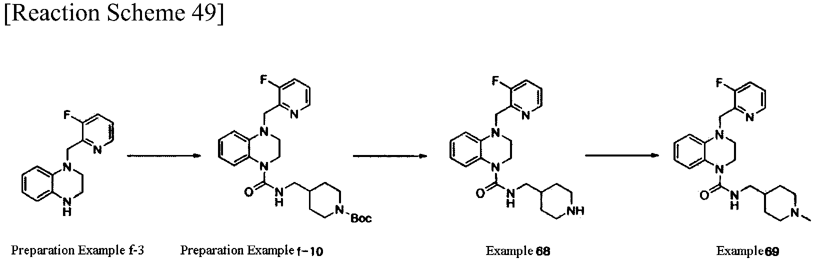

### <Preparation Example f-10> Preparation of tert-butyl 4-((4-((3-fluoropyridin-2yl)methyl)-1,2,3,4-tetrahydroquinoxaline-1-carboxamido)methyl)piperidin-1-carboxylate

Tert-butyl 4-((4-((3-fluoropyridin-2yl)methyl)-1,2,3,4-tetrahydroquinoxaline-1-carboxamido)methyl)piperidin-1-carboxylate was prepared in the same manner as described in Preparation Example f-8 using the 1-((3-fluoropyridin-2-yl)methyl)-1,2,3,4-tetrahydroquinoxaline obtained in Preparation Example f-3 instead of 1-((5-fluoropyridin-2-yl)methyl)-1,2,3,4-tetrahydroquinoxaline.

Brown oil (yield: 9%); ¹H NMR (400 MHz, CDCl₃) δ 8.35 (ddd, J = 4.6, 3.0, 1.5 Hz, 1H), 7.37 (ddd, J = 9.7, 8.3, 1.5 Hz, 1H), 7.24-7.20 (m, 1H), 7.08 (dd, J = 7.8, 1.6 Hz, 1H), 7.00 (ddd, J = 8.6, 7.2, 1.6 Hz, 1H), 6.92 (dd, J = 8.6, 1.5 Hz, 1H), 6.66-6.62 (m, 1H), 5.37 (t, J = 6.0 Hz, 1H), 4.69 (d, J = 1.9 Hz, 2H), 4.14-4.08 (m, 1H), 3.86-3.83 (m, 2H), 3.58 (t, J = 5.2 Hz, 2H), 3.14-3.10 (m, 2H), 2.72-2.61 (m, 2H), 1.66-1.60 (m, 3H), 1.45 (s, 9H), 1.16-1.07 (m, 3H).

### <Example 68> Synthesis of 4-((3-fluoropyridin-2-yl)methyl)-N-(piperidin-4-ylmethyl)-3,4-dihydroquinoxaline-1(2H)-carboxamide

4-((3-fluoropyridin-2-yl)methyl)-N-(piperidin-4-ylmethyl)-3,4-dihydroquinoxaline-1(2H)-carboxamide was synthesized by deprotecting the Boc group of the tert-butyl 4-((4-((3-fluoropyridin-2yl)methyl)-1,2,3,4-tetrahydroquinoxaline-1-carboxamido)methyl)piperidin-1-carboxylate obtained in Preparation Example f-10 with TFA.

White solid (yield: 86%); ¹H NMR (400 MHz, CDCl₃) δ 8.35 (ddd, J = 4.6, 3.0, 1.5 Hz, 1H), 7.37 (ddd, J = 9.8, 8.3, 1.5 Hz, 1H), 7.23-7.19 (m, 1H), 7.10 (dd, J = 7.8, 1.6 Hz, 1H), 6.99 (ddd, J = 8.7, 7.2, 1.6 Hz, 1H), 6.92 (dd, J = 8.7, 1.4 Hz, 1H), 6.64 (ddd, J = 7.8, 7.2, 1.4 Hz, 1H), 5.36 (t, J = 5.9 Hz, 1H), 4.69 (d, J = 1.9 Hz, 2H), 3.84 (dd, J = 6.9, 3.5 Hz, 2H), 3.58 (t, J = 5.1 Hz, 2H), 3.12-3.08 (m, 3H), 3.07-3.05 (m, 1H), 2.61-2.54 (m, 2H), 1.67-1.60 (m, 3H), 1.15-1.05 (m, 2H); LC/MS ESI (+): 384.3 (M+1).

### <Example 69> Synthesis of 4-((3-fluoropyridine-2-yl)methyl)-N-((1-methylpiperidin-4-yl)methyl)-3,4-dihydroquinoxaline-1(2H)-carboxamide

The 4-((3-Fluoropyridin-2-yl)methyl)-N-(piperidin-4-ylmethyl)-3,4-dihydroquinoxaline-1(2H)-carboxamide (1.0 equiv.) obtained in Example 68 was dissolved in dichloromethane (0.3 M), sodium triacetoxyborohydride (1.3 equiv.) and formaldehyde (1.1 equiv.) were added, and the resulting mixture was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize 4-((5-fluoropyridin-2-yl)methyl)-N-((1-methylpiperidin-4-yl)methyl)-3,4-dihydroquinoxaline-1(2H)-carboxamide.

White solid (yield: 82%); ¹H NMR (400 MHz, CDCl₃) δ 8.35 (ddd, J = 4.6, 3.0, 1.5 Hz, 1H), 7.37 (ddd, J = 9.8, 8.3, 1.4 Hz, 1H), 7.23-7.19 (m, 1H), 7.10 (dd, J = 7.8, 1.6 Hz, 1H), 6.99 (ddd, J = 8.6, 7.2, 1.6 Hz, 1H), 6.92 (dd, J = 8.3, 1.5 Hz, 1H), 6.64 (ddd, J = 7.8, 7.2, 1.5 Hz, 1H), 5.36 (t, J = 6.0 Hz, 1H), 4.69 (d, J = 1.9 Hz, 2H), 3.84 (t, J = 5.1 Hz, 2H), 3.58 (t, J = 5.1 Hz, 2H), 3.14-3.11 (m, 2H), 2.85-2.81 (m, 2H), 2.25 (s, 3H), 1.92-1.85 (m, 2H), 1.68-1.64 (m, 2H), 1.51-1.43 (m, 1H), 1.29-1.20 (m, 2H); LC/MS ESI (+): 398.3 (M+1).

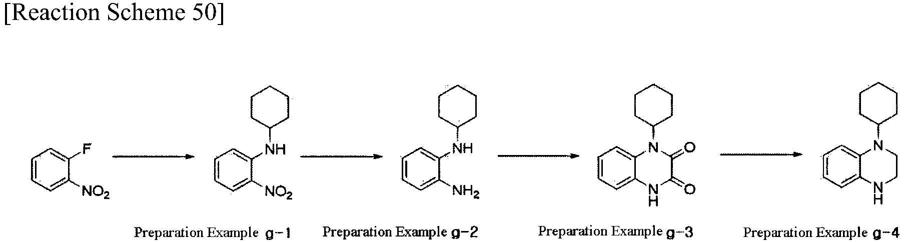

### <Preparation Method g> Preparation of 1-cyclohexyl-1,2,3,4-tetrahydroquinoxaline

1-cyclohexyl-1,2,3,4-tetrahydroquinoxaline according to the present invention was synthesized through the procedures described in Preparation Examples g-1 to g-4.

### <Preparation Example g-1> Preparation of N-cyclohexyl-2-nitroaniline

A solution in which 1-fluoro-2-nitrobenzene (1 g, 7.09 mmol) and cyclohexylamine (842.9 mg, 8.50 mmol) were mixed was stirred for 6 hours at 110 °C and then cooled to room temperature. After terminating the reaction by adding water to the reaction solution, the resulting solution n was extracted with dichloromethane. An organic layer was washed with brine, dried over MgSO₄, filtered and then concentrated under reduced pressure. The residue obtained thereby was purified by silica column chromatography using a dichloromethane/methanol mixture to prepare N-cyclohexyl-2-nitroaniline.

Orange solid (yield: 99%); ¹H NMR (400 MHz, CDCl₃) δ 8.15 (dd, J = 8.8, 1.7 Hz, 1H), 8.12 (brs, NH), 7.39 (ddd, J = 8.9, 6.6, 1.7 Hz, 1H), 6.87 (dd, J = 8.9, 1.2 Hz, 1H), 6.59 (ddd, J = 8.8, 6.6, 1.2 Hz, 1H), 3.56-3.48 (m, 1H), 2.10-2.01 (m, 2H), 1.84-1.78 (m, 2H), 1.69-1.64 (m, 1H), 1.48-1.29 (m, 5H); LC/MS ESI (+): 221.1 (M+1).

### <Preparation Example g-2> Preparation of N¹-cyclohexylbenzene-1,2-diamine

The N-cyclohexyl-2-nitroaniline (1.6 g, 7.04 mmol) obtained in Preparation Example g-1 was dissolved in EtOAc/MeOH (1:3 = v/v, 20 mL), 10% Pd/C (74 mg, 0.70 mmol) was added and stirred for 6 hours at room temperature under a hydrogen gas condition. The reaction solution was filtered through a Celite pad and then concentrated under reduced pressure. The residue obtained thereby was purified by silica column chromatography using an n-hexane/ethyl acetate mixture to prepare N¹-cyclohexylbenzene-1,2-diamine.

Brown oil (yield: 86%); ¹H NMR (400 MHz, CDCl₃) δ 6.80 (ddd, J = 7.9, 7.2, 1.6 Hz, 1H), 6.72 (dd, J = 7.9, 1.6 Hz, 1H), 6.67 (dd, J = 7.1, 1.3 Hz, 1H), 6.65-6.63 (m, 1H), 3.26-3.19 (m, 1H), 2.10-2.03 (m, 2H), 1.79-1.75 (m, 2H), 1.69-1.63 (m, 1H), 1.44-1.35 (m, 2H), 1.26-1.18 (m, 3H); LC/MS ESI (+): 191.2 (M+1).

### <Preparation Example g-3> Preparation of 1-cyclohexylquinoxaline-2,3(1H,4H)-dione

A reaction solution in which the N¹-cyclohexylbenzene-1,2-diamine (1.0 g, 5.26 mmol) obtained in Preparation Example g-2 was dissolved in diethyl oxalate (4.3 mL, 31.53 mmol) was stirred for 2 hours at 130 °C. The reaction solution was cooled to room temperature, filtered and dried, thereby preparing 1-cyclohexylquinoxaline-2,3(1H,4H)-dione.

Gray solid (yield: 80%); ¹H NMR (400 MHz, DMSO-d₆) δ 11.96 (s, 1H), 7.61-7.59 (m, 1H), 7.19-7.13 (m, 3H), 4.58-4.39 (m, 1H), 2.47-2.37 (m, 2H), 1.83-1.79 (m, 2H), 1.70-1.67 (m, 3H), 1.50-1.40 (m, 2H), 1.29-1.19 (m, 1H); LC/MS ESI (+): 245.1 (M+1).

### <Preparation Example g-4> Preparation of 1-cyclohexyl-1,2,3,4-tetrahydroquinoxaline

A borane-THF complex (1 M) (24 mL, 24.07 mmol) was slowly added to a solution in which the 1-cyclohexylquinoxaline-2,3(1H,4H)-dione (980 mg, 4.01 mmol) obtained in Preparation Example g-3 was dissolved in THF (7.0 mL) at room temperature, and then stirred for 16 hours at 65 °C. After cooling the reaction solution to room temperature and adding water terminate the reaction, the resulting solution was neutralized with a saturated NaHCO₃ aqueous solute ion and extracted with ethyl acetate. An organic layer was washed with brine, dried over MgSO₄, filtered and then concentrated under reduced pressure. The residue obtained thereby was purified by silica column chromatography using an n-hexane/ethyl acetate mixture to prepare 1-cyclohexyl-1,2,3,4-tetrahydroquinoxaline.

Orange oil (yield: 91%); ¹H NMR (400 MHz, CDCl₃) δ 6.69-6.62 (m, 2H), 6.55-6.48 (m, 2H), 3.59-3.52 (m, 1H), 3.38 (dd, J = 5.9, 3.5 Hz, 2H), 3.29 (dd, J = 5.9, 3.5 Hz, 2H), 1.92-1.82 (m, 4H), 1.75-1.69 (m, 1H), 1.50-1.33 (m, 4H), 1.21-1.10 (m, 1H); LC/MS ESI (+): 217.2 (M+1).

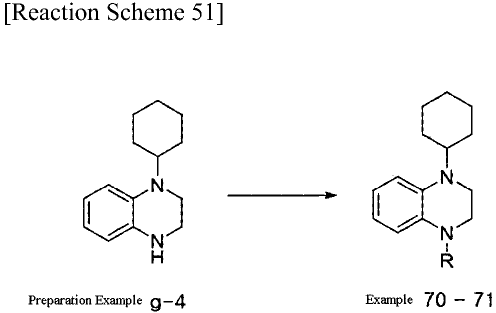

### <Example 70> Synthesis of (4-cyclohexyl-3,4-dihydroquinoxaline-1(2H)-yl)(4-methylpiperazin-1-yl)methanone

The 1-cyclohexyl-1,2,3,4-tetrahydroquinoxaline (1.0 equiv.) obtained in Preparation Example g-4 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and 1-methylpiperazine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to synthesize (4-cyclohexyl-3,4-dihydroquinoxaline-1(2H)-yl)(4-methylpiperazin-1-yl)methanone.

Brown oil (yield: 81%); ¹H NMR (400 MHz, CDCl₃) δ 6.96 (dd, J = 7.9, 1.6 Hz, 1H), 6.90 (ddd, J = 8.3, 7.3, 1.6 Hz, 1H), 6.70 (dd, J = 7.3, 1.1 Hz, 1H), 6.55 (ddd, J = 8.3, 7.9, 1.1 Hz, 1H), 3.63 (dd, J = 7.3, 1.1 Hz, 2H), 3.37-3.33 (m, 6H), 2.26-2.33 (m, 4H), 2.28 (s, 3H), 1.89-1.81 (m, 4H), 1.73-1.70 (m, 2H), 1.50-1.33 (m, 4H), 1.17-1.13 (m, 1H); LC/MS ESI (+): 343.3 (M+1).

### <Example 71> Synthesis of 4-cyclohexyl-3,4-dihydroquinoxaline-1(2H)-yl)(piperidin-1-yl)methanone

4-cyclohexyl-3,4-dihydroquinoxaline-1(2H)-yl)(piperidin-1-yl)methanone was synthesized in the same manner as described in Example 70 using piperidine instead of 1-methylpiperazine.

Red oil (yield: 80%); ¹H NMR (400 MHz, CDCl₃) δ 6.94-6.90 (m, 1H), 6.89-6.87 (m, 1H), 6.71-6.69 (m, 1H), 6.55 (ddd, J = 7.9, 7.3, 1.3 Hz, 1H), 3.61 (dd, J = 5.9, 4.8 Hz, 2H), 3.36 (dd, J = 5.9, 4.8 Hz, 2H), 3.26-3.24 (m, 4H), 1.88-1.82 (m, 4H), 1.73-1.69 (m, 1H), 1.53-1.47 (m, 7H), 1.44-1.34 (m, 4H), 1.21-1.11 (m, 1H); LC/MS ESI (+): 328.3 (M+1).

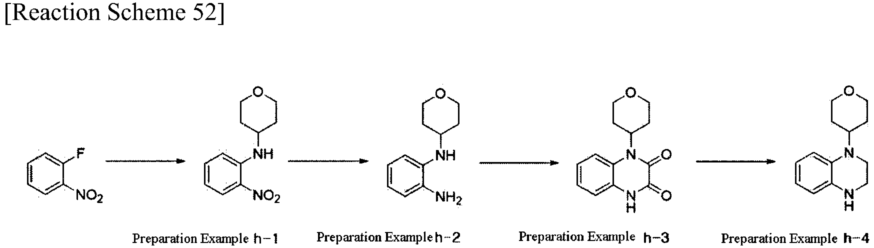

### <Preparation Method h> Preparation of 1-(tetrahydro-2H-pyran-4-yl)-1,2,3,4-tetrahydroquinoxaline

1-(tetrahydro-2H-pyran-4-yl)-1,2,3,4-tetrahydroquinoxaline according to the present invention was prepared through the procedures described in Preparation Examples h-1 to h-4 below.

### <Preparation Example h-1> Preparation of N-(2-nitrophenyl)tetrahydro-2H - pyran-4-amine

A solution in which 1-fluoro-2-nitrobenzene (1.0 equiv.) and tetrahydro-2H-pyran-4-amine (1.2 equiv.) were mixed was stirred for 6 hours at 110 °C and then cooled to room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare N-(2-nitrophenyl)tetrahydro-2H - pyran-4-amine.

Orange solid (yield: 91%); ¹H NMR (400 MHz, CDCl₃) δ 8.19 (dd, J = 8.6, 1.6 Hz, 1H), 8.11 (brs, NH), 7.43 (ddd, J = 8.6, 7.0, 1.2 Hz, 1H), 6.88 (dd, J = 8.6, 1.2 Hz, 1H), 6.65 (ddd, J = 8.4, 7.0, 1.6 Hz, 1H), 4.06-4.01 (m, 2H), 3.79-3.70 (m, 1H), 3.60-3.54 (m, 2H), 2.11-2.04 (m, 2H), 1.73-1.63 (m, 2H); LC/MS ESI (+): 223.1193.2 (M+1).

### <Preparation Example h-2> Preparation of N¹-(tetrahydro-2H-pyran-4-yl)benzene-1,2-diamine

The N-(2-nitrophenyl)tetrahydro-2H-pyran-4-amine (1.0 equiv.) obtained in Preparation Example h-1 was dissolved in EtOAc/MeOH (1:3 = v/v, 20 mL), 10% Pd/C (0.2 equiv.) was added, and then the resulting mixture was stirred for 6 hours at room temperature under a hydrogen gas condition. The reaction solution was filtered through a Celite pad and then concentrated under reduced pressure. The residue obtained thereby was purified by silica column chromatography using an n-hexane/ethyl acetate mixture to prepare N¹-(tetrahydro-2H-pyran-4-yl)benzene-1,2-diamine.

Brown solid (yield: 91%); ¹H NMR (400 MHz, CDCl₃) δ 6.82-6.78 (m, 1H), 6.76-6.73 (m, 1H), 6.71-6.67 (m, 2H), 4.04-3.99 (m, 2H), 3.56-3.49 (m, 2H), 3.48-3.43 (m, 1H), 3.37 (brs, NH2), 3.22 (brs, NH), 2.07-2.01 (m, 2H), 1.58-1.48 (m, 2H); LC/MS ESI (+): 193.2 (M+1).

### <Preparation Example h-3> Preparation of 1-(tetrahydro-2H-pyran-4-yl)-1,4-hydroquinoxaline-2,3-dione

A reaction solution in which the N¹-(tetrahydro-2H-pyran-4-yl)benzene-1,2-diamine (1.0 equiv.) obtained in Preparation Example h-2 was dissolved in diethyl oxalate (6.0 equiv.) was stirred for 2 hours at 130 °C. After cooling the reaction solution to room temperature, it was filtered and dried to 1-(tetrahydro-2H-pyran-4-yl)-1,4-hydroquinoxaline-2,3-dione.

Brown solid (yield: 70%); ¹H NMR (400 MHz, DMSO-d₆) δ 11.99 (brs, NH), 7.68-7.65 (m, 1H), 7.19-7.17 (m, 3H), 4.76-4.69 (m, 1H), 3.98-3.94 (m, 2H), 3.55-3.49 (m, 2H). 2.76-2.66 (m, 2H), 1.66-1.57 (m, 2H); LC/MS ESI (+): 247.1 (M+1).

### <Preparation Example h-4> Preparation of 1-(tetrahydro-2H-pyran-4-yl)-1,2,3,4-tetrahydroquinoxaline

A 1.0 M borane-THF complex (6.0 equiv.) was slowly added to a solution in which 1-(tetrahydro-2H-pyran-4-yl)-1,4-hydroquinoxaline-2,3-dione (1.0 equiv.) obtained in Preparation Example h-3 was dissolved in THF at room temperature, and then stirred for 16 hours at 65 °C. After cooling the reaction solution to room temperature and terminating the reaction by adding water, the resulting solution was neutralized with a saturated NaHCO₃ aqueous solution and extracted with ethyl acetate. An organic layer was washed with brine, dried over MgSO₄, filtered and then concentrated under reduced pressure. The residue obtained thereby was purified by silica column chromatography using an n-hexane/ethyl acetate mixture to prepare 1-(tetrahydro-2H-pyran-4-yl)-1,2,3,4-tetrahydroquinoxaline.

White solid (yield: 79%); ¹H NMR (400 MHz, CD₃OD) δ 6.71 (dd, J = 8.1, 1.0 Hz, 1H), 6.62-6.58 (m, 1H), 6.54-6.47 (m, 2H), 4.06-4.01 (m, 2H), 3.89-3.81 (m, 1H), 3.58-3.51 (m, 2H), 3.29-3.28 (m, 2H), 3.27-3.24 (m, 2H), 1.88-1.78 (m, 2H), 1.72-1.67 (m, 2H); LC/MS ESI (+): 219.2 (M+1).

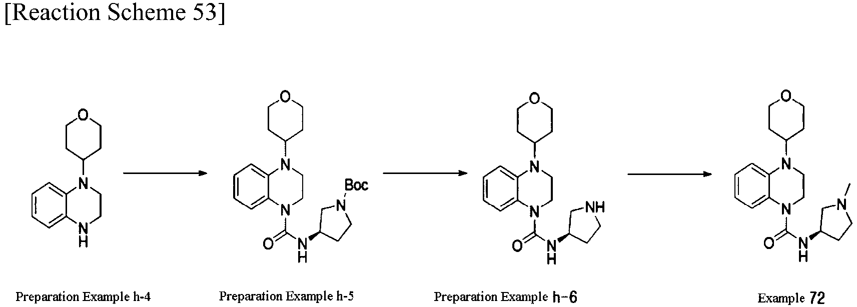

### <Preparation Example h-5> Preparation of tert-butyl (R)-3-(4-(tetrahydro-2H-pyran-4-yl)-1,2,3,4-tetrahydroquinoxaline-1-carboxamido)pyrrolidin-1-carboxylate

The 1-(tetrahydro-2H-pyran-4-yl)-1,2,3,4-tetrahydroquinoxaline (1.0 equiv.) obtained in Preparation Example h-4 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (R)-(+)-1-Boc-3-aminopyrrolidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl (R)-3-(4-(tetrahydro-2H-pyran-4-yl)-1,2,3,4-tetrahydroquinoxaline-1-carboxamido)pyrrolidin-1-carboxylate.

White solid (yield: 94%); ¹H NMR (400 MHz, CDCl₃) δ 7.08-7.04 (m, 2H), 6.82-6.79 (m, 1H), 6.65-6.62 (m, 1H), 5.28 (d, J = 6.7 Hz, 1H), 4.38-4.32 (m, 1H), 4.10-4.08 (m, 2H), 3.92-3.85 (m, 1H), 3.79-3.77 (m, 1H), 3.73-3.71 (m, 1H), 3.65-3.61 (m, 1H), 3.54-3.48 (m, 2H), 3.39-3.34 (m, 2H), 3.32-3.29 (m, 2H), 3.13-3.10 (m, 1H), 2.15-2.10 (m, 1H), 1.91-1.82 (m, 3H), 1.74-1.71 (m, 2H), 1.44 (s, 9H).

### <Preparation Example h-6> Preparation of (R)-N-(pyrrolidin-3-yl)-4-(tetrahydro-2H-pyran-4-yl)-3,4-dihydroquinoxaline-1(2H)-carboxamide

(R)-N-(pyrrolidin-3-yl)-4-(tetrahydro-2H-pyran-4-yl)-3,4-dihydroquinoxaline-1(2H)-carboxamide was prepared by deprotecting the Boc group of the tert-butyl (R)-3-(4-(tetrahydro-2H-pyran-4-yl)-1,2,3,4-tetrahydroquinoxaline-1-carboxamido)pyrrolidin-1-carboxylate obtained in Preparation Example h-5 with TFA.

White solid (yield: 84%); ¹H NMR (400 MHz, CDCl₃) δ 7.13 (dd, J = 7.8, 1.6 Hz, 1H), 7.05 (ddd, J = 7.8, 7.5, 1.2 Hz, 1H), 6.80 (dd, J = 8.3, 1.2 Hz, 1H), 6.64 (ddd, J = 8.3, 7.5, 1.6 Hz, 1H), 5.44 (d, J = 6.7 Hz, 1H), 4.35-4.28 (m, 1H), 4.10-4.07 (m, 2H), 3.75 (dd, J = 5.4, 4.8 Hz, 2H), 3.54-3.47 (m, 2H), 3.31 (dd, J = 5.4, 4.8 Hz, 2H), 3.55-3.47 (m, 2H), 3.34-3.18 (m, 2H), 3.17-3.05 (m, 1H), 3.04-2.96 (m, 1H), 2.91-2.87 (m, 1H), 2.23-2.15 (m, 1H), 1.87-1.79 (m, 1H), 1.74-1.66 (m, 2H); LC/MS ESI (+): 331.2 (M+1).

### <Example 72> Synthesis of (R)-N-(1-methylpyrrolidin-3-yl)-4-(tetrahydro-2H-pyran-4-yl)-3,4-dihydroquinoxaline-1 (2H)-carboxamide

The (R)-N-(pyrrolidin-3-yl)-4-(tetrahydro-2H-pyran-4-yl)-3,4-dihydroquinoxaline-1(2H)-carboxamide (1.0 equiv.) obtained in Preparation Example h-6 was dissolved in dichloromethane (0.3 M), sodium triacetoxyborohydride (1.3 equiv.) and formaldehyde (1.1 equiv.) were added, and the resulting mixture was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (R)-N-(1-methylpyrrolidin-3-yl)-4-(tetrahydro-2H-pyran-4-yl)-3,4-dihydroquinoxaline-1(2H)-carboxamide.

Light yellow oil (yield: 83%); ¹H NMR (400 MHz, CDCl₃) δ 7.11 (dd, J = 7.8, 1.6 Hz, 1H), 7.03 (ddd, J = 8.8, 7.4, 1.6 Hz, 1H), 6.79 (dd, J =8.8, 1.3 Hz, 1H), 6.63 (ddd, J = 7.8, 7.4, 1.3 Hz, 1H), 5.44 (d, J = 7.5, 1H), 4.40-4.32 (m, 1H), 4.1-4.07 (m, 2H), 3.91-3.83 (m, 2H), 3.69-3.67 (m, 1H), 3.54-3.48 (m, 2H), 3.33-3.24 (m, 2H), 2.77-2.71 (m, 1H), 2.58-2.51 (m, 2H), 2.29 (s, 3H), 2.23-2.17 (m, 1H), 1.90-1.80 (m, 2H), 1.75-1.69 (m, 3H), 1.57-1.49 (m, 1H); LC/MS ESI (+): 345.2 (M+1).

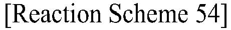

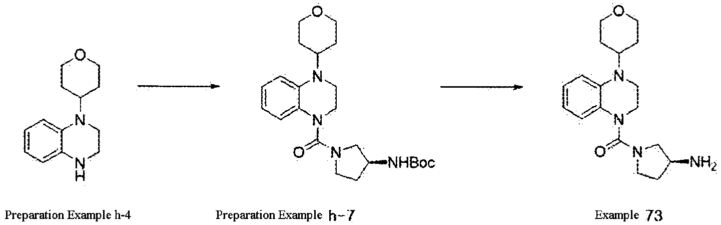

### <Preparation Example h-7> Preparation of tert-butyl (S)-(1-(4-(tetrahydro-2H-pyran-4-yl)-1,2,3,4-tetrahydroquinoxaline-1-carboxamido)pyrrolidin-3-yl)carbamate

Tert-butyl (S)-(1-(4-(tetrahydro-2H-pyran-4-yl)-1,2,3,4-tetrahydroquinoxaline-1-carboxamido)pyrrolidin-3-yl)carbamate was prepared in the same manner as described in Preparation Example h-5 using (S)-(-)-3-(Boc-amino)pyrrolidine instead of (R)-(+)-1-Boc-3-aminopyrrolidine.

Ivory solid (yield: 90%); ¹H NMR (400 MHz, CDCl₃) δ 6.92 (ddd, J = 8.5, 7.3, 1.6 Hz, 1H), 6.86 (dd, J = 7.9, 1.6 Hz, 1H), 6.74 (dd, J = 8.5, 1.1 Hz, 1H), 6.59 (dd, J = 7.9, 7.3, 1.1 Hz, 1H), 4.55-4.52 (m, 1H), 4.14-4.08 (m, 3H), 3.91-3.83 (m, 1H), 3.69-3.67 (m, 2H), 3.55-3.48 (m, 2H), 3.45-3.42 (m, 2H), 3.39-3.36 (m, 2H), 3.07-3.03 (m, 1H), 2.12-2.04 (m, 1H), 1.92-1.80 (m, 2H), 1.77-1.72 (m, 3H), 1.42 (s, 9H).

### <Example 73> Synthesis of (S)-(3-aminopyrrolidin-1-yl)(4-(tetrahydro-2H-pyran-4-yl)-3,4-dihydroquinoxaline-1(2H)-yl)methanone

(S)-(3-aminopyrrolidin-1-yl)(4-(tetrahydro-2H-pyran-4-yl)-3,4-dihydroquinoxaline-1(2H)-yl)methanone was synthesized by deprotecting the Boc group of the tert-butyl (S)-(1-(4-(tetrahydro-2H-pyran-4-yl)-1,2,3,4-tetrahydroquinoxaline-1-carboxamido)pyrrolidin-3-yl)carbamate obtained in Preparation Example h-7 with TFA.

Yellow solid (yield: 70%); ¹H NMR (400 MHz, CDCl₃) δ 6.93-6.87 (m, 2H), 6.73 (dd, J = 8.2, 1.3 Hz, 1H), 6.60 (ddd, J = 7.9, 7.2, 1.3 Hz, 1H), 4.11-4.07 (m, 2H), 3.90-3.82 (m, 1H), 3.76-3.70 (m, 1H), 3.66-3.61 (m, 1H), 3.55-3.42 (m, 4H), 3.39-3.32 (m, 3H), 2.99-2.94 (m, 1H), 1.91-1.81 (m, 2H), 1.76-1.71 (m, 2H), 1.65-1.56 (m, 1H), 1.44-1.37 (m, 2H); LC/MS ESI (+): 331.2 (M+1).

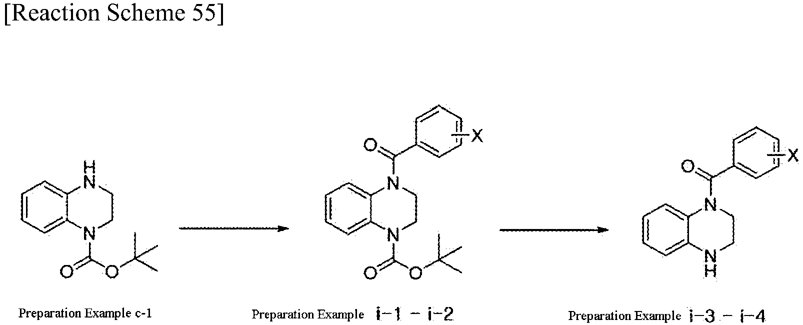

### <Preparation Method i> Preparation of (3,4-dihydroquinoxaline-1(2H)-yl)(phenyl)methanone or halogen-substituted (3,4-dihydroquinoxaline-1(2H)-yl)(phenyl)methanone

(3,4-dihydroquinoxaline-1(2H)-yl)(phenyl)methanone or halogen-substituted (3,4-dihydroquinoxaline-1(2H)-yl)(phenyl)methanone according to the present invention was prepared through the procedures described in Preparation Examples i-1 to i-4 below.

### <Preparation Example i-1> Preparation of tert-butyl 4-benzoyl-3,4-dihydroquinoxaline-1(2H)-carboxylate

The tert-butyl 3,4-dihydroquinoxaline-1(2H)-carboxylate (473.0 mg, 2.02 mmol) obtained in Preparation Example c-1 was dissolved in THF (20 mL), benzoyl chloride (141.9 mg, 1.01 mmol) was added, and the resulting mixture was stirred for 3 hours at room temperature. After terminating the reaction by adding water to the reaction solution, the resulting solution was neutralized with a saturated NaHCO₃ aqueous solution and extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl 4-benzoyl-3,4-dihydroquinoxaline-1(2H)-carboxylate.

Yellow oil (yield: 100%); ¹H NMR (400 MHz, CDCl₃) δ 7.88 (d, J = 8.4 Hz, 1H), 7.49-7.41 (m, 2H), 7.40-7.36 (m, 1H), 7.31-7.27 (m, 2H), 7.05 (ddd, J = 8.1, 7.4, 1.5 Hz, 1H), 6.73 (ddd, J = 8.4, 7.4, 1.5 Hz, 1H), 6.60 (d, J = 8.1 Hz, 1H), 4.06 (t, J = 6.0 Hz, 2H), 3.95 (t, J = 6.0 Hz, 2H), 1.57 (s, 9H).

### <Preparation Example i-2> Preparation of tert-butyl 4-(4-fluorobenzoyl)-3,4-dihydroquinoxaline-1(2H)-carboxylate

Tert-butyl 4-(4-fluorobenzoyl)-3,4-dihydroquinoxaline-1(2H)-carboxylate was prepared in the same manner as described in Preparation Example i-1 using 4-fluorobenzoyl chloride instead of benzoyl chloride.

Colorless oil (yield: 93%); ¹H NMR (400 MHz, CDCl₃) δ 7.89 (d, J = 8.4 Hz, 1H), 7.46-7.41 (m, 2H), 7.07 (ddd, J = 8.4, 7.4, 1.5 Hz, 1H), 6.99-6.94 (m, 2H), 6.75 (ddd, J = 8.1, 7.4, 1.5 Hz, 1H), 6.54 (d, J = 8.1 Hz, 1H), 4.08-4.04 (m, 2H), 3.96-3.93 (m, 2H), 1.57 (s, 9H).

### <Preparation Example i-3> Preparation of (3,4-dihydroquinoxaline-1(2H)-yl)(phenyl)methanone

(3,4-dihydroquinoxaline-1(2H)-yl)(phenyl)methanone was prepared by deprotecting the Boc group of the tert-butyl 4-benzoyl-3,4-dihydroquinoxaline-1(2H)-carboxylate obtained in Preparation Example i-1 with TFA.

Light yellow oil (yield: 92%); ¹H NMR (400 MHz, CDCl₃) δ 7.47-7.44 (m, 2H), 7.39-7.35 (m, 1H), 7.33-7.29 (m, 2H), 6.87 (ddd, J = 8.3, 7.3, 1.4 Hz, 1H), 6.62-6.57 (m, 2H), 6.38-6.34 (m, 1H), 4.13 (brs, NH), 3.97 (t, J = 5.2 Hz, 2H), 3.54 (t, J = 5.2 Hz, 2H); LC/MS ESI (+): 239.1 (M+1).

### <Preparation Example i-4> Preparation of (3,4-dihydroquinoxaline-1(2H)-yl)(4-fluorophenyl)methanone

(3,4-dihydroquinoxaline-1(2H)-yl)(4-fluorophenyl)methanone was prepared by deprotecting the Boc group of the tert-butyl 4-(4-fluorobenzoyl)-3,4-dihydroquinoxaline-1(2H)-carboxylate obtained in Preparation Example i-2 with TFA.

White solid (yield: 93%); ¹H NMR (400 MHz, CDCl₃) δ 7.48-7.43 (m, 2H), 7.01-6.96 (m, 2H), 6.87 (d, J = 8.4, 7.3, 1.4 Hz, 1H), 6.59 (dd, J = 8.1, 1.4 Hz, 1H), 6.54-6.48 (m, 1H), 6.38-6.34 (m, 1H), 4.14 (brs, NH), 3.97 (t, J = 5.5 Hz, 2H), 3.56 (t, J = 5.5 Hz, 2H); LC/MS ESI (+): 257.1 (M+1).

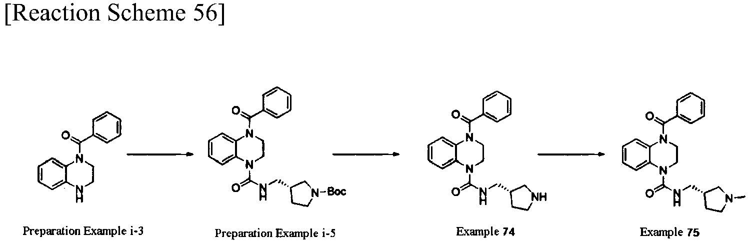

### <Preparation Example i-5> Preparation of tert-butyl (R)-3-((4-benzoyl-1,2,3,4-tetrahydroquinoxaline-1-carboxamido)methyl)pyrrolidin-1-carboxylate

The (3,4-dihydroquinoxaline-1(2H)-yl)(phenyl)methanone (1.0 equiv.) obtained in Preparation Example i-3 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and (R)-1-Boc-3-(aminomethyl)pyrrolidine (1.2 equiv.) were added to the reaction solution, and the resulting solution was stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl (R)-3-((4-benzoyl-1,2,3,4-tetrahydroquinoxaline-1-carboxamido)methyl)pyrrolidin-1-carboxylate.

White solid (yield: 94%); ¹H NMR (400 MHz, CDCl₃) δ 7.43-7.42 (m, 1H), 7.40-7.38 (m, 3H), 7.34-7.31 (m, 2H), 7.12-7.08 (m, 1H), 6.89-6.83 (m, 2H), 5.39-5.27 (m, 1H), 4.07-4.03 (m, 2H), 3.98-3.94 (m, 2H), 3.55-3.43 (m, 3H), 3.33-3.28 (m, 1H), 3.10-2.99 (m, 1H), 2.50-2.45 (m, 1H), 2.05-1.97 (m, 1H), 1.69-1.60 (m, 1H), 1.46 (s, 9H).

### <Example 74> Synthesis of (S)-4-benzoyl-N-(pyrrolidin-3-ylmethyl)-3,4-dihydroquinoxaline-1(2H)-carboxamide

(S)-4-benzoyl-N-(pyrrolidin-3-ylmethyl)-3,4-dihydroquinoxaline-1(2H)-carboxamide was synthesized by deprotecting the Boc group of the tert-butyl (R)-3-((4-benzoyl-1,2,3,4-tetrahydroquinoxaline-1-carboxamido)methyl)pyrrolidin-1-carboxylate obtained in Preparation Example i-5 with TFA.

White solid (yield: 92%); ¹H NMR (400 MHz, CD₃OD) δ 7.55 (dd, J = 8.3, 1.4 Hz, 1H), 7.46-7.39 (m, 3H), 7.35-7.31 (m, 2H), 7.16-7.10 (m, 1H), 6.85-6.81 (m, 1H), 6.69 (d, J = 8.0 Hz, 1H), 4.09-4.05 (m, 2H), 3.94-3.89 (m, 2H), 3.36-3.30 (m, 1H), 3.23-3.16 (m, 2H), 3.12-3.06 (m, 1H), 2.89-2.85 (m, 1H), 2.59-2.51 (m, 1H), 2.11-2.0 (m, 2H), 1.73-1.66 (m, 1H); LC/MS ESI (+): 365.2 (M+1).

### <Example 75> Synthesis of (R)-4-benzoyl-N-((1-methylpirrolidin-3-yl)methyl)-3,4-dihydroquinoxaline-1(2H)-carboxamide

The (S)-4-benzoyl-N-(pyrrolidin-3-ylmethyl)-3,4-dihydroquinoxaline-1(2H)-carboxamide (1.0 equiv.) obtained in Example 74 was dissolved in dichloromethane (0.3 M), sodium triacetoxyborohydride (1.5 equiv.) and formaldehyde (2.0 equiv.) were added, and the resulting mixture was stirred for 1 hour at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using a dichloromethane/methanol mixture to synthesize (R)-4-benzoyl-N-((1-methylpirrolidin-3-yl)methyl)-3,4-dihydroquinoxaline-1(2H)-carboxamide.

Light yellow solid (yield: 82%); ¹H NMR (400 MHz, CD₃OD) δ 7.52 (dd, J = 8.2, 1.2 Hz, 1H), 7.45-7.39 (m, 3H), 7.35-7.31 (m, 2H), 7.13 (ddd, J = 8.1, 7.5, 1.4 Hz, 1H), 6.88 (ddd, J = 8.2, 7.5, 1.4 Hz, 1H), 6.68 (d, J = 8.1 Hz, 1H), 4.08-4.05 (m, 2H), 3.93-3.89 (m, 2H), 3.27 (d, J = 7.1 Hz, 2H), 2.79-2.75 (m, 1H), 2.65-2.62 (m, 2H), 2.58-2.5 (m, 1H), 2.43-2.39 (m, 1H), 2.36 (s, 3H), 2.09-2.00 (m, 1H), 1.66-1.58 (m, 1H); LC/MS ESI (+): 379.1 (M+1).

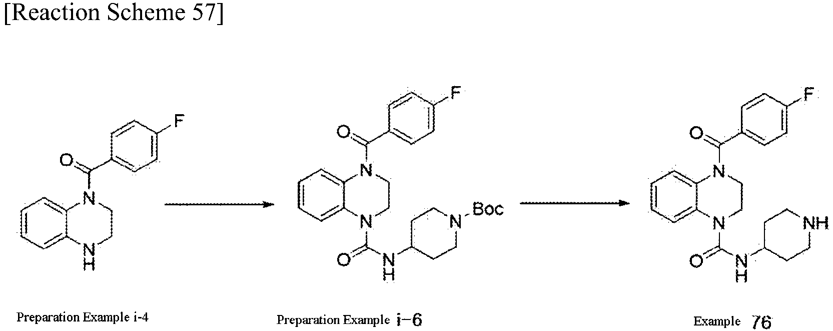

### <Preparation Example i-6> Preparation of tert-butyl-4-(4-(4-fluorobenzoyl)-1,2,3,4-tetrahydroquinoxaline-1-carboxamido)piperidin-1-carboxylate

The (3,4-dihydroquinoxaline-1(2H)-yl)(4-fluorophenyl)methanone (1.0 equiv.) obtained in Preparation Example i-4 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and 1-Boc-4-aminopiperidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl 4-(4-(4-fluorobenzoyl)-1,2,3,4-tetrahydroquinoxaline-1-carboxamido)piperidin-1-carboxylate.

White solid (yield: 96%); ¹H NMR (400 MHz, CDCl₃) δ 7.42-7.37 (m, 3H), 7.10 (ddd, J = 8.0, 7.4, 1.5 Hz, 1H), 7.02-7.08 (m, 2H), 6.88 (ddd, J = 8.4, 7.4, 1.5 Hz, 1H), 6.78-6.76 (m, 1H), 5.01 (d, J = 7.6 Hz, 1H), 4.13-4.05 (m, 4H), 3.97-3.87 (m, 3H), 2.92-2.85 (m, 2H), 2.01-1.96 (m, 2H), 1.45 (s, 9H), 1.39-1.29 (m, 2H).

### <Example 76> Synthesis of 4-(4-fluorobenzoyl)-N-(piperidin-4-yl)-3,4-dihydroquinoxaline-1(2H)-carboxamide

4-(4-fluorobenzoyl)-N-(piperidin-4-yl)-3,4-dihydroquinoxaline-1(2H)-carboxamide was synthesized by deprotecting the Boc group of the (tert-butyl 4-(4-(4-fluorobenzoyl)-1,2,3,4-tetrahydroquinoxaline-1-carboxamido)piperidin-1-carboxylate obtained in Preparation Example i-6 with TFA.

White solid (yield: 87%); ¹H NMR (400 MHz, CD₃OD) δ 7.55 (dd, J = 8.3, 1.1 Hz, 1H), 7.50-7.45 (m, 2H), 7.13 (ddd, J = 8.2, 7.4, 1.5 Hz, 1H), 7.09-7.03 (m, 2H), 6.83 (ddd, J = 8.3, 7.4, 1.5 Hz, 1H), 6.66 (d, J = 8.2 Hz, 1H), 4.07-4.05 (m, 2H), 3.92-3.89 (m, 2H), 3.83-3.76 (m, 1H), 3.14-3.08 (m, 2H), 2.75-2.68 (m, 2H), 1.99-1.96 (m, 2H), 1.58-1.48 (m, 2H); LC/MS ESI (+): 383.3 (M+1).

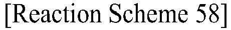

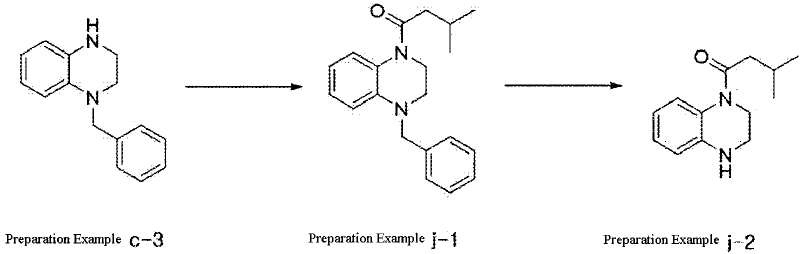

### <Preparation Method j> Preparation of 1-(3,4-dihydroquinoxaline-1(2H)-yl)-3-methylbutan-1-one

1-(3,4-dihydroquinoxaline-1(2H)-yl)-3-methylbutan-1-one according to the present invention was synthesized through the procedures described in Preparation Examples j-1 and j-2 below.

### <Preparation Example j-1> Preparation of 1-(4-benzyl-3,4-dihydroquinoxaline-1(2H)-yl)-3-methylbutan-1-one

The 1-benzyl-1,2,3,4-tetrahydroquinoxaline (1.0 equiv.) obtained in Preparation Example c-3 was dissolved in THF (0.3 M), isovaleryl chloride (1.5 equiv.) and TEA (2.0 equiv.) were slowly added at 0 °C, and the resulting mixture was stirred for 1.5 hours. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare 1-(4-benzyl-3,4-dihydroquinoxaline-1(2H)-yl)-3-methylbutan-1-one.

Light yellow solid (yield: 97%); ¹H NMR (400 MHz, CD₃OD) δ 7.31-7.28 (m, 2H), 7.24-7.19 (m, 3H), 7.07-6.99 (m, 1H), 6.73 (d, J = 8.3 Hz, 1H), 6.65-6.61 (m, 1H), 4.57 (s, 2H), 3.91 (t, J = 5.5 Hz, 2H), 3.45 (t, J = 5.5 Hz, 2H), 2.48 (d, J = 7.2 Hz, 2H), 2.07-2.02 (m, 1H), 0.89 (d, J = 6.7 Hz, 6H); LC/MS ESI (+): 309.3 (M+1).

### <Preparation Example j-2> Preparation of 1-(3,4-dihydroquinoxaline-1(2H)-yl)-3-methylbutan-1-one

The 1-(4-benzyl-3,4-dihydroquinoxaline-1(2H)-yl)-3-methylbutan-1-one (1.0 equiv.) obtained in Preparation Example j-1 was dissolved in MeOH/THF (v/v = 2: 1, 0.4 M), 10% Pd/C (0.5 equiv.) was added, and the resulting mixture was stirred for 1.5 hours at room temperature under a hydrogen gas condition. The reaction solution was filtered through a Celite pad and concentrated under reduced pressure. The residue obtained thereby was purified by silica column chromatography using an n-hexane/ethyl acetate mixture to prepare 1-(3,4-dihydroquinoxaline-1(2H)-yl)-3-methylbutan-1-one.

Yellow solid (yield: 92.5%); ¹H NMR (400 MHz, CD₃OD) δ 7.00-6.96 (m, 2H), 6.63 (dd, J = 8.1, 1.4 Hz, 1H), 6.59-6.55 (m, 1H), 3.77 (t, J = 5.5 Hz, 2H), 3.37 (t, J = 5.5 Hz, 2H), 2.49 (d, J = 7.2 Hz, 2H), 2.10-2.02 (m, 1H), 0.85 (d, J = 6.7 Hz, 6H); LC/MS ESI (+): 219.1 (M+1).

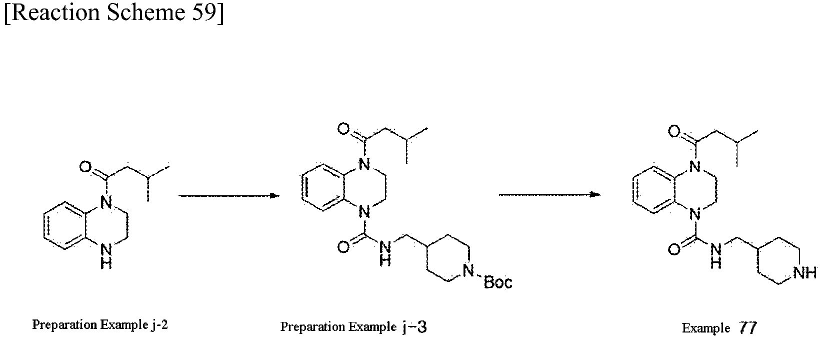

### <Preparation Example j-3> Preparation of tert-butyl 4-((4-(3-methylbutanoyl)-1,2,3,4-tetrahydroquinoxaline-1-carboxamido)methyl)piperidin-1-carboxylate

The 1-(3,4-dihydroquinoxaline-1(2H)-yl)-3-methylbutan-1-one (1.0 equiv.) obtained in Preparation Example j-2 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and 1-Boc-4-(aminomethyl)piperidine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl 4-((4-(3-methylbutanoyl)-1,2,3,4-tetrahydroquinoxaline-1-carboxamido)methyl)piperidin-1-carboxylate.

White solid (yield: 94%); ¹H NMR (400 MHz, CD₃OD) δ 7.55 (d, J = 8.1 Hz, 1H), 7.33-7.24 (m, 2H), 7.16 (ddd, J = 8.1, 7.7, 1.5 Hz, 1H), 4.09-4.04 (m, 2H), 3.95 (t, J = 6.4 Hz, 2H), 3.79 (t, J = 6.4 Hz, 2H), 3.11 (d, J = 6.6 Hz, 2H), 2.77-2.69 (m, 1H), 2.46 (d, J = 7.2 Hz, 2H), 2.08-2.04 (m, 1H), 1.76-1.66 (m, 4H), 1.42 (s, 9H), 1.12-1.03 (m, 2H), 0.85 (d, J = 6.2 Hz, 6H).

### <Example 77> Synthesis of 4-(3-methylbutanoyl)-N-(piperidin-4-ylmethyl)-3,4-dihydroquinoxaline-1(2H)-carboxamide

4-(3-methylbutanoyl)-N-(piperidin-4-ylmethyl)-3,4-dihydroquinoxaline-1(2H)-carboxamide was synthesized by deprotecting the Boc group of the tert-butyl 4-((4-(3-methylbutanoyl)-1,2,3,4-tetrahydroquinoxaline-1-carboxamido)methyl)piperidin-1-carboxylate obtained in Preparation Example j-3 with TFA.

Ivory solid (yield: 95%); ¹H NMR (400 MHz, CD₃OD) δ 7.57 (d, J = 8.1 Hz, 1H), 7.47-7.19 (m, 2H), 7.16 (ddd, J = 8.1, 7.7, 1.4 Hz, 1H), 3.94 (t, J = 6.5 Hz, 2H), 3.79 (t, J = 6.5 Hz, 2H), 3.09 (d, J = 6.4 Hz, 2H), 3.06-3.02 (m, 2H), 2.59-2.52 (m, 2H), 2.45 (d, J = 7.2 Hz, 2H), 2.08-2.02 (m, 1H), 1.73-1.63 (m, 3H), 1.18-1.13 (m, 2H), 0.83 (d, J = 6.2 Hz, 6H); LC/MS ESI (+): 359.0 (M+1).

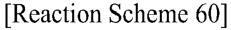

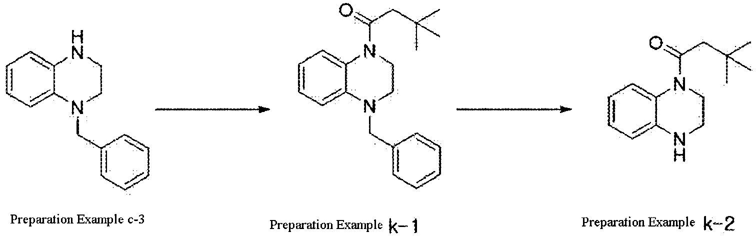

### <Preparation Method k> Preparation of 1-(3,4-dihydroquinoxaline-1(2H)-yl)-3,3-dimethylbutan-1-one

1-(3,4-dihydroquinoxaline-1(2H)-yl)-3,3-dimethylbutan-1-one according to the present invention was prepared through the procedures described in Preparation Examples k-1 and k-2 below.

### <Preparation Example k-1> Preparation of 1-(4-benzyl-3,4-dihydroquinoxaline-1(2H)-yl)-3,3-dimethylbutan-1-one

The 1-benzyl-1,2,3,4-tetrahydroquinoxaline (1.0 equiv.) obtained in Preparation Example c-3 was dissolved in THF (0.4 M), 3,3-dimethylbutyryl chloride (1.5 equiv.) and TEA (2.0 equiv.) were slowly added at 0 °C, and then the resulting mixture was stirred for 1.5 hours. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare 1-(4-benzyl-3,4-dihydroquinoxaline-1(2H)-yl)-3,3-dimethylbutan-1-one.

Yellow oil (yield: 99%); ¹H NMR (400 MHz, CD₃OD) δ 7.32-7.28 (m, 2H), 7.24-7.20 (m, 3H), 7.06-6.99 (m, 2H), 6.68 (dd, J = 8.4, 1.2 Hz, 1H), 6.66-6.61 (m, 1H), 4.57 (s, 2H), 3.91 (t, J = 5.5 Hz, 2H), 3.47 (t, J = 5.5 Hz, 2H), 2.57 (s, 2H), 0.90 (s, 9H); LC/MS ESI (+): 323.3 (M+1).

### <Preparation Example k-2> Preparation of 1-(3,4-dihydroquinoxaline-1(2H)-yl)-3,3-dimethylbutan-1-one

The 1-(4-benzyl-3,4-dihydroquinoxaline-1(2H)-yl)-3,3-dimethylbutan-1-one (1.0 equiv.) obtained in Preparation Example k-1 was dissolved in MeOH/THF (v/v = 2: 1, 0.4 M), 10% Pd/C (0.5 equiv.) was added, and the resulting mixture was stirred for 1.5 hours at room temperature under a hydrogen gas condition. The reaction solution was filtered through a Celite pad and concentrated under reduced pressure. The residue obtained thereby was purified by silica column chromatography using an n-hexane/ethyl acetate mixture to prepare 1-(3,4-dihydroquinoxaline-1(2H)-yl)-3,3-dimethylbutan-1-one.

Light yellow solid (yield: 92%); ¹H NMR (400 MHz, CD₃OD) δ 7.03-6.93 (m, 2H), 6.68 (dd, J = 8.2, 1.4 Hz, 1H), 6.68 (ddd, J = 8.2, 7.6, 1.4 Hz, 1H), 3.76 (t, J = 5.5 Hz, 2H), 3.47 (t, J = 5.5 Hz, 2H), 2.58 (s, 2H), 0.98 (s, 9H); LC/MS ESI (+): 233.0 (M+1).

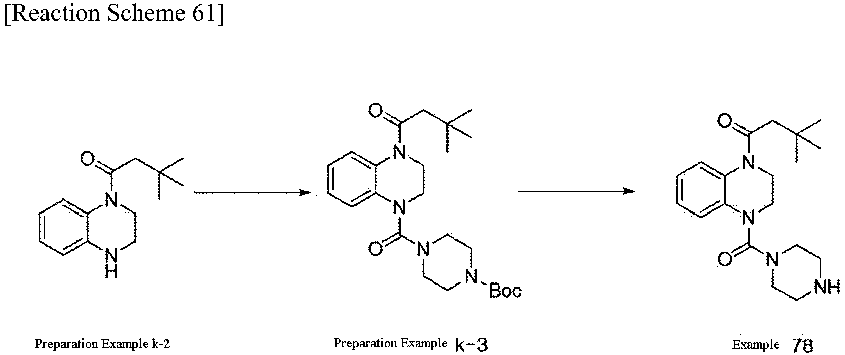

### <Preparation Example k-3> Preparation of tert-butyl 4-((4-(3,3-dimethylbutanoyl)-1,2,3,4-tetrahydroquinoxaline-1-carboxamido)piperazin-1-carboxylate

The 1-(3,4-dihydroquinoxaline-1(2H)-yl)-3,3-dimethylbutan-1-one (1.0 equiv.) obtained in Preparation Example k-2 was dissolved in dichloromethane (0.3 M), TEA (3.0 equiv.) and triphosgene (0.6 equiv.) were slowly added at 0 °C, and the resulting mixture was stirred for 1 hour at 0 °C. TEA (2.0 equiv.) and 1-Boc-piperzine (1.2 equiv.) were added to the reaction solution and stirred for 3 hours at room temperature. After terminating the reaction by adding water to the reaction solution, the resulting solution was extracted with dichloromethane. An organic layer was washed with brine, dried over MgSO₄, filtered and then concentrated under reduced pressure. The residue obtained thereby was purified by silica column chromatography using an n-hexane/ethyl acetate mixture to prepare tert-butyl 4-((4-(3,3-dimethylbutanoyl)-1,2,3,4-tetrahydroquinoxaline-1-carboxamido)piperazin-1-carboxylate.

White solid (yield: 59%); ¹H NMR (400 MHz, CD₃CN) δ 7.38-7.30 (m, 1H), 7.16-7.10 (m, 2H), 7.01 (ddd, J = 7.9, 6.8, 1.9 Hz, 1H), 3.84 (t, J = 6.3 Hz, 2H), 3.66 (t, J = 6.3 Hz, 2H), 3.33-3.31 (m, 4H), 3.23-3.18 (m, 4H), 2.48 (s, 2H), 1.42 (s, 9H), 0.98 (s, 9H).

### <Example 78> Synthesis of 3,3-dimethyl-1-(4-(piperazine-1-carbonyl)-3,4-dihydroquinoxaline-1(2H)-yl)butan-1-one

3,3-dimethyl-1-(4-(piperazine-1-carbonyl)-3,4-dihydroquinoxaline-1(2H)-yl)butan-1-one was synthesized by deprotecting the Boc group of the tert-butyl 4-((4-(3,3-dimethylbutanoyl)-1,2,3,4-tetrahydroquinoxaline-1-carboxamido)piperazin-1-carboxylate obtained in Preparation Example k-3 with TFA.

Colorless oil (yield: 74%); ¹H NMR (400 MHz, CD₃CN) δ 7.39-7.31 (m, 1H), 7.14-7.09 (m, 2H), 6.98 (ddd, J = 7.9, 6.8, 1.9 Hz, 1H), 3.84 (t, J = 6.3 Hz, 2H), 3.64 (t, J = 6.3 Hz, 2H), 3.21-3.19 (m, 4H), 2.69-2.67 (m, 4H), 2.46 (s, 2H), 0.96 (s, 9H); LC/MS ESI (+): 345.2 (M+1).

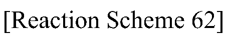

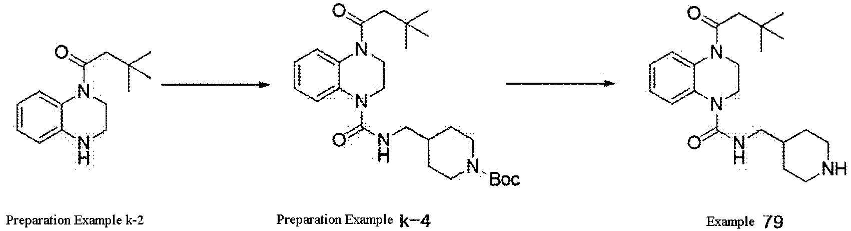

### <Preparation Example k-4> Preparation of tert-butyl 4-((4-(3,3-dimethylbutanoyl)-1,2,3,4-tetrahydroquinoxaline-1-carboxamido)methyl)piperidin-1-carboxylate

Tert-butyl 4-((4-(3,3-dimethylbutanoyl)-1,2,3,4-tetrahydroquinoxaline-1-carboxamido)methyl)piperidin-1-carboxylate was prepared in the same manner as described in Preparation Example k-3 using 1-Boc-4-(aminomethyl)piperidine instead of 1-Boc-piperzine.

White solid (yield: 93%); ¹H NMR (400 MHz, CD₃OD) δ 7.55 (d, J = 8.3 Hz, 1H), 7.37-7.21 (m, 2H), 7.18-7.13 (m, 1H), 4.09-4.04 (m, 2H), 3.95 (t, J = 6.4 Hz, 2H), 3.78 (t, J = 6.4 Hz, 2H), 3.11 (d, J = 6.5 Hz, 2H), 2.68-2.64 (m, 2H), 1.95 (s, 2H), 1.76-1.67 (m, 3H), 1.45 (s, 9H), 1.12-1.03 (m, 2H), 0.92 (s, 9H).

### <Example 79> Synthesis of 4-(3,3-dimethylbutanoyl)-N-(piperidin-4-ylmethyl)-3,4-dihydroquinoxaline-1(2H)-carboxamide

4-(3,3-dimethylbutanoyl)-N-(piperidin-4-ylmethyl)-3,4-dihydroquinoxaline-1(2H)-carboxamide was synthesized by deprotecting the Boc group of the tert-butyl 4-((4-(3,3-dimethylbutanoyl)-1,2,3,4-tetrahydroquinoxaline-1-carboxamido)methyl)piperidin-1-carboxylate obtained in Preparation Example k-4 with TFA.

White solid (yield: 46%); ¹H NMR (400 MHz, CD₃OD) δ 7.59 (d, J = 8.1 Hz, 1H), 7.34-7.20 (m, 2H), 7.18-7.13 (m, 1H), 3.95 (t, J = 6.4 Hz, 2H), 3.80 (t, J = 6.4 Hz, 2H), 3.20-3.16 (m, 2H), 3.13-3.09 (m, 2H), 2.74-2.67 (m, 2H), 2.53 (s, 2H), 1.81-1.69 (m, 3H), 1.30-1.20 (m, 2H), 0.92 (s, 9H); LC/MS ESI (+): 373.1 (M+1).

The compounds of the present invention prepared in the above examples are summarized in Table 1 below. Throughout the specification, the term "example" may be referred to as the term "compound." For example, Example 1 may be referred to as Compound 1.

**[Table 1]**

| **Number** | **Structure** | **IUPAC Name** |
|---|---|---|
| 1 | 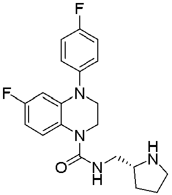 | (*R*)-6-fluoro-4-(4-fluorophenyl)-*N*-(pyrrolidin-2-ylmethyl)-3,4-dihydroquinoxaline-1(*2H*)-carboxamide |
| 2 | 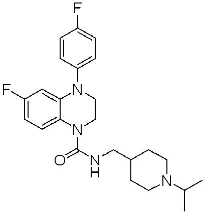 | 6-fluoro-4-(4-fluorophenyl)-*N-*((isopropylpiperidin-4-yl)methyl)-3,4-dihydroquinoxaline-1(2*H*)-carboxamide |
| 3 | 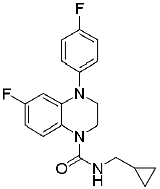 | *N*-(cyclopropylmethyl)-6-fluoro-4-(4-fluorophenyl)-3,4-dihydroquinoxaline-1(*2H*)-carboxamide |
| 4 | 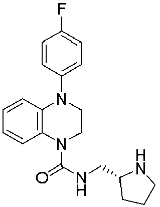 | (*R*)-4-(4-fluorophenyl)-*N*-(pyrrolidin-2-ylmethyl)-3,4-dihydroquinoxaline-1(*2H*)-carboxamide |
| 5 | 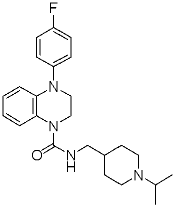 | 4-(4-fluorophenyl)-*N*-((1-isopropylpiperidin-4-yl)methyl)-3,4-dihydroquinoxaline-1(*2H*)-carboxamide |
| 6 | 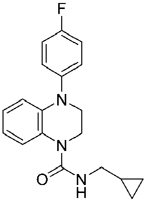 | *N*-(cyclopropylmethyl)-4-(4-fluorophenyl)-3,4-dihydroquinoxaline-1(2*H*)-carboxamide |
| 7 | 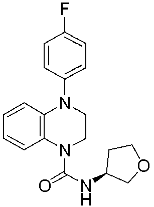 | (*S*)-4-(4-fluorophenyl)-*N*-(tetrahydrofuran-3-yl)-3,4-dihydroquinoxaline-1(*2H*)-carboxamide |
| 8 | 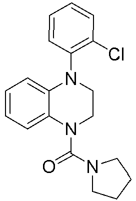 | (4-(2-chlorophenyl)-3,4-dihydroquinoxaline-1(*2H*)-yl)(pyrrolidin-1-yl)methanone |
| 9 | 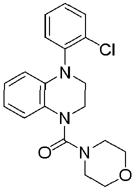 | (4-(2-chlorophenyl)-3,4-dihydroquinoxaline-1(*2H*)-yl)(morpholino)methanone |
| 10 | 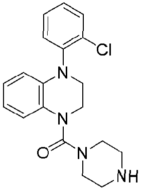 | (4-(2-chlorophenyl)-3,4-dihydroquinoxaline-1(*2H*)-yl)(piperazin-1-yl)methanone |
| 11 | 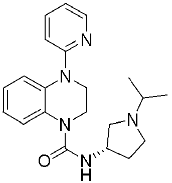 | (*S*)-*N*-(1-isopropylpyrrolidin-3-yl)-4-(pyridin-2-yl)-3,4-dihydroquinoxaline-1(*2H*)-carboxamide |
| 12 | 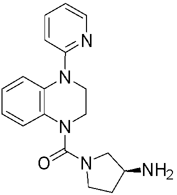 | (*S*)-(3-aminopyrrolidin-1-yl)(4-(pyridin-2-yl)-3,4-dihydroquinoxaline-1(*2H*)-yl)methanone |
| 13 | 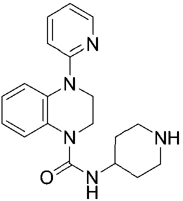 | *N*-(piperidin-4-yl)-4-(pyridin-2-yl)-3,4-dihydroquinoxaline-1(2*H*)-carboxamide |
| 14 | 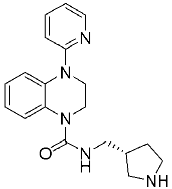 | (*R*)-4-(pyridin-2-yl)-*N*-(pyrrolidin-3-ylmethyl)-3,4-dihydroquinoxaline-1(*2H*)-carboxamide |
| 15 | 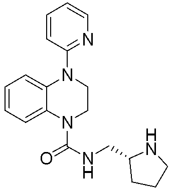 | (*R*)-4-(pyridin-2-yl)-*N*-(pyrrolidin-2-ylmethyl)-3,4-dihydroquinoxaline-1(*2H*)-carboxamide |
| 16 | 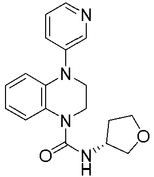 | (*R*)-4-(pyridin-3-yl)-*N*-(tetrahydrofuran-3-yl)-3,4-dihydroquinoxaline-1 (2*H*)-carboxamide |
| 17 | 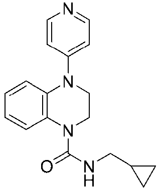 | *N*-(cyclopropylmethyl)-4-(pyridin-4-yl)-3,4-dihydroquinoxaline-1(2*H*)-carboxamide |
| 18 | 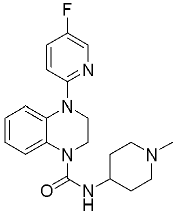 | 4-(5-fluoropyridin-2-yl)-*N*-(1-methylpiperidin-4-yl)-3,4-dihydroquinoxaline-1(*2H*)-carboxamide |
| 19 | 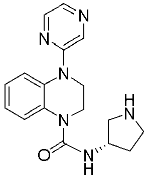 | (*S*)-4-(pyrazin-2-yl)-*N*-(pyrrolidin-3-yl)-3,4-dihydroquinoxaline-1(2*H*)-carboxamide |
| 20 | 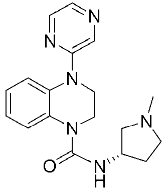 | (*S*)-*N*-(1-methylpyrrolidin-3-yl)-4-(pyrazin-2-yl)-3,4-dihydroquinoxaline-1(*2H*)-carboxamide |
| 21 | 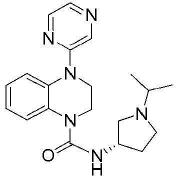 | (*S*)-*N*-(1-isopropylpyrrolidin-3-yl)-4-(pyrazin-2-yl)-3,4-dihydroquinoxaline-1(*2H*)-carboxamide |
| 22 | 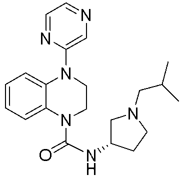 | (*S*)-*N-*(1-isobutylpyrrolidin-3-yl)-4-(pyrazin-2-yl)-3,4-dihydroquinoxaline-1(*2H*)-carboxamide |
| 23 | 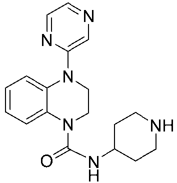 | *N-*(piperidin-4-yl)-4-(pyrazin-2-yl)-3,4-dihydroquinoxaline-1(*2H*)-carboxamide |
| 24 | 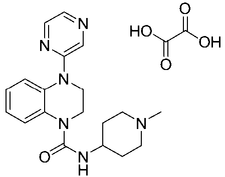 | *N*-(1-methylpiperidin-4-yl)-4-(pyrazin-2-yl)-3,4-dihydroquinoxaline-1(*2H*)-carboxamide oxalic acid salt |
| 25 | 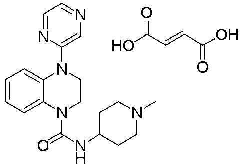 | *N*-(1-methylpiperidin-4-yl)-4-(pyrazin-2-yl)-3,4-dihydroquinoxaline-1(*2H*)-carboxamide fumaric acid salt |
| 26 | 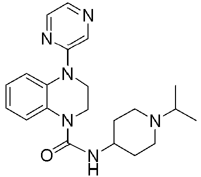 | *N*-(1-isopropylpiperidin-4-yl)-4-(pyrazin-2-yl)-3,4-dihydroquinoxaline-1(*2H*)-carboxamide |
| 27 | 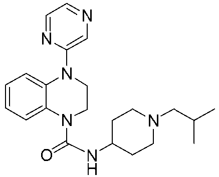 | *N*-(1-isobutylpiperidin-4-yl)-4-(pyrazin-2-yl)-3,4-dihydroquinoxaline-1(*2H*)-carboxamide |
| 28 | 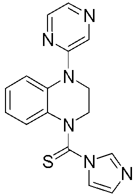 | imidazole-1-yl-(4-pyrazin-2-yl-2,3-dihydroquinoxaline-1-yl)methanethione |
| 29 | 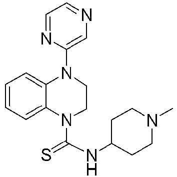 | *N*-(1-methylpiperidin-4-yl)-4-(pyrazin-2-yl)-3,4-dihydroquinoxaline-1 (*2H*)-carbothioamide |
| 30 | 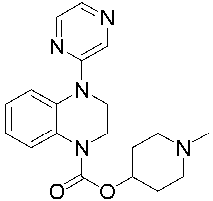 | 1-methylpiperidin-4-yl-4-(pyrazin-2-yl)-3,4-dihydroquinoxaline-1(*2H*)-carboxylate |
| 31 | 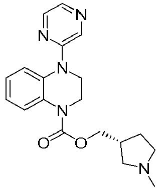 | (*R*)-(1-methylpyrrolidin-3-yl)methyl-4-(pyrazin-2-yl)-3,4-dihydroquinoxaline-1(*2H*)-carboxylate |
| 32 | 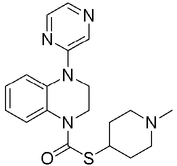 | (*S*)-(1-methylpiperidin-4-yl)-4-(pyrazin-2-yl)-3,4-dihydroquinoxaline-1(2*H*)-carbothioate |
| 33 | 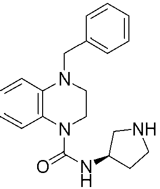 | (*R*)-4-benzyl-*N*-(pyrrolidin-3-yl)-3,4-dihydroquinoxaline-1(*2H*)-carboxamide |
| 34 | 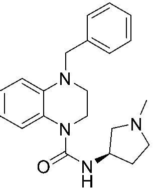 | (*R*)-4-benzyl-*N*-(1-methylpyrrolidin-3-yl)-3,4-dihydroquinoxaline-1(*2H*)-carboxamide |
| 35 | 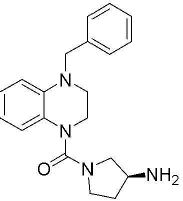 | (*S*)-(3-aminopyrrolidin-1-yl)(4-benzyl-3,4-dihydroquinoxaline-1(*2H*)-yl)methanone |
| 36 | 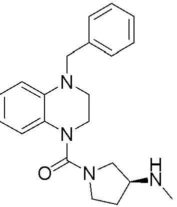 | (*S*)-(4-benzyl-3,4-dihydroquinoxaline-1(*2H*)-yl)(3-(methylamino)pyrrolidin-1-yl)methanone |
| 37 | 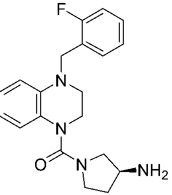 | (S)-(3-aminopyrrolidin-1-yl)(4-(2-fluorobenzyl)-3,4-dihydroquinoxaline-1(*2H*)-yl)methanone |
| 38 | 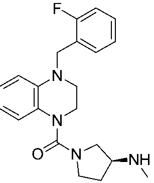 | (*S*)-(4-(2-fluorobenzyl)-3,4-dihydroquinoxaline-1(*2H*)-yl)(3-(methylamino)pyrrolidin-1-yl)methanone |
| 39 | 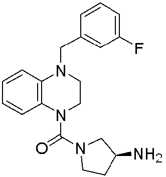 | (*S*)-(3-aminopyrrolidin-1-yl)(4-(3-fluorobenzyl)-3,4-dihydroquinoxaline-1(*2H*)-yl)methanone |
| 40 | 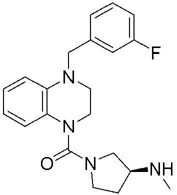 | (*S*)-(4-(3-fluorobenzyl)-3,4-dihydroquinoxaline-1(*2H*)-yl)(3-(methylamino)pyrrolidin-1-yl)methanone |
| 41 | 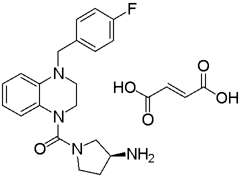 | (*S*)-(3-aminopyrrolidin-1-yl)(4-(4-fluorobenzyl)-3,4-dihydroquinoxaline-1(*2H*)-yl)methanone fumaric acid salt |
| 42 | 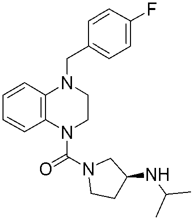 | (*S*)-(4-(4-fluorobenzyl)-3,4-dihydroquinoxaline-1(*2H*)-yl)(3-(isopropylamino)pyrrolidin-1-yl)methanone |
| 43 | 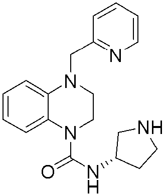 | (*S*)-4-(pyridin-2-ylmethyl)-*N*-(pyrrolidin-3-yl)-3,4-dihydroquinoxaline-1(*2H*)-carboxamide |
| 44 | 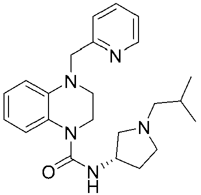 | (*S*)-*N*-(1-isobutylpyrrolidin-3-yl)-4-(pyrazin-2-ylmethyl)-3,4-dihydroquinoxaline*-*1(*2H*)-carboxamide |
| 45 | 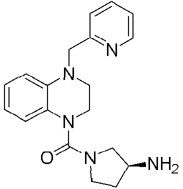 | (*S*)-(3-aminopyrrolidin-1-yl)(4-(pyrazin-2-ylmethyl)-3,4-dihydroquinoxaline-1(*2H*)-yl)methanone |
| 46 | 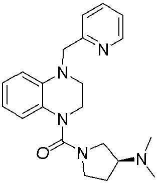 | (*S*)-(3-(dimethylamino)pyrrolidin-1-yl)(4-(pyridin-2-ylmethyl)-3,4-dihydroquinoxaline-1(*2H*)-yl)methanone |
| 47 | 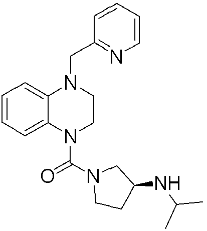 | (*S*)-(3-(isopropylamino)pyrrolidin-1-yl)(4-(pyridin-2-ylmethyl)-3,4-dihydroquinoxaline-1(*2H*)-yl)methanone |
| 48 | 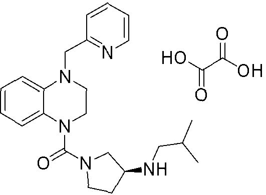 | (*S*)-(3-(isobutylamino)pyrrolidin-1-yl)(4-(pyridin-2-ylmethyl)-3,4-dihydroquinoxaline-1(*2H*)-yl)methanone oxalic acid salt |
| 49 | 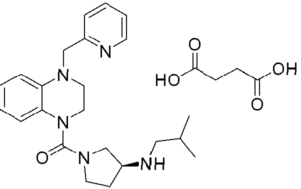 | (*S*)-(3-(isobutylamino)pyrrolidin-1-yl)(4-(pyridin-2-ylmethyl)-3,4-dihydroquinoxaline-1(*2H*)-yl)methanone succinic acid salt |
| 50 | 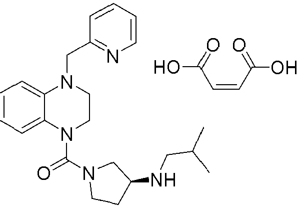 | (S)-(3-(isobutylamino)pyrrolidin-1-yl)(4-(pyridin-2-ylmethyl)-3,4-dihydroquinoxaline-1(*2H*)-yl)methanone maleic acid salt |
| 51 | 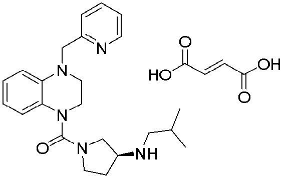 | (*S*)-(3-(isobutylamino)pyrrolidin-1-yl)(4-(pyridin-2-ylmethyl)-3,4-dihydroquinoxaline-1(*2H*)-yl)methanone fumaric acid salt |
| 52 | 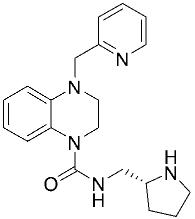 | (*R*)-4-(pyridin-2-ylmethyl)-N-(pyrrolidin-2-ylmethyl)-3,4-dihydroquinoxaline-1(*2H*)-carboxamide |
| 53 | 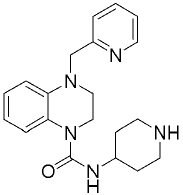 | *N*-(piperidin-4-yl)-4-(pyridin-2-ylmethyl)-3,4-dihydroquinoxaline-1(*2H*)-carboxamide |
| 54 | 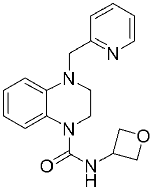 | *N*-(oxetan-3-yl)-4-(pyridin-2-ylmethyl)-3,4-dihydroquinoxaline-1(*2H*)-carboxamide |
| 55 | 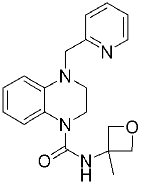 | *N*-(3-Methyloxetan-3-yl)-4-(pyridin-2-ylmethyl)-3,4-dihydroquinoxaline-1(*2H*)-carboxamide |
| 56 | 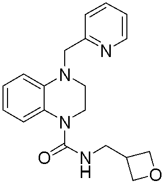 | *N*-(oxetan-3-ylmethyl)-4-(pyridin-2-ylmethyl)-3,4-dihydroquinoxaline-1(*2H*)-carboxamide |
| 57 | 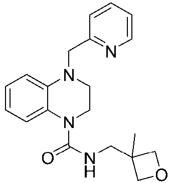 | *N*-((3-methyloxetan-3-yl)methyl)-4-(pyridin-2-ylmethyl)-3,4-dihydroquinoxaline-1(*2H*)-carboxamide |
| 58 | 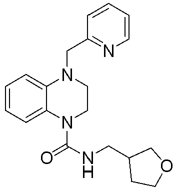 | 4-(pyridin-2-ylmethyl)-*N*-((tetrahydrofuran-3-yl)methyl)-3,4-dihydroquinoxaline-1(*2H*)-carboxamide |
| 59 | 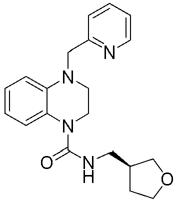 | (*S*)-4-(pyridin-2-ylmethyl)-*N-*((tetrahydrofuran-3-yl)methyl)-3,4-dihydroquinoxaline-1(*2H*)-carboxamide |
| 60 | 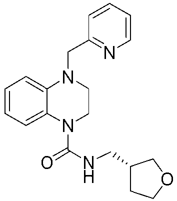 | (*R*)-4-(pyridin-2-ylmethyl)-*N-*((tetrahydrofuran-3-yl)methyl)-3,4-dihydroquinoxaline-1(*2H*)-carboxamide |
| 61 | 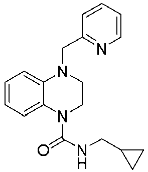 | *N*-(cyclopropylmethyl)-4-(pyridin-2-ylmethyl)-3,4-dihydroquinoxaline-1(*2H*)-carboxamide |
| 62 | 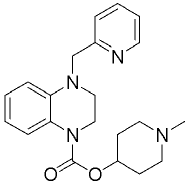 | 1-methylpiperidin-4-yl-4-(pyridin-2-ylmethyl)-3,4-dihydroquinoxaline-1(*2H*)-carboxylate |
| 63 | 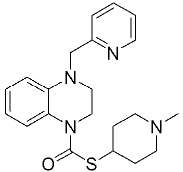 | (*S*)-(1-Methylpiperidin-4-yl)-4-(pyridin-2-ylmethyl)-3,4-dihydroquinoxaline-1(*2H*)-carbothioate |
| 64 | 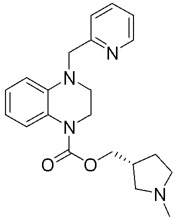 | (*R*)-(1-methylpyrrolidin-3-yl)methyl-4-(pyridin-2-ylmethyl)-3,4-dihydroquinoxaline-1(*2H*)-carboxylate |
| 65 | 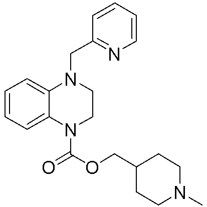 | (1-methylpiperidin-4-yl)methyl-4-(pyridin-2-ylmethyl)-3,4-dihydroquinoxaline-1(*2H*)-carboxylate |
| 66 | 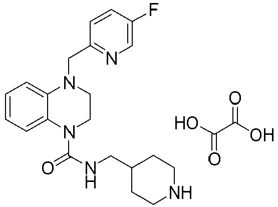 | 4-((5-fluoropyridine-2-yl)methyl)-*N-*(piperidin-4-ylmethyl)-3,4-dihydroquinoxaline-1(*2H*)-carboxamide oxalic acid salt |
| 67 | 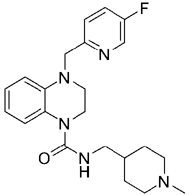 | 4-((5-fluoropyridin-2-yl)methyl)-*N*-((1-methylpiperidin-4-yl)methyl)-3,4-dihydroquinoxaline-1(*2H*)-carboxamide |
| 68 | 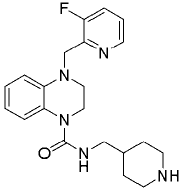 | 4-((3-fluoropyridin-2-yl)methyl)-*N*-(piperidin-4-ylmethyl)-3,4-dihydroquinoxaline-1(*2H*)-carboxamide |
| 69 | 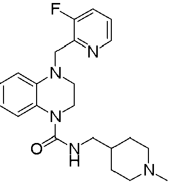 | 4-((3-fluoropyridin-2-yl)methyl)-*N*-((1-methylpiperidin-4-yl)methyl)-3,4-dihydroquinoxaline-1(*2H*)-carboxamide |
| 70 | 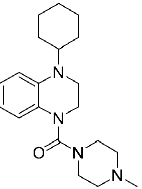 | (4-cyclohexyl-3,4-dihydroquinoxaline-1(2*H*)-yl)(4-methylpiperazin-1-yl)methanone |
| 71 | 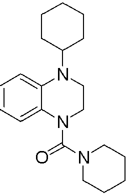 | 4-cyclohexyl-3,4-dihydroquinoxaline-1(*2H*)-yl)(piperidin-1-yl)methanone |
| 72 | 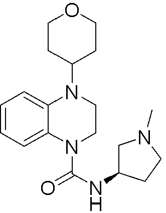 | (*R*)-*N*-(1-methylpyrrolidin-3-yl)-4-(tetrahydro-2H-pyran-4-yl)-3,4-dihydroquinoxaline-1(*2H*)-carboxamide |
| 73 | 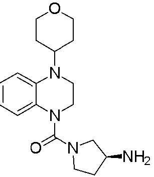 | (*S*)-(3-aminopyrrolidin-1-yl)(4-(tetrahydro-2H-pyran-4-yl)-3,4-dihydroquinoxaline-1(*2H*)-yl)methanone |
| 74 | 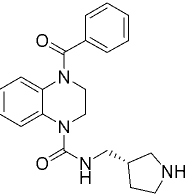 | (*S*)-4-benzoyl-*N*-(pyrrolidin-3-ylmethyl)-3,4-dihydroquinoxaline-1(*2H*)-carboxamide |
| 75 | 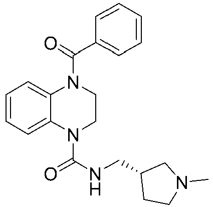 | (*R*)-4-Benzoyl-*N*-((1-methylpirrolidin-3-yl)methyl)-3,4-dihydroquinoxaline-1(*2H*)-carboxamide |
| 76 | 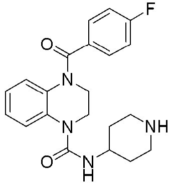 | 4-(4-fluorobenzoyl-*N*-(piperidin-4-yl)-3,4-dihydroquinoxaline-1(*2H*)-carboxamide |
| 77 | 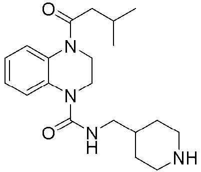 | 4-(3-Methylbutanoyl)-*N*-(piperidin-4-ylmethyl)-3,4-dihydroquinoxaline-1(2*H*)-carboxamide |
| 78 | 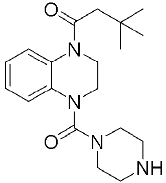 | 3,3-dimethyl-1-(4-(piperazine-1-carbonyl)-3,4-dihydroquinoxaline-1(*2H*)-yl)butan-1-one |
| 79 | 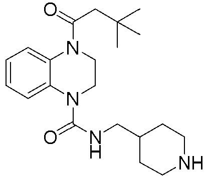 | 4-(3,3-dimethylbutanoyl)-*N*-(piperidin-4-ylmethyl)-3,4-dihydroquinoxaline-1(*2H*)-carboxamide |

### Experimental Example 2. Confirmation of in vitro α-SMA expression inhibitory effect

When scars are generated in tissue, fibroblasts undergo fibroblast-to-myofibroblast transition (FMT), and the resulting myofibroblasts produce the extracellular matrix, leading to tissue fibrosis. α-SMA is a representative biomarker of myofibroblasts, and its expression greatly increases as FMT progresses. Accordingly, through the evaluation of α-SMA expression inhibitory activity, the efficacy of the compounds in inhibiting fibrosis may be evaluated. Therefore, the inhibitory activity of the compounds of the present invention on α-SMA expression was evaluated in cells. To more accurately evaluate the inhibition of α-SMA expression, IC₅₀ values were determined by treating human lung-derived fibroblasts (MRC-5 cells) with the compounds of the present invention in a concentration-dependent manner.

Specifically, 2 hours after the MRC-5 cells were treated with DMSO or compounds dissolved in serum containing 0.5% fetal bovine serum (FBS) in a concentration-dependent manner, fibrosis was induced by treatment with TGF-β1 (5 ng/mL). After 72 hours, the cells were immobilized with a 2% paraformaldehyde solution for 10 minutes, and to impart cell membrane permeability, a 0.1% Triton X-100 solution was treated for 10 minutes, and a 1% bovine serum albumin (BSA) solution was treated to prevent non-specific binding. For immunostaining, anti-GAPDH (BIO-RAD) was first treated as a primary antibody, and Alexa Fluor^{™} Plus 555 for GAPDH labeling was used as a secondary antibody. For α-SMA staining, an FITC fluorescence-tagged α-SMA primary antibody was used, and thus the increase and decrease in α-SMA caused by the compounds were evaluated. The immunohistochemical experimental results were confirmed with a STELLARIS 5 confocal microscope (Leica), and FITC-stained α-SMA was standardized using GAPDH labeled with Alexa FluorTM Plus 555 for quantification. The IC₅₀ of the compounds according to the present invention was calculated through statistical analysis (linear mixed effects model). The α-SMA expression inhibitory effect of each compound is summarized in Table 2 below (A: IC₅₀ <1 µM, B: 1 µM ≤ IC₅₀ < 10 µM, C: 10 µM ≤ IC₅₀ < 30 µM, D: IC₅₀ ≤ 30 µM).

**[Table 2]**

| **Compound Number** | **IC₅₀** | **Compound Number** | **IC₅₀** | **Compound Number** | **IC₅₀** |
|---|---|---|---|---|---|
| **3** | **B** | **29** | **B** | **55** | **A** |
| **6** | **B** | **31** | **B** | **56** | **C** |
| **8** | **C** | **33** | **B** | **57** | **D** |
| **9** | **D** | **34** | **B** | **58** | **A** |
| **10** | **A** | **36** | **C** | **59** | **A** |
| **11** | **B** | **37** | **B** | **60** | **C** |
| **12** | **D** | **38** | **B** | **64** | **A** |
| **13** | **B** | **39** | **A** | **65** | **C** |
| **14** | **B** | **41** | **A** | **66** | **A** |
| **15** | **A** | **42** | **B** | **67** | **D** |
| **16** | **B** | **44** | **D** | **68** | **D** |
| **18** | **B** | **46** | **D** | **69** | **B** |
| **19** | **B** | **47** | **D** | **70** | **B** |
| **20** | **A** | **51** | **A** | **71** | **B** |
| **22** | **D** | **52** | **B** | **77** | **D** |
| **23** | **B** | **53** | **B** | **78** | **D** |
| **27** | **C** | **54** | **A** | **79** | **D** |

### Experimental Example 3. Evaluation of inhibition of F-actin formation of compounds according to the present invention

In this experimental example, 2 hours after human lung-derived fibroblasts (MRC-5 cells) were treated with Compound 41 (10 µM) or Compound 51 (10 µM) according to the present invention, or DMSO, dissolved in 0.5% FBS-containing serum, TGF-β1 (5 ng/mL) was treated to induce fibrosis. After 72 hours, the cells were immobilized with a 2% paraformaldehyde solution for 10 minutes, and to impart cell membrane permeability, a 0.1% Triton X-100 solution was treated for 10 minutes, and a 1% bovine serum albumin (BSA) solution was treated to prevent non-specific binding. For immunostaining, anti-GAPDH (BIO-RAD) was first treated as a primary antibody, and Alexa Fluor^{™} Plus 488 for GAPDH labeling was used as a secondary antibody. For staining of an actin cytoskeleton, particularly, F-actin, rhodamine phalloidin (Cytoskeleton) was used to confirm the increase and decrease in F-actin caused by the compounds. The immunohistochemical experimental results were confirmed with a STELLARIS 5 confocal microscope (Leica), F-actin stained with rhodamine phalloidin was standardized with GAPDH labeled with Alexa Fluor^{™} Plus 488 for quantification.

The degree of inhibition of F-actin formation by the compound according to the present invention is shown in FIG. 1. In TGF-β1-treated cells, intracellular F-actin formation greatly increased compared to a control (control material). On the other hand, in the cells co-treated with Compound 41 or 51 of the present invention, intracellular F-actin formation was reduced to a level similar to that of the group not treated with TGF-β1, confirming that the compounds of the present invention can significantly inhibit F-actin formation mediated by TGF-β1. These results show that the compounds of the present invention can effectively inhibit F-actin formation, which is a key factor in the activation of myofibroblasts and pulmonary fibrosis.

### Experimental Example 4. Evaluation of fibrosis treatment efficacy in liver cells by compound according to the present invention

In this experimental example, it was confirmed whether the compounds according to the present invention exhibit a therapeutic effect on liver fibrosis. Here, to confirm the degree of fibrosis, ACTA2 or COL1A1 mRNA that can express α-SMA or collagen type 1 was used as an indicator. α-SMA is a marker of activated myofibroblasts, which are the main effector cells of fibrosis, and myofibroblasts with increased α-SMA expression are known to cause fibrosis by producing a fibrotic protein such as COL1A1.

Specifically, human hepatic stellate cells (HHSCs) were seeded, and cultured for 24 hours under a nutrient-deficient condition for effective cell activation. Afterward, the cells were treated with the compounds (Compounds 51 and 41) according to the present invention, together with TGF-β1 (5 ng/mL) to induce fibrosis, at 10 and 50 µM for 48 hours, respectively, and then cultured to extract RNA therefrom. The RNA was used to perform qRT-PCR. From the experimental results, the mRNA expression levels of ACTA2 and COL1A1 were confirmed, thereby verifying the therapeutic effects of the compounds.

As shown in FIG. 2, as a result of confirming the ACTA2 mRNA expression level through qRT-PCR, the HSCs treated with the inflammatory cytokine TGF-β1 had a significant increase in ACTA2 expression compared to the non-treated control. On the other hand, it was confirmed that the cells treated with the compound of the present invention showed that the increase in expression of ACTA2 or COL1A1 mRNA by TGF-β1 treatment was inhibited in a concentration-dependent manner.

The above results show that the compounds according to the present invention are able to effectively inhibit liver fibrosis. Particularly, it was demonstrated that both compounds (Compounds 51 and 41) according to the present invention can effectively inhibit the mRNA expression of the key fibrosis marker, α-SMA, thereby effectively inhibiting liver fibrosis.

### Experimental Example 5. Evaluation of fibrosis treatment efficacy in kidney cells by compound according to the present invention

In this example, it was confirmed whether the compounds according to the present invention exhibit a therapeutic effect on renal fibrosis. Here, to confirm the degree of fibrosis, ACTA2 or COL1A1 mRNA, which can express α-SMA or collagen type 1, was used as an indicator. α-SMA is a marker for activated myofibroblasts, which are the main effector cells of fibrosis, and myofibroblasts with increased α-SMA expression are known to cause fibrosis by producing a fibrotic protein such as collagen type 1.

Specifically, human renal fibroblasts (HRFs) were seeded, and cultured for 24 hours under a nutrient-deficient condition for effective cell activation. Afterward, the cells were treated with the compounds (Compounds 51 and 41) according to the present invention, together with TGF-β1 (5 ng/mL) to induce fibrosis, at 10 and 50 µM for 48 hours, respectively, and then cultured to extract RNA therefrom. The RNA was used to perform qRT-PCR. From the experimental results, the mRNA expression levels of ACTA2 and COL1A1 were confirmed, thereby verifying the therapeutic effects of the compounds.

As shown in FIG. 3, as a result of confirming the ACTA2 mRNA expression level through qRT-PCR, the renal fibroblasts treated with the inflammatory cytokine TGF-β1 had greatly increased ACTA2 expression compared to the non-treated control. On the other hand, it was confirmed that the cells treated with the compound of the present invention showed that the increase in expression of ACTA2 or COL1A1 mRNA by TGF-β1 treatment was inhibited in a concentration-dependent manner.

The above results show that the compounds according to the present invention are able to effectively inhibit renal fibrosis. Particularly, it was demonstrated that both compounds (Compounds 51 and 41) according to the present invention can effectively inhibit the mRNA expression of the key fibrosis markers, ACTA2 and COL1A1, thereby effectively inhibiting renal fibrosis.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative in all aspects and not restrictive.

### [Industrial Applicability]

The compound according to the present invention or a pharmaceutically acceptable salt thereof was confirmed to effectively inhibit the expression (formation) of α-SMA and F-actin, which are major causative factors of fibrosis. Therefore, the novel compound according to the present invention, as a therapeutic agent having the effect of improving or inhibiting fibrosis, may be usefully employed for the prevention and treatment of fibrosis-related diseases, and thus has industrial applicability.

## Claims

1. A compound represented by Chemical Formula 1, a stereoisomer thereof, or a pharmaceutically acceptable salt thereof: 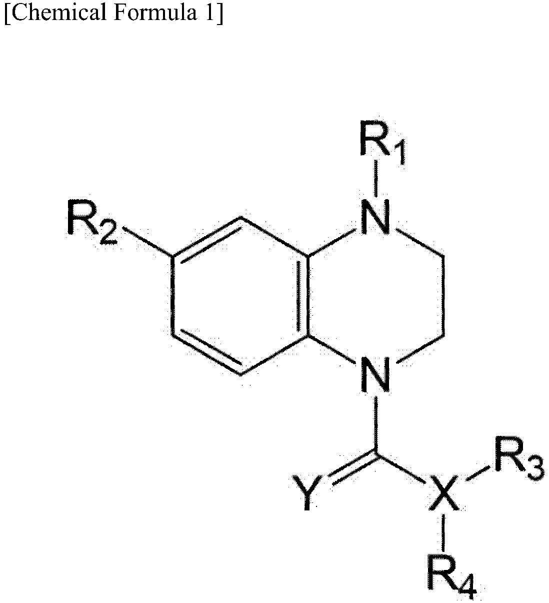 (In Chemical Formula 1,
X is N, S, or O;
Y is O or S;
R₁ is a C₁-C₁₀ alkyl, a C₃-C₂₀ cycloalkyl, a C₂-C₂₀ heterocycloalkyl, a 3- to 10-membered aromatic cyclic group, a 3- to 10-membered aromatic heterocyclic group, - CO-(C₁-C₆ alkyl), or -CO-(halogen-substituted or unsubstituted C₆-C₁₂ aryl),
one or more H atoms of R₁ are substituted with a halogen, a halogen-substituted or unsubstituted C₆-C₁₂ aryl, or a halogen-substituted or unsubstituted C₅-C₁₂ heteroaryl;
R₂ is hydrogen or a halogen;
R₃ and R₄ are linked to each other to form a 5- or 6-membered ring, where the ring has one or more H atoms that are substituted with -NH₂, dimethylamine, -NH-(C₁-C₅ alkyl), or a C₁-C₅ alkyl, and
when R₃ and R₄ do not form a ring, R₄ is absent or hydrogen, R₃ is a C₃-C₁₀ cycloalkyl, a C₂-C₁₀ heterocycloalkyl, -CH₂-(C₃-C₆ cycloalkyl), or -CH₂-(C₂-C₆ heterocycloalkyl), where one or more H atoms of R₃ are substituted with -NH-(C₁-C₅ alkyl), or a C₁-C₅ alkyl; and
the heterocycloalkyl, a heterocyclic group, and heteroaryl each independently comprise one or more heteroatoms selected from the group consisting of N, S, and O.)

2. The compound, stereoisomer thereof, or pharmaceutically acceptable salt thereof of claim 1, wherein R₃ and R₄ are linked to each other to form a 5- or 6-membered heterocycloalkyl, or 5- or 6-membered aromatic heterocyclic, where the 5- or 6-membered heterocycloalkyl or 5- or 6-membered aromatic heterocyclic comprises one or two N or O atoms, and X is N, and
in the 5- or 6-membered heterocycloalkyl or 5- or 6-membered aromatic heterocyclic formed by R₃ and R₄, one or more H atoms are substituted with -NH₂, dimethylamine, -NH-(C₁-C₅ alkyl), or a C₁-C₅ alkyl.

3. The compound, stereoisomer thereof, or pharmaceutically acceptable salt thereof of claim 1, wherein R₁ is a 6-membered aromatic cyclic group, a 6-membered aromatic heterocyclic group, -CH₂-aryl, -CH₂-heteroaryl, a 6-membered cycloalkyl, a 6-membered heterocycloalkyl, -CO-aryl, or -CO-(C₄-C₅ alkyl), where the 6-membered aromatic heterocyclic group and the heteroaryl comprise one or more N atoms, and the 6-membered heterocycloalkyl comprises one or more O atoms, and
one or more H atoms of R₁ are substituted with a halogen.

4. The compound, stereoisomer thereof, or pharmaceutically acceptable salt thereof of claim 1, wherein R₁ is a 6-membered aromatic cyclic group, a 6-membered aromatic heterocyclic group, -CH₂-aryl, -CH₂-heteroaryl, or -CO-(C₅ alkyl), where the 6-membered aromatic heterocyclic group and the heteroaryl comprise one or more N atoms,
one or more H atoms of R₁ are substituted with a halogen;
R₂ is hydrogen;
R₃ and R₄ are linked to each other to form a 5- or 6-membered heterocycloalkyl, or 5- or 6-membered aromatic heterocyclic, where the 5- or 6-membered heterocycloalkyl or 5- or 6-membered aromatic heterocyclic comprises one or more N or O atoms, and X is N, and
in the 5- or 6-membered heterocycloalkyl or 5- or 6-membered aromatic heterocyclic formed by R₃ and R₄, one or more H atoms are substituted with -NH₂, or - NH-(C₁-C₅ alkyl),
when R₃ and R₄ do not form a ring, R₄ is absent or hydrogen, R₃ is a C₄-C₆ heterocycloalkyl, -CH₂-(C₃ cycloalkyl), or -CH₂-(C₄-C₆ heterocycloalkyl), where one or more H atoms of R₃ are substituted with a C₁-C₄ alkyl.

5. The compound, stereoisomer thereof, or pharmaceutically acceptable salt thereof of claim 1, wherein the compound represented by Chemical Formula 1 is any one selected from the group consisting of the following compounds:
(1) (*R*)-6-fluoro-4-(4-fluorophenyl)-*N*-(pyrrolidin-2-ylmethyl)-3,4-dihydroquinoxaline-1(*2H*)-carboxamide;
(2) 6-fluoro-4-(4-fluorophenyl)-*N*-((isopropylpiperidin-4-yl)methyl)-3,4-dihydroquinoxaline-1(*2H*)-carboxamide;
(3) *N*-(cyclopropylmethyl)-6-fluoro-4-(4-fluorophenyl)-3,4-dihydroquinoxaline-1(*2H*)-carboxamide;
(4) (*R*)-4-(4-fluorophenyl)-*N*-(pyrrolidin-2-ylmethyl)-3,4-dihydroquinoxaline-1 (*2H*)-carboxamide;
(5) 4-(4-fluorophenyl)-*N*-((isopropylpiperidin-4-yl)methyl)-3,4-dihydroquinoxaline-1(*2H*)-carboxamide;
(6) *N*-(cyclopropylmethyl)-4-(4-fluorophenyl)-3,4-dihydroquinoxaline-1(*2H*)-carboxamide;
(7) (*S*)-4-(4-fluorophenyl)-*N*-(tetrahydrofuran-3-yl)-3,4-dihydroquinoxaline-1 (*2H*)-carboxamide;
(8) (4-(2-chlorophenyl)-3,4-dihydroquinoxaline-1(*2H*)-yl)(pyrrolidin-1-yl)methanone;
(9) (4-(2-chlorophenyl)-3,4-dihydroquinoxaline-1(*2H*)-yl)(morpholino)methanone;
(10) (4-(2-chlorophenyl)-3,4-dihydroquinoxaline-1(*2H*)-yl)(piperazin-1-yl)methanone;
(11) (*S*)-*N*-(1-isopropylpyrrolidin-3-yl)-4-(pyridin-2-yl)-3,4-dihydroquinoxaline-1(*2H*)-carboxamide;
(12) (*S*)-(3-aminopyrrolidin-1-yl)(4-(pyridin-2-yl)-3,4-dihydroquinoxaline-1(2*H*)-yl)methanone;
(13) *N*-(piperidin-4-yl)-4-(pyridin-2-yl)-3,4-dihydroquinoxaline-1(*2H*)-carboxamide;
(14) (*R*)-4-(pyridin-2-yl)-*N*-(pyrrolidin-3-ylmethyl)-3,4-dihydroquinoxaline-1(*2H*)-carboxamide;
(15) (*R*)-4-(pyridin-2-yl)-*N*-(pyrrolidin-2-ylmethyl)-3,4-dihydroquinoxaline-1(*2H*)-carboxamide;
(16) (*R*)-4-(pyridin-3-yl)-*N*-(tetrahydrofuran-3-yl)-3,4-dihydroquinoxaline-1(*2H*)-carboxamide;
(17) *N*-(cyclopropylmethyl)-4-(pyridin-4-yl)-3,4-dihydroquinoxaline-1(*2H*)-carboxamide;
(18) 4-(5-fluoropyridin-2-yl)-*N*-(1-methylpiperidin-4-yl)-3,4-dihydroquinoxaline-1(*2H*)-carboxamide;
(19) (*S*)-4-(pyrazin-2-yl)-*N*-(pyrrolidin-3-yl)-3,4-dihydroquinoxaline-1(*2H*)-carboxamide;
(20) (*S*)-*N*-(1-methylpyrrolidin-3-yl)-4-(pyrazin-2-yl)-3,4-dihydroquinoxaline-1(*2H*)-carboxamide;
(21) (*S*)-*N*-(1-isopropylpyrrolidin-3-yl)-4-(pyrazin-2-yl)-3,4-dihydroquinoxaline-1(*2H*)-carboxamide;
(22) (*S*)-*N*-(1-isobutylpyrrolidin-3-yl)-4-(pyrazin-2-yl)-3,4-dihydroquinoxaline-1(*2H*)-carboxamide;
(23) *N*-(piperidin-4-yl)-4-(pyrazin-2-yl)-3,4-dihydroquinoxaline-1(*2H*)-carboxamide;
(24) *N*-(1-methylpiperidin-4-yl)-4-(pyrazin-2-yl)-3,4-dihydroquinoxaline-1(*2H*)-carboxamide oxalic acid salt;
(25) *N*-(1-methylpiperidin-4-yl)-4-(pyrazin-2-yl)-3,4-dihydroquinoxaline-1(*2H*)-carboxamide fumaric acid salt;
(26) *N*-(1-isopropylpiperidin-4-yl)-4-(pyrazin-2-yl)-3,4-dihydroquinoxaline-1(*2H*)-carboxamide;
(27) *N*-(1-isobutylpiperidin-4-yl)-4-(pyrazin-2-yl)-3,4-dihydroquinoxaline-1(*2H*)-carboxamide;
(28) imidazole-1-yl-(4-pyrazin-2-yl-2,3-dihydroquinoxaline-1-yl)methanethione;
(29) *N*-(1-methylpiperidin-4-yl)-4-(pyrazin-2-yl)-3,4-dihydroquinoxaline-1(*2H*)-carbothioamide;
(30) 1-methylpiperidin-4-yl-4-(pyrazin-2-yl)-3,4-dihydroquinoxaline-1(*2H*)-carboxylate;
(31) (*R*)-(1-methylpyrrolidin-3-yl)methyl-4-(pyrazin-2-yl)-3,4-dihydroquinoxaline-1(*2H*)-carboxylate;
(32) (*S*)-(1-methylpiperidin-4-yl)-4-(pyrazin-2-yl)-3,4-dihydroquinoxaline-1 (*2H*)-carbothioate;
(33) (*R*)-4-benzyl-*N*-(pyrrolidin-3-yl)-3,4-dihydroquinoxaline-1(2*H*)-carboxamide;
(34) (*R*)-4-benzyl-*N*-(1-methylpyrrolidin-3-yl)-3,4-dihydroquinoxaline-1(*2H*)-carboxamide;
(35) (*S*)-(3-aminopyrrolidin-1-yl)(4-benzyl-3,4-dihydroquinoxaline-1(2*H*)-yl)methanone;
(36) (*S*)-(4-benzyl-3,4-dihydroquinoxaline-1(*2H*)-yl)(3-(methylamino)pyrrolidin-1-yl)methanone;
(37) (*S*)-(3-aminopyrrolidin-1-yl)(4-(2-fluorobenzyl)-3,4-dihydroquinoxaline-1(*2H*)-yl)methanone;
(38) (*S*)-(4-(2-fluorobenzyl)-3,4-dihydroquinoxaline-1(*2H*)-yl)(3-(methylamino)pyrrolidin-1-yl)methanone;
(39) (*S*)-(3-aminopyrrolidin-1-yl)(4-(3-fluorobenzyl)-3,4-dihydroquinoxaline-1(*2H*)-yl)methanone;
(40) (*S*)-(4-(3-fluorobenzyl)-3,4-dihydroquinoxaline-1(*2H*)-yl)(3-(methylamino)pyrrolidin-1-yl)methanone;
(41) (*S*)-(3-aminopyrrolidin-1-yl)(4-(4-fluorobenzyl)-3,4-dihydroquinoxaline-1(2H)-yl)methanone fumaric acid salt;
(42) (*S*)-(4-(4-fluorobenzyl)-3,4-dihydroquinoxaline-1(*2H*)-yl)(3-(isopropylamino)pyrrolidin-1-yl)methanone;
(43) (*S*)-4-(pyridin-2-ylmethyl)-*N*-(pyrrolidin-3-yl)-3,4-dihydroquinoxaline-1(*2H*)-carboxamide;
(44) (*S*)-*N*-(1-isobutylpyrrolidin-3-yl)-4-(pyrazin-2-ylmethyl)-3,4-dihydroquinoxaline-1(*2H*)-carboxamide;
(45) (*S*)-(3-aminopyrrolidin-1-yl)(4-(pyrazin-2-ylmethyl)-3,4-dihydroquinoxaline-1(*2H*)-yl)methanone;
(46) (*S*)-(3-(dimethylamino)pyrrolidin-1-yl)(4-(pyridin-2-ylmethyl)-3,4-dihydroquinoxaline-1(*2H*)-yl)methanone;
(47) (*S*)-(3-(isopropylamino)pyrrolidin-1-yl)(4-(pyridin-2-ylmethyl)-3,4-dihydroquinoxaline-1(*2H*)-yl)methanone;
(48) (*S*)-(3-(isobutylamino)pyrrolidin-1-yl)(4-(pyridin-2-ylmethyl)-3,4-dihydroquinoxaline-1(2H)-yl)methanone oxalic acid salt;
(49) (*S*)-(3-(isobutylamino)pyrrolidin-1-yl)(4-(pyridin-2-ylmethyl)-3,4-dihydroquinoxaline-1(*2H*)-yl)methanone succinic acid salt;
(50) (*S*)-(3-(isobutylamino)pyrrolidin-1-yl)(4-(pyridin-2-ylmethyl)-3,4-dihydroquinoxaline-1(*2H*)-yl)methanone maleic acid salt;
(51) (*S*)-(3-(isobutylamino)pyrrolidin-1-yl)(4-(pyridin-2-ylmethyl)-3,4-dihydroquinoxaline-1(*2H*)-yl)methanone fumaric acid salt;
(52) (*R*)-4-(pyridin-2-ylmethyl)-*N*-(pyrrolidin-2-ylmethyl)-3,4-dihydroquinoxaline-1(*2H*)-carboxamide;
(53) *N*-(piperidin-4-yl)-4-(pyridin-2-ylmethyl)-3,4-dihydroquinoxaline-1(*2*H)-carboxamide;
(54) *N*-(oxetan-3-yl)-4-(pyridin-2-ylmethyl)-3,4-dihydroquinoxaline-1(*2H*)-carboxamide;
(55) *N*-(3-methyloxetan-3-yl)-4-(pyridin-2-ylmethyl)-3,4-dihydroquinoxaline-1 (*2H*)-carboxamide;
(56) *N*-(oxetan-3-ylmethyl)-4-(pyridin-2-ylmethyl)-3,4-dihydroquinoxaline-1(*2H*)-carboxamide;
(57) *N*-((3-methyloxetan-3-yl)methyl)-4-(pyridin-2-ylmethyl)-3,4-dihydroquinoxaline-1(*2H*)-carboxamide;
(58) 4-(pyridin-2-ylmethyl)-*N*-((tetrahydrofuran-3-yl)methyl)-3,4-dihydroquinoxaline-1(*2H*)-carboxamide;
(59) (*S*)-4-(pyridin-2-ylmethyl)-*N*-((tetrahydrofuran-3-yl)methyl)-3,4-dihydroquinoxaline-1(*2H*)-carboxamide;
(60) (*R*)-4-(pyridin-2-ylmethyl)-*N*-((tetrahydrofuran-3-yl)methyl)-3,4-dihydroquinoxaline-1(*2H*)-carboxamide;
(61) *N*-(cyclopropylmethyl)-4-(pyridin-2-ylmethyl)-3,4-dihydroquinoxaline-1(*2H*)-carboxamide;
(62) 1-methylpiperidin-4-yl-4-(pyridin-2-ylmethyl)-3,4-dihydroquinoxaline-1 (*2H*)-carboxylate;
(63) (*S*)-(1-methylpiperidin-4-yl)-4-(pyridin-2-ylmethyl)-3,4-dihydroquinoxaline-1(*2H*)-carbothioate;
(64) (*R*)-(1-methylpyrrolidin-3-yl)methyl-4-(pyridin-2-ylmethyl)-3,4-dihydroquinoxaline-1(*2H*)-carboxylate;
(65) (1-methylpiperidin-4-yl)methyl-4-(pyridin-2-ylmethyl)-3,4-dihydroquinoxaline-1(*2H*)-carboxylate;
(66) 4-((5-fluoropyridine-2-yl)methyl)-*N*-(piperidin-4-ylmethyl)-3,4-dihydroquinoxaline-1(*2H*)-carboxamide oxalic acid salt;
(67) 4-((5-fluoropyridin-2-yl)methyl)-*N*-((1-methylpiperidin-4-yl)methyl)-3,4-dihydroquinoxaline-1(*2H*)-carboxamide;
(68) 4-((3-fluoropyridin-2-yl)methyl)-*N*-(piperidin-4-ylmethyl)-3,4-dihydroquinoxaline-1(*2H*)-carboxamide;
(69) 4-((3-fluoropyridin-2-yl)methyl)-*N*-((1-methylpiperidin-4-yl)methyl)-3,4-dihydroquinoxaline-1(*2H*)-carboxamide;
(70) (4-cyclohexyl-3,4-dihydroquinoxaline-1(*2H*)-yl)(4-methylpiperazin-1-yl)methanone;
(71) 4-cyclohexyl-3,4-dihydroquinoxaline-1(*2H*)-yl)(piperidin-1-yl)methanone;
*(72)* (*R*)*-N*-(1-methylpyrrolidin-3-yl)-4-(tetrahydro-2H-pyran-4-yl)-3,4-dihydroquinoxaline-1(*2H*)-carboxamide;
(73) (*S*)-(3-aminopyrrolidin-1-yl)(4-(tetrahydro-2H-pyran-4-yl)-3,4-dihydroquinoxaline-1(*2H*)-yl)methanone;
(74) (*S*)-4-benzoyl-*N*-(pyrrolidin-3-ylmethyl)-3,4-dihydroquinoxaline-1(*2H*)-carboxamide;
(75) (*R*)-4-benzoyl-*N*-((1-methylpirrolidin-3-yl)methyl)-3,4-dihydroquinoxaline-1(*2H*)-carboxamide;
(76) 4-(4-fluorobenzoyl-*N*-(piperidin-4-yl)-3,4-dihydroquinoxaline-1(2*H*)-carboxamide;
(77) 4-(3-methylbutanoyl)-*N*-(piperidin-4-ylmethyl)-3,4-dihydroquinoxaline-1(*2H*)-carboxamide;
(78) 3,3-dimethyl-1-(4-(piperazine-1-carbonyl)-3,4-dihydroquinoxaline-1(*2H*)-yl)butan-1-one; and
(79) 4-(3,3-dimethylbutanoyl)-*N*-(piperidin-4-ylmethyl)-3,4-dihydroquinoxaline-1(*2H*)-carboxamide.

6. A pharmaceutical composition for preventing or treating a fibrosis-related disease, comprising:
the compound represented by Chemical Formula 1, stereoisomer thereof, or pharmaceutically acceptable salt thereof of claim 1 as an active ingredient.

7. The pharmaceutical composition of claim 6, wherein the composition reduces the expression or activity of one or more mRNAs or proteins selected from the group consisting of α-SMA, COL1A1, and F-actin.

8. The pharmaceutical composition of claim 6, wherein the fibrosis-related disease is a fibrosis-related disease induced by an inflammatory cytokine.

9. The pharmaceutical composition of claim 6, wherein the fibrosis-related disease is a disease associated with liver, renal, or lung fibrosis.

10. The pharmaceutical composition of claim 9, wherein the liver fibrosis-related disease is one or more selected from the group consisting of liver fibrosis, cirrhosis, biliary cirrhosis, alcoholic or non-alcoholic steatohepatitis, viral hepatitis, autoimmune hepatitis, and sclerosing cholangitis.

11. The pharmaceutical composition of claim 9, wherein the renal fibrosis-related disease is one or more selected from the group consisting of renal fibrosis, end-stage kidney disease (ESKD), diabetic nephropathy (DN), IgA nephropathy (IgAN), HIV-related nephropathy, non-diabetic chronic kidney disease, focal segmental glomerulosclerosis (FSGS), minimal change disease (MCD), and xanthine oxidase deficiency.

12. The pharmaceutical composition of claim 9, wherein the lung fibrosis-related disease is one or more selected from the group consisting of lung fibrosis, idiopathic pulmonary fibrosis, desquamative interstitial pneumonia, idiopathic interstitial pneumonia, non-specific interstitial pneumonia, cryptogenic organizing pneumonia, interstitial lung disease associated with respiratory bronchiolitis, acute interstitial pneumonia, lymphocytic interstitial pneumonia, idiopathic parenchymal elastosis, interstitial lung disease, and chronic obstructive pulmonary disease (COPD).

13. A kit for preventing or treating a fibrosis-related disease, comprising the composition of any one of claims 6 to 12.

14. A method of preventing or treating a fibrosis-related disease, comprising:
administering the compound represented by Chemical Formula 1, stereoisomer thereof, or pharmaceutically acceptable salt thereof of claim 1 to a subject in need thereof at a pharmaceutically effective amount.

15. A use of the compound represented by Chemical Formula 1, stereoisomer thereof, or pharmaceutically acceptable salt thereof of claim 1 for the prevention of treatment of a fibrosis-related disease.

16. A use of the compound represented by Chemical Formula 1, stereoisomer thereof, or pharmaceutically acceptable salt thereof of claim 1 for the preparation of a drug for preventing or treating a fibrosis-related disease.
